(19) Europäisches Patentamt / European Patent Office / Office européen des brevets

(11) EP 1 664 122 B1

(12) **EUROPEAN PATENT SPECIFICATION**

(45) Date of publication and mention of the grant of the patent: **17.03.2010 Bulletin 2010/11**

(21) Application number: **04765363.9**

(22) Date of filing: **17.09.2004**

(51) Int Cl.:
***C07K 16/28*** *(2006.01)*
***C12N 15/13*** *(2006.01)*
***C12N 15/62*** *(2006.01)*
***C12N 15/79*** *(2006.01)*
***C12N 5/10*** *(2006.01)*
***A61K 39/395*** *(2006.01)*
***A61P 37/00*** *(2006.01)*

(86) International application number: **PCT/EP2004/010471**

(87) International publication number: **WO 2005/026210 (24.03.2005 Gazette 2005/12)**

# (54) THERAPEUTIC HUMANISED ANTIBODIES AGAINST CD45 ISOFORMS

THERAPEUTISCHE HUMANISIERTE ANTIKÖRPER GEGEN CD45-ISOFORMEN

DES ANTICORPS HUMANISÉS THERAPEUTIQUES CONTRE DES ISOFORMES DE CD45

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **18.09.2003 US 666332**
**25.06.2004 GB 0414309**

(43) Date of publication of application: **07.06.2006 Bulletin 2006/23**

(73) Proprietors:
- **Novartis AG**
  **4056 Basel (CH)**
  Designated Contracting States:
  **BE BG CH CY CZ DE DK EE ES FI FR GB GR HU IE IT LI LU MC NL PL PT RO SE SI SK TR**
- **Novartis Pharma GmbH**
  **1230 Wien (AT)**
  Designated Contracting States:
  **AT**

(72) Inventors:
- **KOLBINGER, Frank**
  **79395 Neuenburg (DE)**
- **CARBALLIDO HERRERA, José, M.**
  **A-2380 Perchtoldsdorf (AT)**
- **ASZODI, András**
  **A-1140 Vienna (AT)**
- **SALDANHA, José W.**
  **Enfield,**
  **London EN1 3BD (GB)**
- **HALL, Bruce, M.**
  **Strathfield, NSW 2135 (AU)**
- **GREGORI, Silvia**
  **I-20090 Buccinasco (IT)**
- **RONCAROLO, Maria Grazia**
  **I-20090 Segrate (IT)**
- **LOUX, Véronique**
  **F-68870 Bartenheim (FR)**
- **AVERSA, Gregorio**
  **Vancouver, BC V6R IV4 (CA)**
- **JESCHKE, Margit**
  **CH-4058 Basel (CH)**

(74) Representative: **Leon, Susanna Iris et al**
**Novartis AG**
**Corporate Intellectual Property**
**P.O. Box**
**4002 Basel (CH)**

(56) References cited:
**WO-A-98/11918** **WO-A-02/072832**

- **G. AVERSA ET AL.: "A monoclonal antibody (A6) recognizing a unique epitope restricted to CD45RO and -RB isoforms of the leukocyte common antigen family identifies functional T cell subsets." CELLULAR IMMUNOLOGY, vol. 158, no. 2, 15 October 1994 (1994-10-15), pages 314-328, XP002228858**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- G. AVERSA ET AL.: "Use of monoclonal antibodies to study in vivo and in vitro activated lymphocytes." TRANSPLANTATION PROCEEDINGS, vol. 21, no. 1(1), February 1989 (1989-02), pages 349-350, XP008017039 NEW YORK, NY, USA
- S. GREGORI ET AL.: "Anti-CD45RO/RB mAb a new tool for tolerance induction." THE FASEB JOURNAL, vol. 17, no. 7, 14 April 2003 (2003-04-14), page C63, XP008046594 BETHESDA, MD, USA
- D. DUNN-WALTERS ET AL.: "Effect of somatic hypermutation on potential N-glycosylation sites in human immunoglobulin heavy chain variable regions." MOLECULAR IMMUNOLOGY, vol. 37, February 2000 (2000-02), pages 107-113, XP002258153
- P. FRIEND ET AL.: "Phase I study of an engineered aglycosylated humanized CD3 antibody in renal transplant rejection." TRANSPLANTATION, vol. 68, no. 11, 15 December 1999 (1999-12-15), pages 1632-1637, XP009011271
- F. KOLBINGER ET AL.: "Humanization of a mouse anti-human IgE antibody: a potential therapeutic for IgE-mediated allergies." PROTEIN ENGINEERING, vol. 6, no. 8, November 1993 (1993-11), pages 971-980, XP000983997 OXFORD, GB
- C. BEDFORD ET AL.: "Glycomics: From glycobiology to diagnostics and therapeutics." DRUG NEWS PERSPECT, vol. 19, no. 3, April 2006 (2006-04), pages 163-172,

## Description

### Field of the Invention

The present invention relates to organic compounds, such as to binding molecules against CD45 antigen isoforms, such as for example monoclonal antibodies (mAbs), and use thereof.

### Background of the Invention

One approach in the treatment of a variety of diseases is to achieve the elimination or the inactivation of pathogenic leukocytes and the potential for induction of tolerance to inactivate pathological immune responses.

Organ, cell and tissue transplant rejection and the various autoimmune diseases are thought to be primarily the result of T-cell mediated immune response triggered by helper T-cells which are capable of recognizing specific antigens which are captured, processed and presented to the helper T cells by antigen presenting cell (APC) such as macrophages and dendritic cells, in the form of an antigen-MHC complex, i.e. the helper T-cell when recognizing specific antigens is stimulated to produce cytokines such as IL-2 and to express or upregulate some cytokine receptors and other activation molecules and to proliferate. Some of these activated helper T-cells may act directly or indirectly, i.e. assisting effector cytotoxic T-cells or B cells, to destroy cells or tissues expressing the selected antigen. After the termination of the immune response some of the mature clonally selected cells remain as memory helper and memory cytotoxic T-cells, which circulate in' the body and rapidly recognize the antigen if appearing again. If the antigen triggering this response is an innocuous environmental antigen the result is allergy, if the antigen is not a foreign antigen, but a self antigen, it can result is autoimmune disease; if the antigen is an antigen from a transplanted organ, the result can be graft rejection.

The immune system has developed to recognize self from non-self. This property enables an organism to survive in an environment exposed to the daily challenges of pathogens. This specificity for non-self and tolerance towards self arises during the development of the T cell repertoire in the thymus through processes of positive and negative selection, which also comprise the recognition and elimination of autoreactive T cells. This type of tolerance is referred to as central tolerance. However, some of these autoreactive cells escape this selective mechanism and pose a potential hazard for the development of autoimmune diseases. To control the autoreactive T cells that have escaped to the periphery, the immune system has peripheral regulatory mechanisms that provide protection against autoimmunity. These mechanisms are a basis for peripheral tolerance.

Cell surface antigens recognized by specific mAbs are generally designated by a CD (Cluster of Differentiation) number assigned by successive International Leukocyte Typing workshops and the term CD45 applied herein refers to the cell surface leukocyte common antigen CD45; and an mAb to that antigen is designated herein as "anti-CD45".

Antibodies against the leukocyte common antigen (LCA) or CD45 are a major component of anti-lymphocyte globulin (ALG). CD45 belongs to the family of transmembrane tyrosine phosphatases and is both a positive and negative regulator of cell activation, depending upon receptor interaction. The phosphatase activity of CD45 appears to be required for activation of Src-family kinases associated with antigen receptor of B and T lymphocytes (Trowbridge IS et al, Annu Rev Immunol. 1994;12:85-116). Thus, in T cell activation, CD45 is essential for signal 1 and CD45-deficicient cells have profound defects in TCR-mediated activation events.

The CD45 antigen exists in different isoforms comprising a family of transmembrane glycoproteins. Distinct isoforms of CD45 differ in their extracellular domain structure which arise from alternative splicing of 3 variable exons coding for part of the CD45 extracellular region (Streuli MF. et al, J. Exp. Med. 1987; 166:1548-1566). The various isoforms of CD45 have different extra-cellular domains, but have the same transmembrane and cytoplasmic segments having two homologous, highly conserved phosphatase domains of approximately 300 residues. Different isoform combinations are differentially expressed on subpopulations of T and B lymphocytes (Thomas ML. et al, Immunol. Today 1988; 9:320-325). Some monoclonal antibodies recognize an epitope common to all the different isoforms, while other mAbs have a restricted (CD45R) specificity, dependent on which of the alternatively spliced exons (A, B or C) they recognize. For example, monoclonal antibodies recognizing the product of exon A are consequently designated CD45RA, those recognizing the various isoforms containing exon B have been designated CD45RB (Beverley PCL et al, Immunol. Supp. 1988; I:3-5). Antibodies such as UCHL1 selectively bind to the 180 kDa isoform CD45RO (without any of the variable exons A, B or C) which appears to be restricted to a subset of activated T cells, memory cells and cortical thymocytes and is not detected on B cells (Terry LA et al, Immunol. 1988; 64:331-336).

WO 2002/072832 discloses monoclonal antibodies (mAbs), which are specific for an epitope on both CD45RB and CD45RO for therapeutic uses against autoimmune diseases and graft rejections.

The variable heavy domain of human antibody may be N-glycosylated (Dunn-Walters D. et al, Molecular Immunology 2000; 37:107-113). Somatic hypermutation introduces predominantly single nucleotide substitution into the variable region genes in order to increase immunoglobulin variability.

N-glycosilation in the variable region is known to affect antigen binding. Not with standing aglycosylated monoclonal antibodies are known, which, while preserving their biological function, by eaching glycosylation lose their adverse side effects (Friends P. et al, Transplantation 1999, 68; 1632-1687)

## Description of the Figures

Figure 1 shows that the inhibition of primary MLR by the "candidate mAb" is dose-dependent in the range of 0.001 and 10 μg/ml. "Concentration" is concentration of the "candidate mAb".

Figures shows the plasmid map of the expression vector HCMV-G1 HuA6-VHQ comprising the heavy chain having the nucleotide sequence SEQ ID NO:12 (3921-4274)-in the complete expression vector nucleotide sequence SEQ ID NO:15.

Figure 3 shows the plasmid map of the-expression vector HCMV-G1 HuA6-VHE-comprising the heavy chain having the nucleotide sequence SEQ ID NO:11 (3921-4274) in the complete expression vector nucleotide sequence SEQ ID NO:16.

Figure 4 shows the plasmid map of the expression vector HCMV-K HuAb-VL1 humV1 comprising the light chain having the nucleotide sequence SEQ ID NO:14 (3964-4284) in the complete expression vector nucleotide sequence SEQ ID NO:17.

Figure 5 shows the plasmid map of the expression vector HCMV-K HuAb- VL1 humV2 comprising the light chain having the nucleotide sequence SEQ ID NO: 13 (3926-4246) in the complete expression vector nucleotide sequence SEQ ID NO:18.

Figure 6 shows the plasmid map of the expression vector LCVL1SP20 comprising the light chain having the nucleotide sequence SEQ ID NO:33 in the complete expression vector nucleotide sequence SEQ ID NO:36.

Figure 7 shows the plasmid map of the expression vector LCVL2SP20 comprising the light chain having the nucleotide sequence SEQ ID NO:13 in the complete expression vector nucleotide sequence SEQ ID NO:39.

Figure 8 shows the plasmid map of the expression vector HCVHEN73D Sp20 comprising the heavy chain having the nucleotide sequence SEQ ID NO:34 in the complete expression vector nucleotide sequence SEQ ID NO:37.

Figure 9 shows the plasmid map of the expression vector HCVHQN73D Sp20 comprising the heavy chain having the nucleotide sequence SEQ ID NO:35 in the complete expression vector nucleotide sequence SEQ ID NO:38.

Figure 10 shows the plasmid-map of the expression vector HCVHESp20 comprising the heavy chain having the Nucleotide sequence SEQ ID NO:11 in the complete-expression vector nucleotide sequence SEQ ID NO:40.

Figure 11 shows the plasmid map of the expression vector HCVHQSp20 comprising the heavy chain having the nucleotide sequence SEQ ID NO:12 in the complete expression vector nucleotide sequence SEQ ID NO:41.

Figure 12 shows Size Exclusion Chromatography analysis of VHE/humV1, VHE/humV2, VHQ/humV1 and VHQ/humV2, and of VHE/humV1 together with VHE-N73D/humV1.

Figure 13 shows Cation Exchange Chromatography of VHE/humV2, VHE/humV1, VHQ/humV2, VHQ/humV1 and of VHE/humV2 together with VHE-N73D/humV1.

Figure 14 shows Reverse Phase Chromatography of VHE/humV2 and VHE-N73D/humV1.

## Description of the Invention

We have now found a humanised antibody having binding specificity for both CD45RO and CD45 RB comprising a heavy chain variable region of SEQ ID NO: 31 or 32 and a light chain variable region of SEQ ID NO:7 or SEQ ID NO:8.

In another aspect the humanized antibody having binding specificity for both CD45RO and CD45RB comprises:

- a heavy chain variable region of SEQ ID NO:31 and a light chain variable region of SEQ ID NO:7,
- a heavy chain variable region of SEQ ID NO:31 and a light chain variable region of SEQ ID NO:8,
- a heavy chain variable region of SEQ ID NO:32 and a light chain variable region of SEQ ID NO:7
- a heavy chain variable region of SEQ ID NO:32 and a light chain variable region of SEQ ID NO:8.

CD45RB/RO humanised antibody may also suppress the inflammatory process that mediates human allograft skin rejection. In addition it is found that CD45RO/RB humanised antibody may suppress the inflammatory process that mediates human allograft skin rejection, in particular, may suppress the inflammatory process that mediates human allograft skin rejection *in vivo* in SCID mice transplanted with human skin and engrafted with mononuclear splenocytes. And furthermore, it is found that CD45RB/RO humanised antibody may lead to prolonged human islet allograft survival by preventing graft-infiltration and by inhibiting the leukocyte-mediated rejection reaction in vivo.

CD45RO/RB humanised antibodies according to the invention are capable of binding specifically to the CD45RB and CD45RO isoforms of the CD45 antigen, either alone or associated with other molecules. The binding reaction may be shown by standard methods (qualitative assay) including for example any kind of binding assay such as direct or indirect immunofluorescence together with fluorescence microscopy or cytofluorimetric (FACS) analysis, enzyme-linked immunosorbent assay (ELISA) or radioimmunoassay in which binding of the molecule to cells expressing a particular CD45 isoform can be visualized. In addition, the binding of this molecule may result in the alteration of the function of the cells expressing these isoforms. For example inhibition of primary or secondary mixed lymphocyte response (MLR) may be determined, such as an in vitro assay or a bioassay for determining the inhibition of primary or secondary MLR in the presence and in the absence of a CD45RO/RB binding molecule and determining the differences in primary MLR inhibition.

Alternatively, the *in vitro* functional modulatory effects can also be determined by measuring the PBMC or T cells or $CD4^+$ T cells proliferation, production of cytokines, change in the expression of cell surface molecules e.g. following cell activation in MLR, or following stimulation with specific antigen such as tetanus toxoid or other antigens, or with polyclonal stimulators such as phytohemagglutinin (PHA) or anti-CD3 and anti-CD28 antibodies or phorbol esters and $Ca^{2+}$ ionophores. The cultures are set up in a similar manner as described for MLR except that instead of allogeneic cells as stimulators soluble antigen or polyclonal stimulators such as those mentioned above are used. T cell proliferation is measured preferably as described above by $^3H$-thymidine incorporation.

Cytokine production is measured preferably by sandwich ELISA where a cytokine capture antibody is coated on the surface of a 96-well plate, the supernatants from the cultures are added and incubated for 1 hr at room temperature and a detecting antibody specific for the particular cytokine is then added, following a second-step antibody conjugated to an enzyme such as Horseradish peroxidase followed by the corresponding substrate and the absorbance is measured in a plate reader. The change in cell surface molecules may be preferably measured by direct or indirect immunofluorescence after staining the target cells with antibodies specific for a particular cell surface molecule. The antibody can be either directly labeled with flourochrome or a fluorescently labeled second step antibody specific for the first antibody can be used, and the cells are analysed with a cytofluorimeter.

The humanised antibody of the invention has a binding specificity for both CD45RO and CD45RB ("CD45RB/RO binding molecule").

Preferably the humanised antibody binds to CD45RO isoforms with a dissociation constant (Kd) <20nM, preferably with a Kd<15nM or <10nM, more preferably with a Kd<5nM. Preferably the humanised antibody binds to CD45RB isoforms with a Kd<50nM, preferably with a Kd<15nM or <10nM, more preferably with a Kd<5nM.

In a further preferred embodiment the humanised antibody of the invention binds those CD45 isoforms which

1) include the A and B epitopes but not the C epitope of the CD45 molecule; and/or

2) include the B epitope but not the A and not the C epitope of the CD45 molecule; and/or

3) do not include any of the A, B or C epitopes of the CD45 molecule.

In yet a further preferred embodiment the binding molecule of the invention does not bind CD45 isoforms which include

1) all of the A, B and C epitopes of the CD45 molecule; and/or

2) both the B and C epitopes but not the A epitope of the CD45 molecule.

In further preferred embodiments the binding molecule of the invention further

1) recognises memory and in vivo alloactivated T cells; and/or
2) binds to its target on human T cells, such as for example PEER cells; wherein said binding preferably is with a Kd<15nM, more preferably with a Kd<10nM, most preferably with a Kd<5nM; and/or
3) inhibits in vitro alloreactive T cell function, preferably with an $IC_{50}$ of about less than 100nM, preferably less than 50nM or 30nM, more preferably with an $IC_{50}$ of about 10 or 5nM, most preferably with an $IC_{50}$ of about 0,5nM or even 0,1nM; and/or
4) induces cell death through apoptosis in human T lymphocytes; and/or
5) induces alloantigen-specific T cell tolerance in vitro; and/or
6) prevents lethal xenogeneic graft versus host disease (GvHD) induced in SCID mice by injection of human PBMC when admistiered in an effective amount; and/or
7) binds to T lymphocytes, monocytes, stem cells, natural killer cells and/or granulocytes, but not to platelets or B lymphocytes; and/or
8) supports the differentiation of T cells with a characteristic T regulatory cell (Treg) phenotype; and/or
9) induces T regulatory cells capable of suppressing naïve T cell activation; and/or
10) suppresses the inflammatory process that mediates human allograft skin rejection, in particular, suppresses the inflammatory process that mediates human allograft skin rejection *in vivo* in SCID mice transplanted with human skin and engrafted with mononuclear splenocytes; and/or
11) prolongs human islet allograft survival in a hu-PBL-NOD/SCID mice model.

In a further preferred embodiment the humanised antibody of the invention binds to the same epitope as the monoclonal antibody "A6" as described by Aversa et al., Cellular Immunology 158, 314-328 (1994).

Due to the above-described binding properties and biological activities, such humanised antibody of the invention are particularly useful in medicine, for therapy and/or prophylaxis. Diseases in which the humanised antibody of the invention are particularly useful include autoimmune diseases, transplant rejection, dermatitis, inflammatory bowel disease and/or allergies, as will be further set out below.

Humanised antibodies may be e.g. derived from antibodies as produced by B-cells or hybridomas and or any fragment thereof, e.g. F(ab')2 and Fab fragments, as well as single chain or single domain antibodies. A single chain antibody consists of the variable regions of antibody heavy and light chains covalently bound by a peptide linker, usually consisting of from 10 to 30 amino acids, preferably from 15 to 25 amino acids. Therefore, such a structure does not include the constant part of the heavy and light chains and it is believed that the small peptide spacer should be less antigenic than a whole constant part. By a humanised antibody is meant an antibody in which the hypervariable regions (CDRs) are of non-human (e.g. murine) origin while all or substantially all the other part, e.g. the constant regions and the highly conserved parts of the variable regions are of human origins. A humanised antibody may however retain a few amino acids of the murine sequence in the parts of the variable regions adjacent to the hypervariable regions.

Hypervariable regions, i.e. CDR's according to the present invention may be associated with any kind of framework regions, e.g. constant parts of the light and heavy chains, of human origin. Suitable framework regions are e.g. described in "Sequences of proteins of immunological interest", Kabat, E.A. et al, US department of health and human services, Public health service, National Institute of health. Preferably the constant part of a human heavy chain may be of the IgG1 type, including subtypes, preferably the constant part of a human light chain may be of the $\kappa$ or $\lambda$ type, more preferably of the $\kappa$ type. Preferably, said heavy chain comprises not more than one glycosylation site, most preferably the glycosylation site is a N-glycosylation site, and most preferably the one glycosylation site is located in the constant part of the heavy chain. Most preferably no glycosylation site is present in the variable region, preferably no glycosylation site in the framework region.

In another aspect the present invention provides a humanised antibody comprising

- a polypeptide of SEQ ID NO:31 and a polypeptide of SEQ ID NO:7 (such as VHEN73D/humV2),
- a polypeptide of SEQ ID NO:31 and a polypeptide of SEQ ID NO:8 (such as VHEN73D/humV1),
- a polypeptide of SEQ ID NO:32 and a polypeptide of SEQ ID NO:7 (such as VHQN73D/humV2), or
- a polypeptide of SEQ ID NO:32 and a polypeptide of SEQ ID NO:8 (such as VHQN73D/humV1).

"Polypeptide", if not otherwise specified herein, includes any peptide or protein comprising amino acids joined to each other by peptide bonds, having an amino acid sequence starting at the N-terminal extremity and ending at the C-terminal extremity. The polypeptide of the present invention is a monoclonal antibody. Preferably is a humanised (CDR-grafted) monoclonal antibody. The humanised (CDR-grafted) monoclonal antibody may or may not include further mutations introduced into the framework (FR) sequences of the acceptor antibody. Preferably the humanized antibody comprises no more than one glycosylation site. Most preferably said one glycosylation site is a N-glycosylation site Most preferably no glycosylation site is present in the variable region, and even more preferably no glycosylation site is present in the variable region of the heavy chain, most preferably no glycosylation site is present in the framework regions (FR's).

The term "covalent modification" includes modifications of a polypeptide according to the present invention, e.g. of a specified sequence; or a fragment thereof with an organic proteinaceous or non-proteinaceous derivatizing agent, fusions to heterologous polypeptide sequences, and post-translational modifications. Covalent modified polypeptides, e.g. of a specified sequence, still have the ability bind to CD45RO and CD45RB by crosslinking. Covalent modifications are traditionally introduced by reacting targeted amino acid residues with an organic derivatizing agent that is capable of reacting with selected sides or terminal residues, or by harnessing mechanisms of post-translational modifications that function in selected recombinant host cells. Certain post-translational modifications are the result of the action of recombinant host cells on the expressed polypeptide. Glutaminyl and asparaginyl residues are frequently post-translationally deamidated to the corresponding glutamyl and aspartyl residues. Alternatively, these residues are deaminated under mildly acidic conditions. Other post-translational modifications include hydroxylation of proline and lysine, phosphorylation of hydroxyl groups of seryl, tyrosine or threonyl residues, methylation of the α-amino groups of lysine, arginine, and histidine side chains, see e.g. T. E. Creighton, Proteins: Structure and Molecular Properties, W. H. Freeman & Co., San Francisco, pp. 79-86 (1983). Covalent modifications e.g. include fusion proteins comprising a polypeptide according to the present invention, e.g. of a specified sequence and their amino acid sequence variants, such as immunoadhesins, and N-terminal fusions to heterologous signal sequences.

"Amino acid(s)" refer to all naturally occurring L-α-amino acids, e.g. and including D-amino acids. The amino acids are identified by either the well known single-letter or three-letter designations.

The term "amino acid sequence variant" refers to molecules with some differences in their amino acid sequences as compared to a polypeptide according to the present invention, e.g. of a specified sequence. Amino acid sequence variants of a polypeptide according to the present invention, e.g. of a specified sequence, still have the ability to bind to CD45RO and CD45RB. Substitutional variants are those that have at least one amino acid residue removed and a different amino acid inserted in its place at the same position in a polypeptide according to the present invention, e.g. of a specified sequence. These substitutions may be single, where only one amino acid in the molecule has been substituted, or they may be multiple, where two or more amino acids have been substituted in the same molecule. Insertional variants are those with one or more amino acids inserted immediately adjacent to an amino acid at a particular position in a polypeptide according to the present invention, e.g. of a specified sequence. Immediately adjacent to an amino acid means connected to either the α-carboxy or α-amino functional group of the amino acid. Deletional variants are those with one or more amino acids in a polypeptide according to the present invention, e.g. of a specified sequence, removed. Ordinarily, deletional variants will have one or two amino acids deleted in a particular region of the molecule.

In another aspect the present invention provides isolated polynucleotides encoding the humanised antibody of the present invention.

Polynucleotides comprising polynucleotides encoding a polypeptide of SEQ ID NO:31 or of SEQ ID NO:32 and/or, preferably and, a polypeptide of SEQ ID NO:7 or of SEQ ID NO:8; e.g. encoding

- a polypeptide of SEQ ID NO:31 and a polypeptide of SEQ ID NO:7,
- a polypeptide of SEQ ID NO:31 and a polypeptide of SEQ ID NO:8,
- a polypeptide of SEQ ID NO:32 and a polypeptide of SEQ ID NO:7, or
- a polypeptide of SEQ ID NO:32 and a polypeptide of SEQ ID NO:8.

"Polynucleotide", if not otherwise specified herein, includes any polyribonucleotide or polydeoxyribonucleotide, which may be unmodified RNA or DNA, or modified RNA or DNA, including without limitation single and double stranded RNA, and RNA that is a mixture of single- and double-stranded regions.

A CD45RO/RB humanised antibody e.g. which is a chimeric or humanised antibody, may be produced by recombinant DNA techniques. Thus, one or more DNA molecules encoding the CD45RO/RB may be constructed, placed under appropriate control sequences and transferred into a suitable host (organism) for expression by an appropriate vector.

In another aspect the present invention provides a polynucleotide which encodes a single, heavy and/or a light chain of a CD45RO/RB humanised antibody according to the present invention; and the use of a polynucleotide according to the present invention for the production of a CD45RO/RB humanised antibody according to the present invention by recombinant means.

A CD45RO/RB-binding molecule may be obtained according, e.g. analogously, to a method as conventional together with the information provided herein, e.g. with the knowledge of the amino acid sequence of the hypervariable or variable regions and the polynucleotide sequences encoding these regions. A method for constructing a variable domain gene is e.g. described in EP 239 400 and may be briefly summarized as follows: A gene encoding a variable region of a mAb of whatever specificity may be cloned. The DNA segments encoding the framework and hypervariable regions are determined and the DNA segments encoding the hypervariable regions are removed. Double stranded synthetic CDR cassettes are prepared by DNA synthesis according to the CDR and CDR' sequences as specified herein. These cassettes are provided with sticky ends so that they can be ligated at junctions of a desired framework of human origin. Polynucleotides encoding single chain antibodies may also be prepared according to, e.g. analogously, to a method as conventional. A polynucleotide according to the present invention thus prepared may be conveniently transferred into an appropriate expression vector.

Appropriate cell lines may be found according, e.g. analogously, to a method as conventional. Expression vectors, e.g. comprising suitable promotor(s) and genes encoding heavy and light chain constant parts are known e.g. and are commercially available. Appropriate hosts are known or may be found according, e.g. analogously, to a method as conventional and include cell culture or transgenic animals.

In another aspect the present invention provides an expression vector comprising polynucleotides according to the present invention, wherein the vector is capable of producing humanised antibody when said vector is present in a compatible host cell.

In another aspect, the present invention provides an isolated host cell which comprises an expression vector capable of producing humanised antibody We have further found that a CD45RO/RB humanised antibody according to the present invention inhibit primary alloimmune responses in a dose-dependent fashion as determined by in vitro MLR. The results indicate that the cells which had been alloactivated in the presence of a CD45RO/RB humanised antibody according to the present invention are impaired in their responses to alloantigen. This confirms the indication that a CD45RO/RB humanised antibody according to the present invention can act directly on the effector alloreactive T cells and modulate their function. In addition, the functional properties of T cells derived from the primary MLR were further studied in restimulation experiments in secondary MLR, using specific stimulator cells or third-party stimulators to assess the specificity of the observed functional effects. We have found that the cells derived from primary MLRs in which a CD45RO/RB humanised antibody according to the present invention is present, were impaired in their ability to respond to subsequent optimal stimulation with specific stimulator cells, although there was no antibody added to the secondary cultures. The specificity of then inhibition was demonstrated by the ability of cells treated with a CD45RO/RB humanised antibody according to the present invention to respond normally to stimulator cells from unrelated third-party donors. Restimulation experiments using T cells derived from primary MLR cultures thus indicate that the cells which had been alloactivated a CD45RO/RB humanised antibody according to the present invention are hyporesponsive, i.e. tolerant, to the original alloantigen. Further biological activities are described in examples 7, and 9 to 13.

Furthermore we have found that cell proliferation in cells pre-treated with a CD45RO/RB humanised antibody according to the present invention could be rescued by exogenous IL-2. This indicates that treatment of alloreactive T cells with a CD45RO/RB humanised antibody according to the present invention induces a state of tolerance. Indeed, the reduced proliferative responses observed in cells treated with a CD45RO/RB humanised antibody according to the present invention, was due to impairement of T cell function, and these cells were able to respond to exogenous IL-2, indicating that these cells are in an anergic, true unresponsive state. The specificity of this response was shown by the ability of cells treated with a CD45RO/RB humanised antibody according to the present invention to proliferate normally to unrelated donor cells to the level of the control treated cells.

In addition experiments indicate that the binding of a CD45RO/RB humanised antibody according to the present invention to CD45RO and CD45RB may inhibit the memory responses of peripheral blood mononuclear cells (PBMC) from immunized donors to specific recall antigen. Binding of a CD45RO/RB humanised antibody according to the present invention to CD45RO and CD45RB thus is also effective in inhibiting memory responses to soluble Ag. The ability of a CD45RO/RB humanised antibody according to the present invention to inhibit recall responses to tetanus in PBMC from immunized donors indicate that a CD45RO/RB binding molecule according to the present invention is able to target and modulate the activation of memory T cells. E.g. these data indicate that a CD45RO/RB humanised antibody according to the present invention in addition to recognizing alloreactive and activated T cells is able to modulate their function, resulting in induction of T cell anergy. This property may be important in treatment of ongoing immune responses to autoantigens and allergens and possibly to alloantigens as seen in autoimmune diseases, allergy and chronic rejection, and diseases, such as psoriasis, inflammatory bowel disease, where memory responses play a role in the maintenance of disease state. It is believed to be an important feature in a disease situation, such as in autoimmune diseases in which memory responses to autoantigens may play a major role for the disease maintenance.

We have also found that a CD45RO/RB humanised antibody according to the present invention may modulate T cell proliferative responses in a mixed lymphocyte response (MLR) *in vivo*, i.e. a CD45RO/RB humanised antibody according to the present invention was found to have corresponding inhibitory properties in vivo testing, such as for example, prevention of lethal xenogeneic graft versus host disease (GvHD) or suppression of inflammatory process that mediates human allograft skin rejection in SCID mice models, or prolonging of human islet allograft survival in a hu-PBL-NOD/SCID mice model.

A CD5RO/RB humanised antibody according to the present invention may thus have immunosuppressive and tolerogenic properties and may be useful for in vivo and ex-vivo tolerance induction to alloantigens, autoantigens, allergens and bacterial flora antigens, e.g. a CD45RO/RB humanised antibody according to the present invention may be useful in the treatment and prophylaxis of diseases e.g. including autoimmune diseases, such as, but not limited to, rheumatoid arthritis, psoriatic arthritis, autoimmune thyroditis, Graves disease, type I and type II diabetes, multiple sclerosis, Crohn's disease (CD), ulcerative colitis (UC), systemic lupus erythematosus, Sjögren syndrome, scleroderma, autoimmune gastritis, glomerulonephritis, transplant rejection, such as, but not limited to, organ and tissue allograft and xenograft rejection, e.g. for the treatment of recipients of e.g. heart, lung, combined heart-lung, liver, kidney, pancreatic, skin or corneal transplants, graft versus host disease (GVHD), such as following bone marrow transplantation, and/or pancreatic islet cell transplant rejection, and/or also psoriasis, dermatitis such as atopic and contact dermatitis including allergic contact dermatitis, inflammatory bowel disease and/or allergies, including allergic asthma.

In another aspect the present invention provides the use of the humanised antibody CD45RO/RB according to the present invention as a pharmaceutical.

In a further aspect the present invention provides the humanised antibody according to the present invention for use in the treatment and/or prophylaxis of autoimmune diseases; transplant rejection, psoriasis, dermatitis, inflammatory bowel disease an/or allergies

In another aspect the present invention provides a humanised antibody according to the present invention in the treatment and/or prophylaxis of graft versus host disease (GVHD).

In another aspect the present invention provides a humanised antibody according to the present invention for the preparation of a medicament in the treatment of pancreatic islet cell transplant rejection.

In another aspect the present invention provides a method of treatment and/or prophylaxis of diseases associated with autoimmune diseases, transplant rejection, psoriasis, dermatitis, inflammatory bowel disease and/or allergies comprising administering to a subject in need of such treatment and/or prophylaxis an effective amount of a GD45RO/RB humanised antibody according to the present invention, e.g. in the form of a pharmaceutical composition according to the present invention.

One embodiment of the present invention provides a method of treatment and/or prophylaxis of a disease associated with islet cell transplant rejection, e.g. islet cell transplant rejection, comprising administering to a subject in need of such treatment and/or prophylaxis an effective amount of a humanised antibody according to the present invention.

In preferred embodiments said CD45RO/RB humanised antibody for use as a pharmaceutical, for the production of a medicament or in a method of treatment and/or prophylaxis of a disease associated with autoimmune diseases, transplant rejection, psoriasis, dermatitis, inflammatory bowel disease and/or allergies comprises a polypeptide of SEQ ID NO:31 or of SEO ID NO:32 and/or a polypeptide of SEQ ID NO:7 or of SEQ ID NO:8. Preferrably the CD45RO/RB humanised antibody comprises a polypeptide of SEQ ID NO:31 and a polypeptide of SEQ ID NO:8.

An "effective amount" of a CD45RO/RB humanised antibody is an amount sufficient to effect beneficial or desired results including clinical results such decreasing one or more symptoms resulting from the autoimmune disease, transplant rejection, psoriasis, dermatitis, inflammatory bowel disease and/or allergy, increasing the quality of life of those suffering from, decreasing the dose of other medications required to treat such diseases, enhancing effect of another medication, delaying the progression of the disease, and/or prolonging survival of patients, either directly or indirectly.

An effective amount can be administered in one or more administrations and may or may not be achieved in conjunction with another drug, compound, or pharmaceutical composition. Thus, an "effective amount" may be considered in the context of administering one or more therapeutic agents, and a single agent may be considered to be given in an effective amount if, in conjunction with one or more other agents, a desirable result may be or is achieved.

Further is provided that a CD45RO/RB humanised antibody of the invention may be administered as the sole active ingredient or together with other drugs in immunomodulating regimens or other anti-inflammatory agents e.g. for the treatment or prevention of diseases associated with autoimmune diseases, transplant rejection, psoriasis, dermatitis inflammatory bowel disease and/or allergies. For example, a CD45RO/RB humanised antibody of the invention may be used in combination with a calcineurin inhibitor, e.g. cyclosporine A, cyclosporine G, FK-506, ABT-281, ASM 981; an mTOR inhibitor, e.g. rapamycin, 40-O-(2-hydroxy)ethyl-rapamycin, CCI779, ABT578, AP23573, AP23464, AP23675, AP23841, TAFA-93, biolimus-7 or bioimus-9; a corticosteroid; cyclophosphamide; azathioprine; methotrexate; a S1P receptor agonist, e.g. FTY 720 or an analogue thereof; leflunomide or analogs thereof; mizoribine; mycophenolic acid; mycophenolate mofetil; 15-deoxyspergualine or analogs thereof; immunosuppressive monoclonal antibodies, e.g., monoclonal antibodies to leukocyte receptors, e.g., MHC, CD2, CDS, CD4, CD11a/CD18. CD7, CD25, CD27, B7, CD40, CD45, CD58, CD137, ICOS, CD150 (SLAM), OX40, 4-1BB or their ligands, e.g. CD154; or other immunomodulatory compounds, e.g. a recombinant binding molecule having at least a portion of the extracellular domain of CTLA4 or a mutant thereof, e.g. an at least extracellular portion of CTLA4 or a mutant thereof joined to a non-CTLA4 protein sequence, e.g. CTLA4lg (e.g. designated ATCC 68629) or a mutant thereof, e.g. LEA29Y, or other adhesion molecule inhibitors, e.g. mAbs or low molecular weight inhibitors including LFA-1 antagonists, Selectin antagonists and VLA-4 antagonists.

An effective amount of a CD45RO/RB humanised antibody of the invention, alone or in conjunction with another drug, compound, or pharmaceutical composition can be administered by any conventional route, including injection or by gradual infusion over time. The administration may, for example, be oral, intravenous, intraperitoneal, intramuscular, intracavity, subcutaneous, topical or transdermal. By "co-administration" is meant administration of the components of the compositions of the invention together or at substantially the same time, either in the same vehicle or in separate vehicles, so that upon oral administration, for example, both compounds are present simultaneously in the gastrointestinal tract. Preferably, the compounds are administered as a fixed combination.

In ,another aspect the present invention provides a pharmaceutical composition comprising a CD45RO/RB humanised antibody according to the present invention in association with at least one pharmaceutically acceptable carrier or diluent.

The term "pharmaceutically-acceptable carrier or diluent" as used herein means one or more compatible solid or liquid fillers, diluents or encapsulating substances which are suitable for administration into a mammals including humans. The term "carrier" denotes an organic or inorganic ingredient, natural or synthetic, with which the active ingredient is combined to facilitate the application.

The term "pharmaceutically acceptable" means a non-toxic material that does not interfere with the effectiveness of the biological activity of the active ingredients. Such preparations may routinely contain pharmaceutically acceptable concentrations of salts, buffering agents, preservatives, compatible carriers, supplementary immune potentiating agents such as adjuvants and cytokines and optionally other therapeutic agents, such as chemotherapeutic agents.

When used in medicine, the salts should be pharmaceutically acceptable, but non-pharmaceutically acceptable salts may conveniently be used to prepare pharmaceutically-acceptable salts thereof and are not excluded from the scope of the invention.

The pharmaceutical compositions may contain suitable buffering agents, including: acetic acid in a salt; citric acid in a salt; boric acid in a salt; and phosphoric acid in a salt. The pharmaceutical compositions also may contain, optionally, suitable preservatives, such as: benzalkonium chloride; chlorobutanol; parabens and thimerosal.

The doses of polypeptide or nucleic acid encoding said polypeptide administered to a subject can be chosen in accordance with different parameters, in particular in accordance with the mode of administration used and the state of the subject. Other factors include the desired period of treatment. In the event that a response in a subject is insufficient at the initial doses applied, higher doses (or effectively higher doses by a different, more localized delivery route) may be employed to the extent that patient tolerance permits.

The pharmaceutical compositions may conveniently be presented in unit dosage form and may be prepared by any of the methods well-known in the art of pharmacy. All methods include the step of bringing the active agent into association with a carrier which constitutes one or more accessory ingredients. In general, the compositions are prepared by uniformly and intimately bringing the active compound into association with a liquid carrier, a finely divided solid carrier, or both, and then, if necessary, shaping the product.

Compositions suitable for oral administration may be presented as discrete units, such as capsules, tablets, lozenges, each containing a predetermined amount of the active compound. Other compositions include suspensions in aqueous liquids or non-aqueous liquids such as a syrup, elixir or an emulsion.

Compositions suitable for parenteral administration conveniently comprise a sterile aqueous or non-aqueous preparation of a polypeptide or nucleic acid encoding the polypeptide, which is preferably isotonic with the blood of the recipient. This preparation may be formulated according to known methods using suitable dispersing or wetting agents and suspending agents. The sterile injectable preparation also may be a sterile injectable solution or suspension in a non-toxic parenterally-acceptable diluent or solvent, for example, as a solution in 1,3-butane diol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or di-glycerides. In addition, fatty acids such as oleic acid may be used in the preparation of injectables. Carrier formulation suitable for oral, subcutaneous, intravenous, intramuscular, etc. administrations can be found in Remington's Pharmaceutical Sciences, Mack Publishing Co., Easton, PA.

A pharmaceutical composition may comprise further, e.g. active, ingredients, e.g. other immunomodulatory antibodies such as, but not confined to anti-ICOS, anti-CD154, anti-CD134L or recombinant proteins such as, but not confined to rCTLA-4 (CD152), rOX40 (CD134), or anti-inflammatory agents or Immunomodulatory compounds such as, but not confined to cyclosporin A, FTY720, RAD, rapamycin, FK506, 15-deoxyspergualin, steroids; as described above. Such pharmaceutical composition may comprise a CD45RO/RB humanised antibody according to the invention and immunomodulating drugs and/or anti-inflammatory agents in separate unit dosage forms, preferably wherein the unit dosage forms are suitable for administration of the component compounds in synergistically effective amounts, together with instruction for use, optionally with further means for facilitating compliance with the administration of the component compounds, e.g. a label or drawings. The compositions of the invention can be administered as a free combination, or can be formulated into a fixed combination. Absolute dosages of the compounds will vary depending on a number of factors, e.g. the individual, the route of administration, the desired duration, the rate of release of the active agent and the nature and severity of the condition to be treated.

Diseases as outlined above to be treated with a CD45RO/RB humanised antibody of the present invention alone or in combination with other drugs include, but are not limited to autoimmune diseases, including rheumatoid arthritis, psoriatic arthritis, autoimmune thyroditis, Graves disease, type I and type II diabetes, multiple sclerosis, Crohn's disease (CD), ulcerative colitis (UC), systemic lupus erythematosus, Sjögren syndrome, scleroderma, autoimmune gastritis and glomerulonephritis; transplant rejection, Including, but are not limited to, organ and tissue allograft and xenograft rejection, e.g. for the treatment of recipients of e.g. heart, lung, combined heart-lung, liver, kidney, pancreatic, skin or corneal transplants, graft versus host disease (GVHD), such as following bone marrow transplantation, and/or pancreatic islet cell transplant rejection; psoriasis; dermatitis such as atopic and contact dermatitis including allergic contact dermatitis; inflammatory bowel disease and/or allergies, including allergic asthma.

## EXAMPLES

The invention will be more fully understood by reference to the following examples. They should not, however, be construed as limiting the scope of the invention. In the following examples all temperatures are in degree Celsius.

The "candidate mAb" or "chimeric antibody" is a CD45RO/RB binding molecule according to the present invention comprising light chain of SEQ ID NO:3 and heavy chain of SEQ ID NO:4.

The "humanised antibody" is a CD45RO/RB binding molecule according to the present invention comprising a polypeptide of SEQ ID NO:8 and polypeptide of SEQ ID NO:9 (VHE/humV1, VHE/VL1 or VHE/VLh), a polypeptide of SEQ ID NO:8 and a polypeptide of SEQ ID NO:10 (VHQ/humV1, VHQ/VL1 or VHQ/VLh); a polypeptide of SEQ ID NO: 7 and polypeptide of SEQ ID NO:9 (VHE/humV2, VHE/VL2 or VHE/VLm); a polypeptide of SEQ ID NO:7 and polypeptide of SEQ ID NO:10 (VHQ/humV2, VHQ/VL2 or VHQ/VLm); a polypeptide of SEQ ID NO:8 and polypeptide of SEQ ID NO: 31 (VHEN73D/humV1, VHEN73D/VL1 or VHEN73D/VLh); a polypeptide of SEQ ID NO:8 and polypeptide of SEQ ID NO:32 (VHQN73D/humV1, VHQN73D/VL1 or VHQN73D/VLh); a polypeptide of SEQ ID NO:7 and polypeptide of SEQ ID NO:31 (VHEN73D/humV2, VHEN73D/VL2 or VHEN73D/VLm); or a polypeptide of SEQ ID NO:7 and polypeptide of SEQ ID NO:32 (VHQN73D/humV2, VHQN73D/VL2 or VHEN73D /VLm).

The following abbreviations are used:

| | |
|---|---|
| APC | antigen presenting cell |
| CEX | cation exchange chromatography |
| c.p.m. | counts per minute |
| dhfr | dihydrofolate reductase |
| EDTA | ethylen dinitrilo tetra acidic acid |
| ELISA | enzyme linked immuno-sorbant assay |
| ESI-Q-TOF | electrospray ionization - quadrupole - time of flight |
| FACS | fluorescence activated cell sorting |
| Fc | fragment crystallizable |
| $F(ab')_2$ | fragment antigen-binding; bivalent |
| FITC | fluorescein isothiocyanate |
| FBS | foetal bovine serum |
| GVHD | graft-vs-host disease |
| HCMV | human cytomegalovirus promoter |
| HPLC | High Performance Liquid Chromatography |
| IFN-y | interferon gamma |
| IgE | immunoglobulin isotype E |
| IgG | immunoglobulin isotype G |
| IL-2 | interleukin-2 |
| IU | international units |
| MALDI-TOF | matrix assisted laser desorption ionization - time of flight |
| MLR | mixed lymphocyte reaction |
| MLC | mixed lymphocyte culture |
| MP1 | matrix protein 1 from hemophilus influenza |
| MTX | methotrexate |
| PBS | phosphate-buffered saline |
| PBL | peripheral blood leukocytes |
| PBMC | peripheral blood mononuclear cells |
| PCR | polymerase chain reaction |
| RP | reverse phase chromatography |
| SEC | size exclusion chromatography |
| SCID | severe combined immunodeficiency |
| $T_{reg}$ | T regulatory cells |
| xGVHD | xeno-graft-vs-host disease |

**Example 1: Primary mixed lymphocyte response (MLR)**

*Cells*

Blood samples are obtained from healthy human donors. Peripheral blood mononuclear cells (PBMC) are isolated by centrifugation over Ficoll-Hypaque (Pharmacia LKB) from leukocytes from whole peripheral blood, leukopheresis or buffy coats with known blood type, but unknown HLA type. In some MLR experiments, PBMC are directly used as the stimulator cells after the irradiation at 40 Gy. In the other experiments, T cells were depleted from PBMC by using CD2 or CD3 Dynabeads (Dynal, Oslo, Norway). Beads and contaminating cells are removed by magnetic field. T cell-depleted PBMC are used as simulator cells after the irradiation.

PBMC, $CD3^+$ T cells or $CD4^+$ T cells are used as the responder cells in MLR. Cells are prepared from different donors to stimulator cells. $CD3^+$ T cells are purified by negative selection using anti-CD16 mAb (Zymed, CA), goat anti-mouse IgG Dynabeads, anti-CD14 Dynabeads, CD19 Dynabeads. In addition anti-CD8 Dynabeads are used to purify $CD4^+$ T cells. The cells obtained are analyzed by FACScan or FACSCalibur (Becton Dickinson & Co., CA) and the purity of the cells obtained was >75%. Cells are suspended in RPMI1640 medium, supplemented with 10 % heat-inactivated FBS, penicillin, streptomycin and L-glutamine.

*Reagents*

The chimeric anti-CD45R0/RB mAb "candidate mAb" and an isotype matched control chimeric antibody is also generated. Mouse (Human) control $IgG_1$ antibody specific for KLH (keyhole limpet hemocyanin) or recombinant human IL-10 is purchased from BD Pharmingen (San Diego, CA). Anti-human CD154 mAb 5c8 is according to Lederman et al 1992.

*Primary Mixed lymphocyte response (MLR)*

Aliquots of 1 x $10^5$ PBMC or 5 X $10^4$ of $CD3^+$ or $CD4^+$ cells are mixed with 1 x $10^5$ irradiated PBMC or 5 x $10^4$ T cells-depleted irradiated (50 Gy) PBMC in the each well of 96-well culture plates (Costar, Cambridge, MA) in the presence of the indicated mAb or absence of Ab. In some experiments, $F(ab')_2$ fragment of goat anti-mouse lg or goat anti-human lg specific for Fc portion (Jackson ImmunoResearch, West Grove, PA) is added at 10 $\mu$g/ml in addition to the candidate mAb To ensure optimal in vitro cross-linking of the target CD45 molecules. The mixed cells are cultured for 4 or 5 days at 37°C in 5% $CO_2$ and proliferation is determined by pulsing the cells with $^3$H-thymidine for the last 16-20 hours of culture.

Other experiments are similar to those described above, but with the following exceptions: 1) Medium used is EX-VIVO (Bio-Whittaker) containing 10% FBS and 1% human plasma; 2) Anti-mouse total IgG (5 $\mu$g/ml) is used as secondary cross-linking step; 3) Irradiation of stimulator cells is 60 Gy.

Primary MLR is performed in the presence of the "candidate mAb" or control chimeric $IgG_1$ (10 $\mu$g/ml) both with a second step reagent, $F(ab')_2$ fragment of goat anti-human lg specific for Fc portion (10 $\mu$g/ml). Percentage inhibition by the "candidate mAb" is calculated in comparison with the cell proliferation in the presence of control $IgG_1$. Results are shown in TABLE 1 below:

TABLE 1

| Inhibition of primary MLR by 10 $\mu$g/ml of a candidate mAb according to the present invention | | |
|---|---|---|
| **Responder** | **Stimulator (Irr. PBMC)** | **% of Inhibition** |
| #211 CD4 | #219 CD3 | 63.51 |
| #220 CD4 | #219 CD3 depl. | 63.07 |
| #227 CD4 | #220 CD3 depl. | 65.96 |
| #229 CD4 | #219 CD3 depl. | 50.76 |
| Average$\pm$ SD | | 60.83$\pm$6.83* |
| * Significantly different from control value (P<0.001) | | |

A candidate mAb according to the present invention inhibits primary MLR as can be seen from TABLE 1. The average inhibitory effect is 60.83 $\pm$ 6.83 % in four different donors-derived $CD4^+$ T cells and statistically significant.

The inhibition of primary MLR by the "candidate mAb" is shown to be dose-dependent in the range of 0.001

and 10 μg/ml of the "candidate mAb" as shown in Figure 1.

The $IC_{50}$ for the inhibition of primary MLR by a "candidate mAb" is determined from the results of three separate MLR experiments using one donor PBMC as responder cells. Thus, responder $CD4^+$ T cells from Donor #229 and #219 and irradiated PBMC depleted of T cells as stimulators are mixed in the presence of a "candidate mAb" or control chimeric Ab with 10 μg/ml of $F(ab')_2$ fragment of goat anti-human Ig. Experiments are repeated 3 times and percentage of proliferation in the presence of a "candidate mAb" is calculated in comparison with the T cell proliferation in the presence of control Ab. $IC_{50}$ value is determined using Origin (V. 6.0®). The cellular activity $IC_{50}$ value is calculated to be 0.87 ± 0.35 nM (0.13 ± 0.052 μg/ml).

**Example 2: Secondary MLR**

In order to assess whether a "candidate mAb" induces unresponsiveness of $CD4^+$ T cells to specific alloantigens, secondary MLR is performed in the absence of any antibodies after the primary MLC. $CD4^+$ T cells are cultured with irradiated allogeneic stimulator cells (T cells-depleted PBMC) in the presence of the indicated antibody in 96-well culture plates for 10 days (primary MLC). Then, cells are collected, layered on a Ficoll-Hypaque gradient to remove dead cells, washed twice with RPMI, and restimulated with the same stimulator, $3^{rd}$ party stimulator cells or IL-2 (50 U/ml). The cells are cultured for 3 days and the proliferative response is determined by pulsing the cells with $^3$H-thymidine for the last 16-20 hours of culture.

Specifically, $CD4^+$ T cells are cultured with irradiated allogeneic stimulator cells (T cells-depleted PBMC taken from other donors) in the presence of 10 μg/ml of the "candidate mAb", control IgG1 chimeric Ab and $F(ab')_2$ fragment of goat anti-human Ig. Primary MLR proliferation is determined on day 5. For secondary MLR, the responder and stimulator cells are cultured for 10 days in the presence of the "candidate mAb", then the cells are harvested, washed twice in RPMI1640 and restimulated with specific stimulator, third-party stimulators or IL-2 (50 U/ml) in the absence of any Ab. Cell proliferation is determined on day 3. Results set out in TABLE 2:

TABLE 2

| Responder CD4+ T cells Donor # | % Inhibition of $2^{ry}$ MLR |
|---|---|
| #211 | 49.90* |
| #220 | 59.33* |
| #227 | 58.68* |
| * Significantly different from control value (p=<0.001 determined by t-test, SigmaStat V.2.03). # p=<0.046 | |

In order to test whether the impaired proliferation is due to unresponsivess as a consequence of the treatment with a "candidate mAb", the cells derived from primary MLR are cultured in the presence of IL-2 (50 U/ml). Addition of IL-2 results in the rescue of proliferative responses of the T cells which had been treated with a "candidate mAb" in primary MLR, to levels similar to those observed in the presence of $IgG_1$ control Ab. These data indicate that the impaired secondary response in T cells treated with a "candidate mAb" is due to to functional alteration of the responder T cells which become unresponsive to the specific stimulator cells.

Percentage inhibition is calculated according to the following formula:

$$\frac{\text{c.p.m. with control Ab} - \text{c.p.m. with "candidate mAb}}{\text{c.p.m. with control Ab}} \times 100$$

Statistical analysis is performed using SigmaStat (Vers. 2.03).

The data is analyzed by two-way ANOVA followed by Dunnett method. In all test procedures probabilities <0.05 are considered as significant. In some experiments t-test is used (SigmaStat V.2.03).

**Example 3: In vivo survival studies in SCID-mice**

*Engraftment of hu-PBL in SCID mice*

Human peripheral blood mononuclear cells (PBMC) are injected intraperitoneally into SCID mice C.B 17 /GbmsTac-*Prkdc*$^{scid}$ *Lyst*$^{bg}$ mice (Taconic, Germantown, NY) in an amount sufficient to induce a lethal xenogeneic graft-versus-host disease (xGvHD) in >90% of the mice within 4 weeks after cell transfer. Such treated SCID mice are hereinafter designated as hu-PBL-SCID mice

*Mab-treatment of hu-PBL-SCID mice*

Hu-PBL-SCID mice are treated with a "candidate mAb" or mouse or chimeric isotype matched mAb controls at day 0, immediately after PBMC injection, at day 3, day 7 and at weekly intervals thereafter. Mabs are delivered subcutaneously in 100 μl PBS at a final concentration of 5 mg/kg body weight. The treatment was stopped when all control mice were dead.

*Evaluation of treatment results*

The main criterion to assess the efficacy of a "candidate mAb" in this study was the survival of the hu-PBL-SCID mice. The significance of the results is evaluated by the statistical method of survival analysis using the Log-rank test (Mantel method) with the help of the Systat v9.01 software. The method of survival analysis is a non-parametric test, which not only consider whether a particular mouse is still alive but also whether if it was sacrificed for reasons irrelevant to the treatment/disease such as the requirement of perform in vitro analysis with its organs/cells. Biopsies of liver, lung, kidney and spleen are obtained from dead mice for further evaluation. In addition, hu-PBL-SCID mice are weighed at the beginning (before cell transfer) and throughout (every two days) the experiment as an indirect estimation of their health status. Linear regression lines were generated using the body weight versus days post-PBMC transfer values obtained from each mouse and subsequently, their slopes (control versus anti-CD45 treated mice) were compared using the non-parametric Mann-Whitney test.

*Results*

All hu-PBL-SCID mice treated with mouse mAb controls had infiltrated human leukocytes in the lung, liver and spleen and died (4/4) within ca. 2 to 3 weeks after cell transfer. Death is a likely consequence of xGvHD. Control mAb-treated mice furthermore lost weight in a linear manner, ca. 10% and more within 3 weeks.

All hu-PBL-SCID mice treated with a "candidate mAb" survived (4/4) without any apparent sign of disease more than 4 weeks, even although "candidate mAb"-treatment was stopped after 3 weeks. "Candidate mAb"-treated mice increased weight in a linear manner, up to ca. 5% within 4 weeks.

**Example 4: Expression of antibodies of the invention**

Expression of humanised antibody comprising a SEQ ID NO:7, SEQ ID NO:8, SEQ ID NO:9 SEQ ID NO:10 SEQ ID NO:31 or SEQ ID NO:32

Expression vectors according to the plasmid maps shown in Figures 2 to 11 are constructed, comprising the corresponding nucleotides encoding the amino acid sequence of humanised light chain variable region humV1 (SEQ ID NO:8; Figures 4 and 6), humanised light chain variable region humV2 (SEQ ID NO:7; Figures 5 and 7), humanised heavy chain variable region VHE (SEQ ID NO:9; Figure 3 and 10), or humanised heavy chain variable region VHQ (SEQ ID NO:10; Figures 2 and 11), humanised heavy chain variable region VHE-N73D (SEQ ID NO:31; Figure 8) or humanised heavy chain variable region VHQ-N73D (SEQ ID NO:32; Figure 9), respectively. These expression vectors have the DNA (nucleotide) sequences SEQ ID NO: 15 and SEQ ID NO: 41 (VHQ), SEQ ID NO: 16 and SEQ ID NO: 40 (VHE), SEQ ID NO: 17 and SEQ ID NO 36 (humV1), SEQ ID NO: 18 and SEQ ID NO: 39 (humV2), or SEQ ID NO 37 (VHE-N73D) and SEQ ID NO 38 (VHQ-N73D).

Construction of humanised antibody heavy and light chain expression vectors for expression in COS cells

*Human kappa light chain expression vectors for versions VLh and VLm*

In order to construct the final expression vector encoding for the complete humanised light chain of human kappa isotype, DNA fragments encoding the complete light chain variable regions (VLh and VLm) were excised from the VLh and VLm containing PCR-Script cloning vectors (Stratagene) (VLm region) using HindIII and BglII. The gel-purified fragments were then subcloned into the HindIII and BamHI sites of C21-HCMV Kappa expression vector which was created during construction of the humanised anti-IgE antibody TESC-21 (Kolbinger et al 1993) and which originally received from M. Bendig (MRC Collaborative Centre, London, UK) (Maeda et al. 1991). The ligation products were purified by phenol/chloroform extraction, and electroporated into electrocoporation-competent Epicurian Coli® XL1-Blue strain (Cat. N° #200228, Stratagene). After plating on LB/amp agar plates overnight at 37°C, each 12 colonies were picked to prepare plasmid DNA from a 3 ml culture using the BioRobot 9600 (Qiagen). This yielded the light chain expression vectors for the humanised antibody versions VLh and VLm, respectively, as further described in the Figures.

*Human gamma-1 heavy chain expression vectors for VHQ*

For the construction of the VHQ expression vector, a step-wise approach was taken. First, the complete variable region of VHQ was assembled by PCR according to the methology as described in Kolbinger et al 1993 (Protein Eng. 1993 Nov; 6(8):971-80) and subcloned into the C21-HCMV-gamma-1 expression from which the C21 insert had been removed using the same enzymes. A HindIII/BamHI fragment of PCRScript clone VHQ containing the complete variable region was then subcloned into expression vector C21-HCMV-gamma-1 cleaved with the same enzymes. This yielded the final expression vector for the humanised antibody version VHQ.

*Human gamma-1 heavy chain expression vectors for VHE*

The construction of the final VHE expression vector encoding for the complete humanised heavy chain of human gamma-1 isotype was achieved by directly ligating a Hindlll and BamHI restricted PCR fragment encoding the variable region into the HindIII and BamHI sites of C21-HCMV gamma-1 expression vector which was created during construction of the humanised anti-IgE antibody TESC-21 (Kolbinger et al. 1993) and which was also originally received from M. Bendig (MRC Collaborative Centre, London, UK) (Maeda et al. 1991).

Transient expression in COS cells

The following transfection protocol is adapted for adherent COS cells in 150 mm cell culture dishes, using SuperFect™ Transfection Reagent (Cat. N°301305, Qiagen). The four different expression vectors described above are used for transient transfection of cells. For expression of humanised antibody, each of two clones containing heavy chain inserts (VHE or VHQ, respectively) are co-transfected into cells with each of the two clones encoding for the light chains (humV1 or humV2, respectively), in total 4 different combinations of heavy and light chain expression vectors (VHE/humV1, VHE/humV2, VHQ/humV1 and VHQ/humV2). Before transfection, the plasmids are linearized with the restriction endonuclease Pvul which cleaves in the region encoding the resistance gene for ampicillin. The day before transfection, 4 x $10^6$ COS cells in 30 ml of fresh culture medium are seeded in 150 mm cell culture dishes. Seeding at this cell density generally yielded 80% confluency after 24 hours. On the day of transfection, four different combinations of linearized heavy-and light-chain DNA expression vectors (15 μg each) are diluted in a total volume of 900 μl of fresh medium without serum and antibiotics. 180 μl of SuperFect Transfection Reagent is then mixed thoroughly with the DNA solution. The DNA mixture is incubated for 10 min at room temperature to allow complex formation. While complex formation takes place, the growth medium is removed from COS cell cultures, and cells are washed once with PBS. 9 ml of fresh culture medium (containing 10% FBS and antibiotics) are then added to each reaction tube containing the transfection complexes and well mixed. The final preparation is immediately transferred to each of 4 cultures to be transfected and gently mixed. Cell cultures are then incubated with the DNA complexes for 3 hours at 37°C and 5% CO2. After incubation, the medium containing transfection complexes is removed and replaced with 30 ml of fresh culture medium. At 48 hr post transfection, the culture supernatants are harvested.

Concentration of culture supernatants

For ELISA and FACS analysis, the culture supernatants collected from COS cells transfected with heavy- and light- chain plasmids are concentrated as follows. 10 ml of each supernatant are added to Centriprep YM-50 Centrifugal Filter Devices (Cat. N° 4310, Millipore) as described by the manufacturer. The Centriprep filters are centrifuged for 10 min at 3000 rpm at room temperature. The centrifugation step is then repeated again with the remaining 20 ml of supernatant using only 5 min of centrifugation and supervising the concentration evolution. The intermediate 500 μl of concentrated supernatant is recovered, transferred to new Microcon Centrifugal Filter Devices (Cat. N° 42412, Microcon) and further concentrated following the manufacturer's protocol. The concentrated supernatants are centrifuged four times for 24 min at 3000 rpm at room temperature, one time for 10 min at 6000 rpm and then, three times for 5 min, always supervising the concentration evolution. The final volume of concentrated conditioned medium achieved is 100-120 μl corresponding to a 250 to 300-fold concentration of original culture medium and is stored at 4°C until use. For comparison and control, culture medium from untransfected cells is similarly concentrated, using the same centrifugation protocol described above.

Generation of stable Sp2/0 myeloma transfectants secreting humanised anti-CD45RO/RB antibodies

The mouse myeloma cell line Sp2/0 (ATCC, CRL-1581) is electroporated with the above described CHO-expression vectors encoding heavy (VHE or VHQ) and light (humV1 or humV2) chain of the CD45RO/RB binding humanised antibodies. Four different combinations of heavy and light chain expression vectors (VHE/humV1, VHE/humV2, VHQ/humV1 and VHQ/humV2) are used for transfection according to the following protocol: 20 μg supercoiled DNA of each plasmid is mixed in an electroporation cuvette (0.4 cm gap) with 8 x $10^6$ live Sp2/0 cells suspended in DMEM / 10%FCS culture medium. Electroporation settings are 1500 V, 25 μF using a BioRad GenePulser instrument. After electroporation, cells are cultured for 20 h in culture medium (DMEM supplemented with 10% FCS penicillin, streptomycin and L-glutamine). On day two the selection drug G418 (Cat. N° 10131-019, Gibco) is added to a final concentration of 1 mg active drug / ml and the cells are distributed into one 96-well plate, 200 μl each well with approx. $10^5$ cells per well. Ten to 15 days later, G418-surviving clones are expanded in G418-containing medium. Secretion of humanised mAbs from these transfectants is assessed by ELISA, using a coating antibody goat anti-human IgG/Fcγ (Cat. N° 109-005-098, Jackson Labs) and a peroxidase-coupled antibody against human kappa light chain (Cat. N° A-7164, Sigma). Transfectants, which score positive in this assay are selected for a comparison of productivity on a per cell per day basis, again using ELISA (see below). The best clone of each transfectant is selected for immediate subcloning by limiting dilution, using a seeding density of 1 cell per well. Productivity of G418-surviving subclones is again determined as described above.

Subclones are expanded in G418-containing selection medium, until the culture volume reaches 150 ml, at which stage the culture is continued without G418 in flasks destined to feed roller bottles.

After the first transfection and selection, stable transfectants grow out of the 96-well plates at a frequency of 20.8 % for VHE/humV1, 11.5 % for VHQ/humV1, 18.8 % for VHE/humV2 and 7.3 % for VHQ/humV2. After two rounds of subcloning the best two producers are clone 1.33.25 (3.87 pg/cell/day) and clone 1.33.26 (3.43 pg/cell/day) for VHE/humV1 and clone 12.1.4 (1.19 pg/cell/day) and clone 12.1.20 (1.05 pg/cell/day) for VHQ/humV1. The stable Sp2/0 transfectants for VHE/humV1 and VHQ/humV1 are subsequently expanded for antibody production and purification.

The antibodies are purified from supernatants of stably transfected SP2/0 myeloma cell lines containing 10% FCS by a combination of affinity chromatography using an immobilized anti-human IgGFc matrix and size-exclusion chromatography. If required, endotoxin is removed using an Acticlean Etox column (Sterogene Bioseparations).

Construction of humanised antibody heavy and light chain expression vectors for expression in Sp2/0 cells

*Human kappa light chain expression vectors for versions VLh and VLm:*

The humV1 (= VL1) or humV2 (= VL2) cDNA is amplified by PCR from the CHO expression plasmid SEQ ID NO: 17 or SEQ ID NO: 18 respectively, using the primers HuCD45LC-Mlu (5'- AAAACGCGTTGTGACATTCTGCTGACCCAGTCT - 3'; SEQ ID NO: 42) and HuCD45LC-Hind (5'- AAAAAAGCTTGGTCCCCTGGCCGAACGTGAA - 3'; SEQ ID NO: 43). Each 321 bp PCR fragment is digested with *Mlu*I and *Hin*dIII and ligated directly into the light chain expression vector chA6HCk.dhfr which is digested with the same enzymes. The resulting plasmids are named LCVL1Sp20 (SEQ ID NO: 36; Figure 6) and LCVL2Sp20 (SEQ ID NO: 39; Figure 7) respectively. LCVL1Sp20 is then used for the expression in Sp2/0 cells of the humanised antibody VHE/humV1, VHQ/humV1 or VHE-N73D/humV1; LCVL2Sp20 may then used for the expression in Sp2/0 cells of the humanised antibody VHE/humV2 VHQ/humV2 or VHE-N73D/humV2.

*Human gamma-1 heavy chain expression vectors for VHQ and for VHE*

The two humanised $V_H$ cDNA regions are amplified by PCR from the recombinant plasmids HCMV-G1 HuA6-VHE (SEQ ID NO: 16; Figure 3) and HCMV-G1 HuA6-VHQ (SEQ ID NO: 15 Figure 2) respectively, using the PCR primers HuCD45HCEup (5'-CAGGCAGAGGTGCAGCTGGTGGAGTCA - 3'; SEQ ID NO: 44) or HuCD45HCQup (5'-CAGGCACAGGTGCAGCTGGTGGAGTCA - 3'; SEQ ID NO: 45) and HuCD45HClo (5' - AAATCCTTCTAGAACTCACCTGAGGAGAC - 3'; SEQ ID NO: 46). The PCR fragments' 3' ends were digested with BstEII. Each PCR fragment is then cloned into the heavy chain cassette vector HCcassREAL cut with BstEII and the blunt end cutter *Hin*cII. The resulting plasmids are intermediates to the final expression constructs and are named HCcassHVESP20 and HCcassHVQSP20, respectively. This subcloning also leads to a change of the leader sequence which is associated to both of the $V_H$ regions in the original vectors. Whereas the old leader sequence's amino acid sequence is MDWTWRVFCLLAVVAPGAHS (SEQ ID NO: 47), it has been replaced during the subcloning with MAWVWTLPFLMAAAQSVQA (SEQ ID NO: 48).

The intermediates HCcassVHESP20 or HCcassVHQSP20 are digested with ScaI and subsequenctly, the plasmids are digested with *Bam*HI and *Eco*RI. The 2.9 kb fragments containing the Ig heavy chain promoter and the $V_H$ region are purified and ligated with the 8.7 kb fragment of *Bam*HI- and *Eco*RI-digested of a heavy chain expression construct. The resulting plasmids are named HCVHESp20 (SEQ ID NO: 40; Figure 10) and HCVHQSp20 (SEQ ID NO: 41; 11), respectively; and are used for the expression in Sp2/0 cells of the humanised antibody VHE/ humV1. VHQ/humV1.

*Human gamma-1 heavy chain expression vector for VHEN73D*

Expression vectors HCVHESp20 (SEQ ID NO: 40; Figure 10) and HCVHQSp20 (SEQ ID NO: 41; Figure 11), are used as templates for site-directed mutagenesis to achieve a N73D mutation, (exchange of an asparagine to aspartate in amino acid position 73 of the huminised antibody heavy chain). This mutation eliminate a putative N-glycosylation site. Mutagenesis is performed with the QuikChange® Multi-Site Directed Mutagenesis kit (Stratagene) and the primer aCD45H-N73D (5'-phospho GCCACACTAACTGCAGACAAATCCATCAGCACAGC - 3'; SEQ ID NO: 49) according to the kit manual. The sequence of the resulting constructs HCVHEN73DSp20 and HCVHQN73DSp20 is confirmed and is disclosed as SEQ NO: 37 and SEQ NO: 38, and Figures 8 and 9, respectively. HCVHEN73DSp20 (SEQ NO: 37) together with the light chain expression construct LCVL1 SP20 (SEQ NO: 36) are used for the expression in Sp2/0 cells of the humanised antibody VHE-N73D/humV1.

Generation of stable Sp2/0 myeloma transfectants secreting humanised anti-CD45RO/RB antibody VHE-N73D/humV1

The mouse myeloma cell line Sp2/0 Ag14.10 is electroporated with vectors encoding heavy (HCVHEN73DSp20; SEQ ID NO: 37) and light (LCVL1SP20; SEQ ID NO: 36) chain of the humanised antibody VHEN73D/humV1. For transfection, cells in exponential growth phase with a viability higher than 95% are used. Cells are washed twice with cold TF-buffer (272 mM sucrose, 1 mM $MgCl_2$,7 mM phosphate buffer pH 7.4) and cell concentration is adjusted to 2 x $10^7$ cells/ml in TF-buffer. 0.8 ml cell suspension is mixed with 15 $\mu$g each of the heavy and light chain plasmid construct and placed on ice for 10 minutes. Five transfections are done by electroporation using the Biorad Gene Pulser (280V and 25 $\mu$F). After electroporation cells are placed on ice for 15 minutes followed by transfer into 50 ml cold culture medium (RPMI-based medium without FCS) and incubated for 2 days at 37° C and 5% $CO_2$.

For selection of transfectants, cells were cultivated in the presence of 1.1 mg/ml G418 (Geneticin, Gibco lot 3069464) for approximately 2 to 3 weeks. The dhfr (dihydrofolate reductase) amplification marker located on the light chain construct allows amplification of the dhfr gene as well as of the transgene by the folic acid analogue methotrexate (MTX). For gene amplification, G418 resistant cells are cultivated in the presence of 200 nM MTX for a period of 2-3 weeks followed by a further increase in the MTX concentration at 1 $\mu$M resulting in an amplified heterogeneous cell pool. The antibody concentration is determined by analytical ProteinA HPLC. The best producing pools are selected for cloning by limiting dilution, using a seeding density of 0.3 cell per well to isolate high producer clones.

**Example 5: Determination of recombinant human IgG expression by ELISA**

Characterization of VHE/humV1, VHE/humV2, VHQ/humV1 and VHQ/humV2

To determine IgG concentrations of recombinant human antibody expressed in the culture supernatants, a sandwich ELISA protocol has been developed and optimized using human IgG as standard. Flat bottom 96-well microtiter plates (Cat. N° 4-39454, Nunc Immunoplate Maxisorp) are coated overnight at 4°C with 100 $\mu$l of goat anti-human IgG (whole molecule, Cat. N° I1011, SIGMA) at the final concentration of 0.5 $\mu$g/ml in PBS. Wells are then washed 3 times with washing buffer (PBS containing 0.05% Tween 20) and blocked for 1.5 hours at 37°C with blocking buffer (0.5% BSA in PBS). After 3 washing cycles, the antibody samples and the standard human IgG (Cat.No. 14506, SIGMA) are prepared by serial 1.5-fold dilution in blocking buffer. 100 $\mu$l of diluted samples or standard are transfered in duplicate to the coated plate and incubated for 1 hour at room temperature. After incubation, the plates are washed 3 times with washing buffer and subsequently incubated for 1 hour with 100 $\mu$l of horseradish peroxidase-conjugated goat anti-human IgG kappa-light chain (Cat. N° A-7164, SIGMA) diluted at 1/4000 in blocking buffer. Control wells received 100 $\mu$l of blocking buffer or concentrated normal culture medium. After washing, the colorimetric quantification of bound peroxidase in the sample and standard wells is performed, using a TMB Peroxidase EIA Substrate Kit (Cat. N° 172-1067, Bio-Rad) according to the manufacturer's instructions. The peroxidase mixture is added at 100 $\mu$l per well and incubated for 30 min at room temperature in the dark. The colorimetric reaction is stopped by addition of 100 $\mu$l of 1 M sulfuric acid and the absorbance in each well is read at 450 nm, using an ELISA plate reader (Model 3350-UV, BioRad).

With a correlation coefficient of 0.998 for the IgG standard curve, the following concentrations are determined for the four different culture concentrates (ca. 250-300 fold concentrated) obtained from transfected COS cells:

VHE/humV1 supernatant = 8.26 μg/ml
VHE/humV2 supernatant = 6.27 μg/ml
VHQ/humV1 supernatant = 5.3 μg/ml
VHQ/humV2 supernatant = 5.56 μg/ml

*Size Exclusion Chromatography analysis (SEC)*

The affinity-purified antibodies VHE/humV1, VHE/humV2, VHQ/humV1 and VHQ/humV2 are analyzed by Size Exclusion Chromatography (SEC) on a TSKgel Super SW3000SWXL in order to determine the protein content, the percentage of small aggregates (oligomeric antibodies) as well as possible by- and degradation products. The chromatograms show for VHE/humV1, VHE/humV2, VHQ/humV1 and VHQ/humV2 that the main peak is split and that the separation is more pronounced for pools with heavy chain E (Figure 12). The results suggest that there are at least two molecules with a retention time typical for an IgG antibody present in each sample.

*SDS-PAGE under reducing conditions*

Analysis of the humanized CD45RO/RB binding molecules VHE/humV1, VHE/humV2, VHQ/humV1 and VHQ/humV2 by SDS-PAGE under reducing conditions (gradient gel, 4 to 20% Tris-Glycine gel, Novex) shows the presence of an unexpected additional band, migrating slightly above the band corresponding to the heavy chain band in each sample.
The difference in mass is estimated to be in the range of 2 to 4 kDa. Western Blot analysis reveals that the upper band is recognized by anti-human (H + L) antibodies suggesting that the additional band is a heavy chain variant.

*Cation Exchange Chromatography (CEX)*

The charge heterogeneity is evaluated by cation exchange chromatography (CEX) using a PolyCatA column (PolyLC Inc). While the chimeric mAb is essentially free of charge heterogeneity other than the C-terminal lysine variants, all humanized CD45RO/RB binding molecules, i.e. VHE/humV1, VHE/humV2, VHQ/humV1 and VHQ/humV2, are found to be very heterogeneous in charge. A common reason for charge heterogeneity in antibodies is the presence/absence of Lys at the C-terminal end of the antibody heavy chain resulting in three peaks in CEX. The high number of peaks visible on each chromatogram (>10; Figure 13) indicates that at least one additional modification is present in all four CD45RO/RB binding molecules.

*SDS-PAGE*

In order to evaluate the molecular differences between the two antibody species found in the protein pools of VHE/humV1, VHE/humV2, VHQ/humV1 and VHQ/humV2, VHE/humV2 is chosen to be further analysed. The two SEC peaks of VHE/humV2 are collected in a semi-preparative mode and re-analyzed by SEC to confirm their purity. The fractions collected by SEC (Fraction 1 and Fraction 2) are then analyzed by SDS-PAGE under reducing conditions. The proportion of the unexpected additional band at about 49 kDa is different between the two fractions. Fraction 2 is almost free of this additional higher band.

*ESI- Q-TOF Mass Spectrometry*

SEC fractions F1, F2 and the protein pool of VHE/humV2 are then further analyzed by ESI-Q-TOF mass spectrometry. In the spectrograms of the protein pool and Fraction 2, the same groups of signals are observed. The first group of signals at about 148'000 Da, is found in the protein pool and the Fraction 2, is not detected in Fraction 1. In Fraction 1, a second group of signals (at about 150'300 Da) is detected in addition to another group of signals at about 152'500 Da. The second group of signals at about 150'320 Da could not be correlated to any usually observed antibody forms. These findings suggests that the two peaks in SEC and the two upper bands in reduced SDS-PAGE correspond each to variants of an expected protein.

*Reverse Phase Chromatography*

To obtain a less complex pattern in mass spectrometry, the antibody chains are further separately analyzed. The fractions are reduced and alkylated. In order to confirm completeness of the reactions the reduced and alkylated samples are analyzed by reverse phased (RP) chromatography (SORBAX, Poroshell 300SB-C8). After reduction and alkylation, the peak shape is almost the same as after reduction only. A shift in retention time is observed. Similar patterns are obtained for the SEC Fraction 1 and the Fraction 2 reduced and alkylated samples. The VHE/humV2 protein pool sample, SEC Fraction 1 and SEC Fraction 2 (reduced & alkylated) are analyzed with ESI- Q-TOF mass spectrometry. Several mass peaks could be assigned to expected antibody forms. The same major peaks are found in all the three samples (protein pool, F1 and F2) but their intensities in Fraction 2 are very low (Figure 14).

*Carbohydrates analysis of heavy chain variants*

The reduced and alkylated antibody is injected on the reverse phase (RP) chromatography column and the two peaks corresponding the heavy chain variants are collected. The oligosaccharide profiles of these RP-fractionated antibodies are then determined. Among the anticipated G0 and G1 oligosaccharides residues found in all samples, several other peaks are detected in the chromatogram of the Fraction 1 (traces of those peaks are also detectable in chromatograms of Fraction 2 and protein pool).

Taken together, the results suggest that the larger antibody species contain additional glycosylation, not typically found in SP2/0 expressed monoclonal antibodies, accounting for the mass difference. Since the large, complex glycosylation found by carbohydrate analysis and mass spectrometry would be very unlikely for the conserved glycosylation site in the constant region of the antibody, a search for other possible sites is made in the amino acid sequence of the humanized antibodies. A potential site (N73) for N-glycosylation (N-X-S) is identified in the variable domain of the two heavy chain variants.

*Preparative fractionation by CEX*

Further analyses is performed using material purified from pool VHE/humV1. To reduce the heterogeneity of the material, the C-terminal -lysine is removed by carboxypeptidase-B treatment. The VHE/humV1 antibody is fractionated by preparative cation exchange chromatography using SP Sepharose (Pharmacia). The column is equilibrated with 25 mM sodium phosphate buffer, pH 6.0 (= buffer A) and bound protein is eluted with 250 mM NaCl in buffer A (= buffer B; gradient from 0-65% B). The purity of the fractions is analysed by using CEX. The fractions collected are re-injected on the SEC column to look for a correlation between the results obtained with the two techniques. The chromatogram indicates that the first group of peaks in CEX correlates with the pre-peak in SEC analysis of the antibody, that the second group of peaks in CEX leads together to the first peak in SEC analysis of the protein pool (or Fraction 1), and that the last peak in CEX elutes as the last peak in SEC (or Fraction 2).

In conclusion, the results described above (oligosaccharide profile for the SEC Fractions 1 and 2, CEX pattern obtained after de-carboxylation, SEC pattern obtained for the fractions collected in CEX, mass spectrometry) strongly suggest the presence of antibody species with complex oligosaccharides causing a) mass heterogeneity, leading to a double peak in SEC and b) the observed double heavy chain band in reduced SDS-PAGE charge heterogeneity, leading to the CEX pattern.

*Analytical characterization of CEX fractions*

Since the larger antibody species elute earlier on CEX, showing that they are less positively charged, the presence of sialic acid (a component of complex glycosylation and negatively charged) is measured in the different fractions obtained by preparative CEX. Sialic acid is present in all 4 samples to varying degrees. The sialic acid found is most likely the N-glycolyl-neuraminic acid form (based on the shorter retention time as compared to the N-acetyl-neuraminic acid standard). The very low presence of sialic acid in one CEX fraction in contrast to its very high abundance in another CEX fraction strongly suggest that the negatively charged sugar component contributes to the mass/charge heterogeneity. The CEX fractions are analyzed by ESI-Q-TOF mass spectrometry.

*SDS-PAGE*

The same fractions are also analyzed by SDS-PAGE under reducing conditions. It can be concluded that the upper heavy chain band corresponds to the portion of antibody with a complex oligosaccharide profile containing sialic acid. The lower heavy chain band corresponds to the expected antibody displaying a conventional oligosaccharide profile.

*Separation on Mono-S column of papaïn digested CEX fractions*

In order to confirm the usage of the potential glycosylation site in the variable domain of the heavy chain, about 1 mg of each CEX fraction is treated with papain to separate the Fab and the Fc part of the antibody. Preparative chromatography is performed on a Mono-S column. Each of the sub-fraction collected is re-injected onto the RP column to identify its content in terms of Fab and/or Fc domains. Each of the sub-fraction collected is than analyzed with ESI-Q-TOF mass spectrometry. The results obtained show that a) the conserved glycosylation site in the Fc part of the heavy chain is occupied by bi-antennary oligosaccharide forms with none (G0), one (G1) or two terminal (G2) galactose units; b) that in contrast, the irregular glycosylation site (N73) carries complex oligosaccharides containing N-glycolyl neuraminic acid.

In conclusion, the asparagine residue N73 in the variable domain of the heavy chain is found to be partially N-glycosylated. These sugar species cause mass heterogeneity as well as charge heterogeneity that are detected by SDS-PAGE, size exclusion chromatography and cation exchange chromatography.

Characterization of VHE-N73D/humV1

In order to eliminate the heterogenicity of the humanized VHE/humV1, VHE/humV2, VHQ/humV1 and VHQ/humV2 antibodies the asparagine residue at position N73 in the variable domain of the heavy chain is substituted by an aspartic acid residue (see example 4). The VHE-N73D/humV1 is then further analysed:

*Size Exclusion Chromatography analysis (SEC)*

SEC is performed as described above. A clear difference between VHE/humV1 and VHE-N73D /humV1 is observed in SEC. In contrast to the double peak obtained for VHE/humV1, only one peak is obtained for VHE-N73D /humV1 (Figure 12). Approximately 0.2% of aggregates are quantified by SEC.

*SDS-PAGE under reducing conditions*

VHE/humV2 and VHE-N73D/humV1 are further analysed by SDS-PAGE under reducing conditions as outlined above. Only one band is visible for VHE-N73D/humV1 at the expected heavy chain (HC) position (about 50 kDa). The HC-band observed for VHE-N73D/humV1 corresponds to the lower band of the doublet observed for VHE/humV2. The position of the light chain band is the same for the proteins analyzed.

*Cation Exchange Chromatography (CEX)*

Cation Exchange Chromatography (CEX) is performed as outlined above. Results obtained for CEX analysis show that the charge heterogeneity of VHE-N73D/humV1 is reduced to the C-terminal Lysine variants (three peaks) as compared to the high charge heterogeneity of VHE/humV2 (>10; Figure 13)

*MALDI TOF analyse (mass spectrometry)*

The detected mass for the heavy chain and the light chain obtained by MALDI TOF analyse (mass spectrometry) are in close agreement with the expected mass, deduced from the amino acid sequence of VHE-N73D/humV1.

*Reverse Phase chromatography (RP)*

After reduction with DDT the two humanized antibodies VHE/humV2 and VHE-N73D/humV1 are analyzed by Reverse Phase chromatography (RP). Due to the partial glycosylation on asparagine N73, two "heavy chains" are observed for VHE/humV2 (double peak at about 18.5 min) amongst the peak corresponding to the light chain (at about 17.3 min). For VHE-N73D/humV1, only one peak is observed for the heavy chain (Figure 14).

**<u>Example 6:</u> FACS competition analysis (binding affinity)**

The human T-cell line PEER is chosen as the target cell for FACS analysis because it expressed the CD45 antigen on its cell surface. To analyze the binding affinity of humanised antibody supernatants, competition experiments using FITC-labeled chimeric antibody as a reference are performed and compared with the inhibition of purified mouse antibody and of chimeric antibody. PEER cell cultures are centrifuged for 10 seconds at 3000 rpm and the medium is removed. Cells are resuspended in FACS buffer (PBS containing 1% FBS and 0.1 % sodium azide) and seeded into 96-well round-bottom microtitter plate at a cell density of $1x10^5$ cells per well. The plate is centrifuged and the supernatant is discarded. For blocking studies, 25 μl of concentrated untransfected medium or isotype matched control antibody (negative controls), unlabeled mouse antibody or chimeric antibody (positive controls) as well as concentrated supernatant containing the various combinations of humanised antibody (samples), is first added in each well at the indicated concentrations in the text. After 1 hour of incubation at 4°C, PEER cells are washed with 200 μl of FACS buffer by centrifugation. Cells are subsequently incubated for 1 hour at 4°C with chimeric antibody conjugated with FITC in 25 μl of FACS buffer at the final concentration of 20 μg/ml. Cells are washed and resuspended in 300 μl of FACS buffer containing 2 μg/ml propidium iodide which allows gating of viable cells. The cell preparations are analyzed on a flow cytometer (FACSCalibur, Becton Dickinson).

FACS analyses indicate a dose-dependent blockade of fluorochrome-labeled chimeric antibody by the concentrated humanised antibody culture supernatants. No dose-dependent blockade of chimeric antibody binding is seen with the isotype matched control antibody, indicating that the blocking effect by the different humanised antibody combinations is epitope specific and that epitope specificity appears to be retained after the humanisation process.

Undiluted supernatant from the above mentioned SP2/0 transfectants or chimeric antibody (positive controls) or isotype matched control antibody (negative controls) at 2 μg/ml in culture medium are incubated with $1.5x10^5$ PEER cells in 100 μl for 30 min at 4°C. Then, 100 μl PBS containing FITC-labeled chimeric antibody is added to each sample and incubation at 4°C continues for another 30 minutes. After washing, cells are resuspended in FACS-PBS containing 1 μg/ml 7-Amino-Actinomycin D and analyzed by flow cytometry using a Becton Dickinson FACSCalibur instrument and the CellQuest Pro Software. Gating was on live cells, i.e. 7-Amino-Actinomycin D - negative events.

FACS analyses show that unlabeled humanised CD45RB/RO binding molecules, e.g. VHE/humV1 and VHQ/humV1 but not the isotype matched control antibody compete with FITC-labeled chimeric antibody for binding to the human CD45-positive T cell line PEER.

*Specificity of VHE-N73D/humV1*

To evaluate whether the modification of the humanised CD45R0/RB binding molecule VHE/humV1, i.e. the exchange of the asparagine to aspartate in amino acid position 73 of the CD45RO/RB-binding molecule's heavy chain, modified the reactivity with the cognate epitope, the reactivity of VHE-N73D/humV1 with a CD45-expressing human T cell line PEER is analysed in a competition binding experiment.

PEER cells are incubated with VHE-N73D/humV1, with its chimeric predecessor or with a non-binding isotype control IgG1 antibody. Unbound antibody is washed off and the cells are subjected to an incubation with fluorochrome fluorescein isothiocyanate (FITC) labeled chimeric anti-CD45R0/RB mAb or FITC-labeled isotype IgG1 control antibody. After washing, the cells are subjected to flow cytometry analysis to quantitate the amount of FITC-labeled antibody bound to the pre-incubated PEER cell: PEER cell cultures are centrifuged for 10 min at 400 times the standard gravity force (g) and the medium is removed. Cells are resuspended in FACS buffer (PBS containing 1% v/v FBS, 0.1 w/v EDTA and 0.1% w/v sodium azide) and seeded into 96-well V-bottom microtiter plates at a cell density of $1x10^5$ cells per well. The plate is centrifuged and the supernatant is discarded. Each sample of cells is incubated for 30 minutes at 4°C in 50 μl of FACS buffer containing 20 μg/ml of either chimeric anti-CD45R0/RB mAb, VHE-N73D/humV1 or IgG1 control Ab. Next, PEER cells are washed twice with 150 μl of FACS buffer by centrifugation. Cells are subsequently incubated for 30 minutes at 4°C in 50 μl of FACS buffer containing 20 μg/ml of either FITC-conjugated chimeric mAb or FITC-conjugated 3G5 control Ab. Finally, cells are washed and resuspended in 200 μl of FACS buffer containing 7-amino actinomycin D (7-AAD) at 1 μg/ml, which allows identification of viable cells during analysis. The cell preparations are analysed on a FACSCalibur flow cytometer (Becton Dickinson).

It is observed that the unlabeled VHE-N73D/humV1 and the unlabeled chimeric anti-CD45R0/RB mAb but not the IgG1 control antibody prevent the FITC-labeled chimeric anti-CD45R0/RB mAb from binding to CD45-expressing PEER cells as determined by a decreased fluorescence signal. In conclusion, it is found that the modification of the VHE/humV1 antibody to VHE-N73D/humV1 does not change the epitope specificity of the humanised anti-CD45R0/RB binding molecule.

Affinity of VHE-N73D/humV1 for human or cynomolgus CD45

To determine the affinity of the VHE-N73D/humV1 antibody for its epitope, the reactivity of VHE-N73D/humV1 with a human CD45-expressing T cell line PEER or the cynomolgus monkey CD45-expressing T cell line HSC-F can be quantitated in a competition binding experiment, similar to the procedure described in Daley et al., 1995; J. Mol. Biol. 253:243.

Briefly, the staining intensity of PEER or HSC-F cells with FITC-labeled antibody in the presence of various concentrations of unlabeled competitor antibody is used to calculate the unlabeled antibodies' affinities. More precisely, in a first step, the concentration and fluorochrome labeling stochiometry of the FITC-labeled mouse-derived anti-human CD45RB/RO antibody A6 (mA6) is measured. Next, the concentration of the target molecule, CD45, on the surface of the PEER or HSC-F cell line is be determined with the help of the above mentioned FITC-conjugated mA6 yes (mouse-derived anti-huCD45) antibody and using fluorescent beads with known molecular labeling degree. With the concentration of cellular CD45 receptors and its FITC-conjugated ligand known, the affinity of FITC-conjugated mA6 to cellular CD45 receptors is determined by a FACS based equilibrium titration procedure. Finally, the affinity of the unlabeled antibody VHE-N73D/humV1 is determined from competition staining reactions with FITC-conjugated mA6 on PEER or HSC-F cells measuring the increase in FITC fluorescence as a function of the total concentration of VHE-N73D/humV1. If the cubic equation which represents the algebraically explicit description of binding equilibria in a mixture of two competing ligands binding to one receptor is implemented into the program Origin 7.5, the software program is used to calculate the dissociation constant (Kd) value for VHE-N73D/humV1 upon reiterative curve fitting analyses.

In one experiment the dissociation constant of VHE-N73D/humV1 for the human PEER cellular target is calculated as 2.44 $\pm$ 0.07 nM - for the cynomolgus HSC-F cell target it is calculated as 1.67 $\pm$ 0.00 nM, assuming divalent antibody binding to the target CD45RO/RB molecules.

**<u>Example 7:</u> Biological activities of CD45RB/RO binding molecules**

In this study, we have addressed whether CD45RB/RO binding chimeric antibody, when present in cultures of polyclonally activated primary human T cells (i) supports the differentiation of T cells with a characteristic Treg phenotype, (ii) prevents or enhances apoptosis following T cell activation, and (iii) affects expression of subset-specific antigens and receptors after restimulation.

**CD45RB/RO binding chimeric antibody enhances cell death in polyclonally activated T cells**

Primary T cells (mixture of CD4+ and CD8+ T subsets) were subjected to activation by anti-CD3 plus anti-CD28 mAb (200 ng/ml each) in the presence or absence (=control) of CD45RB/RO binding chimeric antibody. Excess antibodies were removed by washing on day 2. 7-amino-actinomycin D (7-AAD) as a DNA-staining dye taken up by apoptotic and necrotic cells was used to measure cell death following activation. The results show that activation of T cells in the presence of CD45RB/RO binding chimeric antibody increased the fraction of 7-AAD positive cells than two-fold on day 2 after activation. On day 7, the portion of 7-AAD positive cells was again similar in CD45RB/RO binding chimeric antibody-treated and control cultures.

**CD45RB/RO binding chimeric antibody but not control mAb treated T cells display a T regulatory cell (Treg) phenotype**

Increased expression of CD25 and the negative regulatory protein CTLA-4 (CD152) is a marker of Treg cells. Functional suppression of primary and secondary T cell responses by CD45RB/RO binding chimeric antibody may be due to the induction of Treg cells. To address this issue, T cells were activated by anti-CD3 + CD28 mAbs and cultured in the presence of CD45RB/RO binding chimeric antibody or anti-LPS control mAb. The time course of CTLA-4 and CD25 expression reveals marked differences between controls and CD45RB/RO binding chimeric antibody-treated T cells on days 1 and 3 after secondary stimulation, indicating a Treg phenotype.

**Intracellular CTLA-4 expression is sustained in the presence of CD45RB/RO binding chimeric antibody**

It has been reported that substantial amounts of CTLA-4 can also be found intracellularly. Therefore, in parallel to surface CTLA-4 staining, intracellular CTLA-4 expression was analyzed. Moderate differences between T cell cultures were seen on day 4 after stimulation. After prolonged culture, however, high levels of intracellular CTLA-4 were sustained only in CD45RB/RO binding chimeric antibody-treated but not in control T cells.

**CD45RB/RO binding chimeric antibody-treated T cells become double positive for CD4 and CD8**

Following stimulation, T cells induce and upregulate the expression of several surface receptors, such as CD25, CD152 (CTLA-4), CD154 (CD40-Ligand) and others. In contrast, the level of expression of CD4 or CD8 is thought to stay relatively constant. We reproducibly observed a strong increase of both CD4 and CD8 antigens on CD45RB/RO binding chimeric antibody-treated but not on control Ab-treated T cells after activation. The emergence of a CD4/CD8 double-positive T cell population seems to be due to the upregulation of CD4 on the CD8+ subset and conversely, CD8 on the CD4+ subset. This contrasts with a moderately low percentage of double positive T cells in control cultures.

**High IL-2 receptor a lpha-chain, but very low beta-chain expression by CD45RB/RO binding chimeric antibody-treated T cells**

Treg cells are known to be constitutively positive for CD25, the IL-2 receptor alpha-chain. The regulation of other subunits of the trimeric IL-2 receptor on Treg cells is not known. Recently we have compared the expression of the beta-chain of IL-2 receptor, e.g. CD122, on T cells activated and propagated in the presence or absence of CD45RB/RO binding chimeric antibody. The results show that CD45RB/RO binding chimeric antibody-treated T cells have about ten-fold lower CD122 expression as compared to T cells in control cultures. This difference may indicate that Treg cells require factors other than IL-2 to proliferate.

**Example 8: Sequences of the invention (CDR sequences of the invention are underlined)**

**SEQ ID NO:1**
**Part of the amino acid sequence of chimeric light chain**

DILLTQSPAILSVSPGERVSFSCRASQNIGTSIQWYQQRTNGSPRLLIRSSSESISGIPSRFSG
SGSGTDFTLSINSVESEDIADYYCQQSNTWPFTFGSGTKLEIK

**SEQ ID NO:2**
**Part of the amino acid sequence of chimeric heavy chain**

EVQLQQSGPELVKPGASVKMSCKASGYTFTNYIIHWVKQEPGQGLEWIGYFNPYNHGTKY
NEKFKGRATLTADKSSNTAYMDLSSLTSEDSAIYYCARSGPYAWFDTWGQGTTVTVSS

**SEQ ID NO:3**
**Amino acid sequence of chimeric light chain**

DILLTQSPAILSVSPGERVSFSCRASQNIGTSIQWYQQRTNGSPRLLIRSSSESISGIPSRFSG
SGSGTDFTLSINSVESEDIADYYCQQSNTWPFTFGSGTKLEIKRTVAAPSVFIFPPSDEQLKS
GTASVVCLLNNFYPREAKVQWKVDNALQSGNSQESVTEQDSKDSTYSLSSTLTLSKADYE
KHKVYACEVTHQGLSSPVTKSFNRGEC

**SEQ ID NO:4**
**Amino acid sequence of chimeric heavy chain**

EVQLQQSGPELVKPGASVKMSCKASGYTFTNYIIHWVKQEPGQGLEWIGYFNPYNHGTKY
NEKFKGRATLTADKSSNTAYMDLSSLTSEDSAIYYCARSGPYAWFDTWGQGTTVTVSSAS
TKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVFL
FPPKPKDTLMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSREEMTKNQVS
LTCLVKGFYPSDIAVEWESNGQPENNYKTTPPVLDSDGSFFLYSKLTVDKSRWQQGNVFS
CSVMHEALHNHYTQKSLSLSPGK

**SEQ ID NO:5**
**Nucleotide sequence encoding a polypeptide of SEQ ID NO:1**

GACATTCTGCTGACCCAGTCTCCAGCCATCCTGTCTGTGAGTCCAGGAGAAAGAGTCA
GTTTCTCCTGCAGGGCCAGTCAGAACATTGGCACAAGCATACAGTGGTATCAACAAAGA
ACAAATGGTTCTCCAAGGCTTCTCATAAGGTCTTCTTCTGAGTCTATCTCTGGGATCCCT
TCCAGGTTTAGTGGCAGTGGATCAGGGACAGATTTTACTCTTAGCATCAACAGTGTGGA
GTCTGAAGATATTGCAGATTATTACTGTCAACAAAGTAATACCTGGCCATTCACGTTCGG
CTCGGGGACCAAGCTTGAAATCAAA

**SEQ ID NO:6**
**Nucleotide sequence encoding a polypeptide of SEQ ID NO:2**

GAGGTGCAGCTGCAGCAGTCAGGACCTGAACTGGTAAAGCCTGGGGCTTCAGTGAAG
ATGTCCTGCAAGGCCTCTGGATACACATTCACTAATTATATTATCCACTGGGTGAAGCA
GGAGCCTGGTCAGGGCCTTGAATGGATTGGATATTTTAATCCTTACAATCATGGTACTA
AGTACAATGAGAAGTTCAAAGGCAGGGCCACACTAACTGCAGACAAATCCTCCAACACA
GCCTACATGGACCTCAGCAGCCTGACCTCTGAGGACTCTGCGATCTACTACTGTGCAA
GATCAGGACCCTATGCCTGGTTTGACACCTGGGGCCAAGGGACCACGGTCACCGTCTC
CTCA

**SEQ ID NO:7**
**Part of amino acid sequence of humanised light chain designated humV2 (humV2 =** VLm)

DILLTQSPAT LSLSPGERAT FSCRASQNIG TSIQWYQQKT NGAPRLLIRS SSESISGIPS
RFSGSGSGTD FTLTISSLEP EDFAVYYCQQ SNTWPFTFGQ GTKLEIK

**SEQ ID NO:8**
**Part of amino acid sequence of humanised light chain designated humV1 (humV1 =** VLh)

DILLTQSPAT LSLSPGERAT LSCRASQNIG TSIQWYQQKP GQAPRLLIRS SSESISGIPS
RFSGSGSGTD FTLTISSLEP EDFAVYYCQQ SNTWPFTFGQ GTKLEIK

**SEQ ID NO:9**
**Part of amino acid sequence of humanised heavy chain designated VHE**

EVQLVESGAE VKKPGASVKV SCKASGYTFT NYIIHWVKQE PGQGLEWIGY
FNPYNHGTKY NEKFKGRATL TANKSISTAY MELSSLRSED TAVYYCARSG
PYAWFDTWGQ GTTVTVSS

**SEQ ID NO:10**
**Part of amino acid sequence of humanised heavy chain designated VHQ**

QVQLVESGAE VKKPGASVKV SCKASGYTFT NYIIHWVKQE PGQGLEWIGY FNPYNHGTKY NEKFKGRATL TANKSISTAY MELSSLRSED TAVYYCARSG PYAWFDTWGQ GTTVTVSS

**SEQ ID NO:11**
**Nucleotide sequence encoding amino acid sequence SEQ ID NO:9**

GAGGTGCAGCTGGTGGAGTCAGGAGCCGAAGTGAAAAAGCCTGGGGCTTCAGTGAAG
GTGTCCTGCAAGGCCTCTGGATACACATTCACTAATTATATTATCCACTGGGTGAAGCA
GGAGCCTGGTCAGGGCCTTGAATGGATTGGATATTTTAATCCTTACAATCATGGTACTA
AGTACAATGAGAAGTTCAAAGGCAGGGCCACACTAACTGCAAACAAATCCATCAGCACA
GCCTACATGGAGCTCAGCAGCCTGCGCTCTGAGGACACTGCGGTCTACTACTGTGCAA
GATCAGGACCCTATGCCTGGTTTGACACCTGGGGCCAAGGGACCACGGTCACCGTCTC
CTCA

**SEQ ID NO:12**
**Nucleotide sequence encoding amino acid sequence SEQ ID NO:10**

CAGGTGCAGCTGGTGGAGTCAGGAGCCGAAGTGAAAAAGCCTGGGGCTTCAGTGAAG
GTGTCCTGCAAGGCCTCTGGATACACATTCACTAATTATATTATCCACTGGGTGAAGCA
GGAGCCTGGTCAGGGCCTTGAATGGATTGGATATTTTAATCCTTACAATCATGGTACTA
AGTACAATGAGAAGTTCAAAGGCAGGGCCACACTAACTGCAAACAAATCCATCAGCACA
GCCTACATGGAGCTCAGCAGCCTGCGCTCTGAGGACACTGCGGTCTACTACTGTGCAA
GATCAGGACCCTATGCCTGGTTTGACACCTGGGGCCAAGGGACCACGGTCACCGTCTC
CTCA

**SEQ ID NO:13**
**Nucleotide sequence encoding amino acid sequence SEQ ID NO:7**

GACATTCTGCTGACCCAGTCTCCAGCCACCCTGTCTCTGAGTCCAGGAGAAAGAGCCA
CTTTCTCCTGCAGGGCCAGTCAGAACATTGGCACAAGCATACAGTGGTATCAACAAAAA
ACAAATGGTGCTCCAAGGCTTCTCATAAGGTCTTCTTCTGAGTCTATCTCTGGGATCCC
TTCCAGGTTTAGTGGCAGTGGATCAGGGACAGATTTTACTCTTACCATCAGCAGTCTGG
AGCCTGAAGATTTTGCAGTGTATTACTGTCAACAAAGTAATACCTGGCCATTCACGTTC
GGCCAGGGGACCAAGCTGGAGATCAAA

**SEQ ID NO:14**
**Nucleotide sequence encoding amino acid sequence SEQ ID NO:8**

GACATTCTGCTGACCCAGTCTCCAGCCACCCTGTCTCTGAGTCCAGGAGAAAGAGCCA
CTCTCTCCTGCAGGGCCAGTCAGAACATTGGCACAAGCATACAGTGGTATCAACAAAAA
CCAGGTCAGGCTCCAAGGCTTCTCATAAGGTCTTCTTCTGAGTCTATCTCTGGGATCCC

TTCCAGGTTTAGTGGCAGTGGATCAGGGACAGATTTTACTCTTACCATCAGCAGTCTGG
AGCCTGAAGATTTTGCAGTGTATTACTGTCAACAAAGTAATACCTGGCCATTCACGTTC
GGCCAGGGGACCAAGCTGGAGATCAAA

**SEQ ID NO:15**

**Nucleotide sequence of the expression vector HCMV-G1 HuA6-VHQ (Complete DNA Sequence of a humanised heavy chain expression vector comprising SEQ ID NO:12 (VHQ) from 3921-4274)**

1 AGCTTTTTGC AAAAGCCTAG GCCTCCAAAA AAGCCTCCTC ACTACTTCTG
51 GAATAGCTCA GAGGCCGAGG CGGCCTCGGC CTCTGCATAA ATAAAAAAAA
101 TTAGTCAGCC ATGGGGCGGA GAATGGGCGG AACTGGGCGG AGTTAGGGGC
151 GGGATGGGCG GAGTTAGGGG CGGGACTATG GTTGCTGACT AATTGAGATG
201 CATGCTTTGC ATACTTCTGC CTGCTGGGGA GCCTGGTTGC TGACTAATTG
251 AGATGCATGC TTTGCATACT TCTGCCTGCT GGGGAGCCTG GGGACTTTCC
301 ACACCCTAAC TGACACACAT TCCACAGCTG CCTCGCGCGT TTCGGTGATG
351 ACGGTGAAAA CCTCTGACAC ATGCAGCTCC CGGAGACGGT CACAGCTTGT
401 CTGTAAGCGG ATGCCGGGAG CAGACAAGCC CGTCAGGGCG CGTCAGCGGG
451 TGTTGGCGGG TGTCGGGGCG CAGCCATGAC CCAGTCACGT AGCGATAGCG
501 GAGTGTATAC TGGCTTAACT ATGCGGCATC AGAGCAGATT GTACTGAGAG
551 TGCACCATAT GCGGTGTGAA ATACCGCACA GATGCGTAAG GAGAAAATAC
601 CGCATCAGGC GCTCTTCCGC TTCCTCGCTC ACTGACTCGC TGCGCTCGGT
651 CGTTCGGCTG CGGCGAGCGG TATCAGCTCA CTCAAAGGCG GTAATACGGT
701 TATCCACAGA ATCAGGGGAT AACGCAGGAA AGAACATGTG AGCAAAAGGC
751 CAGCAAAAGG CCAGGAACCG TAAAAAGGCC GCGTTGCTGG CGTTTTTCCA
801 TAGGCTCCGC CCCCCTGACG AGCATCACAA AAATCGACGC TCAAGTCAGA
851 GGTGGCGAAA CCCGACAGGA CTATAAAGAT ACCAGGCGTT TCCCCCTGGA
901 AGCTCCCTCG TGCGCTCTCC TGTTCCGACC CTGCCGCTTA CCGGATACCT
951 GTCCGCCTTT CTCCCTTCGG GAAGCGTGGC GCTTTCTCAT AGCTCACGCT
1001 GTAGGTATCT CAGTTCGGTG TAGGTCGTTC GCTCCAAGCT GGGCTGTGTG
1051 CACGAACCCC CCGTTCAGCC CGACCGCTGC GCCTTATCCG GTAACTATCG
1101 TCTTGAGTCC AACCCGGTAA GACACGACTT ATCGCCACTG GCAGCAGCCA
1151 CTGGTAACAG GATTAGCAGA GCGAGGTATG TAGGCGGTGC TACAGAGTTC
1201 TTGAAGTGGT GGCCTAACTA CGGCTACACT AGAAGGACAG TATTTGGTAT
1251 CTGCGCTCTG CTGAAGCCAG TTACCTTCGG AAAAAGAGTT GGTAGCTCTT
1301 GATCCGGCAA ACAAACCACC GCTGGTAGCG GTGGTTTTTT TGTTTGCAAG

```
1351  CAGCAGATTA CGCGCAGAAA AAAAGGATCT CAAGAAGATC CTTTGATCTT
1401  TTCTACGGGG TCTGACGCTC AGTGGAACGA AAACTCACGT TAAGGGATTT
1451  TGGTCATGAG ATTATCAAAA AGGATCTTCA CCTAGATCCT TTTAAATTAA
1501  AAATGAAGTT TTAAATCAAT CTAAAGTATA TATGAGTAAA CTTGGTCTGA
1551  CAGTTACCAA TGCTTAATCA GTGAGGCACC TATCTCAGCG ATCTGTCTAT
1601  TTCGTTCATC CATAGTTGCC TGACTCCCCG TCGTGTAGAT AACTACGATA
1651  CGGGAGGGCT TACCATCTGG CCCCAGTGCT GCAATGATAC CGCGAGACCC
1701  ACGCTCACCG GCTCCAGATT TATCAGCAAT AAACCAGCCA GCCGGAAGGG
1751  CCGAGCGCAG AAGTGGTCCT GCAACTTTAT CCGCCTCCAT CCAGTCTATT
1801  AATTGTTGCC GGGAAGCTAG AGTAAGTAGT TCGCCAGTTA ATAGTTTGCG
1851  CAACGTTGTT GCCATTGCTG CAGGCATCGT GGTGTCACGC TCGTCGTTTG
1901  GTATGGCTTC ATTCAGCTCC GGTTCCCAAC GATCAAGGCG AGTTACATGA
1951  TCCCCCATGT TGTGCAAAAA AGCGGTTAGC TCCTTCGGTC CTCCGATCGT
2001  TGTCAGAAGT AAGTTGGCCG CAGTGTTATC ACTCATGGTT ATGGCAGCAC
2051  TGCATAATTC TCTTACTGTC ATGCCATCCG TAAGATGCTT TTCTGTGACT
2101  GGTGAGTACT CAACCAAGTC ATTCTGAGAA TAGTGTATGC GGCGACCGAG
2151  TTGCTCTTGC CCGGCGTCAA CACGGGATAA TACCGCGCCA CATAGCAGAA
2201  CTTTAAAAGT GCTCATCATT GGAAAACGTT CTTCGGGGCG AAAACTCTCA
2251  AGGATCTTAC CGCTGTTGAG ATCCAGTTCG ATGTAACCCA CTCGTGCACC
2301  CAACTGATCT TCAGCATCTT TTACTTTCAC CAGCGTTTCT GGGTGAGCAA
2351  AAACAGGAAG GCAAAATGCC GCAAAAAAGG GAATAAGGGC GACACGGAAA
2401  TGTTGAATAC TCATACTCTT CCTTTTTCAA TATTATTGAA GCATTTATCA
2451  GGGTTATTGT CTCATGAGCG GATACATATT TGAATGTATT TAGAAAAATA
2501  AACAAATAGG GGTTCCGCGC ACATTTCCCC GAAAAGTGCC ACCTGACGTC
2551  TAAGAAACCA TTATTATCAT GACATTAACC TATAAAAATA GGCGTATCAC
2601  GAGGCCCTTT CGTCTTCAAG AATTCAGCTT GGCTGCAGTG AATAATAAAA
2651  TGTGTGTTTG TCCGAAATAC GCGTTTTGAG ATTTCTGTCG CCGACTAAAT
2701  TCATGTCGCG CGATAGTGGT GTTTATCGCC GATAGAGATG GCGATATTGG
2751  AAAAATCGAT ATTTGAAAAT ATGGCATATT GAAAATGTCG CCGATGTGAG
2801  TTTCTGTGTA ACTGATATCG CCATTTTTCC AAAAGTGATT TTTGGGCATA
2851  CGCGATATCT GGCGATAGCG CTTATATCGT TTACGGGGGA TGGCGATAGA
2901  CGACTTTGGT GACTTGGGCG ATTCTGTGTG TCGCAAATAT CGCAGTTTCG
2951  ATATAGGTGA CAGACGATAT GAGGCTATAT CGCCGATAGA GGCGACATCA
3001  AGCTGGCACA TGGCCAATGC ATATCGATCT ATACATTGAA TCAATATTGG
3051  CCATTAGCCA TATTATTCAT TGGTTATATA GCATAAATCA ATATTGGCTA
3101  TTGGCCATTG CATACGTTGT ATCCATATCA TAATATGTAC ATTTATATTG
3151  GCTCATGTCC AACATTACCG CCATGTTGAC ATTGATTATT GACTAGTTAT
```

3201 TAATAGTAAT CAATTACGGG GTCATTAGTT CATAGCCCAT ATATGGAGTT
3251 CCGCGTTACA TAACTTACGG TAAATGGCCC GCCTGGCTGA CCGCCCAACG
3301 ACCCCCGCCC ATTGACGTCA ATAATGACGT ATGTTCCCAT AGTAACGCCA
3351 ATAGGGACTT TCCATTGACG TCAATGGGTG GAGTATTTAC GGTAAACTGC
3401 CCACTTGGCA GTACATCAAG TGTATCATAT GCCAAGTACG CCCCCTATTG
3451 ACGTCAATGA CGGTAAATGG CCCGCCTGGC ATTATGCCCA GTACATGACC
3501 TTATGGGACT TTCCTACTTG GCAGTACATC TACGTATTAG TCATCGCTAT
3551 TACCATGGTG ATGCGGTTTT GGCAGTACAT CAATGGGCGT GGATAGCGGT
3601 TTGACTCACG GGGATTTCCA AGTCTCCACC CCATTGACGT CAATGGGAGT
3651 TTGTTTTGGC ACCAAAATCA ACGGGACTTT CCAAAATGTC GTAACAACTC
3701 CGCCCCATTG ACGCAAATGG GCGGTAGGCG TGTACGGTGG GAGGTCTATA
3751 TAAGCAGAGC TCGTTTAGTG AACCGTCAGA TCGCCTGGAG ACGCCATCCA
3801 CGCTGTTTTG ACCTCCATAG AAGACACCGG GACCGATCCA GCCTCCGCAA
3851 GCTTGCCGCC ACCATGGACT GGACCTGGAG GGTGTTCTGC CTGCTGGCCG
3901 TGGCCCCCGG CGCCCACAGC CAGGTGCAGC TGGTGGAGTC AGGAGCCGAA
3951 GTGAAAAAGC CTGGGGCTTC AGTGAAGGTG TCCTGCAAGG CCTCTGGATA
4001 CACATTCACT AATTATATTA TCCACTGGGT GAAGCAGGAG CCTGGTCAGG
4051 GCCTTGAATG GATTGGATAT TTTAATCCTT ACAATCATGG TACTAAGTAC
4101 AATGAGAAGT TCAAAGGCAG GGCCACACTA ACTGCAAACA AATCCATCAG
4151 CACAGCCTAC ATGGAGCTCA GCAGCCTGCG CTCTGAGGAC ACTGCGGTCT
4201 ACTACTGTGC AAGATCAGGA CCCTATGCCT GGTTTGACAC CTGGGGCCAA
4251 GGGACCACGG TCACCGTCTC CTCAGGTGAG TTCTAGAAGG ATCCCAAGCT
4301 AGCTTTCTGG GGCAGGCCAG GCCTGACCTT GGCTTTGGGG CAGGGAGGGG
4351 GCTAAGGTGA GGCAGGTGGC GCCAGCCAGG TGCACACCCA ATGCCCATGA
4401 GCCCAGACAC TGGACGCTGA ACCTCGCGGA CAGTTAAGAA CCCAGGGGCC
4451 TCTGCGCCCT GGGCCCAGCT CTGTCCCACA CCGCGGTCAC ATGGCACCAC
4501 CTCTCTTGCA GCCTCCACCA AGGGCCCATC GGTCTTCCCC CTGGCACCCT
4551 CCTCCAAGAG CACCTCTGGG GGCACAGCGG CCCTGGGCTG CCTGGTCAAG
4601 GACTACTTCC CCGAACCGGT GACGGTGTCG TGGAACTCAG GCGCCCTGAC
4651 CAGCGGCGTG CACACCTTCC CGGCTGTCCT ACAGTCCTCA GGACTCTACT
4701 CCCTCAGCAG CGTGGTGACC GTGCCCTCCA GCAGCTTGGG CACCCAGACC
4751 TACATCTGCA ACGTGAATCA CAAGCCCAGC AACACCAAGG TGGACAAGAA
4801 AGTTGGTGAG AGGCCAGCAC AGGGAGGGAG GGTGTCTGCT GGAAGCCAGG
4851 CTCAGCGCTC CTGCCTGGAC GCATCCCGGC TATGCAGCCC CAGTCCAGGG
4901 CAGCAAGGCA GGCCCCGTCT GCCTCTTCAC CCGGAGGCCT CTGCCCGCCC
4951 CACTCATGCT CAGGGAGAGG GTCTTCTGGC TTTTTCCCCA GGCTCTGGGC
5001 AGGCACAGGC TAGGTGCCCC TAACCCAGGC CCTGCACACA AAGGGGCAGG

5051 TGCTGGGCTC AGACCTGCCA AGAGCCATAT CCGGGAGGAC CCTGCCCCTG
5101 ACCTAAGCCC ACCCCAAAGG CCAAACTCTC CACTCCCTCA GCTCGGACAC
5151 CTTCTCTCCT CCCAGATTCC AGTAACTCCC AATCTTCTCT CTGCAGAGCC
5201 CAAATCTTGT GACAAAACTC ACACATGCCC ACCGTGCCCA GGTAAGCCAG
5251 CCCAGGCCTC GCCCTCCAGC TCAAGGCGGG ACAGGTGCCC TAGAGTAGCC
5301 TGCATCCAGG GACAGGCCCC AGCCGGGTGC TGACACGTCC ACCTCCATCT
5351 CTTCCTCAGC ACCTGAACTC CTGGGGGGAC CGTCAGTCTT CCTCTTCCCC
5401 CCAAAACCCA AGGACACCCT CATGATCTCC CGGACCCCTG AGGTCACATG
5451 CGTGGTGGTG GACGTGAGCC ACGAAGACCC TGAGGTCAAG TTCAACTGGT
5501 ACGTGGACGG CGTGGAGGTG CATAATGCCA AGACAAAGCC GCGGGAGGAG
5551 CAGTACAACA GCACGTACCG TGTGGTCAGC GTCCTCACCG TCCTGCACCA
5601 GGACTGGCTG AATGGCAAGG AGTACAAGTG CAAGGTCTCC AACAAAGCCC
5651 TCCCAGCCCC CATCGAGAAA ACCATCTCCA AAGCCAAAGG TGGGACCCGT
5701 GGGGTGCGAG GGCCACATGG ACAGAGGCCG GCTCGGCCCA CCCTCTGCCC
5751 TGAGAGTGAC CGCTGTACCA ACCTCTGTCC CTACAGGGCA GCCCCGAGAA
5801 CCACAGGTGT ACACCCTGCC CCCATCCCGG GATGAGCTGA CCAAGAACCA
5851 GGTCAGCCTG ACCTGCCTGG TCAAAGGCTT CTATCCCAGC GACATCGCCG
5901 TGGAGTGGGA GAGCAATGGG CAGCCGGAGA ACAACTACAA GACCACGCCT
5951 CCCGTGCTGG ACTCCGACGG CTCCTTCTTC CTCTACAGCA AGCTCACCGT
6001 GGACAAGAGC AGGTGGCAGC AGGGGAACGT CTTCTCATGC TCCGTGATGC
6051 ATGAGGCTCT GCACAACCAC TACACGCAGA AGAGCCTCTC CCTGTCTCCG
6101 GGTAAATGAG TGCGACGGCC GGCAAGCCCC CGCTCCCCGG GCTCTCGCGG
6151 TCGCACGAGG ATGCTTGGCA CGTACCCCCT GTACATACTT CCCGGGCGCC
6201 CAGCATGGAA ATAAAGCACC CAGCGCTGCC CTGGGCCCCT GCGAGACTGT
6251 GATGGTTCTT TCCACGGGTC AGGCCGAGTC TGAGGCCTGA GTGGCATGAG
6301 ATCTGATATC ATCGATGAAT TCGAGCTCGG TACCCGGGGA TCGATCCAGA
6351 CATGATAAGA TACATTGATG AGTTTGGACA AACCACAACT AGAATGCAGT
6401 GAAAAAAATG CTTTATTTGT GAAATTTGTG ATGCTATTGC TTTATTTGTA
6451 ACCATTATAA GCTGCAATAA ACAAGTTAAC AACAACAATT GCATTCATTT
6501 TATGTTTCAG GTTCAGGGGG AGGTGTGGGA GGTTTTTTAA AGCAAGTAAA
6551 ACCTCTACAA ATGTGGTATG GCTGATTATG ATCTCTAGTC AAGGCACTAT
6601 ACATCAAATA TTCCTTATTA ACCCCTTTAC AAATTAAAAA GCTAAAGGTA
6651 CACAATTTTT GAGCATAGTT ATTAATAGCA GACACTCTAT GCCTGTGTGG
6701 AGTAAGAAAA AACAGTATGT TATGATTATA ACTGTTATGC CTACTTATAA
6751 AGGTTACAGA ATATTTTTCC ATAATTTTCT TGTATAGCAG TGCAGCTTTT
6801 TCCTTTGTGG TGTAAATAGC AAAGCAAGCA AGAGTTCTAT TACTAAACAC
6851 AGCATGACTC AAAAAACTTA GCAATTCTGA AGGAAAGTCC TTGGGGTCTT

6901 CTACCTTTCT CTTCTTTTTT GGAGGAGTAG AATGTTGAGA GTCAGCAGTA
6951 GCCTCATCAT CACTAGATGG CATTTCTTCT GAGCAAAACA GGTTTTCCTC
7001 ATTAAAGGCA TTCCACCACT GCTCCCATTC ATCAGTTCCA TAGGTTGGAA
7051 TCTAAAATAC ACAAACAATT AGAATCAGTA GTTTAACACA TTATACACTT
7101 AAAAATTTTA TATTTACCTT AGAGCTTTAA ATCTCTGTAG GTAGTTTGTC
7151 CAATTATGTC ACACCACAGA AGTAAGGTTC CTTCACAAAG ATCCGGGACC
7201 AAAGCGGCCA TCGTGCCTCC CCACTCCTGC AGTTCGGGGG CATGGATGCG
7251 CGGATAGCCG CTGCTGGTTT CCTGGATGCC GACGGATTTG CACTGCCGGT
7301 AGAACTCCGC GAGGTCGTCC AGCCTCAGGC AGCAGCTGAA CCAACTCGCG
7351 AGGGGATCGA GCCCGGGGTG GGCGAAGAAC TCCAGCATGA GATCCCCGCG
7401 CTGGAGGATC ATCCAGCCGG CGTCCCGGAA AACGATTCCG AAGCCCAACC
7451 TTTCATAGAA GGCGGCGGTG GAATCGAAAT CTCGTGATGG CAGGTTGGGC
7501 GTCGCTTGGT CGGTCATTTC GAACCCCAGA GTCCCGCTCA GAAGAACTCG
7551 TCAAGAAGGC GATAGAAGGC GATGCGCTGC GAATCGGGAG CGGCGATACC
7601 GTAAAGCACG AGGAAGCGGT CAGCCCATTC GCCGCCAAGC TCTTCAGCAA
7651 TATCACGGGT AGCCAACGCT ATGTCCTGAT AGCGGTCCGC CACACCCAGC
7701 CGGCCACAGT CGATGAATCC AGAAAAGCGG CCATTTTCCA CCATGATATT
7751 CGGCAAGCAG GCATCGCCAT GGGTCACGAC GAGATCCTCG CCGTCGGGCA
7801 TGCGCGCCTT GAGCCTGGCG AACAGTTCGG CTGGCGCGAG CCCCTGATGC
7851 TCTTCGTCCA GATCATCCTG ATCGACAAGA CCGGCTTCCA TCCGAGTACG
7901 TGCTCGCTCG ATGCGATGTT TCGCTTGGTG GTCGAATGGG CAGGTAGCCG
7951 GATCAAGCGT ATGCAGCCGC CGCATTGCAT CAGCCATGAT GGATACTTTC
8001 TCGGCAGGAG CAAGGTGAGA TGACAGGAGA TCCTGCCCCG GCACTTCGCC
8051 CAATAGCAGC CAGTCCCTTC CCGCTTCAGT GACAACGTCG AGCACAGCTG
8101 CGCAAGGAAC GCCCGTCGTG GCCAGCCACG ATAGCCGCGC TGCCTCGTCC
8151 TGCAGTTCAT TCAGGGCACC GGACAGGTCG GTCTTGACAA AAAGAACCGG
8201 GCGCCCCTGC GCTGACAGCC GGAACACGGC GGCATCAGAG CAGCCGATTG
8251 TCTGTTGTGC CCAGTCATAG CCGAATAGCC TCTCCACCCA AGCGGCCGGA
8301 GAACCTGCGT GCAATCCATC TTGTTCAATC ATGCGAAACG ATCCTCATCC
8351 TGTCTCTTGA TCAGATCTTG ATCCCCTGCG CCATCAGATC CTTGGCGGCA
8401 AGAAAGCCAT CCAGTTTACT TTGCAGGGCT TCCCAACCTT ACCAGAGGGC
8451 GCCCCAGCTG GCAATTCCGG TTCGCTTGCT GTCCATAAAA CCGCCCAGTC
8501 TAGCTATCGC CATGTAAGCC CACTGCAAGC TACCTGCTTT CTCTTTGCGC
8551 TTGCGTTTTC CCTTGTCCAG ATAGCCCAGT AGCTGACATT CATCCGGGGT
8601 CAGCACCGTT TCTGCGGACT GGCTTTCTAC GTGTTCCGCT TCCTTTAGCA
8651 GCCCTTGCGC CCTGAGTGCT TGCGGCAGCG TGAAGCT

SEQ ID NO:16

**Nucleotide sequence of the expression vector HCMV-G1 HuA6-VHE (Complete DNA Sequence of a humanised heavy chain expression vector comprising SEQ ID NO: 11 (VHE) from 3921-4274)**

```
   1 AGCTTTTTGC AAAAGCCTAG GCCTCCAAAA AAGCCTCCTC ACTACTTCTG
  51 GAATAGCTCA GAGGCCGAGG CGGCCTCGGC CTCTGCATAA ATAAAAAAAA
 101 TTAGTCAGCC ATGGGGCGGA GAATGGGCGG AACTGGGCGG AGTTAGGGGC
 151 GGGATGGGCG GAGTTAGGGG CGGGACTATG GTTGCTGACT AATTGAGATG
 201 CATGCTTTGC ATACTTCTGC CTGCTGGGGA GCCTGGTTGC TGACTAATTG
 251 AGATGCATGC TTTGCATACT TCTGCCTGCT GGGGAGCCTG GGGACTTTCC
 301 ACACCCTAAC TGACACACAT TCCACAGCTG CCTCGCGCGT TTCGGTGATG
 351 ACGGTGAAAA CCTCTGACAC ATGCAGCTCC CGGAGACGGT CACAGCTTGT
 401 CTGTAAGCGG ATGCCGGGAG CAGACAAGCC CGTCAGGGCG CGTCAGCGGG
 451 TGTTGGCGGG TGTCGGGGCG CAGCCATGAC CCAGTCACGT AGCGATAGCG
 501 GAGTGTATAC TGGCTTAACT ATGCGGCATC AGAGCAGATT GTACTGAGAG
 551 TGCACCATAT GCGGTGTGAA ATACCGCACA GATGCGTAAG GAGAAAATAC
 601 CGCATCAGGC GCTCTTCCGC TTCCTCGCTC ACTGACTCGC TGCGCTCGGT
 651 CGTTCGGCTG CGGCGAGCGG TATCAGCTCA CTCAAAGGCG GTAATACGGT
 701 TATCCACAGA ATCAGGGGAT AACGCAGGAA AGAACATGTG AGCAAAAGGC
 751 CAGCAAAAGG CCAGGAACCG TAAAAAGGCC GCGTTGCTGG CGTTTTTCCA
 801 TAGGCTCCGC CCCCCTGACG AGCATCACAA AAATCGACGC TCAAGTCAGA
 851 GGTGGCGAAA CCCGACAGGA CTATAAAGAT ACCAGGCGTT TCCCCCTGGA
 901 AGCTCCCTCG TGCGCTCTCC TGTTCCGACC CTGCCGCTTA CCGGATACCT
 951 GTCCGCCTTT CTCCCTTCGG GAAGCGTGGC GCTTTCTCAT AGCTCACGCT
1001 GTAGGTATCT CAGTTCGGTG TAGGTCGTTC GCTCCAAGCT GGGCTGTGTG
1051 CACGAACCCC CCGTTCAGCC CGACCGCTGC GCCTTATCCG GTAACTATCG
1101 TCTTGAGTCC AACCCGGTAA GACACGACTT ATCGCCACTG GCAGCAGCCA
1151 CTGGTAACAG GATTAGCAGA GCGAGGTATG TAGGCGGTGC TACAGAGTTC
1201 TTGAAGTGGT GGCCTAACTA CGGCTACACT AGAAGGACAG TATTTGGTAT
1251 CTGCGCTCTG CTGAAGCCAG TTACCTTCGG AAAAAGAGTT GGTAGCTCTT
1301 GATCCGGCAA ACAAACCACC GCTGGTAGCG GTGGTTTTTT TGTTTGCAAG
1351 CAGCAGATTA CGCGCAGAAA AAAAGGATCT CAAGAAGATC CTTTGATCTT
1401 TTCTACGGGG TCTGACGCTC AGTGGAACGA AAACTCACGT TAAGGGATTT
1451 TGGTCATGAG ATTATCAAAA AGGATCTTCA CCTAGATCCT TTTAAATTAA
1501 AAATGAAGTT TTAAATCAAT CTAAAGTATA TATGAGTAAA CTTGGTCTGA
```

1551 CAGTTACCAA TGCTTAATCA GTGAGGCACC TATCTCAGCG ATCTGTCTAT
1601 TTCGTTCATC CATAGTTGCC TGACTCCCCG TCGTGTAGAT AACTACGATA
1651 CGGGAGGGCT TACCATCTGG CCCCAGTGCT GCAATGATAC CGCGAGACCC
1701 ACGCTCACCG GCTCCAGATT TATCAGCAAT AAACCAGCCA GCCGGAAGGG
1751 CCGAGCGCAG AAGTGGTCCT GCAACTTTAT CCGCCTCCAT CCAGTCTATT
1801 AATTGTTGCC GGGAAGCTAG AGTAAGTAGT TCGCCAGTTA ATAGTTTGCG
1851 CAACGTTGTT GCCATTGCTG CAGGCATCGT GGTGTCACGC TCGTCGTTTG
1901 GTATGGCTTC ATTCAGCTCC GGTTCCCAAC GATCAAGGCG AGTTACATGA
1951 TCCCCCATGT TGTGCAAAAA AGCGGTTAGC TCCTTCGGTC CTCCGATCGT
2001 TGTCAGAAGT AAGTTGGCCG CAGTGTTATC ACTCATGGTT ATGGCAGCAC
2051 TGCATAATTC TCTTACTGTC ATGCCATCCG TAAGATGCTT TTCTGTGACT
2101 GGTGAGTACT CAACCAAGTC ATTCTGAGAA TAGTGTATGC GGCGACCGAG
2151 TTGCTCTTGC CCGGCGTCAA CACGGGATAA TACCGCGCCA CATAGCAGAA
2201 CTTTAAAAGT GCTCATCATT GGAAAACGTT CTTCGGGGCG AAAACTCTCA
2251 AGGATCTTAC CGCTGTTGAG ATCCAGTTCG ATGTAACCCA CTCGTGCACC
2301 CAACTGATCT TCAGCATCTT TTACTTTCAC CAGCGTTTCT GGGTGAGCAA
2351 AAACAGGAAG GCAAAATGCC GCAAAAAAGG GAATAAGGGC GACACGGAAA
2401 TGTTGAATAC TCATACTCTT CCTTTTTCAA TATTATTGAA GCATTTATCA
2451 GGGTTATTGT CTCATGAGCG GATACATATT TGAATGTATT TAGAAAAATA
2501 AACAAATAGG GGTTCCGCGC ACATTTCCCC GAAAAGTGCC ACCTGACGTC
2551 TAAGAAACCA TTATTATCAT GACATTAACC TATAAAAATA GGCGTATCAC
2601 GAGGCCCTTT CGTCTTCAAG AATTCAGCTT GGCTGCAGTG AATAATAAAA
2651 TGTGTGTTTG TCCGAAATAC GCGTTTTGAG ATTTCTGTCG CCGACTAAAT
2701 TCATGTCGCG CGATAGTGGT GTTTATCGCC GATAGAGATG GCGATATTGG
2751 AAAAATCGAT ATTTGAAAAT ATGGCATATT GAAAATGTCG CCGATGTGAG
2801 TTTCTGTGTA ACTGATATCG CCATTTTTCC AAAAGTGATT TTTGGGCATA
2851 CGCGATATCT GGCGATAGCG CTTATATCGT TTACGGGGGA TGGCGATAGA
2901 CGACTTTGGT GACTTGGGCG ATTCTGTGTG TCGCAAATAT CGCAGTTTCG
2951 ATATAGGTGA CAGACGATAT GAGGCTATAT CGCCGATAGA GGCGACATCA
3001 AGCTGGCACA TGGCCAATGC ATATCGATCT ATACATTGAA TCAATATTGG
3051 CCATTAGCCA TATTATTCAT TGGTTATATA GCATAAATCA ATATTGGCTA
3101 TTGGCCATTG CATACGTTGT ATCCATATCA TAATATGTAC ATTTATATTG
3151 GCTCATGTCC AACATTACCG CCATGTTGAC ATTGATTATT GACTAGTTAT
3201 TAATAGTAAT CAATTACGGG GTCATTAGTT CATAGCCCAT ATATGGAGTT
3251 CCGCGTTACA TAACTTACGG TAAATGGCCC GCCTGGCTGA CCGCCCAACG
3301 ACCCCCGCCC ATTGACGTCA ATAATGACGT ATGTTCCCAT AGTAACGCCA
3351 ATAGGGACTT TCCATTGACG TCAATGGGTG GAGTATTTAC GGTAAACTGC

3401 CCACTTGGCA GTACATCAAG TGTATCATAT GCCAAGTACG CCCCCTATTG
3451 ACGTCAATGA CGGTAAATGG CCCGCCTGGC ATTATGCCCA GTACATGACC
3501 TTATGGGACT TTCCTACTTG GCAGTACATC TACGTATTAG TCATCGCTAT
3551 TACCATGGTG ATGCGGTTTT GGCAGTACAT CAATGGGCGT GGATAGCGGT
3601 TTGACTCACG GGGATTTCCA AGTCTCCACC CCATTGACGT CAATGGGAGT
3651 TTGTTTTGGC ACCAAAATCA ACGGGACTTT CCAAAATGTC GTAACAACTC
3701 CGCCCCATTG ACGCAAATGG GCGGTAGGCG TGTACGGTGG GAGGTCTATA
3751 TAAGCAGAGC TCGTTTAGTG AACCGTCAGA TCGCCTGGAG ACGCCATCCA
3801 CGCTGTTTTG ACCTCCATAG AAGACACCGG GACCGATCCA GCCTCCGCAA
3851 GCTTGCCGCC ACCATGGACT GGACCTGGAG GGTGTTCTGC CTGCTGGCCG
3901 TGGCCCCCGG CGCCCACAGC GAGGTGCAGC TGGTGGAGTC AGGAGCCGAA
3951 GTGAAAAAGC CTGGGGCTTC AGTGAAGGTG TCCTGCAAGG CCTCTGGATA
4001 CACATTCACT AATTATATTA TCCACTGGGT GAAGCAGGAG CCTGGTCAGG
4051 GCCTTGAATG GATTGGATAT TTTAATCCTT ACAATCATGG TACTAAGTAC
4101 AATGAGAAGT TCAAAGGCAG GGCCACACTA ACTGCAAACA AATCCATCAG
4151 CACAGCCTAC ATGGAGCTCA GCAGCCTGCG CTCTGAGGAC ACTGCGGTCT
4201 ACTACTGTGC AAGATCAGGA CCCTATGCCT GGTTTGACAC CTGGGGCCAA
4251 GGGACCACGG TCACCGTCTC CTCAGGTGAG TTCTAGAAGG ATCCCAAGCT
4301 AGCTTTCTGG GGCAGGCCAG GCCTGACCTT GGCTTTGGGG CAGGGAGGGG
4351 GCTAAGGTGA GGCAGGTGGC GCCAGCCAGG TGCACACCCA ATGCCCATGA
4401 GCCCAGACAC TGGACGCTGA ACCTCGCGGA CAGTTAAGAA CCCAGGGGCC
4451 TCTGCGCCCT GGGCCCAGCT CTGTCCCACA CCGCGGTCAC ATGGCACCAC
4501 CTCTCTTGCA GCCTCCACCA AGGGCCCATC GGTCTTCCCC CTGGCACCCT
4551 CCTCCAAGAG CACCTCTGGG GGCACAGCGG CCCTGGGCTG CCTGGTCAAG
4601 GACTACTTCC CCGAACCGGT GACGGTGTCG TGGAACTCAG GCGCCCTGAC
4651 CAGCGGCGTG CACACCTTCC CGGCTGTCCT ACAGTCCTCA GGACTCTACT
4701 CCCTCAGCAG CGTGGTGACC GTGCCCTCCA GCAGCTTGGG CACCCAGACC
4751 TACATCTGCA ACGTGAATCA CAAGCCCAGC AACACCAAGG TGGACAAGAA
4801 AGTTGGTGAG AGGCCAGCAC AGGGAGGGAG GGTGTCTGCT GGAAGCCAGG
4851 CTCAGCGCTC CTGCCTGGAC GCATCCCGGC TATGCAGCCC CAGTCCAGGG
4901 CAGCAAGGCA GGCCCCGTCT GCCTCTTCAC CCGGAGGCCT CTGCCCGCCC
4951 CACTCATGCT CAGGGAGAGG GTCTTCTGGC TTTTTCCCCA GGCTCTGGGC
5001 AGGCACAGGC TAGGTGCCCC TAACCCAGGC CCTGCACACA AAGGGGCAGG
5051 TGCTGGGCTC AGACCTGCCA AGAGCCATAT CCGGGAGGAC CCTGCCCCTG
5101 ACCTAAGCCC ACCCCAAAGG CCAAACTCTC CACTCCCTCA GCTCGGACAC
5151 CTTCTCTCCT CCCAGATTCC AGTAACTCCC AATCTTCTCT CTGCAGAGCC
5201 CAAATCTTGT GACAAAACTC ACACATGCCC ACCGTGCCCA GGTAAGCCAG

5251 CCCAGGCCTC GCCCTCCAGC TCAAGGCGGG ACAGGTGCCC TAGAGTAGCC
5301 TGCATCCAGG GACAGGCCCC AGCCGGGTGC TGACACGTCC ACCTCCATCT
5351 CTTCCTCAGC ACCTGAACTC CTGGGGGGAC CGTCAGTCTT CCTCTTCCCC
5401 CCAAAACCCA AGGACACCCT CATGATCTCC CGGACCCCTG AGGTCACATG
5451 CGTGGTGGTG GACGTGAGCC ACGAAGACCC TGAGGTCAAG TTCAACTGGT
5501 ACGTGGACGG CGTGGAGGTG CATAATGCCA AGACAAAGCC GCGGGAGGAG
5551 CAGTACAACA GCACGTACCG TGTGGTCAGC GTCCTCACCG TCCTGCACCA
5601 GGACTGGCTG AATGGCAAGG AGTACAAGTG CAAGGTCTCC AACAAAGCCC
5651 TCCCAGCCCC CATCGAGAAA ACCATCTCCA AAGCCAAAGG TGGGACCCGT
5701 GGGGTGCGAG GGCCACATGG ACAGAGGCCG GCTCGGCCCA CCCTCTGCCC
5751 TGAGAGTGAC CGCTGTACCA ACCTCTGTCC CTACAGGGCA GCCCCGAGAA
5801 CCACAGGTGT ACACCCTGCC CCCATCCCGG GATGAGCTGA CCAAGAACCA
5851 GGTCAGCCTG ACCTGCCTGG TCAAAGGCTT CTATCCCAGC GACATCGCCG
5901 TGGAGTGGGA GAGCAATGGG CAGCCGGAGA ACAACTACAA GACCACGCCT
5951 CCCGTGCTGG ACTCCGACGG CTCCTTCTTC CTCTACAGCA AGCTCACCGT
6001 GGACAAGAGC AGGTGGCAGC AGGGGAACGT CTTCTCATGC TCCGTGATGC
6051 ATGAGGCTCT GCACAACCAC TACACGCAGA AGAGCCTCTC CCTGTCTCCG
6101 GGTAAATGAG TGCGACGGCC GGCAAGCCCC CGCTCCCCGG GCTCTCGCGG
6151 TCGCACGAGG ATGCTTGGCA CGTACCCCCT GTACATACTT CCCGGGCGCC
6201 CAGCATGGAA ATAAAGCACC CAGCGCTGCC CTGGGCCCCT GCGAGACTGT
6251 GATGGTTCTT TCCACGGGTC AGGCCGAGTC TGAGGCCTGA GTGGCATGAG
6301 ATCTGATATC ATCGATGAAT TCGAGCTCGG TACCCGGGGA TCGATCCAGA
6351 CATGATAAGA TACATTGATG AGTTTGGACA AACCACAACT AGAATGCAGT
6401 GAAAAAAATG CTTTATTTGT GAAATTTGTG ATGCTATTGC TTTATTTGTA
6451 ACCATTATAA GCTGCAATAA ACAAGTTAAC AACAACAATT GCATTCATTT
6501 TATGTTTCAG GTTCAGGGGG AGGTGTGGGA GGTTTTTTAA AGCAAGTAAA
6551 ACCTCTACAA ATGTGGTATG GCTGATTATG ATCTCTAGTC AAGGCACTAT
6601 ACATCAAATA TTCCTTATTA ACCCCTTTAC AAATTAAAAA GCTAAAGGTA
6651 CACAATTTTT GAGCATAGTT ATTAATAGCA GACACTCTAT GCCTGTGTGG
6701 AGTAAGAAAA AACAGTATGT TATGATTATA ACTGTTATGC CTACTTATAA
6751 AGGTTACAGA ATATTTTTCC ATAATTTTCT TGTATAGCAG TGCAGCTTTT
6801 TCCTTTGTGG TGTAAATAGC AAAGCAAGCA AGAGTTCTAT TACTAAACAC
6851 AGCATGACTC AAAAAACTTA GCAATTCTGA AGGAAAGTCC TTGGGGTCTT
6901 CTACCTTTCT CTTCTTTTTT GGAGGAGTAG AATGTTGAGA GTCAGCAGTA
6951 GCCTCATCAT CACTAGATGG CATTTCTTCT GAGCAAAACA GGTTTTCCTC
7001 ATTAAAGGCA TTCCACCACT GCTCCCATTC ATCAGTTCCA TAGGTTGGAA
7051 TCTAAAATAC ACAAACAATT AGAATCAGTA GTTTAACACA TTATACACTT

```
7101 AAAAATTTTA TATTTACCTT AGAGCTTTAA ATCTCTGTAG GTAGTTTGTC
7151 CAATTATGTC ACACCACAGA AGTAAGGTTC CTTCACAAAG ATCCGGGACC
7201 AAAGCGGCCA TCGTGCCTCC CCACTCCTGC AGTTCGGGGG CATGGATGCG
7251 CGGATAGCCG CTGCTGGTTT CCTGGATGCC GACGGATTTG CACTGCCGGT
7301 AGAACTCCGC GAGGTCGTCC AGCCTCAGGC AGCAGCTGAA CCAACTCGCG
7351 AGGGGATCGA GCCCGGGGTG GGCGAAGAAC TCCAGCATGA GATCCCCGCG
7401 CTGGAGGATC ATCCAGCCGG CGTCCCGGAA AACGATTCCG AAGCCCAACC
7451 TTTCATAGAA GGCGGCGGTG GAATCGAAAT CTCGTGATGG CAGGTTGGGC
7501 GTCGCTTGGT CGGTCATTTC GAACCCCAGA GTCCCGCTCA GAAGAACTCG
7551 TCAAGAAGGC GATAGAAGGC GATGCGCTGC GAATCGGGAG CGGCGATACC
7601 GTAAAGCACG AGGAAGCGGT CAGCCCATTC GCCGCCAAGC TCTTCAGCAA
7651 TATCACGGGT AGCCAACGCT ATGTCCTGAT AGCGGTCCGC CACACCCAGC
7701 CGGCCACAGT CGATGAATCC AGAAAAGCGG CCATTTTCCA CCATGATATT
7751 CGGCAAGCAG GCATCGCCAT GGGTCACGAC GAGATCCTCG CCGTCGGGCA
7801 TGCGCGCCTT GAGCCTGGCG AACAGTTCGG CTGGCGCGAG CCCCTGATGC
7851 TCTTCGTCCA GATCATCCTG ATCGACAAGA CCGGCTTCCA TCCGAGTACG
7901 TGCTCGCTCG ATGCGATGTT TCGCTTGGTG GTCGAATGGG CAGGTAGCCG
7951 GATCAAGCGT ATGCAGCCGC CGCATTGCAT CAGCCATGAT GGATACTTTC
8001 TCGGCAGGAG CAAGGTGAGA TGACAGGAGA TCCTGCCCCG GCACTTCGCC
8051 CAATAGCAGC CAGTCCCTTC CCGCTTCAGT GACAACGTCG AGCACAGCTG
8101 CGCAAGGAAC GCCCGTCGTG GCCAGCCACG ATAGCCGCGC TGCCTCGTCC
8151 TGCAGTTCAT TCAGGGCACC GGACAGGTCG GTCTTGACAA AAAGAACCGG
8201 GCGCCCCTGC GCTGACAGCC GGAACACGGC GGCATCAGAG CAGCCGATTG
8251 TCTGTTGTGC CCAGTCATAG CCGAATAGCC TCTCCACCCA AGCGGCCGGA
8301 GAACCTGCGT GCAATCCATC TTGTTCAATC ATGCGAAACG ATCCTCATCC
8351 TGTCTCTTGA TCAGATCTTG ATCCCCTGCG CCATCAGATC CTTGGCGGCA
8401 AGAAAGCCAT CCAGTTTACT TTGCAGGGCT TCCCAACCTT ACCAGAGGGC
8451 GCCCCAGCTG GCAATTCCGG TTCGCTTGCT GTCCATAAAA CCGCCCAGTC
8501 TAGCTATCGC CATGTAAGCC CACTGCAAGC TACCTGCTTT CTCTTTGCGC
8551 TTGCGTTTTC CCTTGTCCAG ATAGCCCAGT AGCTGACATT CATCCGGGGT
8601 CAGCACCGTT TCTGCGGACT GGCTTTCTAC GTGTTCCGCT TCCTTTAGCA
8651 GCCCTTGCGC CCTGAGTGCT TGCGGCAGCG TGAAGCT
```

**SEQ ID NO:17**

**Nucleotide sequence of the expression vector HCMV-K HuAb-VL1 hum V1 (Complete DNA Sequence of a humanised light chain expression vector comprising SEQ ID NO: 14 (humV1=VLh) from 3964-4284)**

```
   1 CTAGCTTTTT GCAAAAGCCT AGGCCTCCAA AAAAGCCTCC TCACTACTTC
  51 TGGAATAGCT CAGAGGCCGA GGCGGCCTCG GCCTCTGCAT AAATAAAAAA
 101 AATTAGTCAG CCATGGGGCG GAGAATGGGC GGAACTGGGC GGAGTTAGGG
 151 GCGGGATGGG CGGAGTTAGG GGCGGGACTA TGGTTGCTGA CTAATTGAGA
 201 TGCATGCTTT GCATACTTCT GCCTGCTGGG GAGCCTGGTT GCTGACTAAT
 251 TGAGATGCAT GCTTTGCATA CTTCTGCCTG CTGGGGAGCC TGGGGACTTT
 301 CCACACCCTA ACTGACACAC ATTCCACAGC TGCCTCGCGC GTTTCGGTGA
 351 TGACGGTGAA AACCTCTGAC ACATGCAGCT CCCGGAGACG GTCACAGCTT
 401 GTCTGTAAGC GGATGCCGGG AGCAGACAAG CCCGTCAGGG CGCGTCAGCG
 451 GGTGTTGGCG GGTGTCGGGG CGCAGCCATG ACCCAGTCAC GTAGCGATAG
 501 CGGAGTGTAT ACTGGCTTAA CTATGCGGCA TCAGAGCAGA TTGTACTGAG
 551 AGTGCACCAT ATGCGGTGTG AAATACCGCA CAGATGCGTA AGGAGAAAAT
 601 ACCGCATCAG GCGCTCTTCC GCTTCCTCGC TCACTGACTC GCTGCGCTCG
 651 GTCGTTCGGC TGCGGCGAGC GGTATCAGCT CACTCAAAGG CGGTAATACG
 701 GTTATCCACA GAATCAGGGG ATAACGCAGG AAAGAACATG TGAGCAAAAG
 751 GCCAGCAAAA GGCCAGGAAC CGTAAAAAGG CCGCGTTGCT GGCGTTTTTC
 801 CATAGGCTCC GCCCCCCTGA CGAGCATCAC AAAAATCGAC GCTCAAGTCA
 851 GAGGTGGCGA AACCCGACAG GACTATAAAG ATACCAGGCG TTTCCCCCTG
 901 GAAGCTCCCT CGTGCGCTCT CCTGTTCCGA CCCTGCCGCT TACCGGATAC
 951 CTGTCCGCCT TTCTCCCTTC GGGAAGCGTG GCGCTTTCTC ATAGCTCACG
1001 CTGTAGGTAT CTCAGTTCGG TGTAGGTCGT TCGCTCCAAG CTGGGCTGTG
1051 TGCACGAACC CCCCGTTCAG CCCGACCGCT GCGCCTTATC CGGTAACTAT
1101 CGTCTTGAGT CCAACCCGGT AAGACACGAC TTATCGCCAC TGGCAGCAGC
1151 CACTGGTAAC AGGATTAGCA GAGCGAGGTA TGTAGGCGGT GCTACAGAGT
1201 TCTTGAAGTG GTGGCCTAAC TACGGCTACA CTAGAAGGAC AGTATTTGGT
1251 ATCTGCGCTC TGCTGAAGCC AGTTACCTTC GGAAAAAGAG TTGGTAGCTC
1301 TTGATCCGGC AAACAAACCA CCGCTGGTAG CGGTGGTTTT TTTGTTTGCA
1351 AGCAGCAGAT TACGCGCAGA AAAAAAGGAT CTCAAGAAGA TCCTTTGATC
1401 TTTTCTACGG GGTCTGACGC TCAGTGGAAC GAAAACTCAC GTTAAGGGAT
1451 TTTGGTCATG AGATTATCAA AAAGGATCTT CACCTAGATC CTTTTAAATT
1501 AAAAATGAAG TTTTAAATCA ATCTAAAGTA TATATGAGTA AACTTGGTCT
```

1551 GACAGTTACC AATGCTTAAT CAGTGAGGCA CCTATCTCAG CGATCTGTCT
1601 ATTTCGTTCA TCCATAGTTG CCTGACTCCC CGTCGTGTAG ATAACTACGA
1651 TACGGGAGGG CTTACCATCT GGCCCCAGTG CTGCAATGAT ACCGCGAGAC
1701 CCACGCTCAC CGGCTCCAGA TTTATCAGCA ATAAACCAGC CAGCCGGAAG
1751 GGCCGAGCGC AGAAGTGGTC CTGCAACTTT ATCCGCCTCC ATCCAGTCTA
1801 TTAATTGTTG CCGGGAAGCT AGAGTAAGTA GTTCGCCAGT TAATAGTTTG
1851 CGCAACGTTG TTGCCATTGC TGCAGGCATC GTGGTGTCAC GCTCGTCGTT
1901 TGGTATGGCT TCATTCAGCT CCGGTTCCCA ACGATCAAGG CGAGTTACAT
1951 GATCCCCCAT GTTGTGCAAA AAAGCGGTTA GCTCCTTCGG TCCTCCGATC
2001 GTTGTCAGAA GTAAGTTGGC CGCAGTGTTA TCACTCATGG TTATGGCAGC
2051 ACTGCATAAT TCTCTTACTG TCATGCCATC CGTAAGATGC TTTTCTGTGA
2101 CTGGTGAGTA CTCAACCAAG TCATTCTGAG AATAGTGTAT GCGGCGACCG
2151 AGTTGCTCTT GCCCGGCGTC AACACGGGAT AATACCGCGC CACATAGCAG
2201 AACTTTAAAA GTGCTCATCA TTGGAAAACG TTCTTCGGGG CGAAAACTCT
2251 CAAGGATCTT ACCGCTGTTG AGATCCAGTT CGATGTAACC CACTCGTGCA
2301 CCCAACTGAT CTTCAGCATC TTTTACTTTC ACCAGCGTTT CTGGGTGAGC
2351 AAAAACAGGA AGGCAAAATG CCGCAAAAAA GGGAATAAGG GCGACACGGA
2401 AATGTTGAAT ACTCATACTC TTCCTTTTTC AATATTATTG AAGCATTTAT
2451 CAGGGTTATT GTCTCATGAG CGGATACATA TTTGAATGTA TTTAGAAAAA
2501 TAAACAAATA GGGGTTCCGC GCACATTTCC CCGAAAAGTG CCACCTGACG
2551 TCTAAGAAAC CATTATTATC ATGACATTAA CCTATAAAAA TAGGCGTATC
2601 ACGAGGCCCT TTCGTCTTCA AGAATTCAGC TTGGCTGCAG TGAATAATAA
2651 AATGTGTGTT TGTCCGAAAT ACGCGTTTTG AGATTTCTGT CGCCGACTAA
2701 ATTCATGTCG CGCGATAGTG GTGTTTATCG CCGATAGAGA TGGCGATATT
2751 GGAAAAATCG ATATTTGAAA ATATGGCATA TTGAAAATGT CGCCGATGTG
2801 AGTTTCTGTG TAACTGATAT CGCCATTTTT CCAAAAGTGA TTTTTGGGCA
2851 TACGCGATAT CTGGCGATAG CGCTTATATC GTTTACGGGG GATGGCGATA
2901 GACGACTTTG GTGACTTGGG CGATTCTGTG TGTCGCAAAT ATCGCAGTTT
2951 CGATATAGGT GACAGACGAT ATGAGGCTAT ATCGCCGATA GAGGCGACAT
3001 CAAGCTGGCA CATGGCCAAT GCATATCGAT CTATACATTG AATCAATATT
3051 GGCCATTAGC CATATTATTC ATTGGTTATA TAGCATAAAT CAATATTGGC
3101 TATTGGCCAT TGCATACGTT GTATCCATAT CATAATATGT ACATTTATAT
3151 TGGCTCATGT CCAACATTAC CGCCATGTTG ACATTGATTA TTGACTAGTT
3201 ATTAATAGTA ATCAATTACG GGGTCATTAG TTCATAGCCC ATATATGGAG
3251 TTCCGCGTTA CATAACTTAC GGTAAATGGC CCGCCTGGCT GACCGCCCAA
3301 CGACCCCCGC CCATTGACGT CAATAATGAC GTATGTTCCC ATAGTAACGC
3351 CAATAGGGAC TTTCCATTGA CGTCAATGGG TGGAGTATTT ACGGTAAACT

3401 GCCCACTTGG CAGTACATCA AGTGTATCAT ATGCCAAGTA CGCCCCCTAT
3451 TGACGTCAAT GACGGTAAAT GGCCCGCCTG GCATTATGCC CAGTACATGA
3501 CCTTATGGGA CTTTCCTACT TGGCAGTACA TCTACGTATT AGTCATCGCT
3551 ATTACCATGG TGATGCGGTT TTGGCAGTAC ATCAATGGGC GTGGATAGCG
3601 GTTTGACTCA CGGGGATTTC CAAGTCTCCA CCCCATTGAC GTCAATGGGA
3651 GTTTGTTTTG GCACCAAAAT CAACGGGACT TTCCAAAATG TCGTAACAAC
3701 TCCGCCCCAT TGACGCAAAT GGGCGGTAGG CGTGTACGGT GGGAGGTCTA
3751 TATAAGCAGA GCTCGTTTAG TGAACCGTCA GATCGCCTGG AGACGCCATC
3801 CACGCTGTTT TGACCTCCAT AGAAGACACC GGGACCGATC CAGCCTCCGC
3851 AAGCTTGATA TCGAATTCCT GCAGCCCGGG GGATCCGCCC GCTTGCCGCC
3901 ACCATGGAGA CCCCCGCCCA GCTGCTGTTC CTGCTGCTGC TGTGGCTGCC
3951 CGACACCACC GGCGACATTC TGCTGACCCA GTCTCCAGCC ACCCTGTCTC
4001 TGAGTCCAGG AGAAAGAGCC ACTCTCTCCT GCAGGGCCAG TCAGAACATT
4051 GGCACAAGCA TACAGTGGTA TCAACAAAAA CCAGGTCAGG CTCCAAGGCT
4101 TCTCATAAGG TCTTCTTCTG AGTCTATCTC TGGGATCCCT TCCAGGTTTA
4151 GTGGCAGTGG ATCAGGGACA GATTTTACTC TTACCATCAG CAGTCTGGAG
4201 CCTGAAGATT TTGCAGTGTA TTACTGTCAA CAAAGTAATA CCTGGCCATT
4251 CACGTTCGGC CAGGGGACCA AGCTGGAGAT CAAACGTGAG TATTCTAGAA
4301 AGATCCTAGA ATTCTAAACT CTGAGGGGGT CGGATGACGT GGCCATTCTT
4351 TGCCTAAAGC ATTGAGTTTA CTGCAAGGTC AGAAAAGCAT GCAAAGCCCT
4401 CAGAATGGCT GCAAAGAGCT CCAACAAAAC AATTTAGAAC TTTATTAAGG
4451 AATAGGGGGA AGCTAGGAAG AAACTCAAAA CATCAAGATT TTAAATACGC
4501 TTCTTGGTCT CCTTGCTATA ATTATCTGGG ATAAGCATGC TGTTTTCTGT
4551 CTGTCCCTAA CATGCCCTGT GATTATCCGC AAACAACACA CCCAAGGGCA
4601 GAACTTTGTT ACTTAAACAC CATCCTGTTT GCTTCTTTCC TCAGGAACTG
4651 TGGCTGCACC ATCTGTCTTC ATCTTCCCGC CATCTGATGA GCAGTTGAAA
4701 TCTGGAACTG CCTCTGTTGT GTGCCTGCTG AATAACTTCT ATCCCAGAGA
4751 GGCCAAAGTA CAGTGGAAGG TGGATAACGC CCTCCAATCG GGTAACTCCC
4801 AGGAGAGTGT CACAGAGCAG GACAGCAAGG ACAGCACCTA CAGCCTCAGC
4851 AGCACCCTGA CGCTGAGCAA AGCAGACTAC GAGAAACACA AAGTCTACGC
4901 CTGCGAAGTC ACCCATCAGG GCCTGAGCTC GCCCGTCACA AAGAGCTTCA
4951 ACAGGGGAGA GTGTTAGAGG GAGAAGTGCC CCCACCTGCT CCTCAGTTCC
5001 AGCCTGACCC CCTCCCATCC TTTGGCCTCT GACCCTTTTT CCACAGGGGA
5051 CCTACCCCTA TTGCGGTCCT CCAGCTCATC TTTCACCTCA CCCCCCTCCT
5101 CCTCCTTGGC TTTAATTATG CTAATGTTGG AGGAGAATGA ATAAATAAAG
5151 TGAATCTTTG CACCTGTGGT TTCTCTCTTT CCTCATTTAA TAATTATTAT
5201 CTGTTGTTTA CCAACTACTC AATTTCTCTT ATAAGGGACT AAATATGTAG

5251 TCATCCTAAG GCGCATAACC ATTTATAAAA ATCATCCTTC ATTCTATTTT
5301 ACCCTATCAT CCTCTGCAAG ACAGTCCTCC CTCAAACCCA CAAGCCTTCT
5351 GTCCTCACAG TCCCCTGGGC CATGGTAGGA GAGACTTGCT TCCTTGTTTT
5401 CCCCTCCTCA GCAAGCCCTC ATAGTCCTTT TTAAGGGTGA CAGGTCTTAC
5451 AGTCATATAT CCTTTGATTC AATTCCCTGA GAATCAACCA AAGCAAATTT
5501 TTCAAAAGAA GAAACCTGCT ATAAAGAGAA TCATTCATTG CAACATGATA
5551 TAAAATAACA ACACAATAAA AGCAATTAAA TAAACAAACA ATAGGGAAAT
5601 GTTTAAGTTC ATCATGGTAC TTAGACTTAA TGGAATGTCA TGCCTTATTT
5651 ACATTTTTAA ACAGGTACTG AGGGACTCCT GTCTGCCAAG GGCCGTATTG
5701 AGTACTTTCC ACAACCTAAT TTAATCCACA CTATACTGTG AGATTAAAAA
5751 CATTCATTAA AATGTTGCAA AGGTTCTATA AAGCTGAGAG ACAAATATAT
5801 TCTATAACTC AGCAATCCCA CTTCTAGATG ACTGAGTGTC CCCACCCACC
5851 AAAAAACTAT GCAAGAATGT TCAAAGCAGC TTTATTTACA AAAGCCAAAA
5901 ATTGGAAATA GCCCGATTGT CCAACAATAG AATGAGTTAT TAAACTGTGG
5951 TATGTTTATA CATTAGAATA CCCAATGAGG AGAATTAACA AGCTACAACT
6001 ATACCTACTC ACACAGATGA ATCTCATAAA AATAATGTTA CATAAGAGAA
6051 ACTCAATGCA AAAGATATGT TCTGTATGTT TTCATCCATA TAAAGTTCAA
6101 AACCAGGTAA AAATAAAGTT AGAAATTTGG ATGGAAATTA CTCTTAGCTG
6151 GGGGTGGGCG AGTTAGTGCC TGGGAGAAGA CAAGAAGGGG CTTCTGGGGT
6201 CTTGGTAATG TTCTGTTCCT CGTGTGGGGT TGTGCAGTTA TGATCTGTGC
6251 ACTGTTCTGT ATACACATTA TGCTTCAAAA TAACTTCACA TAAAGAACAT
6301 CTTATACCCA GTTAATAGAT AGAAGAGGAA TAAGTAATAG GTCAAGACCA
6351 CGCAGCTGGT AAGTGGGGGG GCCTGGGATC AAATAGCTAC CTGCCTAATC
6401 CTGCCCTCTT GAGCCCTGAA TGAGTCTGCC TTCCAGGGCT CAAGGTGCTC
6451 AACAAAACAA CAGGCCTGCT ATTTTCCTGG CATCTGTGCC CTGTTTGGCT
6501 AGCTAGGAGC ACACATACAT AGAAATTAAA TGAAACAGAC CTTCAGCAAG
6551 GGGACAGAGG ACAGAATTAA CCTTGCCCAG ACACTGGAAA CCCATGTATG
6601 AACACTCACA TGTTTGGGAA GGGGGAAGGG CACATGTAAA TGAGGACTCT
6651 TCCTCATTCT ATGGGGCACT CTGGCCCTGC CCCTCTCAGC TACTCATCCA
6701 TCCAACACAC CTTTCTAAGT ACCTCTCTCT GCCTACACTC TGAAGGGGTT
6751 CAGGAGTAAC TAACACAGCA TCCCTTCCCT CAAATGACTG ACAATCCCTT
6801 TGTCCTGCTT TGTTTTTCTT TCCAGTCAGT ACTGGGAAAG TGGGGAAGGA
6851 CAGTCATGGA GAAACTACAT AAGGAAGCAC CTTGCCCTTC TGCCTCTTGA
6901 GAATGTTGAT GAGTATCAAA TCTTTCAAAC TTTGGAGGTT TGAGTAGGGG
6951 TGAGACTCAG TAATGTCCCT TCCAATGACA TGAACTTGCT CACTCATCCC
7001 TGGGGGCCAA ATTGAACAAT CAAAGGCAGG CATAATCCAG CTATGAATTC
7051 TAGGATCGAT CCAGACATGA TAAGATACAT TGATGAGTTT GGACAAACCA

7101 CAACTAGAAT GCAGTGAAAA AAATGCTTTA TTTGTGAAAT TTGTGATGCT
7151 ATTGCTTTAT TTGTAACCAT TATAAGCTGC AATAAACAAG TTAACAACAA
7201 CAATTGCATT CATTTTATGT TTCAGGTTCA GGGGGAGGTG TGGGAGGTTT
7251 TTTAAAGCAA GTAAAACCTC TACAAATGTG GTATGGCTGA TTATGATCTC
7301 TAGTCAAGGC ACTATACATC AAATATTCCT TATTAACCCC TTTACAAATT
7351 AAAAAGCTAA AGGTACACAA TTTTTGAGCA TAGTTATTAA TAGCAGACAC
7401 TCTATGCCTG TGTGGAGTAA GAAAAAACAG TATGTTATGA TTATAACTGT
7451 TATGCCTACT TATAAAGGTT ACAGAATATT TTTCCATAAT TTTCTTGTAT
7501 AGCAGTGCAG CTTTTTCCTT TGTGGTGTAA ATAGCAAAGC AAGCAAGAGT
7551 TCTATTACTA AACACAGCAT GACTCAAAAA ACTTAGCAAT TCTGAAGGAA
7601 AGTCCTTGGG GTCTTCTACC TTTCTCTTCT TTTTTGGAGG AGTAGAATGT
7651 TGAGAGTCAG CAGTAGCCTC ATCATCACTA GATGGCATTT CTTCTGAGCA
7701 AAACAGGTTT TCCTCATTAA AGGCATTCCA CCACTGCTCC CATTCATCAG
7751 TTCCATAGGT TGGAATCTAA AATACACAAA CAATTAGAAT CAGTAGTTTA
7801 ACACATTATA CACTTAAAAA TTTTATATTT ACCTTAGAGC TTTAAATCTC
7851 TGTAGGTAGT TTGTCCAATT ATGTCACACC ACAGAAGTAA GGTTCCTTCA
7901 CAAAGATCCG GGACCAAAGC GGCCATCGTG CCTCCCCACT CCTGCAGTTC
7951 GGGGGCATGG ATGCGCGGAT AGCCGCTGCT GGTTTCCTGG ATGCCGACGG
8001 ATTTGCACTG CCGGTAGAAC TCCGCGAGGT CGTCCAGCCT CAGGCAGCAG
8051 CTGAACCAAC TCGCGAGGGG ATCGAGCCCG GGGTGGGCGA AGAACTCCAG
8101 CATGAGATCC CCGCGCTGGA GGATCATCCA GCCGGCGTCC CGGAAAACGA
8151 TTCCGAAGCC CAACCTTTCA TAGAAGGCGG CGGTGGAATC GAAATCTCGT
8201 GATGGCAGGT TGGGCGTCGC TTGGTCGGTC ATTTCGAACC CCAGAGTCCC
8251 GCTCAGAAGA ACTCGTCAAG AAGGCGATAG AAGGCGATGC GCTGCGAATC
8301 GGGAGCGGCG ATACCGTAAA GCACGAGGAA GCGGTCAGCC CATTCGCCGC
8351 CAAGCTCTTC AGCAATATCA CGGGTAGCCA ACGCTATGTC CTGATAGCGG
8401 TCCGCCACAC CCAGCCGGCC ACAGTCGATG AATCCAGAAA AGCGGCCATT
8451 TTCCACCATG ATATTCGGCA AGCAGGCATC GCCATGGGTC ACGACGAGAT
8501 CCTCGCCGTC GGGCATGCGC GCCTTGAGCC TGGCGAACAG TTCGGCTGGC
8551 GCGAGCCCCT GATGCTCTTC GTCCAGATCA TCCTGATCGA CAAGACCGGC
8601 TTCCATCCGA GTACGTGCTC GCTCGATGCG ATGTTTCGCT TGGTGGTCGA
8651 ATGGGCAGGT AGCCGGATCA AGCGTATGCA GCCGCCGCAT TGCATCAGCC
8701 ATGATGGATA CTTTCTCGGC AGGAGCAAGG TGAGATGACA GGAGATCCTG
8751 CCCCGGCACT TCGCCCAATA GCAGCCAGTC CCTTCCCGCT TCAGTGACAA
8801 CGTCGAGCAC AGCTGCGCAA GGAACGCCCG TCGTGGCCAG CCACGATAGC
8851 CGCGCTGCCT CGTCCTGCAG TTCATTCAGG GCACCGGACA GGTCGGTCTT
8901 GACAAAAAGA ACCGGGCGCC CCTGCGCTGA CAGCCGGAAC ACGGCGGCAT

8951 CAGAGCAGCC GATTGTCTGT TGTGCCCAGT CATAGCCGAA TAGCCTCTCC
9001 ACCCAAGCGG CCGGAGAACC TGCGTGCAAT CCATCTTGTT CAATCATGCG
9051 AAACGATCCT CATCCTGTCT CTTGATCAGA TCTTGATCCC CTGCGCCATC
9101 AGATCCTTGG CGGCAAGAAA GCCATCCAGT TTACTTTGCA GGGCTTCCCA
9151 ACCTTACCAG AGGGCGCCCC AGCTGGCAAT TCCGGTTCGC TTGCTGTCCA
9201 TAAAACCGCC CAGTCTAGCT ATCGCCATGT AAGCCCACTG CAAGCTACCT
9251 GCTTTCTCTT TGCGCTTGCG TTTTCCCTTG TCCAGATAGC CCAGTAGCTG
9301 ACATTCATCC GGGGTCAGCA CCGTTTCTGC GGACTGGCTT TCTACGTGTT
9351 CCGCTTCCTT TAGCAGCCCT TGCGCCCTGA GTGCTTGCGG CAGCGTGAAG

**SEQ ID NO:18**

**Nucleotide sequence of the expression vector HCMV-K HuAb-VL1 hum V2 (Complete DNA Sequence of a humanised light chain expression vector comprising SEQ ID NO: 13 (humV2=VLm) from 3926-4246)**

1 CTAGCTTTTT GCAAAAGCCT AGGCCTCCAA AAAAGCCTCC TCACTACTTC
51 TGGAATAGCT CAGAGGCCGA GGCGGCCTCG GCCTCTGCAT AAATAAAAAA
101 AATTAGTCAG CCATGGGGCG GAGAATGGGC GGAACTGGGC GGAGTTAGGG
151 GCGGGATGGG CGGAGTTAGG GGCGGGACTA TGGTTGCTGA CTAATTGAGA
201 TGCATGCTTT GCATACTTCT GCCTGCTGGG GAGCCTGGTT GCTGACTAAT
251 TGAGATGCAT GCTTTGCATA CTTCTGCCTG CTGGGGAGCC TGGGGACTTT
301 CCACACCCTA ACTGACACAC ATTCCACAGC TGCCTCGCGC GTTTCGGTGA
351 TGACGGTGAA AACCTCTGAC ACATGCAGCT CCCGGAGACG GTCACAGCTT
401 GTCTGTAAGC GGATGCCGGG AGCAGACAAG CCCGTCAGGG CGCGTCAGCG
451 GGTGTTGGCG GGTGTCGGGG CGCAGCCATG ACCCAGTCAC GTAGCGATAG
501 CGGAGTGTAT ACTGGCTTAA CTATGCGGCA TCAGAGCAGA TTGTACTGAG
551 AGTGCACCAT ATGCGGTGTG AAATACCGCA CAGATGCGTA AGGAGAAAAT
601 ACCGCATCAG GCGCTCTTCC GCTTCCTCGC TCACTGACTC GCTGCGCTCG
651 GTCGTTCGGC TGCGGCGAGC GGTATCAGCT CACTCAAAGG CGGTAATACG
701 GTTATCCACA GAATCAGGGG ATAACGCAGG AAAGAACATG TGAGCAAAAG
751 GCCAGCAAAA GGCCAGGAAC CGTAAAAAGG CCGCGTTGCT GGCGTTTTTC
801 CATAGGCTCC GCCCCCCTGA CGAGCATCAC AAAAATCGAC GCTCAAGTCA
851 GAGGTGGCGA AACCCGACAG GACTATAAAG ATACCAGGCG TTTCCCCCTG
901 GAAGCTCCCT CGTGCGCTCT CCTGTTCCGA CCCTGCCGCT TACCGGATAC
951 CTGTCCGCCT TTCTCCCTTC GGGAAGCGTG GCGCTTTCTC ATAGCTCACG
1001 CTGTAGGTAT CTCAGTTCGG TGTAGGTCGT TCGCTCCAAG CTGGGCTGTG

1051 TGCACGAACC CCCCGTTCAG CCCGACCGCT GCGCCTTATC CGGTAACTAT
1101 CGTCTTGAGT CCAACCCGGT AAGACACGAC TTATCGCCAC TGGCAGCAGC
1151 CACTGGTAAC AGGATTAGCA GAGCGAGGTA TGTAGGCGGT GCTACAGAGT
1201 TCTTGAAGTG GTGGCCTAAC TACGGCTACA CTAGAAGGAC AGTATTTGGT
1251 ATCTGCGCTC TGCTGAAGCC AGTTACCTTC GGAAAAAGAG TTGGTAGCTC
1301 TTGATCCGGC AAACAAACCA CCGCTGGTAG CGGTGGTTTT TTTGTTTGCA
1351 AGCAGCAGAT TACGCGCAGA AAAAAAGGAT CTCAAGAAGA TCCTTTGATC
1401 TTTTCTACGG GGTCTGACGC TCAGTGGAAC GAAAACTCAC GTTAAGGGAT
1451 TTTGGTCATG AGATTATCAA AAAGGATCTT CACCTAGATC CTTTTAAATT
1501 AAAAATGAAG TTTTAAATCA ATCTAAAGTA TATATGAGTA AACTTGGTCT
1551 GACAGTTACC AATGCTTAAT CAGTGAGGCA CCTATCTCAG CGATCTGTCT
1601 ATTTCGTTCA TCCATAGTTG CCTGACTCCC CGTCGTGTAG ATAACTACGA
1651 TACGGGAGGG CTTACCATCT GGCCCCAGTG CTGCAATGAT ACCGCGAGAC
1701 CCACGCTCAC CGGCTCCAGA TTTATCAGCA ATAAACCAGC CAGCCGGAAG
1751 GGCCGAGCGC AGAAGTGGTC CTGCAACTTT ATCCGCCTCC ATCCAGTCTA
1801 TTAATTGTTG CCGGGAAGCT AGAGTAAGTA GTTCGCCAGT TAATAGTTTG
1851 CGCAACGTTG TTGCCATTGC TGCAGGCATC GTGGTGTCAC GCTCGTCGTT
1901 TGGTATGGCT TCATTCAGCT CCGGTTCCCA ACGATCAAGG CGAGTTACAT
1951 GATCCCCCAT GTTGTGCAAA AAAGCGGTTA GCTCCTTCGG TCCTCCGATC
2001 GTTGTCAGAA GTAAGTTGGC CGCAGTGTTA TCACTCATGG TTATGGCAGC
2051 ACTGCATAAT TCTCTTACTG TCATGCCATC CGTAAGATGC TTTTCTGTGA
2101 CTGGTGAGTA CTCAACCAAG TCATTCTGAG AATAGTGTAT GCGGCGACCG
2151 AGTTGCTCTT GCCCGGCGTC AACACGGGAT AATACCGCGC CACATAGCAG
2201 AACTTTAAAA GTGCTCATCA TTGGAAAACG TTCTTCGGGG CGAAAACTCT
2251 CAAGGATCTT ACCGCTGTTG AGATCCAGTT CGATGTAACC CACTCGTGCA
2301 CCCAACTGAT CTTCAGCATC TTTTACTTTC ACCAGCGTTT CTGGGTGAGC
2351 AAAAACAGGA AGGCAAAATG CCGCAAAAAA GGGAATAAGG GCGACACGGA
2401 AATGTTGAAT ACTCATACTC TTCCTTTTTC AATATTATTG AAGCATTTAT
2451 CAGGGTTATT GTCTCATGAG CGGATACATA TTTGAATGTA TTTAGAAAAA
2501 TAAACAAATA GGGGTTCCGC GCACATTTCC CCGAAAAGTG CCACCTGACG
2551 TCTAAGAAAC CATTATTATC ATGACATTAA CCTATAAAAA TAGGCGTATC
2601 ACGAGGCCCT TTCGTCTTCA AGAATTCAGC TTGGCTGCAG TGAATAATAA
2651 AATGTGTGTT TGTCCGAAAT ACGCGTTTTG AGATTTCTGT CGCCGACTAA
2701 ATTCATGTCG CGCGATAGTG GTGTTTATCG CCGATAGAGA TGGCGATATT
2751 GGAAAAATCG ATATTTGAAA ATATGGCATA TTGAAAATGT CGCCGATGTG
2801 AGTTTCTGTG TAACTGATAT CGCCATTTTT CCAAAAGTGA TTTTTGGGCA
2851 TACGCGATAT CTGGCGATAG CGCTTATATC GTTTACGGGG GATGGCGATA

2901 GACGACTTTG GTGACTTGGG CGATTCTGTG TGTCGCAAAT ATCGCAGTTT
2951 CGATATAGGT GACAGACGAT ATGAGGCTAT ATCGCCGATA GAGGCGACAT
3001 CAAGCTGGCA CATGGCCAAT GCATATCGAT CTATACATTG AATCAATATT
3051 GGCCATTAGC CATATTATTC ATTGGTTATA TAGCATAAAT CAATATTGGC
3101 TATTGGCCAT TGCATACGTT GTATCCATAT CATAATATGT ACATTTATAT
3151 TGGCTCATGT CCAACATTAC CGCCATGTTG ACATTGATTA TTGACTAGTT
3201 ATTAATAGTA ATCAATTACG GGGTCATTAG TTCATAGCCC ATATATGGAG
3251 TTCCGCGTTA CATAACTTAC GGTAAATGGC CCGCCTGGCT GACCGCCCAA
3301 CGACCCCCGC CCATTGACGT CAATAATGAC GTATGTTCCC ATAGTAACGC
3351 CAATAGGGAC TTTCCATTGA CGTCAATGGG TGGAGTATTT ACGGTAAACT
3401 GCCCACTTGG CAGTACATCA AGTGTATCAT ATGCCAAGTA CGCCCCCTAT
3451 TGACGTCAAT GACGGTAAAT GGCCCGCCTG GCATTATGCC CAGTACATGA
3501 CCTTATGGGA CTTTCCTACT TGGCAGTACA TCTACGTATT AGTCATCGCT
3551 ATTACCATGG TGATGCGGTT TTGGCAGTAC ATCAATGGGC GTGGATAGCG
3601 GTTTGACTCA CGGGGATTTC CAAGTCTCCA CCCCATTGAC GTCAATGGGA
3651 GTTTGTTTTG GCACCAAAAT CAACGGGACT TTCCAAAATG TCGTAACAAC
3701 TCCGCCCCAT TGACGCAAAT GGGCGGTAGG CGTGTACGGT GGGAGGTCTA
3751 TATAAGCAGA GCTCGTTTAG TGAACCGTCA GATCGCCTGG AGACGCCATC
3801 CACGCTGTTT TGACCTCCAT AGAAGACACC GGGACCGATC CAGCCTCCGC
3851 AAGCTTGCCG CCACCATGGA GACCCCCGCC CAGCTGCTGT TCCTGCTGCT
3901 GCTGTGGCTG CCCGACACCA CCGGCGACAT TCTGCTGACC CAGTCTCCAG
3951 CCACCCTGTC TCTGAGTCCA GGAGAAAGAG CCACTTTCTC CTGCAGGGCC
4001 AGTCAGAACA TTGGCACAAG CATACAGTGG TATCAACAAA AAACAAATGG
4051 TGCTCCAAGG CTTCTCATAA GGTCTTCTTC TGAGTCTATC TCTGGGATCC
4101 CTTCCAGGTT TAGTGGCAGT GGATCAGGGA CAGATTTTAC TCTTACCATC
4151 AGCAGTCTGG AGCCTGAAGA TTTTGCAGTG TATTACTGTC AACAAAGTAA
4201 TACCTGGCCA TTCACGTTCG GCCAGGGGAC CAAGCTGGAG ATCAAACGTG
4251 AGTATTCTAG AAAGATCCTA GAATTCTAAA CTCTGAGGGG GTCGGATGAC
4301 GTGGCCATTC TTTGCCTAAA GCATTGAGTT TACTGCAAGG TCAGAAAAGC
4351 ATGCAAAGCC CTCAGAATGG CTGCAAAGAG CTCCAACAAA ACAATTTAGA
4401 ACTTTATTAA GGAATAGGGG GAAGCTAGGA AGAAACTCAA AACATCAAGA
4451 TTTTAAATAC GCTTCTTGGT CTCCTTGCTA TAATTATCTG GGATAAGCAT
4501 GCTGTTTTCT GTCTGTCCCT AACATGCCCT GTGATTATCC GCAAACAACA
4551 CACCCAAGGG CAGAACTTTG TTACTTAAAC ACCATCCTGT TTGCTTCTTT
4601 CCTCAGGAAC TGTGGCTGCA CCATCTGTCT TCATCTTCCC GCCATCTGAT
4651 GAGCAGTTGA AATCTGGAAC TGCCTCTGTT GTGTGCCTGC TGAATAACTT
4701 CTATCCCAGA GAGGCCAAAG TACAGTGGAA GGTGGATAAC GCCCTCCAAT

4751 CGGGTAACTC CCAGGAGAGT GTCACAGAGC AGGACAGCAA GGACAGCACC

4801 TACAGCCTCA GCAGCACCCT GACGCTGAGC AAAGCAGACT ACGAGAAACA

4851 CAAAGTCTAC GCCTGCGAAG TCACCCATCA GGGCCTGAGC TCGCCCGTCA

4901 CAAAGAGCTT CAACAGGGGA GAGTGTTAGA GGGAGAAGTG CCCCCACCTG

4951 CTCCTCAGTT CCAGCCTGAC CCCCTCCCAT CCTTTGGCCT CTGACCCTTT

5001 TTCCACAGGG GACCTACCCC TATTGCGGTC CTCCAGCTCA TCTTTCACCT

5051 CACCCCCCTC CTCCTCCTTG GCTTTAATTA TGCTAATGTT GGAGGAGAAT

5101 GAATAAATAA AGTGAATCTT TGCACCTGTG GTTTCTCTCT TTCCTCATTT

5151 AATAATTATT ATCTGTTGTT TACCAACTAC TCAATTTCTC TTATAAGGGA

5201 CTAAATATGT AGTCATCCTA AGGCGCATAA CCATTTATAA AAATCATCCT

5251 TCATTCTATT TTACCCTATC ATCCTCTGCA AGACAGTCCT CCCTCAAACC

5301 CACAAGCCTT CTGTCCTCAC AGTCCCCTGG GCCATGGTAG GAGAGACTTG

5351 CTTCCTTGTT TTCCCCTCCT CAGCAAGCCC TCATAGTCCT TTTTAAGGGT

5401 GACAGGTCTT ACAGTCATAT ATCCTTTGAT TCAATTCCCT GAGAATCAAC

5451 CAAAGCAAAT TTTTCAAAAG AAGAAACCTG CTATAAAGAG AATCATTCAT

5501 TGCAACATGA TATAAAATAA CAACACAATA AAAGCAATTA AATAAACAAA

5551 CAATAGGGAA ATGTTTAAGT TCATCATGGT ACTTAGACTT AATGGAATGT

5601 CATGCCTTAT TTACATTTTT AAACAGGTAC TGAGGGACTC CTGTCTGCCA

5651 AGGGCCGTAT TGAGTACTTT CCACAACCTA ATTTAATCCA CACTATACTG

5701 TGAGATTAAA AACATTCATT AAAATGTTGC AAAGGTTCTA TAAAGCTGAG

5751 AGACAAATAT ATTCTATAAC TCAGCAATCC CACTTCTAGA TGACTGAGTG

5801 TCCCCACCCA CCAAAAAACT ATGCAAGAAT GTTCAAAGCA GCTTTATTTA

5851 CAAAAGCCAA AAATTGGAAA TAGCCCGATT GTCCAACAAT AGAATGAGTT

5901 ATTAAACTGT GGTATGTTTA TACATTAGAA TACCCAATGA GGAGAATTAA

5951 CAAGCTACAA CTATACCTAC TCACACAGAT GAATCTCATA AAAATAATGT

6001 TACATAAGAG AAACTCAATG CAAAAGATAT GTTCTGTATG TTTTCATCCA

6051 TATAAAGTTC AAAACCAGGT AAAAATAAAG TTAGAAATTT GGATGGAAAT

6101 TACTCTTAGC TGGGGGTGGG CGAGTTAGTG CCTGGGAGAA GACAAGAAGG

6151 GGCTTCTGGG GTCTTGGTAA TGTTCTGTTC CTCGTGTGGG GTTGTGCAGT

6201 TATGATCTGT GCACTGTTCT GTATACACAT TATGCTTCAA AATAACTTCA

6251 CATAAAGAAC ATCTTATACC CAGTTAATAG ATAGAAGAGG AATAAGTAAT

6301 AGGTCAAGAC CACGCAGCTG GTAAGTGGGG GGGCCTGGGA TCAAATAGCT

6351 ACCTGCCTAA TCCTGCCCTC TTGAGCCCTG AATGAGTCTG CCTTCCAGGG

6401 CTCAAGGTGC TCAACAAAAC AACAGGCCTG CTATTTTCCT GGCATCTGTG

6451 CCCTGTTTGG CTAGCTAGGA GCACACATAC ATAGAAATTA AATGAAACAG

6501 ACCTTCAGCA AGGGGACAGA GGACAGAATT AACCTTGCCC AGACACTGGA

6551 AACCCATGTA TGAACACTCA CATGTTTGGG AAGGGGGAAG GGCACATGTA

6601 AATGAGGACT CTTCCTCATT CTATGGGGCA CTCTGGCCCT GCCCCTCTCA
6651 GCTACTCATC CATCCAACAC ACCTTTCTAA GTACCTCTCT CTGCCTACAC
6701 TCTGAAGGGG TTCAGGAGTA ACTAACACAG CATCCCTTCC CTCAAATGAC
6751 TGACAATCCC TTTGTCCTGC TTTGTTTTTC TTTCCAGTCA GTACTGGGAA
6801 AGTGGGGAAG GACAGTCATG GAGAAACTAC ATAAGGAAGC ACCTTGCCCT
6851 TCTGCCTCTT GAGAATGTTG ATGAGTATCA AATCTTTCAA ACTTTGGAGG
6901 TTTGAGTAGG GGTGAGACTC AGTAATGTCC CTTCCAATGA CATGAACTTG
6951 CTCACTCATC CCTGGGGGCC AAATTGAACA ATCAAAGGCA GGCATAATCC
7001 AGCTATGAAT TCTAGGATCG ATCCAGACAT GATAAGATAC ATTGATGAGT
7051 TTGGACAAAC CACAACTAGA ATGCAGTGAA AAAAATGCTT TATTTGTGAA
7101 ATTTGTGATG CTATTGCTTT ATTTGTAACC ATTATAAGCT GCAATAAACA
7151 AGTTAACAAC AACAATTGCA TTCATTTTAT GTTTCAGGTT CAGGGGGAGG
7201 TGTGGGAGGT TTTTTAAAGC AAGTAAAACC TCTACAAATG TGGTATGGCT
7251 GATTATGATC TCTAGTCAAG GCACTATACA TCAAATATTC CTTATTAACC
7301 CCTTTACAAA TTAAAAAGCT AAAGGTACAC AATTTTTGAG CATAGTTATT
7351 AATAGCAGAC ACTCTATGCC TGTGTGGAGT AAGAAAAAAC AGTATGTTAT
7401 GATTATAACT GTTATGCCTA CTTATAAAGG TTACAGAATA TTTTTCCATA
7451 ATTTTCTTGT ATAGCAGTGC AGCTTTTTCC TTTGTGGTGT AAATAGCAAA
7501 GCAAGCAAGA GTTCTATTAC TAAACACAGC ATGACTCAAA AAACTTAGCA
7551 ATTCTGAAGG AAAGTCCTTG GGGTCTTCTA CCTTTCTCTT CTTTTTTGGA
7601 GGAGTAGAAT GTTGAGAGTC AGCAGTAGCC TCATCATCAC TAGATGGCAT
7651 TTCTTCTGAG CAAAACAGGT TTTCCTCATT AAAGGCATTC CACCACTGCT
7701 CCCATTCATC AGTTCCATAG GTTGGAATCT AAAATACACA AACAATTAGA
7751 ATCAGTAGTT TAACACATTA TACACTTAAA AATTTTATAT TTACCTTAGA
7801 GCTTTAAATC TCTGTAGGTA GTTTGTCCAA TTATGTCACA CCACAGAAGT
7851 AAGGTTCCTT CACAAAGATC CGGGACCAAA GCGGCCATCG TGCCTCCCCA
7901 CTCCTGCAGT TCGGGGGCAT GGATGCGCGG ATAGCCGCTG CTGGTTTCCT
7951 GGATGCCGAC GGATTTGCAC TGCCGGTAGA ACTCCGCGAG GTCGTCCAGC
8001 CTCAGGCAGC AGCTGAACCA ACTCGCGAGG GGATCGAGCC CGGGGTGGGC
8051 GAAGAACTCC AGCATGAGAT CCCCGCGCTG GAGGATCATC CAGCCGGCGT
8101 CCCGGAAAAC GATTCCGAAG CCCAACCTTT CATAGAAGGC GGCGGTGGAA
8151 TCGAAATCTC GTGATGGCAG GTTGGGCGTC GCTTGGTCGG TCATTTCGAA
8201 CCCCAGAGTC CCGCTCAGAA GAACTCGTCA AGAAGGCGAT AGAAGGCGAT
8251 GCGCTGCGAA TCGGGAGCGG CGATACCGTA AAGCACGAGG AAGCGGTCAG
8301 CCCATTCGCC GCCAAGCTCT TCAGCAATAT CACGGGTAGC CAACGCTATG
8351 TCCTGATAGC GGTCCGCCAC ACCCAGCCGG CCACAGTCGA TGAATCCAGA
8401 AAAGCGGCCA TTTTCCACCA TGATATTCGG CAAGCAGGCA TCGCCATGGG

8451 TCACGACGAG ATCCTCGCCG TCGGGCATGC GCGCCTTGAG CCTGGCGAAC
8501 AGTTCGGCTG GCGCGAGCCC CTGATGCTCT TCGTCCAGAT CATCCTGATC
8551 GACAAGACCG GCTTCCATCC GAGTACGTGC TCGCTCGATG CGATGTTTCG
8601 CTTGGTGGTC GAATGGGCAG GTAGCCGGAT CAAGCGTATG CAGCCGCCGC
8651 ATTGCATCAG CCATGATGGA TACTTTCTCG GCAGGAGCAA GGTGAGATGA
8701 CAGGAGATCC TGCCCCGGCA CTTCGCCCAA TAGCAGCCAG TCCCTTCCCG
8751 CTTCAGTGAC AACGTCGAGC ACAGCTGCGC AAGGAACGCC CGTCGTGGCC
8801 AGCCACGATA GCCGCGCTGC CTCGTCCTGC AGTTCATTCA GGGCACCGGA
8851 CAGGTCGGTC TTGACAAAAA GAACCGGGCG CCCCTGCGCT GACAGCCGGA
8901 ACACGGCGGC ATCAGAGCAG CCGATTGTCT GTTGTGCCCA GTCATAGCCG
8951 AATAGCCTCT CCACCCAAGC GGCCGGAGAA CCTGCGTGCA ATCCATCTTG
9001 TTCAATCATG CGAAACGATC CTCATCCTGT CTCTTGATCA GATCTTGATC
9051 CCCTGCGCCA TCAGATCCTT GGCGGCAAGA AAGCCATCCA GTTTACTTTG
9101 CAGGGCTTCC CAACCTTACC AGAGGGCGCC CCAGCTGGCA ATTCCGGTTC
9151 GCTTGCTGTC CATAAAACCG CCCAGTCTAG CTATCGCCAT GTAAGCCCAC
9201 TGCAAGCTAC CTGCTTTCTC TTTGCGCTTG CGTTTTCCCT TGTCCAGATA
9251 GCCCAGTAGC TGACATTCAT CCGGGGTCAG CACCGTTTCT GCGGACTGGC
9301 TTTCTACGTG TTCCGCTTCC TTTAGCAGCC CTTGCGCCCT GAGTGCTTGC
9351 GGCAGCGTGA AG

**SEQ ID NO:31:**
**Part of amino acid sequence of humanised heavy chain designated VHE-N73D**

EVQLVESGAE VKKPGASVKV SCKASGYTFT NYIIHWVKQE PGQGLEWIGY
FNPYNHGTKY NEKFKGRATL TADKSISTAY MELSSLRSED TAVYYCARSG
PYAWFDTWGQ GTTVTVSS

**SEQ ID NO:32:**
**Part of amino acid sequence of humanised heavy chain designated VHQ-N73D**

QVQLVESGAE VKKPGASVKV SCKASGYTFT NYIIHWVKQE PGQGLEWIGY
FNPYNHGTKY NEKFKGRATL TADKSISTAY MELSSLRSED TAVYYCARSG
PYAWFDTWGQ GTTVTVSS

**SEQ ID NO:33:**
**Nucleotide sequence encoding amino acid sequence SEQ ID NO: 8**

GACATTCTGCTGACCCAGTCTCCAGCCACCCTGTCTCTGAGTCCAGGAGAAAGAGCCA
CTCTCTCCTGCAGGGCCAGTCAGAACATTGGCACAAGCATACAGTGGTATCAACAAAAA
CCAGGTCAGGCTCCAAGGCTTCTCATAAGGTCTTCTTCTGAGTCTATCTCTGGGATCCC
TTCCAGGTTTAGTGGCAGTGGATCAGGGACAGATTTTACTCTTACCATCAGCAGTCTGG
AGCCTGAAGATTTTGCAGTGTATTACTGTCAACAAAGTAATACCTGGCCATTCACGTTC
GGCCAGGGGACCAAGCTTGAAATCAAA

**SEQ ID NO:34:**
**Nucleotide sequence encoding amino acid sequence SEQ ID NO: 31**

GAGGTGCAGCTGGTGGAGTCAGGAGCCGAAGTGAAAAAGCCTGGGGCTTCAGTGAAG
GTGTCCTGCAAGGCCTCTGGATACACATTCACTAATTATATTATCCACTGGGTGAAGCA
GGAGCCTGGTCAGGGCCTTGAATGGATTGGATATTTTAATCCTTACAATCATGGTACTA
AGTACAATGAGAAGTTCAAAGGCAGGGCCACACTAACTGCAGACAAATCCATCAGCACA
GCCTACATGGAGCTCAGCAGCCTGCGCTCTGAGGACACTGCGGTCTACTACTGTGCAA
GATCAGGACCCTATGCCTGGTTTGACACCTGGGGCCAAGGGACCACGGTCACCGTCTC
CTCA

**SEQ ID NO:35**
**Nucleotide sequence encoding amino acid sequence SEQ ID NO: 32**

CAGGTGCAGCTGGTGGAGTCAGGAGCCGAAGTGAAAAAGCCTGGGGCTTCAGTGAAG
GTGTCCTGCAAGGCCTCTGGATACACATTCACTAATTATATTATCCACTGGGTGAAGCA
GGAGCCTGGTCAGGGCCTTGAATGGATTGGATATTTTAATCCTTACAATCATGGTACTA
AGTACAATGAGAAGTTCAAAGGCAGGGCCACACTAACTGCAGACAAATCCATCAGCACA
GCCTACATGGAGCTCAGCAGCCTGCGCTCTGAGGACACTGCGGTCTACTACTGTGCAA
GATCAGGACCCTATGCCTGGTTTGACACCTGGGGCCAAGGGACCACGGTCACCGTCTC
CTCA

**SEQ ID No 36**
**Nucleotide sequence of expression vector LCVL1Sp20**

```
  1   CTAGAGTCCT AGAGAGGTCT GGTGGAGCCT GCAAAAGTCC AGCTTTCAAA
 51   GGAACACAGA AGTATGTGTA TGGAATATTA GAAGATGTTG CTTTTACTCT
101   TAAGTTGGTT CCTAGGAAAA ATAGTTAAAT ACTGTGACTT TAAAATGTGA
```

151 GAGGGTTTTC AAGTACTCAT TTTTTTAAAT GTCCAAAATT TTTGTCAATC
201 AATTTGAGGT CTTGTTTGTG TAGAACTGAC ATTACTTAAA GTTTAACCGA
251 GGAATGGGAG TGAGGCTCTC TCATACCCTA TTCAGAACTG ACTTTTAACA
301 ATAATAAATT AAGTTTAAAA TATTTTTAAA TGAATTGAGC AATGTTGAGT
351 TGGAGTCAAG ATGGCCGATC AGAACCAGAA CACCTGCAGC AGCTGGCAGG
401 AAGCAGGTCA TGTGGCAAGG CTATTTGGGG AAGGGAAAAT AAAACCACTA
451 GGTAAACTTG TAGCTGTGGT TTGAAGAAGT GGTTTTGAAA CACTCTGTCC
501 AGCCCCACCA AACCGAAAGT CCAGGCTGAG CAAAACACCA CCTGGGTAAT
551 TTGCATTTCT AAAATAAGTT GAGGATTCAG CCGAAACTGG AGAGGTCCTC
601 TTTTAACTTA TTGAGTTCAA CCTTTTAATT TTAGCTTGAG TAGTTCTAGT
651 TTCCCCAAAC TTAAGTTTAT CGACTTCTAA AATGTATTTA GAACTCATTT
701 TCAAAATTAG GTTATGTAAG AAATTGAAGG ACTTTAGTGT CTTTAATTTC
751 TAATATATTT AGAAAACTTC TTAAAATTAC TCTATTATTC TTCCCTCTGA
801 TTATTGGTCT CCATTCAATT CTTTTCCAAT ACCCGAAGCA TTTACAGTGA
851 CTTTGTTCAT GATCTTTTTT AGTTGTTTGT TTTGCCTTAC TATTAAGACT
901 TTGACATTCT GGTCAAAACG GCTTCACAAA TCTTTTTCAA GACCACTTTC
951 TGAGTATTCA TTTTAGGAGA AATACTTTTT TTTTAAATGA ATGCAATTAT
1001 CTAGGACCTG CAGGCATGCT GTTTTCTGTC TGTCCCTAAC ATGCCCTGTG
1051 ATTATCCGCA AACAACACAC CCAAGGGCAG AACTTTGTTA CTTAAACACC
1101 ATCCTGTTTG CTTCTTTCCT CAGGAACTGT GGCTGCACCA TCTGTCTTCA
1151 TCTTCCCGCC ATCTGATGAG CAGTTGAAAT CTGGAACTGC CTCTGTTGTG
1201 TGCCTGCTGA ATAACTTCTA TCCCAGAGAG GCCAAAGTAC AGTGGAAGGT
1251 GGATAACGCC CTCCAATCGG GTAACTCCCA GGAGAGTGTC ACAGAGCAGG
1301 ACAGCAAGGA CAGCACCTAC AGCCTCAGCA GCACCCTGAC GCTGAGCAAA
1351 GCAGACTACG AGAAACACAA AGTCTACGCC TGCGAAGTCA CCCATCAGGG
1401 CCTGAGCTCG CCCGTCACAA AGAGCTTCAA CAGGGGAGAG TGTTAGAGGG
1451 AGAAGTGCCC CCACCTGCTC CTCAGTTCCA GCCTGACCCC CTCCCATCCT
1501 TTGGCCTCTG ACCCTTTTTC CACAGGGGAC CTACCCCTAT TGCGGTCCTC
1551 CAGCTCATCT TTCACCTCAC CCCCCTCCTC CTCCTTGGCT TTAATTATGC
1601 TAATGTTGGA GGAGAATGAA TAAATAAAGT GAATCTTTGC ACCTGTGGTT
1651 TCTCTCTTTC CTCATTTAAT AATTATTATC TGTTGTTTTA CCAACTACTC
1701 AATTTCTCTT ATAAGGGACT AAATATGTAG TCATCCTAAG GCGGGATATC
1751 GAGATCTGAA GCTGATCCAG ACATGATAAG ATACATTGAT GAGTTTGGAC
1801 AAACCACAAC TAGAATGCAG TGAAAAAAAT GCTTTATTTG TGAAATTTGT
1851 GATGCTATTG CTTTATTTGT AACCATTATA AGCTGCAATA AACAAGTTAA
1901 CAACAACAAT TGCATTCATT TTATGTTTCA GGTTCAGGGG GAGGTGTGGG
1951 AGGTTTTTTA AAGCAAGTAA AACCTCTACA AATGTGGTAT GGCTGATTAT

2001 GATCTCTAGT CAAGGCACTA TACATCAAAT ATTCCTTATT AACCCCTTTA
2051 CAAATTAAAA AGCTAAAGGT ACACAATTTT TGAGCATAGT TATTAATAGC
2101 AGACACTCTA TGCCTGTGTG GAGTAAGAAA AAACAGTATG TTATGATTAT
2151 AACTGTTATG CCTACTTATA AAGGTTACAG AATATTTTTC CATAATTTTC
2201 TTGTATAGCA GTGCAGCTTT TTCCTTTGTG GTGTAAATAG CAAAGCAAGC
2251 AAGAGTTCTA TTACTAAACA CAGCATGACT CAAAAAACTT AGCAATTCTG
2301 AAGGAAAGTC CTTGGGGTCT TCTACCTTTC TCTTCTTTTT TGGAGGAGTA
2351 GAATGTTGAG AGTCAGCAGT AGCCTCATCA TCACTAGATG GCATTTCTTC
2401 TGAGCAAAAC AGGTTTTCCT CATTAAAGGC ATTCCACCAC TGCTCCCATT
2451 CATCAGTTCC ATAGGTTGGA ATCTAAAATA CACAAACAAT TAGAATCAGT
2501 AGTTTAACAC ATTATACACT TAAAAATTTT ATATTTACCT TAGAGCTTTA
2551 AATCTCTGTA GGTAGTTTGT CCAATTATGT CACACCACAG AAGTAAGGTT
2601 CCTTCACAAA GATCCGGACC AAAGCGGCCA TCGTGCCTCC CCACTCCTGC
2651 AGTTCGGGGG CATGGATGCG CGGATAGCCG CTGCTGGTTT CCTGGATGCC
2701 GACGGATTTG CACTGCCGGT AGAACTCCGC GAGGTCGTCC AGCCTCAGGC
2751 AGCAGCTGAA CCAACTCGCG AGGGGATCGA GCATCCCCCA TGGTCTTATA
2801 AAAATGCATA GCTTTAGGAG GGGAGCAGAG AACTTGAAAG CATCTTCCTG
2851 TTAGTCTTTC TTCTCGTAGA CTTCAAACTT ATACTTGATG CCTTTTTCCT
2901 CCTGGACCTC AGAGAGGACG CCTGGGTATT CTGGGAGAAG TTTATATTTC
2951 CCCAAATCAA TTTCTGGGAA AAACGTGTCA CTTTCAAATT CCTGCATGAT
3001 CCTTGTCACA AAGAGTCTGA GGTGGCCTGG TTGATTCATG GCTTCCTGGT
3051 AAACAGAACT GCCTCCGACT ATCCAAACCA TGTCTACTTT ACTTGCCAAT
3101 TCCGGTTGTT CAATAAGTCT TAAGGCATCA TCCAAACTTT TGGCAAGAAA
3151 ATGAGCTCCT CGTGGTGGTT CTTTGAGTTC TCTACTGAGA ACTATATTAA
3201 TTCTGTCCTT TAAAGGTCGA TTCTTCTCAG GAATGGAGAA CCAGGTTTTC
3251 CTACCCATAA TCACCAGATT CTGTTTACCT TCCACTGAAG AGGTTGTGGT
3301 CATTCTTTGG AAGTACTTGA ACTCGTTCCT GAGCGGAGGC CAGGGTCGGT
3351 CTCCGTTCTT GCCAATCCCC ATATTTTGGG ACACGGCGAC GATGCAGTTC
3401 AATGGTCGAA CCATGATGGC AGCGGGGATA AAATCCTACC AGCCTTCACG
3451 CTAGGATTGC CGTCAAGTTT GGGGGTACCG AGCTCGAATT AGCTTTTTGC
3501 AAAAGCCTAG GCCTCCAAAA AAGCCTCCTC ACTACTTCTG GAATAGCTCA
3551 GAGGGCCGAG GCGGCCTCGG CCTCTGCATA AATAAAAAAA ATTAGTCAGC
3601 CATGGGGCGG AGAATGGGCG GAACTGGGCG GAGTTAGGGG CGGGATGGGC
3651 GGAGTTAGGG GCGGGACTAT GGTTGCTGAC TAATTGAGAT GCATGCTTTG
3701 CATACTTCTG CCTGCTGGGG AGCCTGGGGA CTTTCCACAC CTGGTTGCTG
3751 ACTAATTGAG ATGCATGCTT TGCATACTTC TGCCTGCTGG GGAGCCTGGG
3801 GACTTTCCAC ACCCTAACTG ACACACATTC CACAGCTGCC TCGCGCGTTT

3851 CGGTGATGAC GGTGAAAACC TCTGACACAT GCAGCTCCCG GAGACGGTCA
3901 CAGCTTGTCT GTAAGCGGAT GCCGGGAGCA GACAAGCCCG TCAGGGCGCG
3951 TCAGCGGGTG TTGGCGGGTG TCGGGGCGCA GCCATGACCC AGTCACGTAG
4001 CGATAGCGGA GTGTATACTG GCTTAACTAT GCGGCATCAG AGCAGATTGT
4051 ACTGAGAGTG CACCATATGC GGCCGCATAT GCGGTGTGAA ATACCGCACA
4101 GATGCGTAAG GAGAAAATAC CGCATCAGGC GCTCTTCCGC TTCCTCGCTC
4151 ACTGACTCGC TGCGCTCGGT CGTTCGGCTG CGGCGAGCGG TATCAGCTCA
4201 CTCAAAGGCG GTAATACGGT TATCCACAGA ATCAGGGGAT AACGCAGGAA
4251 AGAACATGTG AGCAAAAGGC CAGCAAAAGG CCAGGAACCG TAAAAAGGCC
4301 GCGTTGCTGG CGTTTTTCCA TAGGCTCCGC CCCCCTGACG AGCATCACAA
4351 AAATCGACGC TCAAGTCAGA GGTGGCGAAA CCCGACAGGA CTATAAAGAT
4401 ACCAGGCGTT TCCCCCTGGA AGCTCCCTCG TGCGCTCTCC TGTTCCGACC
4451 CTGCCGCTTA CCGGATACCT GTCCGCCTTT CTCCCTTCGG GAAGCGTGGC
4501 GCTTTCTCAT AGCTCACGCT GTAGGTATCT CAGTTCGGTG TAGGTCGTTC
4551 GCTCCAAGCT GGGCTGTGTG CACGAACCCC CCGTTCAGCC CGACCGCTGC
4601 GCCTTATCCG GTAACTATCG TCTTGAGTCC AACCCGGTAA GACACGACTT
4651 ATCGCCACTG GCAGCAGCCA CTGGTAACAG GATTAGCAGA GCGAGGTATG
4701 TAGGCGGTGC TACAGAGTTC TTGAAGTGGT GGCCTAACTA CGGCTACACT
4751 AGAAGGACAG TATTTGGTAT CTGCGCTCTG CTGAAGCCAG TTACCTTCGG
4801 AAAAAGAGTT GGTAGCTCTT GATCCGGCAA ACAAACCACC GCTGGTAGCG
4851 GTGGTTTTTT TGTTTGCAAG CAGCAGATTA CGCGCAGAAA AAAAGGATCT
4901 CAAGAAGATC CTTTGATCTT TTCTACGGGG TCTGACGCTC AGTGGAACGA
4951 AAACTCACGT TAAGGGATTT TGGTCATGAG ATTATCAAAA AGGATCTTCA
5001 CCTAGATCCT TTTAAATTAA AAATGAAGTT TTAAATCAAT CTAAAGTATA
5051 TATGAGTAAA CTTGGTCTGA CAGTTACCAA TGCTTAATCA GTGAGGCACC
5101 TATCTCAGCG ATCTGTCTAT TTCGTTCATC CATAGTTGCC TGACTCCCCG
5151 TCGTGTAGAT AACTACGATA CGGGAGGGCT TACCATCTGG CCCCAGTGCT
5201 GCAATGATAC CGCGAGACCC ACGCTCACCG GCTCCAGATT TATCAGCAAT
5251 AAACCAGCCA GCCGGAAGGG CCGAGCGCAG AAGTGGTCCT GCAACTTTAT
5301 CCGCCTCCAT CCAGTCTATT AATTGTTGCC GGGAAGCTAG AGTAAGTAGT
5351 TCGCCAGTTA ATAGTTTGCG CAACGTTGTT GCCATTGCTG CAGGCATCGT
5401 GGTGTCACGC TCGTCGTTTG GTATGGCTTC ATTCAGCTCC GGTTCCCAAC
5451 GATCAAGGCG AGTTACATGA TCCCCCATGT TGTGCAAAAA AGCGGTTAGC
5501 TCCTTCGGTC CTCCGATCGT TGTCAGAAGT AAGTTGGCCG CAGTGTTATC
5551 ACTCATGGTT ATGGCAGCAC TGCATAATTC TCTTACTGTC ATGCCATCCG
5601 TAAGATGCTT TTCTGTGACT GGTGAGTACT CAACCAAGTC ATTCTGAGAA
5651 TAGTGTATGC GGCGACCGAG TTGCTCTTGC CCGGCGTCAA CACGGGATAA

5701 TACCGCGCCA CATAGCAGAA CTTTAAAAGT GCTCATCATT GGAAAACGTT
5751 CTTCGGGGCG AAAACTCTCA AGGATCTTAC CGCTGTTGAG ATCCAGTTCG
5801 ATGTAACCCA CTCGTGCACC CAACTGATCT TCAGCATCTT TTACTTTCAC
5851 CAGCGTTTCT GGGTGAGCAA AAACAGGAAG GCAAAATGCC GCAAAAAAGG
5901 GAATAAGGGC GACACGGAAA TGTTGAATAC TCATACTCTT CCTTTTTCAA
5951 TATTATTGAA GCATTTATCA GGGTTATTGT CTCATGAGCG GATACATATT
6001 TGAATGTATT TAGAAAAATA AACAAATAGG GGTTCCGCGC ACATTTCCCC
6051 GAAAAGTGCC ACCTGACGTC TAAGAAACCA TTATTATCAT GACATTAACC
6101 TATAAAAATA GGCGTATCAC GAGGCCCTTT CGTCTTCAAG AATTCAGCTG
6151 CTCGAGGAAG AGCTCAAACC CATGCTACTC TCTGGCTTGA TGGAAGCAAC
6201 GCTTTCATAG CTGAGCTGTC ATAAATAATA AAGAGATTTT TTTATTAATA
6251 TTGAAAAGAT GGGTTATTTA TGTAAGACTC TGTCTTCATT TTAAAAACCA
6301 CACCTTCCAG TAGTATTCTG TTACTGTTCT GGCAATCACT GTGATCAAGA
6351 AGCTACACGG TGAGTTGTGC TTCTCAGTCC TAAGGGATAC ATCTACAAGA
6401 GGCTCCCATA CTCGAAGCTC AGGAAACATT GTAGAAAAGG AGGCAAAAGA
6451 CTGACAGAGC CAGAGGACCA AGAAATTTGT TGTGAGGTTG TGTCTCCTAC
6501 TAACAATATA AGCAATATCT ATAAATTGTT GATATCATGG CTACTAAAAT
6551 GTGAGTTGAA CGAGGAGGAC ACAAATGAAC ATGACAATCA GAATGAGGCC
6601 TCTCACCTGC AAAAAACACT ATAGAGAAGC AGATAAAGCT GTCAGCAGAA
6651 GAGGCGCACC TCCTTATAGA AGAAGCCTAC CAGGTTTGAT ATATCAGCCT
6701 TGAAAACCTA CATAGTATTT ACATTATATC GAGTCTATGA GACATATTTA
6751 GTAATGCATA TGTATGTGTG TGTGTGCATG TATGTGTGTA AATACATATG
6801 TTCATAGAAA AATGTGTAAA AAGAGATCAT GAATTTAAGA GAGAACTGGG
6851 ACAATTTTTT TCAGGGAGTT GTAATCAGGA AAGTTAAGGG AAAAATGTTG
6901 TAATTAAAAT TCAGGCTCAG AAACAAACAA AGGAAAAGAA AAAAAAACAA
6951 CAACAACAAC AAAAAAACAA AACAAAGGAG AAGCTGTATG GCCACAATAG
7001 CATCTACAGC TAACTGTGAA AGGATAATGG AACAAGTTAT GTACTGCCTA
7051 GAGCAGTATG ATGCCTAAAT CATCTCGACA TGGAGGAAAA TAGAACAAAG
7101 ACACTCTACA TAGACTATGA TAGAAATCAA AATAAGGTGT AAGACATAGA
7151 ACATTAGTTT TGTTTGTTGT TCAAAGAGAC TCACATTCCC ACAAAAAAAT
7201 CTGTGGGATT TTACAGGTCT GCAATAAGCT GCTGACCTGA TGATTTCTGC
7251 AGCTGTGCCT ACCCTTTGCT GATTTGCATG TACCCAAAGC ATAGCTTACT
7301 GACATGAGGA TTTCTTCATA GTCAGGTCAC ACCCTTTGCT GGAGTCAGAA
7351 TCACACTGAT CACACACAGT CATGAGTGTG CTCACTCAGG TCCTGGCGTT
7401 GCTGCTGCTG TGGCTTACAG GTAATGAAGA CAGCACTAGA ATTTTATTGA
7451 GCTTCCTGTA CACTGTGCTG CTTGTCTCTG TGAAAATTCT CTTGTGAATT
7501 AATCATGTGG GGATCTGTTT TCAATTTTTC AATTGTAGGT ACGCGTTGTG

```
7551  ACATTCTGCT GACCCAGTCT CCAGCCACCC TGTCTCTGAG TCCAGGAGAA
7601  AGAGCCACTC TCTCCTGCAG GGCCAGTCAG AACATTGGCA CAAGCATACA
7651  GTGGTATCAA CAAAAACCAG GTCAGGCTCC AAGGCTTCTC ATAAGGTCTT
7701  CTTCTGAGTC TATCTCTGGG ATCCCTTCCA GGTTTAGTGG CAGTGGATCA
7751  GGGACAGATT TTACTCTTAC CATCAGCAGT CTGGAGCCTG AAGATTTTGC
7801  AGTGTATTAC TGTCAACAAA GTAATACCTG GCCATTCACG TTCGGCCAGG
7851  GGACCAAGCT TGAAATCAAA CGTAAGTAGA ATCCAAAGTC TCTTTCTTCC
7901  GTTGTCTATG TCTGTGGCTT CTATGTCTAA AAATGATGTA TAAAATCTTA
7951  CTCTGAAACC AGATTCTGGC ACTCTCCAAG GCAAAGATAC AGAGTAACTC
8001  CGTAAGCAAA GCTGGGAATA GGCTAGACAT GTTCTCTGGA GAATGAATGC
8051  CAGTGTAATA ATTAACACAA GTGATAGTTT CAGAAATGCT CTAGTT
```

**SEQ ID NO:37**

**Nucleotide sequence of the expression vector HCVHEN73DSp20**

```
   1 ctagagaggt ctggtggagc ctgcaaaagt ccagctttca aaggaacaca gaagtatgtg
  61 tatggaatat tagaagatgt tgcttttact cttaagttgg ttcctaggaa aaatagttaa
 121 atactgtgac tttaaaatgt gagagggttt tcaagtactc atttttttaa atgtccaaaa
 181 tttttgtcaa tcaatttgag gtcttgtttg tgtagaactg acattactta aagtttaacc
 241 gaggaatggg agtgaggctc tctcataccc tattcagaac tgacttttaa caataataaa
 301 ttaagtttaa aatattttta aatgaattga gcaatgttga gttggagtca agatggccga
 361 tcagaaccag aacacctgca gcagctggca ggaagcaggt catgtggcaa ggctatttgg
 421 ggaagggaaa ataaaaccac taggtaaact tgtagctgtg gtttgaagaa gtggttttga
 481 aacactctgt ccagccccac caaaccgaaa gtccaggctg agcaaaacac cacctgggta
 541 atttgcattt ctaaaataag ttgaggattc agccgaaact ggagaggtcc tcttttaact
 601 tattgagttc aacctttaa ttttagcttg agtagttcta gtttccccaa acttaagttt
 661 atcgacttct aaaatgtatt taagctttct ggggcaggcc aggcctgacc ttggctttgg
 721 ggcagggagg gggctaaggt gaggcaggtg gcgccagcca ggtgcacacc caatgcccat
 781 gagcccagac actggacgct gaacctcgcg gacagttaag aacccagggg cctctgcgcc
 841 ctgggcccag ctctgtccca caccgcggtc acatggcacc acctctcttg cagcctccac
 901 caagggccca tcggtcttcc ccctggcacc ctcctccaag agcacctctg ggggcacagc
 961 ggccctgggc tgcctggtca aggactactt ccccgaaccg gtgacggtgt cgtggaactc
1021 aggcgccctg accagcggcg tgcacacctt cccggctgtc ctacagtcct caggactcta
1081 ctccctcagc agcgtggtga ccgtgccctc cagcagcttg ggcacccaga cctacatctg
1141 caacgtgaat cacaagccca gcaacaccaa ggtggacaag agagttggtg agaggccagc
1201 acagggaggg agggtgtctg ctggaagcca ggctcagcgc tcctgcctgg acgcatcccg
1261 gctatgcagt cccagtccag ggcagcaagg caggccccgt ctgcctcttc acccggaggc
1321 ctctgcccgc cccactcatg ctcagggaga gggtcttctg gctttttccc caggctctgg
1381 gcaggcacag gctaggtgcc cctaacccag gccctgcaca caaaggggca ggtgctgggc
1441 tcagacctgc caagagccat atccgggagg accctgcccc tgacctaagc ccaccccaaa
```

1501 ggccaaactc tccactccct cagctcggac accttctctc ctcccagatt ccagtaactc
1561 ccaatcttct ctctgcagag cccaaatctt gtgacaaaac tcacacatgc ccaccgtgcc
1621 caggtaagcc agcccaggcc tcgccctcca gctcaaggcg ggacaggtgc cctagagtag
1681 cctgcatcca gggacaggcc ccagccgggt gctgacacgt ccacctccat ctcttcctca
1741 gcacctgaac tcctgggggg accgtcagtc ttcctcttcc ccccaaaacc caaggacacc
1801 ctcatgatct cccggacccc tgaggtcaca tgcgtggtgg tggacgtgag ccacgaagac
1861 cctgaggtca agttcaactg gtacgtggac ggcgtggagg tgcataatgc caagacaaag
1921 ccgcgggagg agcagtacaa cagcacgtac cgtgtggtca gcgtcctcac cgtcctgcac
1981 caggactggc tgaatggcaa ggagtacaag tgcaaggtct ccaacaaagc cctcccagcc
2041 cccatcgaga aaaccatctc caaagccaaa ggtgggaccc gtggggtgcg agggccacat
2101 ggacagaggc cggctcggcc caccctctgc cctgagagtg accgctgtac caacctctgt
2161 ccctacaggg cagccccgag aaccacaggt gtacaccctg cccccatccc gggaggagat
2221 gaccaagaac caggtcagcc tgacctgcct ggtcaaaggc ttctatccca gcgacatcgc
2281 cgtggagtgg gagagcaatg ggcagccgga gaacaactac aagaccacgc ctcccgtgct
2341 ggactccgac ggctccttct tcctctatag caagctcacc gtggacaaga gcaggtggca
2401 gcaggggaac gtcttctcat gctccgtgat gcatgaggct ctgcacaacc actacacgca
2461 gaagagcctc tccctgtccc cgggtaaatg agtgcgacgg ccggcaagcc cccgctcccc
2521 gggctctcgc ggtcgcacga ggatgcttgg cacgtacccc gtctacatac ttcccaggca
2581 cccagcatgg aaataaagca cccaccactg ccctgggccc ctgcgagact gtgatggttc
2641 tttccacggg tcaggccgag tctgaggcct gagtggcatg agggaggcag agcgggtccc
2701 actgtcccca cactggccca ggctgtgcag gtgtgcctgg gccgcctagg gtggggctca
2761 gccaggggct gccctcggca gggtggggga tttgccagcg tggccctccc tccagcagca
2821 gctgccctgg gctgggccac gagaagccct aggagcccct ggggacagac acacagcccc
2881 tgcctctgta ggagactgtc ctgtcctgtg agcgccctgt cctccgaccc cgatgcccac
2941 tcgggggcat gcctagtcca tgcgcgtagg gacaggccct ccctcaccca tctacccccca
3001 cggcactaac ccctggcagc cctgcccagc ctcgcacccg catggggaca caaccgactc
3061 cggggacatg cactctcggg ccctgtggag ggactggtgc agatgcccac acacacactc
3121 agcccagacc cgttcaacaa accccgcact gaggttggtc gagcgggagt gcggccagag
3181 cctgcctcgg ccgtcaggga ggactcccgg gctcactcga aggaggtgcc accatttcag
3241 ctttggtagc ttttcttctt cttttaaatt ttctaaagct cattaattgt ctttgatgtt
3301 tcttttgtga tgacaataaa atatcctttt taagtcttgt acttcgtgat gggagccgcc
3361 ttcctgtgtc cacgcgcctc ctgcccccgg tgggaagcac ggtcaggagg aggctggtcc
3421 agctgcacct cgggggctcc ctgcactcgc ccccgcctc ctgcagccac acgcattgcc
3481 cgagcgaccc tccctggccc ctgtcactac atggacccct ggggcttctc ctcttttcta
3541 catggatgca gtttctcctc ctgctgggca cggtgctgcc tgccctggtc actctgcggg
3601 ggacagggcc tccagggaaa gctgggtcga ggctgggagc tggctcaggc tggccaggca
3661 gagccacagg gagggccttc cagaaccaac catggtccga agcgagaggt gggtgtcaga
3721 tccagacatg ataagataca ttgatgagtt tggacaaacc acaactagaa tgcagtgaaa
3781 aaaatgcttt atttgtgaaa tttgtgatgc tattgcttta tttgtaacca ttataagctg
3841 caataaacaa gttaacaaca acaattgcat tcattttatg tttcaggttc agggggaggt
3901 gtgggaggtt ttttaaagca agtaaaacct ctacaaatgt ggtatggctg attatgatct

3961 ctagtcaagg cactatacat caaatattcc ttattaaccc ctttacaaat taaaaagcta
4021 aaggtacaca atttttgagc atagttatta atagcagaca ctctatgcct gtgtggagta
4081 agaaaaaaca gtatgttatg attataactg ttatgcctac ttataaaggt tacagaatat
4141 ttttccataa ttttcttgta tagcagtgca gctttttcct ttgtggtgta aatagcaaag
4201 caagcaagag ttctattact aaacacagca tgactcaaaa aacttagcaa ttctgaagga
4261 aagtccttgg ggtcttctac ctttctcttc ttttttggag gagtagaatg ttgagagtca
4321 gcagtagcct catcatcact agatggcatt tcttctgagc aaaacaggtt ttcctcatta
4381 aaggcattcc accactgctc ccattcatca gttccatagg ttggaatcta aaatacacaa
4441 acaattagaa tcagtagttt aacacattat acacttaaaa attttatatt taccttagag
4501 ctttaaatct ctgtaggtag tttgtccaat tatgtcacac cacagaagta aggttccttc
4561 acaaagatcc ggaccaaagc ggccatcgtg cctccccact cctgcagttc gggggcatgg
4621 atgcgcggat agccgctgct ggtttcctgg atgccgacgg atttgcactg ccggtagaac
4681 tccgcgaggt cgtccagcct caggcagcag ctgaaccaac tcgcgagggg atcgagcccg
4741 gggtgggcga agaactccag catgagatcc ccgcgctgga ggatcatcca gccggcgtcc
4801 cggaaaacga ttccgaagcc caacctttca tagaaggcgg cggtggaatc gaaatctcgt
4861 gatggcaggt tgggcgtcgc ttggtcggtc atttcgaacc ccagagtccc gctcagaaga
4921 actcgtcaag aaggcgatag aaggcgatgc gctgcgaatc gggagcggcg ataccgtaaa
4981 gcacgaggaa gcggtcagcc cattcgccgc caagctcttc agcaatatca cgggtagcca
5041 acgctatgtc ctgatagcgg tccgccacac ccagccggcc acagtcgatg aatccagaaa
5101 agcggccatt ttccaccatg atattcggca agcaggcatc gccatgggtc acgacgagat
5161 cctcgccgtc gggcatgcgc gccttgagcc tggcgaacag ttcggctggc gcgagcccct
5221 gatgctcttc gtccagatca tcctgatcga caagaccggc ttccatccga gtacgtgctc
5281 gctcgatgcg atgtttcgct tggtggtcga atgggcaggt agccggatca agcgtatgca
5341 gccgccgcat tgcatcagcc atgatggata ctttctcggc aggagcaagg tgagatgaca
5401 ggagatcctg ccccggcact tcgcccaata gcagccagtc ccttcccgct tcagtgacaa
5461 cgtcgagcac agctgcgcaa ggaacgcccg tcgtggccag ccacgatagc cgcgctgcct
5521 cgtcctgcag ttcattcagg gcaccggaca ggtcggtctt gacaaaaaga accgggcgcc
5581 cctgcgctga cagccggaac acggcggcat cagagcagcc gattgtctgt tgtgcccagt
5641 catagccgaa tagcctctcc acccaagcgg ccggagaacc tgcgtgcaat ccatcttgtt
5701 caatcatgcg aaacgatcct catcctgtct cttgatcaga tcttgatccc ctgcgccatc
5761 agatccttgg cggcaagaaa gccatccagt ttactttgca gggcttccca accttaccag
5821 agggcgcccc agctggcaat tccggttcgc ttgctgtcca taaaaccgcc cagtctagct
5881 atcgccatgt aagcccactg caagctacct gctttctctt tgcgcttgcg ttttcccttg
5941 tccagatagc ccagtagctg acattcatcc ggggtcagca ccgtttctgc ggactggctt
6001 tctacgtgtt ccgcttcctt tagcagccct tgcgccctga gtgcttgcgg cagcgtgaag
6061 ctttttgcaa aagcctaggc ctccaaaaaa gcctcctcac tacttctgga atagctcaga
6121 ggccgaggcg gcctcggcct ctgcataaat aaaaaaaatt agtcagccat ggggcggaga
6181 atgggcggaa ctgggcggag ttaggggcgg gatgggcgga gttaggggcg ggactatggt
6241 tgctgactaa ttgagatgca tgctttgcat acttctgcct gctggggagc ctggggactt
6301 tccacacctg gttgctgact aattgagatg catgctttgc atacttctgc ctgctgggga
6361 gcctggggac tttccacacc ctaactgaca cacattccac agctgcctcg cgcgtttcgg

6421 tgatgacggt gaaaacctct gacacatgca gctcccggag acggtcacag cttgtctgta
6481 agcggatgcc gggagcagac aagcccgtca gggcgcgtca gcgggtgttg gcgggtgtcg
6541 gggcgcagcc atgacccagt cacgtagcga tagcggagtg tatactggct taactatgcg
6601 gcatcagagc agattgtact gagagtgcac catatgcggt gtgaaatacc gcacagatgc
6661 gtaaggagaa aataccgcat caggcgctct tccgcttcct cgctcactga ctcgctgcgc
6721 tcggtcgttc ggctgcggcg agcggtatca gctcactcaa aggcggtaat acggttatcc
6781 acagaatcag gggataacgc aggaaagaac atgtgagcaa aaggccagca aaaggccagg
6841 aaccgtaaaa aggccgcgtt gctggcgttt ttccataggc tccgcccccc tgacgagcat
6901 cacaaaaatc gacgctcaag tcagaggtgg cgaaacccga caggactata aagataccag
6961 gcgtttcccc ctggaagctc cctcgtgcgc tctcctgttc cgaccctgcc gcttaccgga
7021 tacctgtccg cctttctccc ttcgggaagc gtggcgcttt ctcatagctc acgctgtagg
7081 tatctcagtt cggtgtaggt cgttcgctcc aagctgggct gtgtgcacga accccccgtt
7141 cagcccgacc gctgcgcctt atccggtaac tatcgtcttg agtccaaccc ggtaagacac
7201 gacttatcgc cactggcagc agccactggt aacaggatta gcagagcgag gtatgtaggc
7261 ggtgctacag agttcttgaa gtggtggcct aactacggct acactagaag gacagtattt
7321 ggtatctgcg ctctgctgaa gccagttacc ttcggaaaaa gagttggtag ctcttgatcc
7381 ggcaaacaaa ccaccgctgg tagcggtggt ttttttgttt gcaagcagca gattacgcgc
7441 agaaaaaaag gatctcaaga agatcctttg atcttttcta cggggtctga cgctcagtgg
7501 aacgaaaact cacgttaagg gattttggtc atgagattat caaaaaggat cttcacctag
7561 atccttttaa attaaaaatg aagttttaaa tcaatctaaa gtatatatga gtaaacttgg
7621 tctgacagtt accaatgctt aatcagtgag gcacctatct cagcgatctg tctatttcgt
7681 tcatccatag ttgcctgact ccccgtcgtg tagataacta cgatacggga gggcttacca
7741 tctggcccca gtgctgcaat gataccgcga gacccacgct caccggctcc agatttatca
7801 gcaataaacc agccagccgg aagggccgag cgcagaagtg gtcctgcaac tttatccgcc
7861 tccatccagt ctattaattg ttgccgggaa gctagagtaa gtagttcgcc agttaatagt
7921 ttgcgcaacg ttgttgccat tgctgcaggc atcgtggtgt cacgctcgtc gtttggtatg
7981 gcttcattca gctccggttc ccaacgatca aggcgagtta catgatcccc catgttgtgc
8041 aaaaaagcgg ttagctcctt cggtcctccg atcgttgtca gaagtaagtt ggccgcagtg
8101 ttatcactca tggttatggc agcactgcat aattctctta ctgtcatgcc atccgtaaga
8161 tgcttttctg tgactggtga gtactcaacc aagtcattct gagaatagtg tatgcggcga
8221 ccgagttgct cttgcccggc gtcaacacgg gataataccg cgccacatag cagaacttta
8281 aaagtgctca tcattggaaa acgttcttcg gggcgaaaac tctcaaggat cttaccgctg
8341 ttgagatcca gttcgatgta acccactcgt gcacccaact gatcttcagc atcttttact
8401 ttcaccagcg tttctgggtg agcaaaaaca ggaaggcaaa atgccgcaaa aaagggaata
8461 agggcgacac ggaaatgttg aatactcata ctcttccttt ttcaatatta ttgaagcatt
8521 tatcagggtt attgtctcat gagcggatac atatttgaat gtatttagaa aaataaacaa
8581 ataggggttc cgcgcacatt tccccgaaaa gtgccacctg acgtctaaga aaccattatt
8641 atcatgacat taacctataa aaataggcgt atcacgaggc cctttcgtct tcaagaattc
8701 gagctcggta cccatcagcc aaaaagcatg cctgccacac aacatcaatt tctggaaaac
8761 gctacactta attatttcta gtagaacagc tctttggttt gccaaaaaga atcacctata
8821 gtggcatcta agcacaaaaa ggagaaaaaa atcacaaaga aatgattgag aggcataata

8881 aaaattatca aaaaattatg agttttacga tttcatcttt ttccaagttg aaatcatagg
8941 gtggctttaa cacagtgaca aggaatgtgc atgctgccat tatggtgctc tgcctaaaat
9001 ggttggagcc ttgtcatgct acagagaaac tgtcatacag caggggggtgc caaatttcca
9061 tattttttta tatcattgag caggtgcaca gaagaccaga aagcactttc tatcaggctg
9121 gccttcctct tcctttccag tatgaagcaa aaactgccaa tgaaactagc aattgttaaa
9181 ttcctttttc aaacagtatt tgtgctatca gaacatagtg cattcaaaag tctagcctga
9241 gagaacaacc cagttttatt cattcctcct actacctctc tcattcccac tgtttgtgtt
9301 ctccctccca ttttaattgt ctatctagtc caaactaagc acacgatcca gtccacatta
9361 aacaacatgt tttcacttta agtcaaatac aagacacctt taatatcagc ccttgttcat
9421 aatcgtgctt ctagtgactt aatgtacatg tcacactgta ctgttgggtt ttgtgtctca
9481 tcatgaacaa tgttgtgaag gtattaagtg gagagtaagc agaattagat tcctctaatg
9541 atgcacaccc acactaagag cagaaataat attaaaaata gaaaaaaaag ttttacatga
9601 gatttcaaat acccaggtat gagctgcagt ttcttcaagt taaagcatcg aggttgtcag
9661 ttacactatt acaggaaaca tatgcagagt ttttatttta gtatattagt tttcacatat
9721 gtggaattac tattaaacta ttctttcttt tcaaatgctt accattgtaa atgagtttgt
9781 gactttgtgt aggtgagtgc acatgactct ggatgcctaa gaggactgaa gaagttggag
9841 ttataggtag ttttattcta cttgactgtt cagtgctaaa aatacaactg aggtccttta
9901 aactgctgtt catgaacttc ttaattgata tatctcatga gatctctaaa ctatttttat
9961 tatgacacgt ttcaccattt tcactgtaac gatttttatg ttttatatta atgtaactat
10021 atgacacttc ccaaaatccc catattcaca attgaactgt ttcaaagttt taccttgact
10081 tatgggaaat gaaaacccac atttataaat tttaaaatga aatgtttatt ttatatttct
10141 gcaaatttca caaggaaaga ttagtcactg gtgtgtgaga gcagaggagc ataagagttc
10201 aggaatagaa tccattatga ttctggaggc aaggaagaac tgatgccaag gtttcagtat
10261 aagagcagta tccactggaa aggataaagt cactacatct gagcacagag caggacatct
10321 acataatgag tggtcactaa tgggccactg ttacactgtt atatgtataa ggctcaagaa
10381 tgagcactga ggctgtaagg tgtatgggtg aggacatcag gatgtaaacc cagctcaggt
10441 agaggactca gaggacagca cagtcagcat gaactaataa acatcagata agataaggca
10501 caagctcagc tatatagggt aagggatctt tgtaaatctg attgtgcatc cagtctagtt
10561 caatgtgact taggaagccc agtcatatgc aaatctagag aagactttag agtagaaatc
10621 tgaggctcac ctcacatacc agcaagcgag tgaccagtta gtcttaaggc accacttctt
10681 agacatcatg gcttgggtgt ggaccttgcc attcctgatg gcagctgccc aaagtaagac
10741 atcagaaaaa agagttccaa ggggaattga agcagttcca tgaatactca ccttcctgtg
10801 ttcttttcac aggtgtccag gcagaggtgc agctggtgga gtcaggagcc gaagtgaaaa
10861 agcctggggc ttcagtgaag gtgtcctgca aggcctctgg atacacattc actaattata
10921 ttatccactg ggtgaagcag gagcctggtc agggccttga atggattgga tattttaatc
10981 cttacaatca tggtactaag tacaatgaga agttcaaagg cagggccaca ctaactgcag
11041 acaaatccat cagcacagcc tacatggagc tcagcagcct gcgctctgag gacactgcgg
11101 tctactactg tgcaagatca ggaccctatg cctggtttga cacctggggc caagggacca
11161 cggtcaccgt ctcctcaggt aagaatggcc actctagggc ctttgttttc tgctgctgcc
11221 tgtgggattt catgagcatt gcaaagttgt cctcgggaca tgttccgagg ggacctgggc
11281 ggactggcca ggaggggacg ggcactgggg tgccttgagg atctgggagc ctctgtggat

11341 tttccgatgc ctttggaaaa tgggactgag gttgggtgcg tctgagacag taactcagcc
11401 tgggggcttg gtgaagatcg ccgcacagca gcgagtccgt gaaatatctt atttagactt
11461 gtgaggtgcg ctgtgtgtca atttacatct taaatccttt attggctgga aagagaattg
11521 ttggagtggg tgaatccagc caggagggac gcggggggat cca

SEQ ID NO: 38

**Nucleotide sequence of the expression vector HCVHQN73DSp20**

1 CTAGAGAGGT CTGGTGGAGC CTGCAAAAGT CCAGCTTTCA AAGGAACACA GAAGTATGTG
61 TATGGAATAT TAGAAGATGT TGCTTTTACT CTTAAGTTGG TTCCTAGGAA AAATAGTTAA
121 ATACTGTGAC TTTAAAATGT GAGAGGGTTT TCAAGTACTC ATTTTTTTAA ATGTCCAAAA
181 TTTTTGTCAA TCAATTTGAG GTCTTGTTTG TGTAGAACTG ACATTACTTA AAGTTTAACC
241 GAGGAATGGG AGTGAGGCTC TCTCATACCC TATTCAGAAC TGACTTTTAA CAATAATAAA
301 TTAAGTTTAA AATATTTTTA AATGAATTGA GCAATGTTGA GTTGGAGTCA AGATGGCCGA
361 TCAGAACCAG AACACCTGCA GCAGCTGGCA GGAAGCAGGT CATGTGGCAA GGCTATTTGG
421 GGAAGGGAAA ATAAAACCAC TAGGTAAACT TGTAGCTGTG GTTTGAAGAA GTGGTTTTGA
481 AACACTCTGT CCAGCCCCAC CAAACCGAAA GTCCAGGCTG AGCAAAACAC CACCTGGGTA
541 ATTTGCATTT CTAAAATAAG TTGAGGATTC AGCCGAAACT GGAGAGGTCC TCTTTTAACT
601 TATTGAGTTC AACCTTTTAA TTTTAGCTTG AGTAGTTCTA GTTTCCCCAA ACTTAAGTTT
661 ATCGACTTCT AAAATGTATT TAAGCTTTCT GGGGCAGGCC AGGCCTGACC TTGGCTTTGG
721 GGCAGGGAGG GGGCTAAGGT GAGGCAGGTG GCGCCAGCCA GGTGCACACC CAATGCCCAT
781 GAGCCCAGAC ACTGGACGCT GAACCTCGCG GACAGTTAAG AACCCAGGGG CCTCTGCGCC
841 CTGGGCCCAG CTCTGTCCCA CACCGCGGTC ACATGGCACC ACCTCTCTTG CAGCCTCCAC
901 CAAGGGCCCA TCGGTCTTCC CCCTGGCACC CTCCTCCAAG AGCACCTCTG GGGGCACAGC
961 GGCCCTGGGC TGCCTGGTCA AGGACTACTT CCCCGAACCG GTGACGGTGT CGTGGAACTC
1021 AGGCGCCCTG ACCAGCGGCG TGCACACCTT CCCGGCTGTC CTACAGTCCT CAGGACTCTA
1081 CTCCCTCAGC AGCGTGGTGA CCGTGCCCTC CAGCAGCTTG GGCACCCAGA CCTACATCTG
1141 CAACGTGAAT CACAAGCCCA GCAACACCAA GGTGGACAAG AGAGTTGGTG AGAGGCCAGC
1201 ACAGGGAGGG AGGGTGTCTG CTGGAAGCCA GGCTCAGCGC TCCTGCCTGG ACGCATCCCG
1261 GCTATGCAGT CCCAGTCCAG GGCAGCAAGG CAGGCCCCGT CTGCCTCTTC ACCCGGAGGC
1321 CTCTGCCCGC CCCACTCATG CTCAGGGAGA GGGTCTTCTG GCTTTTTCCC CAGGCTCTGG
1381 GCAGGCACAG GCTAGGTGCC CCTAACCCAG GCCCTGCACA CAAAGGGGCA GGTGCTGGGC
1441 TCAGACCTGC CAAGAGCCAT ATCCGGGAGG ACCCTGCCCC TGACCTAAGC CCACCCCAAA
1501 GGCCAAACTC TCCACTCCCT CAGCTCGGAC ACCTTCTCTC CTCCCAGATT CCAGTAACTC
1561 CCAATCTTCT CTCTGCAGAG CCCAAATCTT GTGACAAAAC TCACACATGC CCACCGTGCC
1621 CAGGTAAGCC AGCCCAGGCC TCGCCCTCCA GCTCAAGGCG GGACAGGTGC CCTAGAGTAG
1681 CCTGCATCCA GGGACAGGCC CCAGCCGGGT GCTGACACGT CCACCTCCAT CTCTTCCTCA
1741 GCACCTGAAC TCCTGGGGGG ACCGTCAGTC TTCCTCTTCC CCCCAAAACC CAAGGACACC
1801 CTCATGATCT CCCGGACCCC TGAGGTCACA TGCGTGGTGG TGGACGTGAG CCACGAAGAC
1861 CCTGAGGTCA AGTTCAACTG GTACGTGGAC GGCGTGGAGG TGCATAATGC CAAGACAAAG
1921 CCGCGGGAGG AGCAGTACAA CAGCACGTAC CGTGTGGTCA GCGTCCTCAC CGTCCTGCAC
1981 CAGGACTGGC TGAATGGCAA GGAGTACAAG TGCAAGGTCT CCAACAAAGC CCTCCCAGCC

```
2041   CCCATCGAGA AAACCATCTC CAAAGCCAAA GGTGGGACCC GTGGGGTGCG AGGGCCACAT
2101   GGACAGAGGC CGGCTCGGCC CACCCTCTGC CCTGAGAGTG ACCGCTGTAC CAACCTCTGT
2161   CCCTACAGGG CAGCCCCGAG AACCACAGGT GTACACCCTG CCCCCATCCC GGGAGGAGAT
2221   GACCAAGAAC CAGGTCAGCC TGACCTGCCT GGTCAAAGGC TTCTATCCCA GCGACATCGC
2281   CGTGGAGTGG GAGAGCAATG GGCAGCCGGA GAACAACTAC AAGACCACGC CTCCCGTGCT
2341   GGACTCCGAC GGCTCCTTCT TCCTCTATAG CAAGCTCACC GTGGACAAGA GCAGGTGGCA
2401   GCAGGGGAAC GTCTTCTCAT GCTCCGTGAT GCATGAGGCT CTGCACAACC ACTACACGCA
2461   GAAGAGCCTC TCCCTGTCCC CGGGTAAATG AGTGCGACGG CCGGCAAGCC CCCGCTCCCC
2521   GGGCTCTCGC GGTCGCACGA GGATGCTTGG CACGTACCCC GTCTACATAC TTCCCAGGCA
2581   CCCAGCATGG AAATAAAGCA CCCACCACTG CCCTGGGCCC CTGCGAGACT GTGATGGTTC
2641   TTTCCACGGG TCAGGCCGAG TCTGAGGCCT GAGTGGCATG AGGGAGGCAG AGCGGGTCCC
2701   ACTGTCCCCA CACTGGCCCA GGCTGTGCAG GTGTGCCTGG GCCGCCTAGG GTGGGGCTCA
2761   GCCAGGGGCT GCCCTCGGCA GGGTGGGGGA TTTGCCAGCG TGGCCCTCCC TCCAGCAGCA
2821   GCTGCCCTGG GCTGGGCCAC GAGAAGCCCT AGGAGCCCCT GGGGACAGAC ACACAGCCCC
2881   TGCCTCTGTA GGAGACTGTC CTGTCCTGTG AGCGCCCTGT CCTCCGACCC CGATGCCCAC
2941   TCGGGGGCAT GCCTAGTCCA TGCGCGTAGG GACAGGCCCT CCCTCACCCA TCTACCCCCA
3001   CGGCACTAAC CCCTGGCAGC CCTGCCCAGC CTCGCACCCG CATGGGGACA CAACCGACTC
3061   CGGGGACATG CACTCTCGGG CCCTGTGGAG GGACTGGTGC AGATGCCCAC ACACACACTC
3121   AGCCCAGACC CGTTCAACAA ACCCCGCACT GAGGTTGGTC GAGCGGGAGT GCGGCCAGAG
3181   CCTGCCTCGG CCGTCAGGGA GGACTCCCGG GCTCACTCGA AGGAGGTGCC ACCATTTCAG
3241   CTTTGGTAGC TTTTCTTCTT CTTTTAAATT TTCTAAAGCT CATTAATTGT CTTTGATGTT
3301   TCTTTTGTGA TGACAATAAA ATATCCTTTT TAAGTCTTGT ACTTCGTGAT GGGAGCCGCC
3361   TTCCTGTGTC CACGCGCCTC CTGCCCCCGG TGGGAAGCAC GGTCAGGAGG AGGCTGGTCC
3421   AGCTGCACCT CGGGGGCTCC CTGCACTCGC CCCCCGCCTC CTGCAGCCAC ACGCATTGCC
3481   CGAGCGACCC TCCCTGGCCC CTGTCACTAC ATGGACCCCT GGGGCTTCTC CTCTTTTCTA
3541   CATGGATGCA GTTTCTCCTC CTGCTGGGCA CGGTGCTGCC TGCCCTGGTC ACTCTGCGGG
3601   GGACAGGGCC TCCAGGGAAA GCTGGGTCGA GGCTGGGAGC TGGCTCAGGC TGGCCAGGCA
3661   GAGCCACAGG GAGGGCCTTC CAGAACCAAC CATGGTCCGA AGCGAGAGGT GGGTGTCAGA
3721   TCCAGACATG ATAAGATACA TTGATGAGTT TGGACAAACC ACAACTAGAA TGCAGTGAAA
3781   AAAATGCTTT ATTTGTGAAA TTTGTGATGC TATTGCTTTA TTTGTAACCA TTATAAGCTG
3841   CAATAAACAA GTTAACAACA ACAATTGCAT TCATTTTATG TTTCAGGTTC AGGGGGAGGT
3901   GTGGGAGGTT TTTTAAAGCA AGTAAAACCT CTACAAATGT GGTATGGCTG ATTATGATCT
3961   CTAGTCAAGG CACTATACAT CAAATATTCC TTATTAACCC CTTTACAAAT TAAAAAGCTA
4021   AAGGTACACA ATTTTTGAGC ATAGTTATTA ATAGCAGACA CTCTATGCCT GTGTGGAGTA
4081   AGAAAAAACA GTATGTTATG ATTATAACTG TTATGCCTAC TTATAAAGGT TACAGAATAT
4141   TTTTCCATAA TTTTCTTGTA TAGCAGTGCA GCTTTTTCCT TTGTGGTGTA AATAGCAAAG
4201   CAAGCAAGAG TTCTATTACT AAACACAGCA TGACTCAAAA AACTTAGCAA TTCTGAAGGA
4261   AAGTCCTTGG GGTCTTCTAC CTTTCTCTTC TTTTTTGGAG GAGTAGAATG TTGAGAGTCA
4321   GCAGTAGCCT CATCATCACT AGATGGCATT TCTTCTGAGC AAAACAGGTT TTCCTCATTA
4381   AAGGCATTCC ACCACTGCTC CCATTCATCA GTTCCATAGG TTGGAATCTA AAATACACAA
4441   ACAATTAGAA TCAGTAGTTT AACACATTAT ACACTTAAAA ATTTTATATT TACCTTAGAG
```

4501 CTTTAAATCT CTGTAGGTAG TTTGTCCAAT TATGTCACAC CACAGAAGTA AGGTTCCTTC
4561 ACAAAGATCC GGACCAAAGC GGCCATCGTG CCTCCCCACT CCTGCAGTTC GGGGGCATGG
4621 ATGCGCGGAT AGCCGCTGCT GGTTTCCTGG ATGCCGACGG ATTTGCACTG CCGGTAGAAC
4681 TCCGCGAGGT CGTCCAGCCT CAGGCAGCAG CTGAACCAAC TCGCGAGGGG ATCGAGCCCG
4741 GGGTGGGCGA AGAACTCCAG CATGAGATCC CCGCGCTGGA GGATCATCCA GCCGGCGTCC
4801 CGGAAAACGA TTCCGAAGCC CAACCTTTCA TAGAAGGCGG CGGTGGAATC GAAATCTCGT
4861 GATGGCAGGT TGGGCGTCGC TTGGTCGGTC ATTTCGAACC CCAGAGTCCC GCTCAGAAGA
4921 ACTCGTCAAG AAGGCGATAG AAGGCGATGC GCTGCGAATC GGGAGCGGCG ATACCGTAAA
4981 GCACGAGGAA GCGGTCAGCC CATTCGCCGC CAAGCTCTTC AGCAATATCA CGGGTAGCCA
5041 ACGCTATGTC CTGATAGCGG TCCGCCACAC CCAGCCGGCC ACAGTCGATG AATCCAGAAA
5101 AGCGGCCATT TTCCACCATG ATATTCGGCA AGCAGGCATC GCCATGGGTC ACGACGAGAT
5161 CCTCGCCGTC GGGCATGCGC GCCTTGAGCC TGGCGAACAG TTCGGCTGGC GCGAGCCCCT
5221 GATGCTCTTC GTCCAGATCA TCCTGATCGA CAAGACCGGC TTCCATCCGA GTACGTGCTC
5281 GCTCGATGCG ATGTTTCGCT TGGTGGTCGA ATGGGCAGGT AGCCGGATCA AGCGTATGCA
5341 GCCGCCGCAT TGCATCAGCC ATGATGGATA CTTTCTCGGC AGGAGCAAGG TGAGATGACA
5401 GGAGATCCTG CCCCGGCACT TCGCCCAATA GCAGCCAGTC CCTTCCCGCT TCAGTGACAA
5461 CGTCGAGCAC AGCTGCGCAA GGAACGCCCG TCGTGGCCAG CCACGATAGC CGCGCTGCCT
5521 CGTCCTGCAG TTCATTCAGG GCACCGGACA GGTCGGTCTT GACAAAAAGA ACCGGGCGCC
5581 CCTGCGCTGA CAGCCGGAAC ACGGCGGCAT CAGAGCAGCC GATTGTCTGT TGTGCCCAGT
5641 CATAGCCGAA TAGCCTCTCC ACCCAAGCGG CCGGAGAACC TGCGTGCAAT CCATCTTGTT
5701 CAATCATGCG AAACGATCCT CATCCTGTCT CTTGATCAGA TCTTGATCCC CTGCGCCATC
5761 AGATCCTTGG CGGCAAGAAA GCCATCCAGT TTACTTTGCA GGGCTTCCCA ACCTTACCAG
5821 AGGGCGCCCC AGCTGGCAAT TCCGGTTCGC TTGCTGTCCA TAAAACCGCC CAGTCTAGCT
5881 ATCGCCATGT AAGCCCACTG CAAGCTACCT GCTTTCTCTT TGCGCTTGCG TTTTCCCTTG
5941 TCCAGATAGC CCAGTAGCTG ACATTCATCC GGGGTCAGCA CCGTTTCTGC GGACTGGCTT
6001 TCTACGTGTT CCGCTTCCTT TAGCAGCCCT TGCGCCCTGA GTGCTTGCGG CAGCGTGAAG
6061 CTTTTTGCAA AAGCCTAGGC CTCCAAAAAA GCCTCCTCAC TACTTCTGGA ATAGCTCAGA
6121 GGCCGAGGCG GCCTCGGCCT CTGCATAAAT AAAAAAAATT AGTCAGCCAT GGGGCGGAGA
6181 ATGGGCGGAA CTGGGCGGAG TTAGGGGCGG GATGGGCGGA GTTAGGGGCG GGACTATGGT
6241 TGCTGACTAA TTGAGATGCA TGCTTTGCAT ACTTCTGCCT GCTGGGGAGC CTGGGGACTT
6301 TCCACACCTG GTTGCTGACT AATTGAGATG CATGCTTTGC ATACTTCTGC CTGCTGGGGA
6361 GCCTGGGGAC TTTCCACACC CTAACTGACA CACATTCCAC AGCTGCCTCG CGCGTTTCGG
6421 TGATGACGGT GAAAACCTCT GACACATGCA GCTCCCGGAG ACGGTCACAG CTTGTCTGTA
6481 AGCGGATGCC GGGAGCAGAC AAGCCCGTCA GGGCGCGTCA GCGGGTGTTG GCGGGTGTCG
6541 GGGCGCAGCC ATGACCCAGT CACGTAGCGA TAGCGGAGTG TATACTGGCT TAACTATGCG
6601 GCATCAGAGC AGATTGTACT GAGAGTGCAC CATATGCGGT GTGAAATACC GCACAGATGC
6661 GTAAGGAGAA AATACCGCAT CAGGCGCTCT TCCGCTTCCT CGCTCACTGA CTCGCTGCGC
6721 TCGGTCGTTC GGCTGCGGCG AGCGGTATCA GCTCACTCAA AGGCGGTAAT ACGGTTATCC
6781 ACAGAATCAG GGGATAACGC AGGAAAGAAC ATGTGAGCAA AAGGCCAGCA AAAGGCCAGG
6841 AACCGTAAAA AGGCCGCGTT GCTGGCGTTT TTCCATAGGC TCCGCCCCCC TGACGAGCAT
6901 CACAAAAATC GACGCTCAAG TCAGAGGTGG CGAAACCCGA CAGGACTATA AAGATACCAG

6961 GCGTTTCCCC CTGGAAGCTC CCTCGTGCGC TCTCCTGTTC CGACCCTGCC GCTTACCGGA
7021 TACCTGTCCG CCTTTCTCCC TTCGGGAAGC GTGGCGCTTT CTCATAGCTC ACGCTGTAGG
7081 TATCTCAGTT CGGTGTAGGT CGTTCGCTCC AAGCTGGGCT GTGTGCACGA ACCCCCCGTT
7141 CAGCCCGACC GCTGCGCCTT ATCCGGTAAC TATCGTCTTG AGTCCAACCC GGTAAGACAC
7201 GACTTATCGC CACTGGCAGC AGCCACTGGT AACAGGATTA GCAGAGCGAG GTATGTAGGC
7261 GGTGCTACAG AGTTCTTGAA GTGGTGGCCT AACTACGGCT ACACTAGAAG GACAGTATTT
7321 GGTATCTGCG CTCTGCTGAA GCCAGTTACC TTCGGAAAAA GAGTTGGTAG CTCTTGATCC
7381 GGCAAACAAA CCACCGCTGG TAGCGGTGGT TTTTTTGTTT GCAAGCAGCA GATTACGCGC
7441 AGAAAAAAAG GATCTCAAGA AGATCCTTTG ATCTTTTCTA CGGGGTCTGA CGCTCAGTGG
7501 AACGAAAACT CACGTTAAGG GATTTTGGTC ATGAGATTAT CAAAAAGGAT CTTCACCTAG
7561 ATCCTTTTAA ATTAAAAATG AAGTTTTAAA TCAATCTAAA GTATATATGA GTAAACTTGG
7621 TCTGACAGTT ACCAATGCTT AATCAGTGAG GCACCTATCT CAGCGATCTG TCTATTTCGT
7681 TCATCCATAG TTGCCTGACT CCCCGTCGTG TAGATAACTA CGATACGGGA GGGCTTACCA
7741 TCTGGCCCCA GTGCTGCAAT GATACCGCGA GACCCACGCT CACCGGCTCC AGATTTATCA
7801 GCAATAAACC AGCCAGCCGG AAGGGCCGAG CGCAGAAGTG GTCCTGCAAC TTTATCCGCC
7861 TCCATCCAGT CTATTAATTG TTGCCGGGAA GCTAGAGTAA GTAGTTCGCC AGTTAATAGT
7921 TTGCGCAACG TTGTTGCCAT TGCTGCAGGC ATCGTGGTGT CACGCTCGTC GTTTGGTATG
7981 GCTTCATTCA GCTCCGGTTC CCAACGATCA AGGCGAGTTA CATGATCCCC CATGTTGTGC
8041 AAAAAAGCGG TTAGCTCCTT CGGTCCTCCG ATCGTTGTCA GAAGTAAGTT GGCCGCAGTG
8101 TTATCACTCA TGGTTATGGC AGCACTGCAT AATTCTCTTA CTGTCATGCC ATCCGTAAGA
8161 TGCTTTTCTG TGACTGGTGA GTACTCAACC AAGTCATTCT GAGAATAGTG TATGCGGCGA
8221 CCGAGTTGCT CTTGCCCGGC GTCAACACGG GATAATACCG CGCCACATAG CAGAACTTTA
8281 AAAGTGCTCA TCATTGGAAA ACGTTCTTCG GGGCGAAAAC TCTCAAGGAT CTTACCGCTG
8341 TTGAGATCCA GTTCGATGTA ACCCACTCGT GCACCCAACT GATCTTCAGC ATCTTTTACT
8401 TTCACCAGCG TTTCTGGGTG AGCAAAAACA GGAAGGCAAA ATGCCGCAAA AAAGGGAATA
8461 AGGGCGACAC GGAAATGTTG AATACTCATA CTCTTCCTTT TTCAATATTA TTGAAGCATT
8521 TATCAGGGTT ATTGTCTCAT GAGCGGATAC ATATTTGAAT GTATTTAGAA AAATAAACAA
8581 ATAGGGGTTC CGCGCACATT TCCCCGAAAA GTGCCACCTG ACGTCTAAGA AACCATTATT
8641 ATCATGACAT TAACCTATAA AAATAGGCGT ATCACGAGGC CCTTTCGTCT TCAAGAATTC
8701 GAGCTCGGTA CCCATCAGCC AAAAAGCATG CCTGCCACAC AACATCAATT TCTGGAAAAC
8761 GCTACACTTA ATTATTTCTA GTAGAACAGC TCTTTGGTTT GCCAAAAAGA ATCACCTATA
8821 GTGGCATCTA AGCACAAAAA GGAGAAAAAA ATCACAAAGA AATGATTGAG AGGCATAATA
8881 AAAATTATCA AAAAATTATG AGTTTTACGA TTTCATCTTT TTCCAAGTTG AAATCATAGG
8941 GTGGCTTTAA CACAGTGACA AGGAATGTGC ATGCTGCCAT TATGGTGCTC TGCCTAAAAT
9001 GGTTGGAGCC TTGTCATGCT ACAGAGAAAC TGTCATACAG CAGGGGGTGC CAAATTTCCA
9061 TATTTTTTTA TATCATTGAG CAGGTGCACA GAAGACCAGA AAGCACTTTC TATCAGGCTG
9121 GCCTTCCTCT TCCTTTCCAG TATGAAGCAA AAACTGCCAA TGAAACTAGC AATTGTTAAA
9181 TTCCTTTTTC AAACAGTATT TGTGCTATCA GAACATAGTG CATTCAAAAG TCTAGCCTGA
9241 GAGAACAACC CAGTTTTATT CATTCCTCCT ACTACCTCTC TCATTCCCAC TGTTTGTGTT
9301 CTCCCTCCCA TTTTAATTGT CTATCTAGTC CAAACTAAGC ACACGATCCA GTCCACATTA
9361 AACAACATGT TTTCACTTTA AGTCAAATAC AAGACACCTT TAATATCAGC CCTTGTTCAT

9421 AATCGTGCTT CTAGTGACTT AATGTACATG TCACACTGTA CTGTTGGGTT TTGTGTCTCA
9481 TCATGAACAA TGTTGTGAAG GTATTAAGTG GAGAGTAAGC AGAATTAGAT TCCTCTAATG
9541 ATGCACACCC ACACTAAGAG CAGAAATAAT ATTAAAAATA GAAAAAAAAG TTTTACATGA
9601 GATTTCAAAT ACCCAGGTAT GAGCTGCAGT TTCTTCAAGT TAAAGCATCG AGGTTGTCAG
9661 TTACACTATT ACAGGAAACA TATGCAGAGT TTTTATTTTA GTATATTAGT TTTCACATAT
9721 GTGGAATTAC TATTAAACTA TTCTTTCTTT TCAAATGCTT ACCATTGTAA ATGAGTTTGT
9781 GACTTTGTGT AGGTGAGTGC ACATGACTCT GGATGCCTAA GAGGACTGAA GAAGTTGGAG
9841 TTATAGGTAG TTTTATTCTA CTTGACTGTT CAGTGCTAAA AATACAACTG AGGTCCTTTA
9901 AACTGCTGTT CATGAACTTC TTAATTGATA TATCTCATGA GATCTCTAAA CTATTTTTAT
9961 TATGACACGT TTCACCATTT TCACTGTAAC GATTTTTATG TTTTATATTA ATGTAACTAT
10021 ATGACACTTC CCAAAATCCC CATATTCACA ATTGAACTGT TTCAAAGTTT TACCTTGACT
10081 TATGGGAAAT GAAAACCCAC ATTTTATAAT TTTAAAATGA AATGTTTATT TTATATTTCT
10141 GCAAATTTCA CAAGGAAAGA TTAGTCACTG GTGTGTGAGA GCAGAGGAGC ATAAGAGTTC
10201 AGGAATAGAA TCCATTATGA TTCTGGAGGC AAGGAAGAAC TGATGCCAAG GTTTCAGTAT
10261 AAGAGCAGTA TCCACTGGAA AGGATAAAGT CACTACATCT GAGCACAGAG CAGGACATCT
10321 ACATAATGAG TGGTCACTAA TGGGCCACTG TTACACTGTT ATATGTATAA GGCTCAAGAA
10381 TGAGCACTGA GGCTGTAAGG TGTATGGGTG AGGACATCAG GATGTAAACC CAGCTCAGGT
10441 AGAGGACTCA GAGGACAGCA CAGTCAGCAT GAACTAATAA ACATCAGATA AGATAAGGCA
10501 CAAGCTCAGC TATATAGGGT AAGGGATCTT TGTAAATCTG ATTGTGCATC CAGTCTAGTT
10561 CAATGTGACT TAGGAAGCCC AGTCATATGC AAATCTAGAG AAGACTTTAG AGTAGAAATC
10621 TGAGGCTCAC CTCACATACC AGCAAGCGAG TGACCAGTTA GTCTTAAGGC ACCACTTCTT
10681 AGACATCATG GCTTGGGTGT GGACCTTGCC ATTCCTGATG GCAGCTGCCC AAAGTAAGAC
10741 ATCAGAAAAA AGAGTTCCAA GGGGAATTGA AGCAGTTCCA TGAATACTCA CCTTCCTGTG
10801 TTCTTTTCAC AGGTGTCCAG GCACAGGTGC AGCTGGTGGA GTCAGGAGCC GAAGTGAAAA
10861 AGCCTGGGGC TTCAGTGAAG GTGTCCTGCA AGGCCTCTGG ATACACATTC ACTAATTATA
10921 TTATCCACTG GGTGAAGCAG GAGCCTGGTC AGGGCCTTGA ATGGATTGGA TATTTTAATC
10981 CTTACAATCA TGGTACTAAG TACAATGAGA AGTTCAAAGG CAGGGCCACA CTAACTGCAG
11041 ACAAATCCAT CAGCACAGCC TACATGGAGC TCAGCAGCCT GCGCTCTGAG GACACTGCGG
11101 TCTACTACTG TGCAAGATCA GGACCCTATG CCTGGTTTGA CACCTGGGGC CAAGGGACCA
11161 CGGTCACCGT CTCCTCAGGT AAGAATGGCC ACTCTAGGGC CTTTGTTTTC TGCTGCTGCC
11221 TGTGGGATTT CATGAGCATT GCAAAGTTGT CCTCGGGACA TGTTCCGAGG GGACCTGGGC
11281 GGACTGGCCA GGAGGGGACG GGCACTGGGG TGCCTTGAGG ATCTGGGAGC CTCTGTGGAT
11341 TTTCCGATGC CTTTGGAAAA TGGGACTGAG GTTGGGTGCG TCTGAGACAG TAACTCAGCC
11401 TGGGGGCTTG GTGAAGATCG CCGCACAGCA GCGAGTCCGT GAAATATCTT ATTTAGACTT
11461 GTGAGGTGCG CTGTGTGTCA ATTTACATCT TAAATCCTTT ATTGGCTGGA AAGAGAATTG
11521 TTGGAGTGGG TGAATCCAGC CAGGAGGGAC GCGGGGGGAT CCA

**<u>SEQ ID NO: 39</u>**

**Nucleotide sequence of expression vector LCVL2Sp20**

1 CTAGAGTCCT AGAGAGGTCT GGTGGAGCCT GCAAAAGTCC AGCTTTCAAA
51 GGAACACAGA AGTATGTGTA TGGAATATTA GAAGATGTTG CTTTTACTCT

101 TAAGTTGGTT CCTAGGAAAA ATAGTTAAAT ACTGTGACTT TAAAATGTGA
151 GAGGGTTTTC AAGTACTCAT TTTTTTAAAT GTCCAAAATT TTTGTCAATC
201 AATTTGAGGT CTTGTTTGTG TAGAACTGAC ATTACTTAAA GTTTAACCGA
251 GGAATGGGAG TGAGGCTCTC TCATACCCTA TTCAGAACTG ACTTTTAACA
301 ATAATAAATT AAGTTTAAAA TATTTTTAAA TGAATTGAGC AATGTTGAGT
351 TGGAGTCAAG ATGGCCGATC AGAACCAGAA CACCTGCAGC AGCTGGCAGG
401 AAGCAGGTCA TGTGGCAAGG CTATTTGGGG AAGGGAAAAT AAAACCACTA
451 GGTAAACTTG TAGCTGTGGT TTGAAGAAGT GGTTTTGAAA CACTCTGTCC
501 AGCCCCACCA AACCGAAAGT CCAGGCTGAG CAAAACACCA CCTGGGTAAT
551 TTGCATTTCT AAAATAAGTT GAGGATTCAG CCGAAACTGG AGAGGTCCTC
601 TTTTAACTTA TTGAGTTCAA CCTTTTAATT TTAGCTTGAG TAGTTCTAGT
651 TTCCCCAAAC TTAAGTTTAT CGACTTCTAA AATGTATTTA GAACTCATTT
701 TCAAAATTAG GTTATGTAAG AAATTGAAGG ACTTTAGTGT CTTTAATTTC
751 TAATATATTT AGAAAACTTC TTAAAATTAC TCTATTATTC TTCCCTCTGA
801 TTATTGGTCT CCATTCAATT CTTTTCCAAT ACCCGAAGCA TTTACAGTGA
851 CTTTGTTCAT GATCTTTTTT AGTTGTTTGT TTTGCCTTAC TATTAAGACT
901 TTGACATTCT GGTCAAAACG GCTTCACAAA TCTTTTTCAA GACCACTTTC
951 TGAGTATTCA TTTTAGGAGA AATACTTTTT TTTTAAATGA ATGCAATTAT
1001 CTAGGACCTG CAGGCATGCT GTTTTCTGTC TGTCCCTAAC ATGCCCTGTG
1051 ATTATCCGCA AACAACACAC CCAAGGGCAG AACTTTGTTA CTTAAACACC
1101 ATCCTGTTTG CTTCTTTCCT CAGGAACTGT GGCTGCACCA TCTGTCTTCA
1151 TCTTCCCGCC ATCTGATGAG CAGTTGAAAT CTGGAACTGC CTCTGTTGTG
1201 TGCCTGCTGA ATAACTTCTA TCCCAGAGAG GCCAAAGTAC AGTGGAAGGT
1251 GGATAACGCC CTCCAATCGG GTAACTCCCA GGAGAGTGTC ACAGAGCAGG
1301 ACAGCAAGGA CAGCACCTAC AGCCTCAGCA GCACCCTGAC GCTGAGCAAA
1351 GCAGACTACG AGAAACACAA AGTCTACGCC TGCGAAGTCA CCCATCAGGG
1401 CCTGAGCTCG CCCGTCACAA AGAGCTTCAA CAGGGGAGAG TGTTAGAGGG
1451 AGAAGTGCCC CCACCTGCTC CTCAGTTCCA GCCTGACCCC CTCCCATCCT
1501 TTGGCCTCTG ACCCTTTTTC CACAGGGGAC CTACCCCTAT TGCGGTCCTC
1551 CAGCTCATCT TTCACCTCAC CCCCCTCCTC CTCCTTGGCT TTAATTATGC
1601 TAATGTTGGA GGAGAATGAA TAAATAAAGT GAATCTTTGC ACCTGTGGTT
1651 TCTCTCTTTC CTCATTTAAT AATTATTATC TGTTGTTTTA CCAACTACTC
1701 AATTTCTCTT ATAAGGGACT AAATATGTAG TCATCCTAAG GCGGGATATC
1751 GAGATCTGAA GCTGATCCAG ACATGATAAG ATACATTGAT GAGTTTGGAC
1801 AAACCACAAC TAGAATGCAG TGAAAAAAAT GCTTTATTTG TGAAATTTGT
1851 GATGCTATTG CTTTATTTGT AACCATTATA AGCTGCAATA AACAAGTTAA
1901 CAACAACAAT TGCATTCATT TTATGTTTCA GGTTCAGGGG GAGGTGTGGG
1951 AGGTTTTTTA AAGCAAGTAA AACCTCTACA AATGTGGTAT GGCTGATTAT
2001 GATCTCTAGT CAAGGCACTA TACATCAAAT ATTCCTTATT AACCCCTTTA
2051 CAAATTAAAA AGCTAAAGGT ACACAATTTT TGAGCATAGT TATTAATAGC
2101 AGACACTCTA TGCCTGTGTG GAGTAAGAAA AAACAGTATG TTATGATTAT

```
2151   AACTGTTATG CCTACTTATA AAGGTTACAG AATATTTTTC CATAATTTTC
2201   TTGTATAGCA GTGCAGCTTT TTCCTTTGTG GTGTAAATAG CAAAGCAAGC
2251   AAGAGTTCTA TTACTAAACA CAGCATGACT CAAAAAACTT AGCAATTCTG
2301   AAGGAAAGTC CTTGGGGTCT TCTACCTTTC TCTTCTTTTT TGGAGGAGTA
2351   GAATGTTGAG AGTCAGCAGT AGCCTCATCA TCACTAGATG GCATTTCTTC
2401   TGAGCAAAAC AGGTTTTCCT CATTAAAGGC ATTCCACCAC TGCTCCCATT
2451   CATCAGTTCC ATAGGTTGGA ATCTAAAATA CACAAACAAT TAGAATCAGT
2501   AGTTTAACAC ATTATACACT TAAAAATTTT ATATTTACCT TAGAGCTTTA
2551   AATCTCTGTA GGTAGTTTGT CCAATTATGT CACACCACAG AAGTAAGGTT
2601   CCTTCACAAA GATCCGGACC AAAGCGGCCA TCGTGCCTCC CCACTCCTGC
2651   AGTTCGGGGG CATGGATGCG CGGATAGCCG CTGCTGGTTT CCTGGATGCC
2701   GACGGATTTG CACTGCCGGT AGAACTCCGC GAGGTCGTCC AGCCTCAGGC
2751   AGCAGCTGAA CCAACTCGCG AGGGGATCGA GCATCCCCCA TGGTCTTATA
2801   AAAATGCATA GCTTTAGGAG GGGAGCAGAG AACTTGAAAG CATCTTCCTG
2851   TTAGTCTTTC TTCTCGTAGA CTTCAAACTT ATACTTGATG CCTTTTTCCT
2901   CCTGGACCTC AGAGAGGACG CCTGGGTATT CTGGGAGAAG TTTATATTTC
2951   CCCAAATCAA TTTCTGGGAA AAACGTGTCA CTTTCAAATT CCTGCATGAT
3001   CCTTGTCACA AAGAGTCTGA GGTGGCCTGG TTGATTCATG GCTTCCTGGT
3051   AAACAGAACT GCCTCCGACT ATCCAAACCA TGTCTACTTT ACTTGCCAAT
3101   TCCGGTTGTT CAATAAGTCT TAAGGCATCA TCCAAACTTT TGGCAAGAAA
3151   ATGAGCTCCT CGTGGTGGTT CTTTGAGTTC TCTACTGAGA ACTATATTAA
3201   TTCTGTCCTT TAAAGGTCGA TTCTTCTCAG GAATGGAGAA CCAGGTTTTC
3251   CTACCCATAA TCACCAGATT CTGTTTACCT TCCACTGAAG AGGTTGTGGT
3301   CATTCTTTGG AAGTACTTGA ACTCGTTCCT GAGCGGAGGC CAGGGTCGGT
3351   CTCCGTTCTT GCCAATCCCC ATATTTTGGG ACACGGCGAC GATGCAGTTC
3401   AATGGTCGAA CCATGATGGC AGCGGGGATA AAATCCTACC AGCCTTCACG
3451   CTAGGATTGC CGTCAAGTTT GGGGGTACCG AGCTCGAATT AGCTTTTTGC
3501   AAAAGCCTAG GCCTCCAAAA AAGCCTCCTC ACTACTTCTG GAATAGCTCA
3551   GAGGGCCGAG GCGGCCTCGG CCTCTGCATA AATAAAAAAA ATTAGTCAGC
3601   CATGGGGCGG AGAATGGGCG GAACTGGGCG GAGTTAGGGG CGGGATGGGC
3651   GGAGTTAGGG GCGGGACTAT GGTTGCTGAC TAATTGAGAT GCATGCTTTG
3701   CATACTTCTG CCTGCTGGGG AGCCTGGGGA CTTTCCACAC CTGGTTGCTG
3751   ACTAATTGAG ATGCATGCTT TGCATACTTC TGCCTGCTGG GGAGCCTGGG
3801   GACTTTCCAC ACCCTAACTG ACACACATTC CACAGCTGCC TCGCGCGTTT
3851   CGGTGATGAC GGTGAAAACC TCTGACACAT GCAGCTCCCG GAGACGGTCA
3901   CAGCTTGTCT GTAAGCGGAT GCCGGGAGCA GACAAGCCCG TCAGGGCGCG
3951   TCAGCGGGTG TTGGCGGGTG TCGGGGCGCA GCCATGACCC AGTCACGTAG
4001   CGATAGCGGA GTGTATACTG GCTTAACTAT GCGGCATCAG AGCAGATTGT
4051   ACTGAGAGTG CACCATATGC GGCCGCATAT GCGGTGTGAA ATACCGCACA
4101   GATGCGTAAG GAGAAAATAC CGCATCAGGC GCTCTTCCGC TTCCTCGCTC
4151   ACTGACTCGC TGCGCTCGGT CGTTCGGCTG CGGCGAGCGG TATCAGCTCA
```

4201 CTCAAAGGCG GTAATACGGT TATCCACAGA ATCAGGGGAT AACGCAGGAA
4251 AGAACATGTG AGCAAAAGGC CAGCAAAAGG CCAGGAACCG TAAAAAGGCC
4301 GCGTTGCTGG CGTTTTTCCA TAGGCTCCGC CCCCCTGACG AGCATCACAA
4351 AAATCGACGC TCAAGTCAGA GGTGGCGAAA CCCGACAGGA CTATAAAGAT
4401 ACCAGGCGTT TCCCCCTGGA AGCTCCCTCG TGCGCTCTCC TGTTCCGACC
4451 CTGCCGCTTA CCGGATACCT GTCCGCCTTT CTCCCTTCGG GAAGCGTGGC
4501 GCTTTCTCAT AGCTCACGCT GTAGGTATCT CAGTTCGGTG TAGGTCGTTC
4551 GCTCCAAGCT GGGCTGTGTG CACGAACCCC CCGTTCAGCC CGACCGCTGC
4601 GCCTTATCCG GTAACTATCG TCTTGAGTCC AACCCGGTAA GACACGACTT
4651 ATCGCCACTG GCAGCAGCCA CTGGTAACAG GATTAGCAGA GCGAGGTATG
4701 TAGGCGGTGC TACAGAGTTC TTGAAGTGGT GGCCTAACTA CGGCTACACT
4751 ' AGAAGGACAG TATTTGGTAT CTGCGCTCTG CTGAAGCCAG TTACCTTCGG
4801 AAAAAGAGTT GGTAGCTCTT GATCCGGCAA ACAAACCACC GCTGGTAGCG
4851 GTGGTTTTTT TGTTTGCAAG CAGCAGATTA CGCGCAGAAA AAAAGGATCT
4901 CAAGAAGATC CTTTGATCTT TTCTACGGGG TCTGACGCTC AGTGGAACGA
4951 AAACTCACGT TAAGGGATTT TGGTCATGAG ATTATCAAAA AGGATCTTCA
5001 CCTAGATCCT TTTAAATTAA AAATGAAGTT TTAAATCAAT CTAAAGTATA
5051 TATGAGTAAA CTTGGTCTGA CAGTTACCAA TGCTTAATCA GTGAGGCACC
5101 TATCTCAGCG ATCTGTCTAT TTCGTTCATC CATAGTTGCC TGACTCCCCG
5151 TCGTGTAGAT AACTACGATA CGGGAGGGCT TACCATCTGG CCCCAGTGCT
5201 GCAATGATAC CGCGAGACCC ACGCTCACCG GCTCCAGATT TATCAGCAAT
5251 AAACCAGCCA GCCGGAAGGG CCGAGCGCAG AAGTGGTCCT GCAACTTTAT
5301 CCGCCTCCAT CCAGTCTATT AATTGTTGCC GGGAAGCTAG AGTAAGTAGT
5351 TCGCCAGTTA ATAGTTTGCG CAACGTTGTT GCCATTGCTG CAGGCATCGT
5401 GGTGTCACGC TCGTCGTTTG GTATGGCTTC ATTCAGCTCC GGTTCCCAAC
5451 GATCAAGGCG AGTTACATGA TCCCCCATGT TGTGCAAAAA AGCGGTTAGC
5501 TCCTTCGGTC CTCCGATCGT TGTCAGAAGT AAGTTGGCCG CAGTGTTATC
5551 ACTCATGGTT ATGGCAGCAC TGCATAATTC TCTTACTGTC ATGCCATCCG
5601 TAAGATGCTT TTCTGTGACT GGTGAGTACT CAACCAAGTC ATTCTGAGAA
5651 TAGTGTATGC GGCGACCGAG TTGCTCTTGC CCGGCGTCAA CACGGGATAA
5701 TACCGCGCCA CATAGCAGAA CTTTAAAAGT GCTCATCATT GGAAAACGTT
5751 CTTCGGGGCG AAAACTCTCA AGGATCTTAC CGCTGTTGAG ATCCAGTTCG
5801 ATGTAACCCA CTCGTGCACC CAACTGATCT TCAGCATCTT TTACTTTCAC
5851 CAGCGTTTCT GGGTGAGCAA AAACAGGAAG GCAAAATGCC GCAAAAAAGG
5901 GAATAAGGGC GACACGGAAA TGTTGAATAC TCATACTCTT CCTTTTTCAA
5951 TATTATTGAA GCATTTATCA GGGTTATTGT CTCATGAGCG GATACATATT
6001 TGAATGTATT TAGAAAAATA AACAAATAGG GGTTCCGCGC ACATTTCCCC
6051 GAAAAGTGCC ACCTGACGTC TAAGAAACCA TTATTATCAT GACATTAACC
6101 TATAAAAATA GGCGTATCAC GAGGCCCTTT CGTCTTCAAG AATTCAGCTG
6151 CTCGAGGAAG AGCTCAAACC CATGCTACTC TCTGGCTTGA TGGAAGCAAC
6201 GCTTTCATAG CTGAGCTGTC ATAAATAATA AAGAGATTTT TTTATTAATA

6251 TTGAAAAGAT GGGTTATTTA TGTAAGACTC TGTCTTCATT TTAAAAACCA
6301 CACCTTCCAG TAGTATTCTG TTACTGTTCT GGCAATCACT GTGATCAAGA
6351 AGCTACACGG TGAGTTGTGC TTCTCAGTCC TAAGGGATAC ATCTACAAGA
6401 GGCTCCCATA CTCGAAGCTC AGGAAACATT GTAGAAAAGG AGGCAAAAGA
6451 CTGACAGAGC CAGAGGACCA AGAAATTTGT TGTGAGGTTG TGTCTCCTAC
6501 TAACAATATA AGCAATATCT ATAAATTGTT GATATCATGG CTACTAAAAT
6551 GTGAGTTGAA CGAGGAGGAC ACAAATGAAC ATGACAATCA GAATGAGGCC
6601 TCTCACCTGC AAAAAACACT ATAGAGAAGC AGATAAAGCT GTCAGCAGAA
6651 GAGGCGCACC TCCTTATAGA AGAAGCCTAC CAGGTTTGAT ATATCAGCCT
6701 TGAAAACCTA CATAGTATTT ACATTATATC GAGTCTATGA GACATATTTA
6751 GTAATGCATA TGTATGTGTG TGTGTGCATG TATGTGTGTA AATACATATG
6801 TTCATAGAAA AATGTGTAAA AAGAGATCAT GAATTTAAGA GAGAACTGGG
6851 ACAATTTTTT TCAGGGAGTT GTAATCAGGA AAGTTAAGGG AAAAATGTTG
6901 TAATTAAAAT TCAGGCTCAG AAACAAACAA AGGAAAAGAA AAAAAAACAA
6951 CAACAACAAC AAAAAAACAA AACAAAGGAG AAGCTGTATG GCCACAATAG
7001 CATCTACAGC TAACTGTGAA AGGATAATGG AACAAGTTAT GTACTGCCTA
7051 GAGCAGTATG ATGCCTAAAT CATCTCGACA TGGAGGAAAA TAGAACAAAG
7101 ACACTCTACA TAGACTATGA TAGAAATCAA AATAAGGTGT AAGACATAGA
7151 ACATTAGTTT TGTTTGTTGT TCAAAGAGAC TCACATTCCC ACAAAAAAAT
7201 CTGTGGGATT TTACAGGTCT GCAATAAGCT GCTGACCTGA TGATTTCTGC
7251 AGCTGTGCCT ACCCTTTGCT GATTTGCATG TACCCAAAGC ATAGCTTACT
7301 GACATGAGGA TTTCTTCATA GTCAGGTCAC ACCCTTTGCT GGAGTCAGAA
7351 TCACACTGAT CACACACAGT CATGAGTGTG CTCACTCAGG TCCTGGCGTT
7401 GCTGCTGCTG TGGCTTACAG GTAATGAAGA CAGCACTAGA ATTTTATTGA
7451 GCTTCCTGTA CACTGTGCTG CTTGTCTCTG TGAAAATTCT CTTGTGAATT
7501 AATCATGTGG GGATCTGTTT TCAATTTTTC AATTGTAGGT ACGCGTTGTG
7551 ACATTCTGCT GACCCAGTCT CCAGCCACCC TGTCTCTGAG TCCAGGAGAA
7601 AGAGCCACTT TCTCCTGCAG GGCCAGTCAG AACATTGGCA CAAGCATACA
7651 GTGGTATCAA CAAAAAACAA ATGGTGCTCC AAGGCTTCTC ATAAGGTCTT
7701 CTTCTGAGTC TATCTCTGGG ATCCCTTCCA GGTTTAGTGG CAGTGGATCA
7751 GGGACAGATT TTACTCTTAC CATCAGCAGT CTGGAGCCTG AAGATTTTGC
7801 AGTGTATTAC TGTCAACAAA GTAATACCTG GCCATTCACG TTCGGCCAGG
7851 GGACCAAGCT TGAAATCAAA CGTAAGTAGA ATCCAAAGTC TCTTTCTTCC
7901 GTTGTCTATG TCTGTGGCTT CTATGTCTAA AAATGATGTA TAAAATCTTA
7951 CTCTGAAACC AGATTCTGGC ACTCTCCAAG GCAAAGATAC AGAGTAACTC
8001 CGTAAGCAAA GCTGGGAATA GGCTAGACAT GTTCTCTGGA GAATGAATGC
8051 CAGTGTAATA ATTAACACAA GTGATAGTTT CAGAAATGCT CTAGTT

**SEQ ID NO: 40**

**Nucleotide sequence of expression vector HCVHESp20**

```
   1 CTAGAGAGGT CTGGTGGAGC CTGCAAAAGT CCAGCTTTCA AAGGAACACA
  51 GAAGTATGTG TATGGAATAT TAGAAGATGT TGCTTTTACT CTTAAGTTGG
 101 TTCCTAGGAA AAATAGTTAA ATACTGTGAC TTTAAAATGT GAGAGGGTTT
 151 TCAAGTACTC ATTTTTTTAA ATGTCCAAAA TTTTTGTCAA TCAATTTGAG
 201 GTCTTGTTTG TGTAGAACTG ACATTACTTA AAGTTTAACC GAGGAATGGG
 251 AGTGAGGCTC TCTCATACCC TATTCAGAAC TGACTTTTAA CAATAATAAA
 301 TTAAGTTTAA AATATTTTTA AATGAATTGA GCAATGTTGA GTTGGAGTCA
 351 AGATGGCCGA TCAGAACCAG AACACCTGCA GCAGCTGGCA GGAAGCAGGT
 401 CATGTGGCAA GGCTATTTGG GGAAGGGAAA ATAAAACCAC TAGGTAAACT
 451 TGTAGCTGTG GTTTGAAGAA GTGGTTTTGA AACACTCTGT CCAGCCCCAC
 501 CAAACCGAAA GTCCAGGCTG AGCAAAACAC CACCTGGGTA ATTTGCATTT
 551 CTAAAATAAG TTGAGGATTC AGCCGAAACT GGAGAGGTCC TCTTTTAACT
 601 TATTGAGTTC AACCTTTTAA TTTTAGCTTG AGTAGTTCTA GTTTCCCCAA
 651 ACTTAAGTTT ATCGACTTCT AAAATGTATT TAAGCTTTCT GGGGCAGGCC
 701 AGGCCTGACC TTGGCTTTGG GGCAGGGAGG GGGCTAAGGT GAGGCAGGTG
 751 GCGCCAGCCA GGTGCACACC CAATGCCCAT GAGCCCAGAC ACTGGACGCT
 801 GAACCTCGCG GACAGTTAAG AACCCAGGGG CCTCTGCGCC CTGGGCCCAG
 851 CTCTGTCCCA CACCGCGGTC ACATGGCACC ACCTCTCTTG CAGCCTCCAC
 901 CAAGGGCCCA TCGGTCTTCC CCCTGGCACC CTCCTCCAAG AGCACCTCTG
 951 GGGGCACAGC GGCCCTGGGC TGCCTGGTCA AGGACTACTT CCCCGAACCG
1001 GTGACGGTGT CGTGGAACTC AGGCGCCCTG ACCAGCGGCG TGCACACCTT
1051 CCCGGCTGTC CTACAGTCCT CAGGACTCTA CTCCCTCAGC AGCGTGGTGA
1101 CCGTGCCCTC CAGCAGCTTG GGCACCCAGA CCTACATCTG CAACGTGAAT
1151 CACAAGCCCA GCAACACCAA GGTGGACAAG AGAGTTGGTG AGAGGCCAGC
1201 ACAGGGAGGG AGGGTGTCTG CTGGAAGCCA GGCTCAGCGC TCCTGCCTGG
1251 ACGCATCCCG GCTATGCAGT CCCAGTCCAG GGCAGCAAGG CAGGCCCCGT
1301 CTGCCTCTTC ACCCGGAGGC CTCTGCCCGC CCCACTCATG CTCAGGGAGA
1351 GGGTCTTCTG GCTTTTTCCC CAGGCTCTGG GCAGGCACAG GCTAGGTGCC
1401 CCTAACCCAG GCCCTGCACA CAAAGGGGCA GGTGCTGGGC TCAGACCTGC
1451 CAAGAGCCAT ATCCGGGAGG ACCCTGCCCC TGACCTAAGC CCACCCCAAA
1501 GGCCAAACTC TCCACTCCCT CAGCTCGGAC ACCTTCTCTC CTCCCAGATT
1551 CCAGTAACTC CCAATCTTCT CTCTGCAGAG CCCAAATCTT GTGACAAAAC
1601 TCACACATGC CCACCGTGCC CAGGTAAGCC AGCCCAGGCC TCGCCCTCCA
1651 GCTCAAGGCG GGACAGGTGC CCTAGAGTAG CCTGCATCCA GGGACAGGCC
1701 CCAGCCGGGT GCTGACACGT CCACCTCCAT CTCTTCCTCA GCACCTGAAC
1751 TCCTGGGGGG ACCGTCAGTC TTCCTCTTCC CCCCAAAACC CAAGGACACC
1801 CTCATGATCT CCCGGACCCC TGAGGTCACA TGCGTGGTGG TGGACGTGAG
1851 CCACGAAGAC CCTGAGGTCA AGTTCAACTG GTACGTGGAC GGCGTGGAGG
1901 TGCATAATGC CAAGACAAAG CCGCGGGAGG AGCAGTACAA CAGCACGTAC
```

```
1951  CGTGTGGTCA GCGTCCTCAC CGTCCTGCAC CAGGACTGGC TGAATGGCAA
2001  GGAGTACAAG TGCAAGGTCT CCAACAAAGC CCTCCCAGCC CCCATCGAGA
2051  AAACCATCTC CAAAGCCAAA GGTGGGACCC GTGGGGTGCG AGGGCCACAT
2101  GGACAGAGGC CGGCTCGGCC CACCCTCTGC CCTGAGAGTG ACCGCTGTAC
2151  CAACCTCTGT CCCTACAGGG CAGCCCCGAG AACCACAGGT GTACACCCTG
2201  CCCCCATCCC GGGAGGAGAT GACCAAGAAC CAGGTCAGCC TGACCTGCCT
2251  GGTCAAAGGC TTCTATCCCA GCGACATCGC CGTGGAGTGG GAGAGCAATG
2301  GGCAGCCGGA GAACAACTAC AAGACCACGC CTCCCGTGCT GGACTCCGAC
2351  GGCTCCTTCT TCCTCTATAG CAAGCTCACC GTGGACAAGA GCAGGTGGCA
2401  GCAGGGGAAC GTCTTCTCAT GCTCCGTGAT GCATGAGGCT CTGCACAACC
2451  ACTACACGCA GAAGAGCCTC TCCCTGTCCC CGGGTAAATG AGTGCGACGG
2501  CCGGCAAGCC CCCGCTCCCC GGGCTCTCGC GGTCGCACGA GGATGCTTGG
2551  CACGTACCCC GTCTACATAC TTCCCAGGCA CCCAGCATGG AAATAAAGCA
2601  CCCACCACTG CCCTGGGCCC CTGCGAGACT GTGATGGTTC TTTCCACGGG
2651  TCAGGCCGAG TCTGAGGCCT GAGTGGCATG AGGGAGGCAG AGCGGGTCCC
2701  ACTGTCCCCA CACTGGCCCA GGCTGTGCAG GTGTGCCTGG GCCGCCTAGG
2751  GTGGGGCTCA GCCAGGGGCT GCCCTCGGCA GGGTGGGGGA TTTGCCAGCG
2801  TGGCCCTCCC TCCAGCAGCA GCTGCCCTGG GCTGGGCCAC GAGAAGCCCT
2851  AGGAGCCCCT GGGGACAGAC ACACAGCCCC TGCCTCTGTA GGAGACTGTC
2901  CTGTCCTGTG AGCGCCCTGT CCTCCGACCC CGATGCCCAC TCGGGGGCAT
2951  GCCTAGTCCA TGCGCGTAGG GACAGGCCCT CCCTCACCCA TCTACCCCCA
3001  CGGCACTAAC CCCTGGCAGC CCTGCCCAGC CTCGCACCCG CATGGGGACA
3051  CAACCGACTC CGGGGACATG CACTCTCGGG CCCTGTGGAG GGACTGGTGC
3101  AGATGCCCAC ACACACACTC AGCCCAGACC CGTTCAACAA ACCCCGCACT
3151  GAGGTTGGTC GAGCGGGAGT GCGGCCAGAG CCTGCCTCGG CCGTCAGGGA
3201  GGACTCCCGG GCTCACTCGA AGGAGGTGCC ACCATTTCAG CTTTGGTAGC
3251  TTTTCTTCTT CTTTTAAATT TTCTAAAGCT CATTAATTGT CTTTGATGTT
3301  TCTTTTGTGA TGACAATAAA ATATCCTTTT TAAGTCTTGT ACTTCGTGAT
3351  GGGAGCCGCC TTCCTGTGTC CACGCGCCTC CTGCCCCCGG TGGGAAGCAC
3401  GGTCAGGAGG AGGCTGGTCC AGCTGCACCT CGGGGGCTCC CTGCACTCGC
3451  CCCCCGCCTC CTGCAGCCAC ACGCATTGCC CGAGCGACCC TCCCTGGCCC
3501  CTGTCACTAC ATGGACCCCT GGGGCTTCTC CTCTTTTCTA CATGGATGCA
3551  GTTTCTCCTC CTGCTGGGCA CGGTGCTGCC TGCCCTGGTC ACTCTGCGGG
3601  GGACAGGGCC TCCAGGGAAA GCTGGGTCGA GGCTGGGAGC TGGCTCAGGC
3651  TGGCCAGGCA GAGCCACAGG GAGGGCCTTC CAGAACCAAC CATGGTCCGA
3701  AGCGAGAGGT GGGTGTCAGA TCCAGACATG ATAAGATACA TTGATGAGTT
3751  TGGACAAACC ACAACTAGAA TGCAGTGAAA AAAATGCTTT ATTTGTGAAA
3801  TTTGTGATGC TATTGCTTTA TTTGTAACCA TTATAAGCTG CAATAAACAA
3851  GTTAACAACA ACAATTGCAT TCATTTTATG TTTCAGGTTC AGGGGGAGGT
3901  GTGGGAGGTT TTTTAAAGCA AGTAAAACCT CTACAAATGT GGTATGGCTG
3951  ATTATGATCT CTAGTCAAGG CACTATACAT CAAATATTCC TTATTAACCC
```

4001 CTTTACAAAT TAAAAAGCTA AAGGTACACA ATTTTTGAGC ATAGTTATTA
4051 ATAGCAGACA CTCTATGCCT GTGTGGAGTA AGAAAAAACA GTATGTTATG
4101 ATTATAACTG TTATGCCTAC TTATAAAGGT TACAGAATAT TTTTCCATAA
4151 TTTTCTTGTA TAGCAGTGCA GCTTTTTCCT TTGTGGTGTA AATAGCAAAG
4201 CAAGCAAGAG TTCTATTACT AAACACAGCA TGACTCAAAA AACTTAGCAA
4251 TTCTGAAGGA AAGTCCTTGG GGTCTTCTAC CTTTCTCTTC TTTTTTGGAG
4301 GAGTAGAATG TTGAGAGTCA GCAGTAGCCT CATCATCACT AGATGGCATT
4351 TCTTCTGAGC AAAACAGGTT TTCCTCATTA AAGGCATTCC ACCACTGCTC
4401 CCATTCATCA GTTCCATAGG TTGGAATCTA AAATACACAA ACAATTAGAA
4451 TCAGTAGTTT AACACATTAT ACACTTAAAA ATTTTATATT TACCTTAGAG
4501 CTTTAAATCT CTGTAGGTAG TTTGTCCAAT TATGTCACAC CACAGAAGTA
4551 AGGTTCCTTC ACAAAGATCC GGACCAAAGC GGCCATCGTG CCTCCCCACT
4601 CCTGCAGTTC GGGGGCATGG ATGCGCGGAT AGCCGCTGCT GGTTTCCTGG
4651 ATGCCGACGG ATTTGCACTG CCGGTAGAAC TCCGCGAGGT CGTCCAGCCT
4701 CAGGCAGCAG CTGAACCAAC TCGCGAGGGG ATCGAGCCCG GGGTGGGCGA
4751 AGAACTCCAG CATGAGATCC CCGCGCTGGA GGATCATCCA GCCGGCGTCC
4801 CGGAAAACGA TTCCGAAGCC CAACCTTTCA TAGAAGGCGG CGGTGGAATC
4851 GAAATCTCGT GATGGCAGGT TGGGCGTCGC TTGGTCGGTC ATTTCGAACC
4901 CCAGAGTCCC GCTCAGAAGA ACTCGTCAAG AAGGCGATAG AAGGCGATGC
4951 GCTGCGAATC GGGAGCGGCG ATACCGTAAA GCACGAGGAA GCGGTCAGCC
5001 CATTCGCCGC CAAGCTCTTC AGCAATATCA CGGGTAGCCA ACGCTATGTC
5051 CTGATAGCGG TCCGCCACAC CCAGCCGGCC ACAGTCGATG AATCCAGAAA
5101 AGCGGCCATT TTCCACCATG ATATTCGGCA AGCAGGCATC GCCATGGGTC
5151 ACGACGAGAT CCTCGCCGTC GGGCATGCGC GCCTTGAGCC TGGCGAACAG
5201 TTCGGCTGGC GCGAGCCCCT GATGCTCTTC GTCCAGATCA TCCTGATCGA
5251 CAAGACCGGC TTCCATCCGA GTACGTGCTC GCTCGATGCG ATGTTTCGCT
5301 TGGTGGTCGA ATGGGCAGGT AGCCGGATCA AGCGTATGCA GCCGCCGCAT
5351 TGCATCAGCC ATGATGGATA CTTTCTCGGC AGGAGCAAGG TGAGATGACA
5401 GGAGATCCTG CCCCGGCACT TCGCCCAATA GCAGCCAGTC CCTTCCCGCT
5451 TCAGTGACAA CGTCGAGCAC AGCTGCGCAA GGAACGCCCG TCGTGGCCAG
5501 CCACGATAGC CGCGCTGCCT CGTCCTGCAG TTCATTCAGG GCACCGGACA
5551 GGTCGGTCTT GACAAAAAGA ACCGGGCGCC CCTGCGCTGA CAGCCGGAAC
5601 ACGGCGGCAT CAGAGCAGCC GATTGTCTGT TGTGCCCAGT CATAGCCGAA
5651 TAGCCTCTCC ACCCAAGCGG CCGGAGAACC TGCGTGCAAT CCATCTTGTT
5701 CAATCATGCG AAACGATCCT CATCCTGTCT CTTGATCAGA TCTTGATCCC
5751 CTGCGCCATC AGATCCTTGG CGGCAAGAAA GCCATCCAGT TTACTTTGCA
5801 GGGCTTCCCA ACCTTACCAG AGGGCGCCCC AGCTGGCAAT TCCGGTTCGC
5851 TTGCTGTCCA TAAAACCGCC CAGTCTAGCT ATCGCCATGT AAGCCCACTG
5901 CAAGCTACCT GCTTTCTCTT TGCGCTTGCG TTTTCCCTTG TCCAGATAGC
5951 CCAGTAGCTG ACATTCATCC GGGGTCAGCA CCGTTTCTGC GGACTGGCTT
6001 TCTACGTGTT CCGCTTCCTT TAGCAGCCCT TGCGCCCTGA GTGCTTGCGG

6051 CAGCGTGAAG CTTTTTGCAA AAGCCTAGGC CTCCAAAAAA GCCTCCTCAC
6101 TACTTCTGGA ATAGCTCAGA GGCCGAGGCG GCCTCGGCCT CTGCATAAAT
6151 AAAAAAAATT AGTCAGCCAT GGGGCGGAGA ATGGGCGGAA CTGGGCGGAG
6201 TTAGGGGCGG GATGGGCGGA GTTAGGGGCG GGACTATGGT TGCTGACTAA
6251 TTGAGATGCA TGCTTTGCAT ACTTCTGCCT GCTGGGGAGC CTGGGGACTT
6301 TCCACACCTG GTTGCTGACT AATTGAGATG CATGCTTTGC ATACTTCTGC
6351 CTGCTGGGGA GCCTGGGGAC TTTCCACACC CTAACTGACA CACATTCCAC
6401 AGCTGCCTCG CGCGTTTCGG TGATGACGGT GAAAACCTCT GACACATGCA
6451 GCTCCCGGAG ACGGTCACAG CTTGTCTGTA AGCGGATGCC GGGAGCAGAC
6501 AAGCCCGTCA GGGCGCGTCA GCGGGTGTTG GCGGGTGTCG GGGCGCAGCC
6551 ATGACCCAGT CACGTAGCGA TAGCGGAGTG TATACTGGCT TAACTATGCG
6601 GCATCAGAGC AGATTGTACT GAGAGTGCAC CATATGCGGT GTGAAATACC
6651 GCACAGATGC GTAAGGAGAA AATACCGCAT CAGGCGCTCT TCCGCTTCCT
6701 CGCTCACTGA CTCGCTGCGC TCGGTCGTTC GGCTGCGGCG AGCGGTATCA
6751 GCTCACTCAA AGGCGGTAAT ACGGTTATCC ACAGAATCAG GGGATAACGC
6801 AGGAAAGAAC ATGTGAGCAA AAGGCCAGCA AAAGGCCAGG AACCGTAAAA
6851 AGGCCGCGTT GCTGGCGTTT TTCCATAGGC TCCGCCCCCC TGACGAGCAT
6901 CACAAAAATC GACGCTCAAG TCAGAGGTGG CGAAACCCGA CAGGACTATA
6951 AAGATACCAG GCGTTTCCCC CTGGAAGCTC CCTCGTGCGC TCTCCTGTTC
7001 CGACCCTGCC GCTTACCGGA TACCTGTCCG CCTTTCTCCC TTCGGGAAGC
7051 GTGGCGCTTT CTCATAGCTC ACGCTGTAGG TATCTCAGTT CGGTGTAGGT
7101 CGTTCGCTCC AAGCTGGGCT GTGTGCACGA ACCCCCCGTT CAGCCCGACC
7151 GCTGCGCCTT ATCCGGTAAC TATCGTCTTG AGTCCAACCC GGTAAGACAC
7201 GACTTATCGC CACTGGCAGC AGCCACTGGT AACAGGATTA GCAGAGCGAG
7251 GTATGTAGGC GGTGCTACAG AGTTCTTGAA GTGGTGGCCT AACTACGGCT
7301 ACACTAGAAG GACAGTATTT GGTATCTGCG CTCTGCTGAA GCCAGTTACC
7351 TTCGGAAAAA GAGTTGGTAG CTCTTGATCC GGCAAACAAA CCACCGCTGG
7401 TAGCGGTGGT TTTTTTGTTT GCAAGCAGCA GATTACGCGC AGAAAAAAAG
7451 GATCTCAAGA AGATCCTTTG ATCTTTTCTA CGGGGTCTGA CGCTCAGTGG
7501 AACGAAAACT CACGTTAAGG GATTTTGGTC ATGAGATTAT CAAAAAGGAT
7551 CTTCACCTAG ATCCTTTTAA ATTAAAAATG AAGTTTTAAA TCAATCTAAA
7601 GTATATATGA GTAAACTTGG TCTGACAGTT ACCAATGCTT AATCAGTGAG
7651 GCACCTATCT CAGCGATCTG TCTATTTCGT TCATCCATAG TTGCCTGACT
7701 CCCCGTCGTG TAGATAACTA CGATACGGGA GGGCTTACCA TCTGGCCCCA
7751 GTGCTGCAAT GATACCGCGA GACCCACGCT CACCGGCTCC AGATTTATCA
7801 GCAATAAACC AGCCAGCCGG AAGGGCCGAG CGCAGAAGTG GTCCTGCAAC
7851 TTTATCCGCC TCCATCCAGT CTATTAATTG TTGCCGGGAA GCTAGAGTAA
7901 GTAGTTCGCC AGTTAATAGT TTGCGCAACG TTGTTGCCAT TGCTGCAGGC
7951 ATCGTGGTGT CACGCTCGTC GTTTGGTATG GCTTCATTCA GCTCCGGTTC
8001 CCAACGATCA AGGCGAGTTA CATGATCCCC CATGTTGTGC AAAAAAGCGG
8051 TTAGCTCCTT CGGTCCTCCG ATCGTTGTCA GAAGTAAGTT GGCCGCAGTG

8101 TTATCACTCA TGGTTATGGC AGCACTGCAT AATTCTCTTA CTGTCATGCC
8151 ATCCGTAAGA TGCTTTTCTG TGACTGGTGA GTACTCAACC AAGTCATTCT
8201 GAGAATAGTG TATGCGGCGA CCGAGTTGCT CTTGCCCGGC GTCAACACGG
8251 GATAATACCG CGCCACATAG CAGAACTTTA AAAGTGCTCA TCATTGGAAA
8301 ACGTTCTTCG GGGCGAAAAC TCTCAAGGAT CTTACCGCTG TTGAGATCCA
8351 GTTCGATGTA ACCCACTCGT GCACCCAACT GATCTTCAGC ATCTTTTACT
8401 TTCACCAGCG TTTCTGGGTG AGCAAAAACA GGAAGGCAAA ATGCCGCAAA
8451 AAAGGGAATA AGGGCGACAC GGAAATGTTG AATACTCATA CTCTTCCTTT
8501 TTCAATATTA TTGAAGCATT TATCAGGGTT ATTGTCTCAT GAGCGGATAC
8551 ATATTTGAAT GTATTTAGAA AAATAAACAA ATAGGGGTTC CGCGCACATT
8601 TCCCCGAAAA GTGCCACCTG ACGTCTAAGA AACCATTATT ATCATGACAT
8651 TAACCTATAA AAATAGGCGT ATCACGAGGC CCTTTCGTCT TCAAGAATTC
8701 GAGCTCGGTA CCCATCAGCC AAAAAGCATG CCTGCCACAC AACATCAATT
8751 TCTGGAAAAC GCTACACTTA ATTATTTCTA GTAGAACAGC TCTTTGGTTT
8801 GCCAAAAAGA ATCACCTATA GTGGCATCTA AGCACAAAAA GGAGAAAAAA
8851 ATCACAAAGA AATGATTGAG AGGCATAATA AAAATTATCA AAAAATTATG
8901 AGTTTTACGA TTTCATCTTT TTCCAAGTTG AAATCATAGG GTGGCTTTAA
8951 CACAGTGACA AGGAATGTGC ATGCTGCCAT TATGGTGCTC TGCCTAAAAT
9001 GGTTGGAGCC TTGTCATGCT ACAGAGAAAC TGTCATACAG CAGGGGGTGC
9051 CAAATTTCCA TATTTTTTTA TATCATTGAG CAGGTGCACA GAAGACCAGA
9101 AAGCACTTTC TATCAGGCTG GCCTTCCTCT TCCTTTCCAG TATGAAGCAA
9151 AAACTGCCAA TGAAACTAGC AATTGTTAAA TTCCTTTTTC AAACAGTATT
9201 TGTGCTATCA GAACATAGTG CATTCAAAAG TCTAGCCTGA GAGAACAACC
9251 CAGTTTTATT CATTCCTCCT ACTACCTCTC TCATTCCCAC TGTTTGTGTT
9301 CTCCCTCCCA TTTTAATTGT CTATCTAGTC CAAACTAAGC ACACGATCCA
9351 GTCCACATTA AACAACATGT TTTCACTTTA AGTCAAATAC AAGACACCTT
9401 TAATATCAGC CCTTGTTCAT AATCGTGCTT CTAGTGACTT AATGTACATG
9451 TCACACTGTA CTGTTGGGTT TTGTGTCTCA TCATGAACAA TGTTGTGAAG
9501 GTATTAAGTG GAGAGTAAGC AGAATTAGAT TCCTCTAATG ATGCACACCC
9551 ACACTAAGAG CAGAAATAAT ATTAAAAATA GAAAAAAAAG TTTTACATGA
9601 GATTTCAAAT ACCCAGGTAT GAGCTGCAGT TTCTTCAAGT TAAAGCATCG
9651 AGGTTGTCAG TTACACTATT ACAGGAAACA TATGCAGAGT TTTTATTTTA
9701 GTATATTAGT TTTCACATAT GTGGAATTAC TATTAAACTA TTCTTTCTTT
9751 TCAAATGCTT ACCATTGTAA ATGAGTTTGT GACTTTGTGT AGGTGAGTGC
9801 ACATGACTCT GGATGCCTAA GAGGACTGAA GAAGTTGGAG TTATAGGTAG
9851 TTTTATTCTA CTTGACTGTT CAGTGCTAAA AATACAACTG AGGTCCTTTA
9901 AACTGCTGTT CATGAACTTC TTAATTGATA TATCTCATGA GATCTCTAAA
9951 CTATTTTTAT TATGACACGT TTCACCATTT TCACTGTAAC GATTTTTATG
10001 TTTTATATTA ATGTAACTAT ATGACACTTC CCAAAATCCC CATATTCACA
10051 ATTGAACTGT TTCAAAGTTT TACCTTGACT TATGGGAAAT GAAAACCCAC
10101 ATTTTATAAT TTTAAAATGA AATGTTTATT TTATATTTCT GCAAATTTCA

```
10151   CAAGGAAAGA TTAGTCACTG GTGTGTGAGA GCAGAGGAGC ATAAGAGTTC
10201   AGGAATAGAA TCCATTATGA TTCTGGAGGC AAGGAAGAAC TGATGCCAAG
10251   GTTTCAGTAT AAGAGCAGTA TCCACTGGAA AGGATAAAGT CACTACATCT
10301   GAGCACAGAG CAGGACATCT ACATAATGAG TGGTCACTAA TGGGCCACTG
10351   TTACACTGTT ATATGTATAA GGCTCAAGAA TGAGCACTGA GGCTGTAAGG
10401   TGTATGGGTG AGGACATCAG GATGTAAACC CAGCTCAGGT AGAGGACTCA
10451   GAGGACAGCA CAGTCAGCAT GAACTAATAA ACATCAGATA AGATAAGGCA
10501   CAAGCTCAGC TATATAGGGT AAGGGATCTT TGTAAATCTG ATTGTGCATC
10551   CAGTCTAGTT CAATGTGACT TAGGAAGCCC AGTCATATGC AAATCTAGAG
10601   AAGACTTTAG AGTAGAAATC TGAGGCTCAC CTCACATACC AGCAAGCGAG
10651   TGACCAGTTA GTCTTAAGGC ACCACTTCTT AGACATCATG GCTTGGGTGT
10701   GGACCTTGCC ATTCCTGATG GCAGCTGCCC AAAGTAAGAC ATCAGAAAAA
10751   AGAGTTCCAA GGGGAATTGA AGCAGTTCCA TGAATACTCA CCTTCCTGTG
10801   TTCTTTTCAC AGGTGTCCAG GCAGAGGTGC AGCTGGTGGA GTCAGGAGCC
10851   GAAGTGAAAA AGCCTGGGGC TTCAGTGAAG GTGTCCTGCA AGGCCTCTGG
10901   ATACACATTC ACTAATTATA TTATCCACTG GGTGAAGCAG GAGCCTGGTC
10951   AGGGCCTTGA ATGGATTGGA TATTTTAATC CTTACAATCA TGGTACTAAG
11001   TACAATGAGA AGTTCAAAGG CAGGGCCACA CTAACTGCAA ACAAATCCAT
11051   CAGCACAGCC TACATGGAGC TCAGCAGCCT GCGCTCTGAG GACACTGCGG
11101   TCTACTACTG TGCAAGATCA GGACCCTATG CCTGGTTTGA CACCTGGGGC
11151   CAAGGGACCA CGGTCACCGT CTCCTCAGGT AAGAATGGCC ACTCTAGGGC
11201   CTTTGTTTTC TGCTGCTGCC TGTGGGATTT CATGAGCATT GCAAAGTTGT
11251   CCTCGGGACA TGTTCCGAGG GGACCTGGGC GGACTGGCCA GGAGGGGACG
11301   GGCACTGGGG TGCCTTGAGG ATCTGGGAGC CTCTGTGGAT TTTCCGATGC
11351   CTTTGGAAAA TGGGACTGAG GTTGGGTGCG TCTGAGACAG TAACTCAGCC
11401   TGGGGGCTTG GTGAAGATCG CCGCACAGCA GCGAGTCCGT GAAATATCTT
11451   ATTTAGACTT GTGAGGTGCG CTGTGTGTCA ATTTACATCT TAAATCCTTT
11501   ATTGGCTGGA AAGAGAATTG TTGGAGTGGG TGAATCCAGC CAGGAGGGAC
11551   GCGGGGGGAT CCA
```

**<u>SEQ ID NO:41</u>**

**Nucleotide sequence of expression vector HCVHQSp20**

| | | | | | |
|---|---|---|---|---|---|
| 1 | CTAGAGAGGT | CTGGTGGAGC | CTGCAAAAGT | CCAGCTTTCA | AAGGAACACA |
| 51 | GAAGTATGTG | TATGGAATAT | TAGAAGATGT | TGCTTTTACT | CTTAAGTTGG |
| 101 | TTCCTAGGAA | AAATAGTTAA | ATACTGTGAC | TTTAAAATGT | GAGAGGGTTT |
| 151 | TCAAGTACTC | ATTTTTTTAA | ATGTCCAAAA | TTTTTGTCAA | TCAATTTGAG |
| 201 | GTCTTGTTTG | TGTAGAACTG | ACATTACTTA | AAGTTTAACC | GAGGAATGGG |
| 251 | AGTGAGGCTC | TCTCATACCC | TATTCAGAAC | TGACTTTTAA | CAATAATAAA |
| 301 | TTAAGTTTAA | AATATTTTTA | AATGAATTGA | GCAATGTTGA | GTTGGAGTCA |
| 351 | AGATGGCCGA | TCAGAACCAG | AACACCTGCA | GCAGCTGGCA | GGAAGCAGGT |
| 401 | CATGTGGCAA | GGCTATTTGG | GGAAGGGAAA | ATAAAACCAC | TAGGTAAACT |

451 TGTAGCTGTG GTTTGAAGAA GTGGTTTTGA AACACTCTGT CCAGCCCCAC
501 CAAACCGAAA GTCCAGGCTG AGCAAAACAC CACCTGGGTA ATTTGCATTT
551 CTAAAATAAG TTGAGGATTC AGCCGAAACT GGAGAGGTCC TCTTTTAACT
601 TATTGAGTTC AACCTTTTAA TTTTAGCTTG AGTAGTTCTA GTTTCCCCAA
651 ACTTAAGTTT ATCGACTTCT AAAATGTATT TAAGCTTTCT GGGGCAGGCC
701 AGGCCTGACC TTGGCTTTGG GGCAGGGAGG GGGCTAAGGT GAGGCAGGTG
751 GCGCCAGCCA GGTGCACACC CAATGCCCAT GAGCCCAGAC ACTGGACGCT
801 GAACCTCGCG GACAGTTAAG AACCCAGGGG CCTCTGCGCC CTGGGCCCAG
851 CTCTGTCCCA CACCGCGGTC ACATGGCACC ACCTCTCTTG CAGCCTCCAC
901 CAAGGGCCCA TCGGTCTTCC CCCTGGCACC CTCCTCCAAG AGCACCTCTG
951 GGGGCACAGC GGCCCTGGGC TGCCTGGTCA AGGACTACTT CCCCGAACCG
1001 GTGACGGTGT CGTGGAACTC AGGCGCCCTG ACCAGCGGCG TGCACACCTT
1051 CCCGGCTGTC CTACAGTCCT CAGGACTCTA CTCCCTCAGC AGCGTGGTGA
1101 CCGTGCCCTC CAGCAGCTTG GGCACCCAGA CCTACATCTG CAACGTGAAT
1151 CACAAGCCCA GCAACACCAA GGTGGACAAG AGAGTTGGTG AGAGGCCAGC
1201 ACAGGGAGGG AGGGTGTCTG CTGGAAGCCA GGCTCAGCGC TCCTGCCTGG
1251 ACGCATCCCG GCTATGCAGT CCCAGTCCAG GGCAGCAAGG CAGGCCCCGT
1301 CTGCCTCTTC ACCCGGAGGC CTCTGCCCGC CCCACTCATG CTCAGGGAGA
1351 GGGTCTTCTG GCTTTTTCCC CAGGCTCTGG GCAGGCACAG GCTAGGTGCC
1401 CCTAACCCAG GCCCTGCACA CAAAGGGGCA GGTGCTGGGC TCAGACCTGC
1451 CAAGAGCCAT ATCCGGGAGG ACCCTGCCCC TGACCTAAGC CCACCCCAAA
1501 GGCCAAACTC TCCACTCCCT CAGCTCGGAC ACCTTCTCTC CTCCCAGATT
1551 CCAGTAACTC CCAATCTTCT CTCTGCAGAG CCCAAATCTT GTGACAAAAC
1601 TCACACATGC CCACCGTGCC CAGGTAAGCC AGCCCAGGCC TCGCCCTCCA
1651 GCTCAAGGCG GGACAGGTGC CCTAGAGTAG CCTGCATCCA GGGACAGGCC
1701 CCAGCCGGGT GCTGACACGT CCACCTCCAT CTCTTCCTCA GCACCTGAAC
1751 TCCTGGGGGG ACCGTCAGTC TTCCTCTTCC CCCCAAAACC CAAGGACACC
1801 CTCATGATCT CCCGGACCCC TGAGGTCACA TGCGTGGTGG TGGACGTGAG
1851 CCACGAAGAC CCTGAGGTCA AGTTCAACTG GTACGTGGAC GGCGTGGAGG
1901 TGCATAATGC CAAGACAAAG CCGCGGGAGG AGCAGTACAA CAGCACGTAC
1951 CGTGTGGTCA GCGTCCTCAC CGTCCTGCAC CAGGACTGGC TGAATGGCAA
2001 GGAGTACAAG TGCAAGGTCT CCAACAAAGC CCTCCCAGCC CCCATCGAGA
2051 AAACCATCTC CAAAGCCAAA GGTGGGACCC GTGGGGTGCG AGGGCCACAT
2101 GGACAGAGGC CGGCTCGGCC CACCCTCTGC CCTGAGAGTG ACCGCTGTAC
2151 CAACCTCTGT CCCTACAGGG CAGCCCCGAG AACCACAGGT GTACACCCTG
2201 CCCCCATCCC GGGAGGAGAT GACCAAGAAC CAGGTCAGCC TGACCTGCCT
2251 GGTCAAAGGC TTCTATCCCA GCGACATCGC CGTGGAGTGG GAGAGCAATG
2301 GGCAGCCGGA GAACAACTAC AAGACCACGC CTCCCGTGCT GGACTCCGAC
2351 GGCTCCTTCT TCCTCTATAG CAAGCTCACC GTGGACAAGA GCAGGTGGCA
2401 GCAGGGGAAC GTCTTCTCAT GCTCCGTGAT GCATGAGGCT CTGCACAACC
2451 ACTACACGCA GAAGAGCCTC TCCCTGTCCC CGGGTAAATG AGTGCGACGG

2501 CCGGCAAGCC CCCGCTCCCC GGGCTCTCGC GGTCGCACGA GGATGCTTGG
2551 CACGTACCCC GTCTACATAC TTCCCAGGCA CCCAGCATGG AAATAAAGCA
2601 CCCACCACTG CCCTGGGCCC CTGCGAGACT GTGATGGTTC TTTCCACGGG
2651 TCAGGCCGAG TCTGAGGCCT GAGTGGCATG AGGGAGGCAG AGCGGGTCCC
2701 ACTGTCCCCA CACTGGCCCA GGCTGTGCAG GTGTGCCTGG GCCGCCTAGG
2751 GTGGGGCTCA GCCAGGGGCT GCCCTCGGCA GGGTGGGGGA TTTGCCAGCG
2801 TGGCCCTCCC TCCAGCAGCA GCTGCCCTGG GCTGGGCCAC GAGAAGCCCT
2851 AGGAGCCCCT GGGGACAGAC ACACAGCCCC TGCCTCTGTA GGAGACTGTC
2901 CTGTCCTGTG AGCGCCCTGT CCTCCGACCC CGATGCCCAC TCGGGGGCAT
2951 GCCTAGTCCA TGCGCGTAGG GACAGGCCCT CCCTCACCCA TCTACCCCCA
3001 CGGCACTAAC CCCTGGCAGC CCTGCCCAGC CTCGCACCCG CATGGGGACA
3051 CAACCGACTC CGGGGACATG CACTCTCGGG CCCTGTGGAG GGACTGGTGC
3101 AGATGCCCAC ACACACACTC AGCCCAGACC CGTTCAACAA ACCCCGCACT
3151 GAGGTTGGTC GAGCGGGAGT GCGGCCAGAG CCTGCCTCGG CCGTCAGGGA
3201 GGACTCCCGG GCTCACTCGA AGGAGGTGCC ACCATTTCAG CTTTGGTAGC
3251 TTTTCTTCTT CTTTTAAATT TTCTAAAGCT CATTAATTGT CTTTGATGTT
3301 TCTTTTGTGA TGACAATAAA ATATCCTTTT TAAGTCTTGT ACTTCGTGAT
3351 GGGAGCCGCC TTCCTGTGTC CACGCGCCTC CTGCCCCCGG TGGGAAGCAC
3401 GGTCAGGAGG AGGCTGGTCC AGCTGCACCT CGGGGGCTCC CTGCACTCGC
3451 CCCCCGCCTC CTGCAGCCAC ACGCATTGCC CGAGCGACCC TCCCTGGCCC
3501 CTGTCACTAC ATGGACCCCT GGGGCTTCTC CTCTTTTCTA CATGGATGCA
3551 GTTTCTCCTC CTGCTGGGCA CGGTGCTGCC TGCCCTGGTC ACTCTGCGGG
3601 GGACAGGGCC TCCAGGGAAA GCTGGGTCGA GGCTGGGAGC TGGCTCAGGC
3651 TGGCCAGGCA GAGCCACAGG GAGGGCCTTC CAGAACCAAC CATGGTCCGA
3701 AGCGAGAGGT GGGTGTCAGA TCCAGACATG ATAAGATACA TTGATGAGTT
3751 TGGACAAACC ACAACTAGAA TGCAGTGAAA AAAATGCTTT ATTTGTGAAA
3801 TTTGTGATGC TATTGCTTTA TTTGTAACCA TTATAAGCTG CAATAAACAA
3851 GTTAACAACA ACAATTGCAT TCATTTTATG TTTCAGGTTC AGGGGGAGGT
3901 GTGGGAGGTT TTTTAAAGCA AGTAAAACCT CTACAAATGT GGTATGGCTG
3951 ATTATGATCT CTAGTCAAGG CACTATACAT CAAATATTCC TTATTAACCC
4001 CTTTACAAAT TAAAAAGCTA AAGGTACACA ATTTTTGAGC ATAGTTATTA
4051 ATAGCAGACA CTCTATGCCT GTGTGGAGTA AGAAAAAACA GTATGTTATG
4101 ATTATAACTG TTATGCCTAC TTATAAAGGT TACAGAATAT TTTTCCATAA
4151 TTTTCTTGTA TAGCAGTGCA GCTTTTTCCT TTGTGGTGTA AATAGCAAAG
4201 CAAGCAAGAG TTCTATTACT AAACACAGCA TGACTCAAAA AACTTAGCAA
4251 TTCTGAAGGA AAGTCCTTGG GGTCTTCTAC CTTTCTCTTC TTTTTTGGAG
4301 GAGTAGAATG TTGAGAGTCA GCAGTAGCCT CATCATCACT AGATGGCATT
4351 TCTTCTGAGC AAAACAGGTT TTCCTCATTA AAGGCATTCC ACCACTGCTC
4401 CCATTCATCA GTTCCATAGG TTGGAATCTA AAATACACAA ACAATTAGAA
4451 TCAGTAGTTT AACACATTAT ACACTTAAAA ATTTTATATT TACCTTAGAG
4501 CTTTAAATCT CTGTAGGTAG TTTGTCCAAT TATGTCACAC CACAGAAGTA

4551 AGGTTCCTTC ACAAAGATCC GGACCAAAGC GGCCATCGTG CCTCCCCACT
4601 CCTGCAGTTC GGGGGCATGG ATGCGCGGAT AGCCGCTGCT GGTTTCCTGG
4651 ATGCCGACGG ATTTGCACTG CCGGTAGAAC TCCGCGAGGT CGTCCAGCCT
4701 CAGGCAGCAG CTGAACCAAC TCGCGAGGGG ATCGAGCCCG GGGTGGGCGA
4751 AGAACTCCAG CATGAGATCC CCGCGCTGGA GGATCATCCA GCCGGCGTCC
4801 CGGAAAACGA TTCCGAAGCC CAACCTTTCA TAGAAGGCGG CGGTGGAATC
4851 GAAATCTCGT GATGGCAGGT TGGGCGTCGC TTGGTCGGTC ATTTCGAACC
4901 CCAGAGTCCC GCTCAGAAGA ACTCGTCAAG AAGGCGATAG AAGGCGATGC
4951 GCTGCGAATC GGGAGCGGCG ATACCGTAAA GCACGAGGAA GCGGTCAGCC
5001 CATTCGCCGC CAAGCTCTTC AGCAATATCA CGGGTAGCCA ACGCTATGTC
5051 CTGATAGCGG TCCGCCACAC CCAGCCGGCC ACAGTCGATG AATCCAGAAA
5101 AGCGGCCATT TTCCACCATG ATATTCGGCA AGCAGGCATC GCCATGGGTC
5151 ACGACGAGAT CCTCGCCGTC GGGCATGCGC GCCTTGAGCC TGGCGAACAG
5201 TTCGGCTGGC GCGAGCCCCT GATGCTCTTC GTCCAGATCA TCCTGATCGA
5251 CAAGACCGGC TTCCATCCGA GTACGTGCTC GCTCGATGCG ATGTTTCGCT
5301 TGGTGGTCGA ATGGGCAGGT AGCCGGATCA AGCGTATGCA GCCGCCGCAT
5351 TGCATCAGCC ATGATGGATA CTTTCTCGGC AGGAGCAAGG TGAGATGACA
5401 GGAGATCCTG CCCCGGCACT TCGCCCAATA GCAGCCAGTC CCTTCCCGCT
5451 TCAGTGACAA CGTCGAGCAC AGCTGCGCAA GGAACGCCCG TCGTGGCCAG
5501 CCACGATAGC CGCGCTGCCT CGTCCTGCAG TTCATTCAGG GCACCGGACA
5551 GGTCGGTCTT GACAAAAAGA ACCGGGCGCC CCTGCGCTGA CAGCCGGAAC
5601 ACGGCGGCAT CAGAGCAGCC GATTGTCTGT TGTGCCCAGT CATAGCCGAA
5651 TAGCCTCTCC ACCCAAGCGG CCGGAGAACC TGCGTGCAAT CCATCTTGTT
5701 CAATCATGCG AAACGATCCT CATCCTGTCT CTTGATCAGA TCTTGATCCC
5751 CTGCGCCATC AGATCCTTGG CGGCAAGAAA GCCATCCAGT TTACTTTGCA
5801 GGGCTTCCCA ACCTTACCAG AGGGCGCCCC AGCTGGCAAT TCCGGTTCGC
5851 TTGCTGTCCA TAAAACCGCC CAGTCTAGCT ATCGCCATGT AAGCCCACTG
5901 CAAGCTACCT GCTTTCTCTT TGCGCTTGCG TTTTCCCTTG TCCAGATAGC
5951 CCAGTAGCTG ACATTCATCC GGGGTCAGCA CCGTTTCTGC GGACTGGCTT
6001 TCTACGTGTT CCGCTTCCTT TAGCAGCCCT TGCGCCCTGA GTGCTTGCGG
6051 CAGCGTGAAG CTTTTTGCAA AAGCCTAGGC CTCCAAAAAA GCCTCCTCAC
6101 TACTTCTGGA ATAGCTCAGA GGCCGAGGCG GCCTCGGCCT CTGCATAAAT
6151 AAAAAAAATT AGTCAGCCAT GGGGCGGAGA ATGGGCGGAA CTGGGCGGAG
6201 TTAGGGGCGG GATGGGCGGA GTTAGGGGCG GGACTATGGT TGCTGACTAA
6251 TTGAGATGCA TGCTTTGCAT ACTTCTGCCT GCTGGGGAGC CTGGGGACTT
6301 TCCACACCTG GTTGCTGACT AATTGAGATG CATGCTTTGC ATACTTCTGC
6351 CTGCTGGGGA GCCTGGGGAC TTTCCACACC CTAACTGACA CACATTCCAC
6401 AGCTGCCTCG CGCGTTTCGG TGATGACGGT GAAAACCTCT GACACATGCA
6451 GCTCCCGGAG ACGGTCACAG CTTGTCTGTA AGCGGATGCC GGGAGCAGAC
6501 AAGCCCGTCA GGGCGCGTCA GCGGGTGTTG GCGGGTGTCG GGGCGCAGCC
6551 ATGACCCAGT CACGTAGCGA TAGCGGAGTG TATACTGGCT TAACTATGCG

6601 GCATCAGAGC AGATTGTACT GAGAGTGCAC CATATGCGGT GTGAAATACC
6651 GCACAGATGC GTAAGGAGAA AATACCGCAT CAGGCGCTCT TCCGCTTCCT
6701 CGCTCACTGA CTCGCTGCGC TCGGTCGTTC GGCTGCGGCG AGCGGTATCA
6751 GCTCACTCAA AGGCGGTAAT ACGGTTATCC ACAGAATCAG GGGATAACGC
6801 AGGAAAGAAC ATGTGAGCAA AAGGCCAGCA AAAGGCCAGG AACCGTAAAA
6851 AGGCCGCGTT GCTGGCGTTT TTCCATAGGC TCCGCCCCCC TGACGAGCAT
6901 CACAAAAATC GACGCTCAAG TCAGAGGTGG CGAAACCCGA CAGGACTATA
6951 AAGATACCAG GCGTTTCCCC CTGGAAGCTC CCTCGTGCGC TCTCCTGTTC
7001 CGACCCTGCC GCTTACCGGA TACCTGTCCG CCTTTCTCCC TTCGGGAAGC
7051 GTGGCGCTTT CTCATAGCTC ACGCTGTAGG TATCTCAGTT CGGTGTAGGT
7101 CGTTCGCTCC AAGCTGGGCT GTGTGCACGA ACCCCCCGTT CAGCCCGACC
7151 GCTGCGCCTT ATCCGGTAAC TATCGTCTTG AGTCCAACCC GGTAAGACAC
7201 GACTTATCGC CACTGGCAGC AGCCACTGGT AACAGGATTA GCAGAGCGAG
7251 GTATGTAGGC GGTGCTACAG AGTTCTTGAA GTGGTGGCCT AACTACGGCT
7301 ACACTAGAAG GACAGTATTT GGTATCTGCG CTCTGCTGAA GCCAGTTACC
7351 TTCGGAAAAA GAGTTGGTAG CTCTTGATCC GGCAAACAAA CCACCGCTGG
7401 TAGCGGTGGT TTTTTTGTTT GCAAGCAGCA GATTACGCGC AGAAAAAAAG
7451 GATCTCAAGA AGATCCTTTG ATCTTTTCTA CGGGGTCTGA CGCTCAGTGG
7501 AACGAAAACT CACGTTAAGG GATTTTGGTC ATGAGATTAT CAAAAAGGAT
7551 CTTCACCTAG ATCCTTTTAA ATTAAAAATG AAGTTTTAAA TCAATCTAAA
7601 GTATATATGA GTAAACTTGG TCTGACAGTT ACCAATGCTT AATCAGTGAG
7651 GCACCTATCT CAGCGATCTG TCTATTTCGT TCATCCATAG TTGCCTGACT
7701 CCCCGTCGTG TAGATAACTA CGATACGGGA GGGCTTACCA TCTGGCCCCA
7751 GTGCTGCAAT GATACCGCGA GACCCACGCT CACCGGCTCC AGATTTATCA
7801 GCAATAAACC AGCCAGCCGG AAGGGCCGAG CGCAGAAGTG GTCCTGCAAC
7851 TTTATCCGCC TCCATCCAGT CTATTAATTG TTGCCGGGAA GCTAGAGTAA
7901 GTAGTTCGCC AGTTAATAGT TTGCGCAACG TTGTTGCCAT TGCTGCAGGC
7951 ATCGTGGTGT CACGCTCGTC GTTTGGTATG GCTTCATTCA GCTCCGGTTC
8001 CCAACGATCA AGGCGAGTTA CATGATCCCC CATGTTGTGC AAAAAAGCGG
8051 TTAGCTCCTT CGGTCCTCCG ATCGTTGTCA GAAGTAAGTT GGCCGCAGTG
8101 TTATCACTCA TGGTTATGGC AGCACTGCAT AATTCTCTTA CTGTCATGCC
8151 ATCCGTAAGA TGCTTTTCTG TGACTGGTGA GTACTCAACC AAGTCATTCT
8201 GAGAATAGTG TATGCGGCGA CCGAGTTGCT CTTGCCCGGC GTCAACACGG
8251 GATAATACCG CGCCACATAG CAGAACTTTA AAAGTGCTCA TCATTGGAAA
8301 ACGTTCTTCG GGGCGAAAAC TCTCAAGGAT CTTACCGCTG TTGAGATCCA
8351 GTTCGATGTA ACCCACTCGT GCACCCAACT GATCTTCAGC ATCTTTTACT
8401 TTCACCAGCG TTTCTGGGTG AGCAAAAACA GGAAGGCAAA ATGCCGCAAA
8451 AAAGGGAATA AGGGCGACAC GGAAATGTTG AATACTCATA CTCTTCCTTT
8501 TTCAATATTA TTGAAGCATT TATCAGGGTT ATTGTCTCAT GAGCGGATAC
8551 ATATTTGAAT GTATTTAGAA AAATAAACAA ATAGGGGTTC CGCGCACATT
8601 TCCCCGAAAA GTGCCACCTG ACGTCTAAGA AACCATTATT ATCATGACAT

8651 TAACCTATAA AAATAGGCGT ATCACGAGGC CCTTTCGTCT TCAAGAATTC
8701 GAGCTCGGTA CCCATCAGCC AAAAAGCATG CCTGCCACAC AACATCAATT
8751 TCTGGAAAAC GCTACACTTA ATTATTTCTA GTAGAACAGC TCTTTGGTTT
8801 GCCAAAAAGA ATCACCTATA GTGGCATCTA AGCACAAAAA GGAGAAAAAA
8851 ATCACAAAGA AATGATTGAG AGGCATAATA AAAATTATCA AAAAATTATG
8901 AGTTTTACGA TTTCATCTTT TTCCAAGTTG AAATCATAGG GTGGCTTTAA
8951 CACAGTGACA AGGAATGTGC ATGCTGCCAT TATGGTGCTC TGCCTAAAAT
9001 GGTTGGAGCC TTGTCATGCT ACAGAGAAAC TGTCATACAG CAGGGGGTGC
9051 CAAATTTCCA TATTTTTTTA TATCATTGAG CAGGTGCACA GAAGACCAGA
9101 AAGCACTTTC TATCAGGCTG GCCTTCCTCT TCCTTTCCAG TATGAAGCAA
9151 AAACTGCCAA TGAAACTAGC AATTGTTAAA TTCCTTTTTC AAACAGTATT
9201 TGTGCTATCA GAACATAGTG CATTCAAAAG TCTAGCCTGA GAGAACAACC
9251 CAGTTTTATT CATTCCTCCT ACTACCTCTC TCATTCCCAC TGTTTGTGTT
9301 CTCCCTCCCA TTTTAATTGT CTATCTAGTC CAAACTAAGC ACACGATCCA
9351 GTCCACATTA AACAACATGT TTTCACTTTA AGTCAAATAC AAGACACCTT
9401 TAATATCAGC CCTTGTTCAT AATCGTGCTT CTAGTGACTT AATGTACATG
9451 TCACACTGTA CTGTTGGGTT TTGTGTCTCA TCATGAACAA TGTTGTGAAG
9501 GTATTAAGTG GAGAGTAAGC AGAATTAGAT TCCTCTAATG ATGCACACCC
9551 ACACTAAGAG CAGAAATAAT ATTAAAAATA GAAAAAAAAG TTTTACATGA
9601 GATTTCAAAT ACCCAGGTAT GAGCTGCAGT TTCTTCAAGT TAAAGCATCG
9651 AGGTTGTCAG TTACACTATT ACAGGAAACA TATGCAGAGT TTTTATTTTA
9701 GTATATTAGT TTTCACATAT GTGGAATTAC TATTAAACTA TTCTTTCTTT
9751 TCAAATGCTT ACCATTGTAA ATGAGTTTGT GACTTTGTGT AGGTGAGTGC
9801 ACATGACTCT GGATGCCTAA GAGGACTGAA GAAGTTGGAG TTATAGGTAG
9851 TTTTATTCTA CTTGACTGTT CAGTGCTAAA AATACAACTG AGGTCCTTTA
9901 AACTGCTGTT CATGAACTTC TTAATTGATA TATCTCATGA GATCTCTAAA
9951 CTATTTTTAT TATGACACGT TTCACCATTT TCACTGTAAC GATTTTTATG
10001 TTTTATATTA ATGTAACTAT ATGACACTTC CCAAAATCCC CATATTCACA
10051 ATTGAACTGT TTCAAAGTTT TACCTTGACT TATGGGAAAT GAAAACCCAC
10101 ATTTTATAAT TTTAAAATGA AATGTTTATT TTATATTTCT GCAAATTTCA
10151 CAAGGAAAGA TTAGTCACTG GTGTGTGAGA GCAGAGGAGC ATAAGAGTTC
10201 AGGAATAGAA TCCATTATGA TTCTGGAGGC AAGGAAGAAC TGATGCCAAG
10251 GTTTCAGTAT AAGAGCAGTA TCCACTGGAA AGGATAAAGT CACTACATCT
10301 GAGCACAGAG CAGGACATCT ACATAATGAG TGGTCACTAA TGGGCCACTG
10351 TTACACTGTT ATATGTATAA GGCTCAAGAA TGAGCACTGA GGCTGTAAGG
10401 TGTATGGGTG AGGACATCAG GATGTAAACC CAGCTCAGGT AGAGGACTCA
10451 GAGGACAGCA CAGTCAGCAT GAACTAATAA ACATCAGATA AGATAAGGCA
10501 CAAGCTCAGC TATATAGGGT AAGGGATCTT TGTAAATCTG ATTGTGCATC
10551 CAGTCTAGTT CAATGTGACT TAGGAAGCCC AGTCATATGC AAATCTAGAG
10601 AAGACTTTAG AGTAGAAATC TGAGGCTCAC CTCACATACC AGCAAGCGAG
10651 TGACCAGTTA GTCTTAAGGC ACCACTTCTT AGACATCATG GCTTGGGTGT

```
10701    GGACCTTGCC ATTCCTGATG GCAGCTGCCC AAAGTAAGAC ATCAGAAAAA
10751    AGAGTTCCAA GGGGAATTGA AGCAGTTCCA TGAATACTCA CCTTCCTGTG
10801    TTCTTTTCAC AGGTGTCCAG GCACAGGTGC AGCTGGTGGA GTCAGGAGCC
10851    GAAGTGAAAA AGCCTGGGGC TTCAGTGAAG GTGTCCTGCA AGGCCTCTGG
10901    ATACACATTC ACTAATTATA TTATCCACTG GGTGAAGCAG GAGCCTGGTC
10951    AGGGCCTTGA ATGGATTGGA TATTTTAATC CTTACAATCA TGGTACTAAG
11001    TACAATGAGA AGTTCAAAGG CAGGGCCACA CTAACTGCAA ACAAATCCAT
11051    CAGCACAGCC TACATGGAGC TCAGCAGCCT GCGCTCTGAG GACACTGCGG
11101    TCTACTACTG TGCAAGATCA GGACCCTATG CCTGGTTTGA CACCTGGGGC
11151    CAAGGGACCA CGGTCACCGT CTCCTCAGGT AAGAATGGCC ACTCTAGGGC
11201    CTTTGTTTTC TGCTGCTGCC TGTGGGATTT CATGAGCATT GCAAAGTTGT
11251    CCTCGGGACA TGTTCCGAGG GGACCTGGGC GGACTGGCCA GGAGGGGACG
11301    GGCACTGGGG TGCCTTGAGG ATCTGGGAGC CTCTGTGGAT TTTCCGATGC
11351    CTTTGGAAAA TGGGACTGAG GTTGGGTGCG TCTGAGACAG TAACTCAGCC
11401    TGGGGGCTTG GTGAAGATCG CCGCACAGCA GCGAGTCCGT GAAATATCTT
11451    ATTTAGACTT GTGAGGTGCG CTGTGTGTCA ATTTACATCT TAAATCCTTT
11501    ATTGGCTGGA AAGAGAATTG TTGGAGTGGG TGAATCCAGC CAGGAGGGAC
11551    GCGGGGGGAT CCA
```

**Example 9: In vitro efficacy of CD45RO/RB binding humanised antibodies**

VHE/humV1 and VHQ/humV1

To determine the efficacy of the CD45R0/RB binding humanised antibodies VHE/humV1 and VHQ/humV1 in comparison to the chimeric antibody the ability to induce apoptosis in human T cells and also the ability to inhibit human T cell proliferation is analysed.

*Cells and reagents*

Peripheral blood mononuclear cells (PBMC) are isolated from leukopheresis samples of healthy human donors with known blood type, but unknown HLA type by centrifugation over Ficoll-Hypaque (Pharmacia LKB). PBMC used as stimulators are first depleted of T and NK cells by using CD3-coated ferromagnetic beads (Miltenyi). Beads and contaminating cells are removed by magnetic field. T cell-depleted PBMC are used as stimulator cells after irradiation (50 Gy). $CD4^+$ T cells are used as responder cells in MLR and are isolated from PBMC with a CD4 T cell negative selection kit (Miltenyi).

The obtained cells are analyzed by FACScan or FACSCalibur (Becton Dickinson & Co., CA) and the purity of the obtained cells is >75%. Cells are suspended in RPMI1640 medium supplemented with 10% heat-inactivated FCS, penicillin, streptomycin and L-glutamine.

*Apoptosis assays*

Human PBMC of three healthy voluntary donors are cultured in growth medium (RPMI1640+10%FCS) overnight (<16h) in the presence of CD45R0/RB binding chimeric mAb, humanized antibodies (VHE/humV1 and VHQ/humV1) or anti-LPS control mAb. If indicated, a cross-linking reagent, $F(ab')_2$-fragment of goat anti-human IgG (Cat.No. 109-006-098, JacksonLab) is included at a μg/ml concentration being twice as high as the sample's anti-CD45 antibodies concentration. The PBS-concentration in all wells introduced by the antibody reagents is kept constant among all samples, namely at 20% (v/v) for samples without cross-linker or at 40% (v/v) for samples with cross-linker. Earlier experiments demonstrate that the amount of PBS does not affect the readout.

After overnight culture in the presence of the antibodies, the samples are subjected to flow cytometry analyses and stained with the apoptosis marker AnnexinV-FITC (Cat.No. 556419, BD/Pharmingen) and the T cell marker CD2-PE (Cat.No. 556609, BD/Pharmingen). The samples are run in a Becton Dickinson FACSCalibur instrument and the data are analyzed using the CellQuest Pro Software.

From the data collected, curves are fitted using the software Origin v7.0300 The equation used for fitting is

$$y = \frac{A_1 - A_2}{1 + (x / x_0)^p} + A_2 \qquad \text{("Sigmoid-Logistic")}$$

$A_1$: final value (for fitting sessions set to "shared " and "floating")
$A_2$: initial value (for fitting sessions set to "shared " and "floating")
p: power
$X_0$: $ED_{50}$; $IC_{50}$ (see below).

In the absence of cross-linker, VHE/humV1 is most effective, with an $ED_{50}$ value of 148±71 nM, followed by VHQ/humV1 with 377±219 nM. CD45R0/RB binding chimeric antibody is less effective with an $ED_{50}$ value of 2440±1205 nM.

In the presence of a cross-linking antiserum, the $ED_{50}$ values are shifted dramatically towards higher efficacy by at least two orders of magnitude. In addition, the presence of cross-linker permitted higher levels of apoptosis at very high antibody concentrations, now reaching up to 80 %, whereas the absence of cross-linker only allowed for up to 50% of apoptosis. In the presence of cross-linker, the curves (antibody concentration / % apoptosis) are bi-modal with two plateaus: the first plateau is reached at low antibody concentrations (~ 5nM), where the apoptosis level corresponds to the maximum level obtained in the absence of cross-linker. The second plateau is reached at high antibody concentrations (~ 500 nM) and apoptosis is observed within 70-80% of the T cell population.

Both CD45R0/RB binding humanised mAb are equally effective and better or equal compared to CD45R0/RB binding chimeric mAb with respect to their ability to induce apoptosis in primary human T cells.

*Mixed Lymphocyte Reaction assays*

One x $10^5$ PBMC or 5 x $10^4$ of $CD4^+$ cells are mixed with 1x $10^5$ or 5 x$10^4$ T cells-depleted irradiated (50 Gy) PBMC in each well of 96-well culture plates in the presence or absence of the different concentrations of mAb.

The mixed cells are cultured for 5 days and proliferation is determined by pulsing the cells with $^3$H-thymidine for the last 16-20 hours of culture. MLR inhibition at each antibody concentration is expressed as percentage inhibition as described in Example 2.

The effect of increasing concentrations of VHE/humV1 and VHQ/humV1 on MLR is evaluated in three responder: stimulator combinations. All antibodies inhibit the MLR in a dose-dependent manner. The $IC_{50}$ values (see above) are similar for the humanized Ab VHE/humV1 (7 ± 7 nM) and VHQ/humV1 (39 ± 54 nM). Both humanised antibodies are more potent in inhibiting MLR than the parental chimeric antibody ($IC_{50}$ of 347 ± 434 nM). As usually seen with MLR experiments, donor variability is high in these experiments.

<u>VHE-N73D/humV1</u>

To address the biologic effect of VHE-N73D/humV1 inducing apoptosis in human peripheral blood mononuclear cells (PBMC), the target PBMC are incubated overnight in the presence of various concentrations of VHE-N73D/humV1 and are subsequently analyzed for binding of the apoptosis marker AnnexinV by flow cytometry analysis: Human PBMC are incubated overnight in 1 ml tissue culture medium containing various concentrations of either VHE-N73D/humV1, or another humanized CD45RO/RB binding molecule variant, VHE/humV1, or chimeric anti-CD45RO/RB mAb or an isotype IgG1 control mAb. In addition, crosslinking $F(ab')_2$ fragments of goat-anti-human Ig Fc at 2x the mass centration of each mAb is added, mimicking crosslinking of the CD45RO/RB humanised antibody by Fc-receptors, which can occur in vivo. On the next day, the cells are washed by centrifugation for 10 min at 400 times the standard gravity force (g) and the medium is removed. Cells are resuspended in FACS buffer (PBS containing 1% v/v FBS, 0.1 % w/v EDTA and 0.1% w/v sodium azide) and seeded into 96-well V-bottom microtiter plates at a cell density of 1x$10^5$ cells per well. Each sample of cells is incubated for 30 minutes at 4°C in 50 μl of FACS buffer containing 100 μg/ml of normal mouse serum to block unspecific binding sites on cells and with phycoerythrin (PE)-conjugated CD2 to identify human T cells. After washing twice by centrifugation, cells are resuspended in 100 μl calcium-proficient AnnexinV-staining buffer (Vendor BD/Pharmingen kit 556419) containing 1:100 v/v FITC-labeled AnnexinV. Upon incubation for 15 minutes at room temperature in the dark, 7-amino actinomycin D (7-AAD) is added to give a final concentration of 1μg/ml and analyzed using a FACSCalibur flow cytometer (Becton Dickinson). $ED_{50}$ values for the antibodies' efficacy to induce apoptosis can be calculated from an analysis of the amount of apoptosis (= intensity of the AnnexinV-FITC fluorescence) induced as a function of antibody concentration using the software Origin 7.5 with a Sigmoidal/Logistic curve fitting equation.

Upon such analyses, a biphasic effect of all tested CD45RO/RB humanised antibody is observed: at low concentrations of antibody, a low level of less than 30% T cell apoptosis is found. The $ED_{50}$ value for reaching this level of apoptosis is calculated as 0.31 ± 0.13 nM for VHE-N73D/humV1. At higher concentrations of antibody, apoptosis can be induced in up to 70% of the T cells. The $ED_{50}$ value for reaching this higher level of apoptosis is calculated as 352 ± 83 nM for VHE-N73D/humV1. In summary, it is found that VHE-N73D/humV1 also induces apoptosis in human T cells, which can be enhanced by crosslinking

**Example 10: Specificity of CD45RB/RO binding molecule**

Chimeric CD45RB/RO binding molecule

The CD45 molecule is expressed on all leukocytes. However, different CD45 isoforms are expressed by the various leukocyte subsets. In order to determine the leukocyte subset reactivity of CD45RB/RO binding chimeric antibody molecule immunofluorescent labeling of human leukocytes with subset-specific markers and simultaneous immunofluorescent labeling with a dye-conjugated CD45RB/RO binding chimeric antibody is performed, followed by flow cytometry analysis.

Briefly, specific subsets of a freshly isolated preparation of human peripheral blood mononuclear cells (PBMC), human platelets, human peripheral blood neutrophils or human bone-marrow derived hematopoietic stem cells are identified by incubation with phycoerythrin-coupled antibodies against CD2 (T lymphocytes), CD14 (monocytes), CD19 (B lymphocytes), CD34 (stem cells), CD42a (platelets), CD56 (natural killer cells) or CD66b (granulocytes). Simultaneous binding of a FITC-labeled chimeric CD45RB/RO binding molecule is detected on T lymphocytes, monocytes, stem cells, natural killer cells and granulocytes, but not on platelets or B lymphocytes.

VHE-N73D/humV1

In order to determine the leukocyte subset reactivity of VHE-N73D/humV1 immunofluorescent labeling of human leukocytes with subset-specific markers and simultaneous immunofluorescent labeling with a dye-conjugated VHE-N73D/humV1 is performed, followed by flow cytometry analysis.

Briefly, specific subsets of a freshly isolated preparation of human peripheral blood mononuclear cells (PBMC) or human peripheral blood neutrophils are identified upon incubation with phycoerythrin-coupled antibodies against CD3, CD4, CD8 (T lymphocytes), CD14 (monocytes), CD16 (natural killer cells and monocytes), CD19 (B lymphocytes), or CD66b (granulocytes). Simultaneous binding of a FITC-labeled VHE-N73D/humV1 is detected on T lymphocytes, monocytes, natural killer cells and granulocytes, but not on B lymphocytes. Thus, VHE-N73D/humV1 molecules do not react with human B lymphocytes.

**Example 11: In vitro induction of suppressor T cells (T regulatory cells) and of functionally paralyzed T cells**

To demonstrate the ability of a CD45RO/RB binding chimeric antibody to induce suppressor T cells, the antibody is included at various concentrations during the generation of CD8+ T cell lines reactive with the antigen matrix protein 1 (MP1) of hemophilus influenza. These lines are generated through repeated co-culture of CD8+ human lymphocytes with CD14+ human monocytes pulsed with the antigen. Later on, CD14+ monocytes can be replaced with a human leukocyte antigen-2 positive cell line as an MP1 antigen-presenting cell (APC). If such MP1-specific CD8+ T cells from a culture including CD45RO/RB binding chimeric antibody are mixed with freshly isolated human CD8+ T cells and this mixture of cells is stimulated with the MP1 antigen on APC, the addition of CD8+ T cells from the culture in the presence of CD45RO/RB binding molecule is able to reduce the IFN-γ production in an antibody-dose-dependent fashion. No CD45RO/RB binding chimeric antibody is present during this IFN-γ assay culture, indicating that the pre-treatment with the CD45RO/RB mAb has induced CD8+ T cells capable of suppressing the activation of freshly isolated T cells. Because of this induction of suppressor T regulatory cells by the CD45RO/RB binding chimeric antibody, the antibody may be useful in diseases, where a dysregulated and/or activated T cell population is thought to contribute to the pathology. Examples of such diseases include autoimmune diseases, transplant rejection, psoriasis, dermatitis, inflammatory bowel disease and allergies.

To demonstrate the ability of a chimeric CD45RO/RB binding molecule to render T cells hyporesponsive (anergic) to further stimulation, i.e. to functionally paralyze T cells, the antibody is included during the generation of CD8+ T cell lines reactive with the antigen matrix protein 1 (MP1) of hemophilus influenza as outlined above. Paralysis is assessed by activating the T cells (exposed prior to CD45RO/RB binding chimeric antibody) with MP1 antigen presented by APC. No CD45RO/RB binding molecule is present in this culture. CD8+ T cells not exposed to CD45RO/RB binding chimeric antibody previously produce IFN-γ upon the mentioned stimulus. In contrast, CD8+ T cells pre-treated with CD45RO/RB binding chimeric antibody show a markedly reduced to inexistent production of this cytokine in response to the antigen-stimulus, demonstrating the CD45RO/RB binding chimeric antibody's ability to functionally paralyze human T cells. Because of this induction of functional T cell hyporesponsiveness by the CD45RO/RB binding molecule, the antibody may be used in diseases, such autoimmune diseases, transplant rejection, psoriasis, dermatitis, inflammatory bowel disease or allergies, where an activated T cell population is thought to contribute to the pathology.

**Example 12: In vivo studies in SCID-hu Skin mice**

The therapeutic benefit of the CD45RB/RO binding chimeric antibody in skin inflammation is tested using SCID mice. Human skin from normal individuals is transplanted onto SCID mice (SCID-hu Skin) and the inflammatory process is mimicked by adoptive transferring of mononuclear leukocytes isolated from human individuals unrelated to the human skin donor.

*Transplantation of human adult skin in SCID mice (SCID-hu Skin mice)*

Two small pieces (1 $cm^2$) of human adult skin (obtained from the West Hungarian Regional Tissue Bank; WHRTB, Gyor) consisting of the entire epidermis, the papillary dermis and part of the reticular dermis, are transplanted at the right and left upper-back sides of SCID mice C.B 17 /GbmsTac-*Prkdc*$^{scid}$ *Lyst*$^{bg}$ mice (Taconic, Germantown, NY) in replacement of mouse skin. The quality of the grafts is monitored during 5-6 weeks following transplantation and successfully transplanted mice (SCID-hu Skin mice, generally >85%) are selected for in vivo testing of CD45RB/RO binding chimeric antibody.

*Engraftment of human mononuclear cells in SCID mice*

Mononuclear leukcytes (Spl) are isolated from human adult spleen biopsies (WHRTB, Gyor) after cell suspension (using a cell dissociation sieve equipped with a size 50 mesh) and standard density gradient procedures. Aliquots of ~5 $x10^8$ Spl are re-suspended in 1.5 ml of RPMI-10% FCS and injected intraperitoneally (i.p.), on experimental day 0, into the SCID-hu Skin mice. These Spl numbers have been found in previous experiments to be sufficient to induce a lethal xeno-GvHD in >90% of the mice within 4-6 weeks after cell transfer.

*Antibody treatment of SCID-hu Skin mice*

SCID-hu Skin mice, reconstituted with human Spl, are treated with CD45RB/RO binding chimeric antibody or with anti-LPS control mAb at day 0, immediately after mononuclear cell injection, at days 3 and 7 and at weekly intervals thereafter. Antibodies are delivered subcutaneously (s.c.) in 100 μl PBS at a final concentration of 1 mg/kg body weight (b.w.).

*Evaluation of anti-CD45 treatment*

The efficacy of CD45RB/RO binding chimeric antibody is assessed by the survival of the transplanted mice and by monitoring the rejection of the skin grafts. The significance of the results is evaluated by the statistical method of survival analysis using the Log-rank test (Mantel method) with the help of Systat v10 software. At the end of the experiment biopsies of human skin grafts and mouse liver, lung, kidney and spleen are obtained from sacrificed mice for histological purposes. All mice are weighed at the beginning (before cell transfer) and throughout the experiment (every two days) as an indirect estimation of their health status. Linear regression lines are generated using the body weight versus days post-PBMC transfer values obtained from each mouse and subsequently, their slopes (control versus anti-CD45 treated mice) are compared using the non parametric Mann-Whitney test.

*Results*

The human skin grafts are very well tolerated by the SCID mice. Initially, the grafts undergo a period of keratinocyte hyperproliferation resulting in the formation of hyperkeratotic crusts. About 5 weeks after transplantation, the crusts fall off the grafts and reveal a tissue containing all the characteristic structures observed in normal human skin. During this process, the human skin grafts fuse with the adjacent mouse skin and generate a network of freshly grown human vessels that connect the grafts with the underlying mouse tissue. The circulating human Spl transferred into SCID-hu Skin mice (at experimental day 0, approx. 6 weeks after skin transplantation) infiltrate the skin grafts and after recognition of alloantigen molecules expressed on the human skin mount an inflammatory response, that has resemblance with the skin inflammatory process occurring in psoriatic skin, and that in some cases completely destroy the graft.

Treatment of these mice with CD45RB/RO binding chimeric antibody suppresses the inflammatory process and prevents the rejection of the human skin grafts. In contrast, the sample obtained from the control treated mouse shows a massive infiltration with multiple signs of necrosis and a dramatic destruction of the epidermis. This process is easily monitored by eye and documented by simple photography of the mice.

Six out of six SCID-hu Skin mice transferred with allogeneic human Spl and treated with control anti-LPS mAb show a strong inflammatory response clearly visible by eye 23 days after mononuclear cell transfer. All mice show considerable lesions, including erythema, scaling and pronounced pustules. In contrast the skin grafts of all mice treated with CD45RB/RO binding chimeric antibody have a normal appearance. The dramatic differences between the two groups of mice is specifically due to the antibody treatment since the human skin of all mice have an identical look at the beginning of the experiment. This aspect is not changed until the second week after cell transfer, the time at which the control group started to developed skin lesions. The experiment is terminated at day 34 after mononuclear cell transfer. By that time, one of the control mice is already dead (day 30) and four other are sacrificed (days 27, 27, 27 and 30) due to a strong xeno-GvHD. The pathologic reactions observed in the antibody control treated mice also correlates with a loss of body weight in these animals.

In contrast, the CD45RB/RO binding chimeric antibody treated group displays a healthy status during the whole experimentation time.

**Example 13: In vivo activities of an CD45RB/RO binding chimeric antibody in an human islet cell transplantation model**

*Mice*

NOD/SCID female mice (Charles River Laboratories, Calco, Italy) are kept under specific pathogen free conditions. Glucose level in the venous tail blood is quantified using Glucometer Elite system (Bayer, Germany). Diabates is induced in NOD/SCID mice by intravenous injection of 180 mg/kg of streptozotocin (Sigma, St.Luis, MO). A diagnosis of diabetes is made after 2 concecutive glucose measurements higher than 250 mg/dl.

*Islet preparations and transplantation*

Pancreata are obtained from heart-beating cadaveric multiorgan donors. Islets are isolated according to the method as described in Bertuzzi et al., Diabetes, 1999, 48: 1971-8. The purified islets are cultured in sterile flasks containing 25 ml of M199 medium (Seromed Biochrom, Berlin, Germany) supplemented with 10% FCS, 1% L-glutamine, 100 units/ml penicillin and 100 μg/ml streptomycin (complete medium). The islets are incubated at 30° in 5% $CO_2$ and 95% humidified air. Mice are anestetized wirg an intraperitoneal injection of avertin and aliquots of 1500 IE of human islets are transplanted under the kidney capsule of recipient diabetic NOD/SCID mice as described in Davalli A.M. et al., Diabetes, 1996, 45: 1161-7. After transplantation, $50x10^6$ freshly isolated PBMC are injected intraperitoneally to NOD/SCID mice.

*Treatment of transplanted mice*

Hu-PBL-NOD/SCID transplanted mice are s.c. treated at day 0, +3 and +5 with 1 mg/kg of an CD45RB/RO binding chimeric antibody. Control mice are treated either with saline solution or with IgG purified mAb (Vinci.Biochem, Italy).

*Histological analysis*

Kidney poles containing human islet graft are snap-frozen in Tissue Tek (Miles Lab., IN) and stores at -70°. 5 μm thick frozen sections are stained with biotinylated mAb against human insulin or human CD3 followed by streptavidin-peroxidase conjugate. Diaminobenzidine (DAKO, Carpenteria, CA) is used as chromogen and hematoxilin as counterstain. Lymphocyte infiltration of the grafts is evaluated on hematoxilin and eosin-stained frozen sections.

*Results*

Normal NOD/SCID mice transplanted with human islets remain normoglycemic up to 100 days post-transplantation, whereas the mean rejection time of hu-PBL-NOD/SCID mice transplanted with human islets is 35±13 days. Short treatment of hu-PBL-NOD/SCID mice transplanted with human islets with an mAb of the present invention significantly prolongs human islets survival with a survival rate of >70% at day 60 and 50% at day 100 post-transplantation.

Histological analysis of human islet grafts performed 100 day post-transplantational in hu-PBL-NOD/SCID mice show a massive infiltration of $CD3^+$, $CD4^+$ and $CD8^+$ positive T cells in control recipient mice. In contrast, in recipient mice treated with an CD45RB/RO binding chimeric antibody a significantly lower infiltration of human cells is observed. The positive staining for insulin demonstrates the graft function in hu-PBL-NOD/SCID recipient mice treated with an CD45RB/RO binding chimeric antibody compared to control recipient mice. In islet -transplanted and with an CD45RB/RO binding chimeric antibody treated hu-PBL-NOD/SCID mice lower amounts of human IFN-γ are detected in serum compared to control recipient mice.

These data indicate that short term treatment with an CD45RB/RO binding chimeric antibody leads to prolonged human islet allograft survival by preventing graft-infiltration and by inhibiting the leukocyte-mediated rejection reaction in vivo.

## Claims

1. A humanised antibody having binding specificity for both CD45RO and CD45RB comprising a heavy chain variable region of SEQ ID NO: 31 or 32 and a light chain variable region of SEQ ID NO: 7 or SEQ ID NO: 8.

2. A humanised antibody of claim 1 having binding specificity for both CDR45RO and CD45RB comprising:

   - a heavy chain variable region of SEQ ID NO: 31 and a light chain variable region of SEQ ID NO: 7,
   - a heavy chain variable region of SEQ ID NO: 31 and a light chain variable region of SEQ ID NO: 8,
   - a heavy chain variable region of SEQ ID NO: 32 and a light chain variable region of SEQ ID NO: 7, or
   - a heavy chain variable region of SEQ ID NO: 32 and a light chain variable region of SEQ ID NO: 8.

3. Isolated polynucleotides encoding the humanised antibody of claims 1 or 2.

4. An expression vector comprising the polynucleotides of claims 3 which vector is capable of producing a humanised antibody when said vector is present in a compatible host cell.

5. An isolated host cell which comprises an expression vector of claim 4.

6. A humanised antibody of claims 1 or 2 for use as a pharmaceutical.

7. The antibody of claim 6 for use in the treatment and/or prophylaxis of autoimmune diseases, transplant rejection, psoriasis, dermatitis, inflammatory bowel disease and/or allergies.

8. The antibody of claim 7 in the treatment and/or prophylaxis of graft versus host disease (GVHD).

9. The antibody of claim 7 in the preparation of a medicament for treatment of pancreatic islet cell transplant rejection.

10. A pharmaceutical composition comprising the antibody of claim 1 or 2 in association with at least one pharmaceutically acceptable carrier or diluent.

## Patentansprüche

1. Humanisierter Antikörper mit Bindungsspezifität sowohl für CD45RO als auch für CD45RB, der eine variable schwere Kettenregion der SEQ ID NO: 31 oder 32 und eine variable leichte Kettenregion der SEQ IN NO: 7 oder SEQ ID NO: 8 umfasst.

2. Humanisierter Antikörper nach Anspruch 1 mit Bindungsspezifität sowohl für CD45RO als auch für CD45RB, umfassend:

   - eine variable schwere Kettenregion der SEQ ID NO: 31 und eine variable leichte Kettenregion der SEQ ID NO: 7,
   - eine variable schwere Kettenregion der SEQ ID NO: 32 und eine variable leichte Kettenregion der SEQ ID NO: 8,
   - eine variable schwere Kettenregion der SEQ ID NO: 32 und eine variable leichte Kettenregion der SEQ ID NO: 7, oder
- eine variable schwere Kettenregion der SEQ ID NO: 32 und eine variable leichte Kettenregion der SEQ ID NO: 8.

3. Isolierte Polynukleotide, die den humanisierten Antikörper nach den Ansprüchen 1 und 2 codieren.

4. Expressionsvektor, umfassend die Polynukleotide nach Anspruch 3, wobei der Vektor in der Lange ist, einen humanisierten Antikörper zu produzieren, wenn dieser Vektor in einer kompatiblen Wirtszelle vorliegt.

5. Isolierte Wirtszelle, die einen Expressionsvektor nach Anspruch 4 umfasst.

6. Humanisierter Antikörper nach den Ansprüchen 1 oder 2 für die Verwendung als Pharmazeutikum.

7. Antikörper nach Anspruch 6 für die Verwendung zur Behandlung und/oder Prophylaxe von Autoimmunerkrankungen, Transplantatabstoßung, Psoriasis, Dermatitis, entzündlicher Darmerkrankung und/oder Allergien.

8. Antikörper nach Anspruch 7 zur Behandlung und/oder Prophylaxe von Transplantat-Wirt-Reaktion (GVHD).

9. Antikörper nach Anspruch 7 zur Herstellung eines Arzneimittels für die Behandlung von Abstoßung eines pankreatischen Inselzelltransplantats.

10. Pharmazeutische Zusammensetzung, umfassend den Antikörper nach Anspruch 1 oder 2 in Verbindung mit mindestens einem pharmazeutisch annehmbaren Träger oder Verdünnungsmittel.

## Revendications

1. Anticorps humanisé ayant une spécificité de liaison pour les CD45RO et CD45RB, comprenant une région variable de chaîne lourde de SEQ ID n° : 31 ou 32 et une région variable de chaîne légère de SEQ ID n° : 7 ou SEQ ID n° : 8.

2. Anticorps humanisé, selon la revendication 1, ayant une spécificité de liaison pour les CD45RO et CD45RB, comprenant :

- Une région variable de chaîne lourde de SEQ ID n° : 31 et une région variable de chaîne légère de SEQ ID n° : 7,
- Une région variable de chaîne lourde de SEQ ID n° : 31 et une région variable de chaîne légère de SEQ ID n° : 8,
- Une région variable de chaîne lourde de SEQ ID n° : 32 et une région variable de chaîne légère de SEQ ID n° : 7, ou
- Une région variable de chaîne lourde de SEQ ID n°: 32 et une région variable de chaîne légère de SEQ ID n° : 8.

3. Polynucléotides isolés codant pour l'anticorps humanisé selon les revendications 1 ou 2.

4. Vecteur d'expression comprenant les polynucléotides selon la revendication 3, lequel vecteur est capable de produire un anticorps humanisé quand ledit vecteur est présent dans une cellule hôte compatible.

5. Cellule hôte isolée qui comprend un vecteur d'expression selon la revendication 4.

6. Anticorps humanisé, selon les revendications 1 ou 2, pour être utilisé comme substance pharmaceutique.

7. Anticorps, selon la revendication 6, pour être utilisé dans le traitement et/ou la prophylaxie de maladies autoimmunes, d'un rejet de greffe, du psoriasis, d'une dermatite, d'une maladie inflammatoire de l'intestin et/ou d'allergies.

8. Anticorps, selon la revendication 7, dans le traitement et/ou la prophylaxie de la maladie du rejet du greffon contre l'hôte (GVHD).

9. Anticorps, selon la revendication 7, dans la préparation d'un médicament destiné au traitement d'un rejet de greffe de cellules d'îlots pancréatiques.

10. Composition pharmaceutique comprenant l'anticorps, selon la revendication 1 ou la 2, en association avec au moins un transporteur ou un diluant acceptable d'un point de vue pharmaceutique.

Figure 1

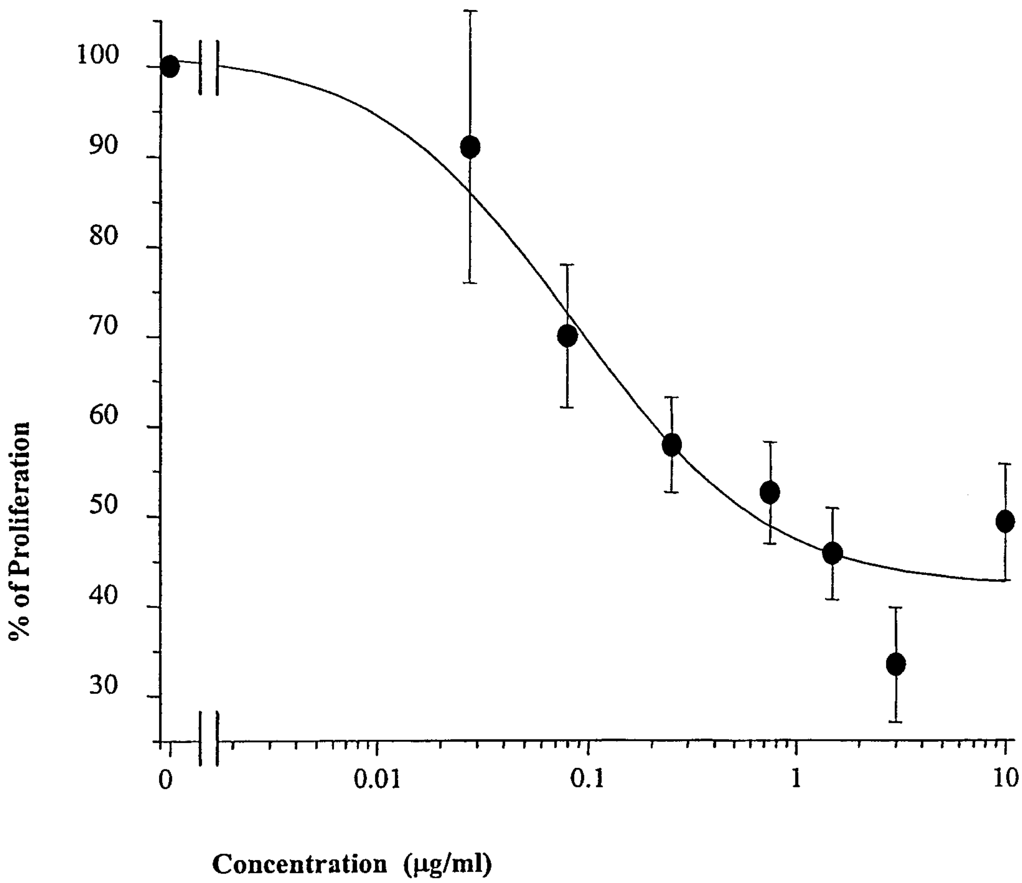
100
90
80
70
60
50
40
30
% of Proliferation
0
0.01
0.1
1
10
Concentration (µg/ml)

Figure 2

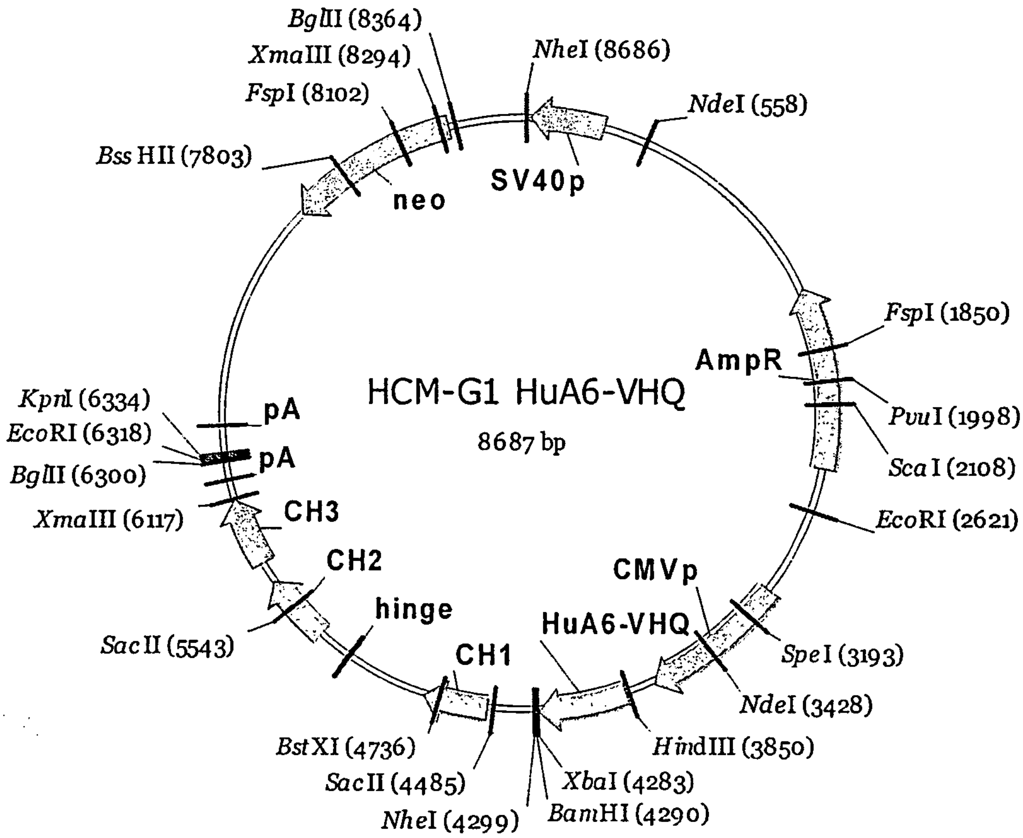
BglII (8364)
XmaIII (8294)
FspI (8102)
BssHII (7803)
NheI (8686)
NdeI (558)
neo
SV40p
FspI (1850)
AmpR
PvuI (1998)
ScaI (2108)
EcoRI (2621)
HCM-G1 HuA6-VHQ
8687 bp
KpnI (6334)
EcoRI (6318)
BglII (6300)
XmaIII (6117)
pA
pA
CH3
CH2
hinge
SacII (5543)
CH1
HuA6-VHQ
CMVp
SpeI (3193)
NdeI (3428)
HindIII (3850)
XbaI (4283)
BamHI (4290)
NheI (4299)
SacII (4485)
BstXI (4736)

Figure 3

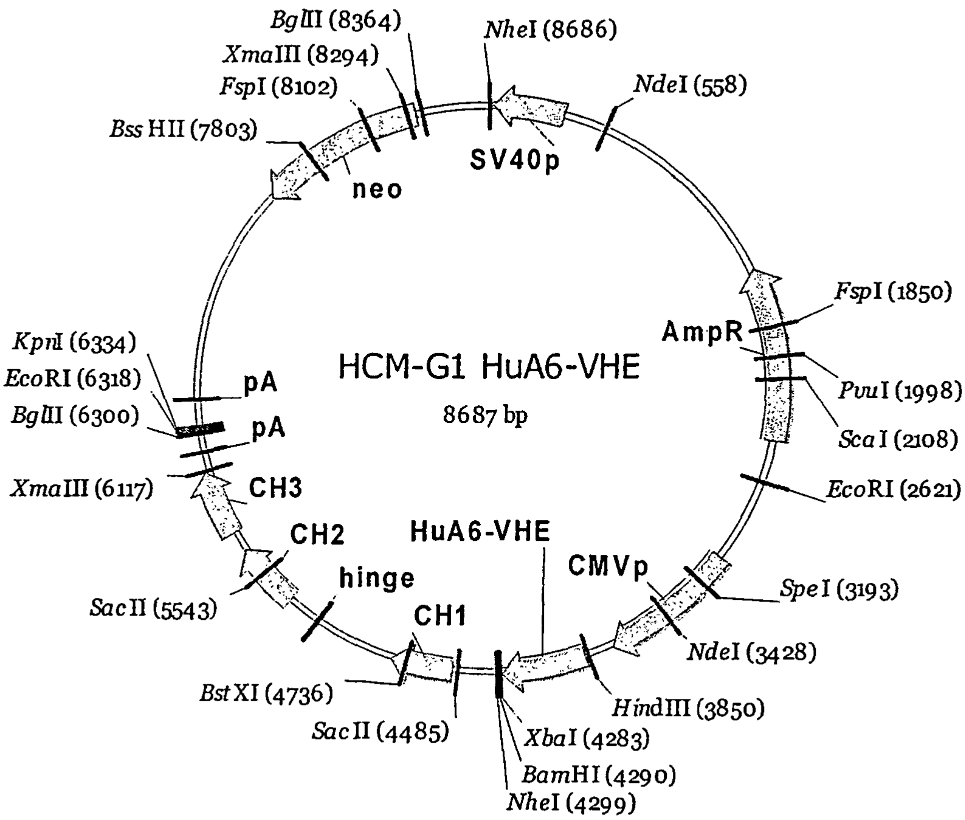
BglII (8364)
XmaIII (8294)
FspI (8102)
BssHII (7803)
NheI (8686)
NdeI (558)
SV40p
neo
FspI (1850)
AmpR
PvuI (1998)
ScaI (2108)
EcoRI (2621)
KpnI (6334)
EcoRI (6318)
BglII (6300)
XmaIII (6117)
pA
pA
CH3
HCM-G1 HuA6-VHE
8687 bp
CH2
HuA6-VHE
CMVp
SpeI (3193)
SacII (5543)
hinge
CH1
NdeI (3428)
BstXI (4736)
HindIII (3850)
SacII (4485)
XbaI (4283)
BamHI (4290)
NheI (4299)

Figure 4

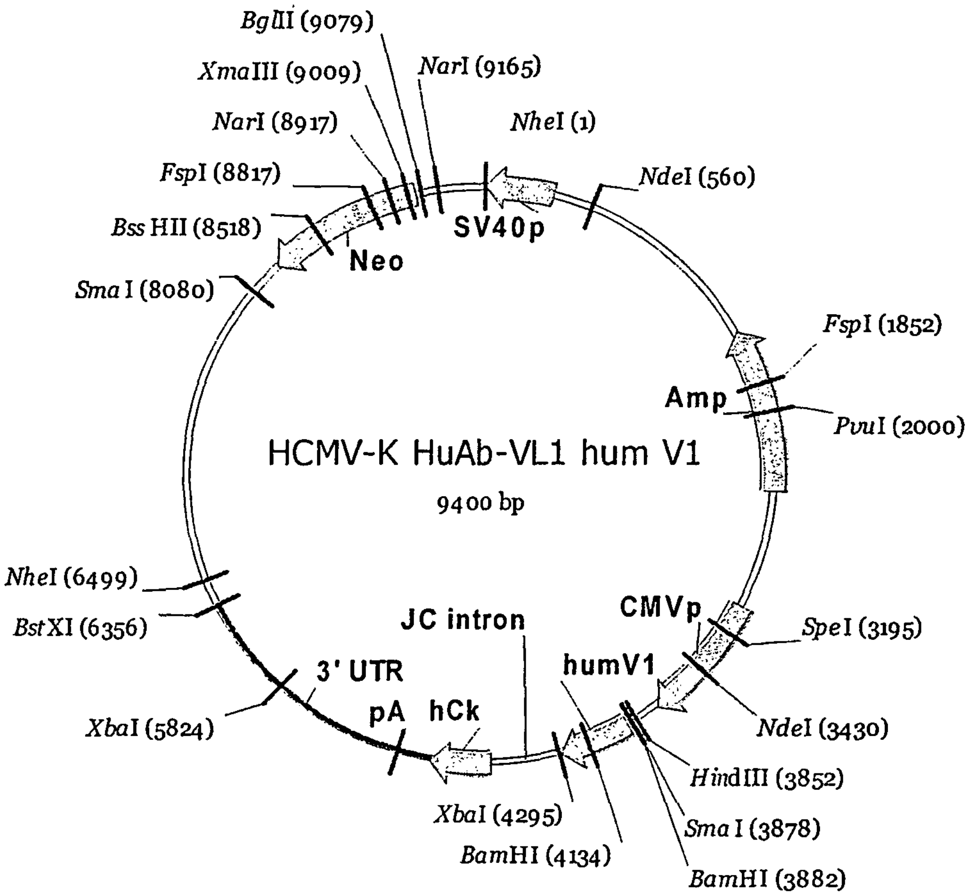
BglII (9079)
XmaIII (9009)
NarI (9165)
NarI (8917)
NheI (1)
FspI (8817)
NdeI (560)
BssHII (8518)
SV40p
Neo
SmaI (8080)
FspI (1852)
Amp
PvuI (2000)
HCMV-K HuAb-VL1 hum V1
9400 bp
NheI (6499)
BstXI (6356)
JC intron
CMVp
SpeI (3195)
3' UTR
humV1
XbaI (5824)
pA
hCk
NdeI (3430)
HindIII (3852)
XbaI (4295)
SmaI (3878)
BamHI (4134)
BamHI (3882)

Figure 5

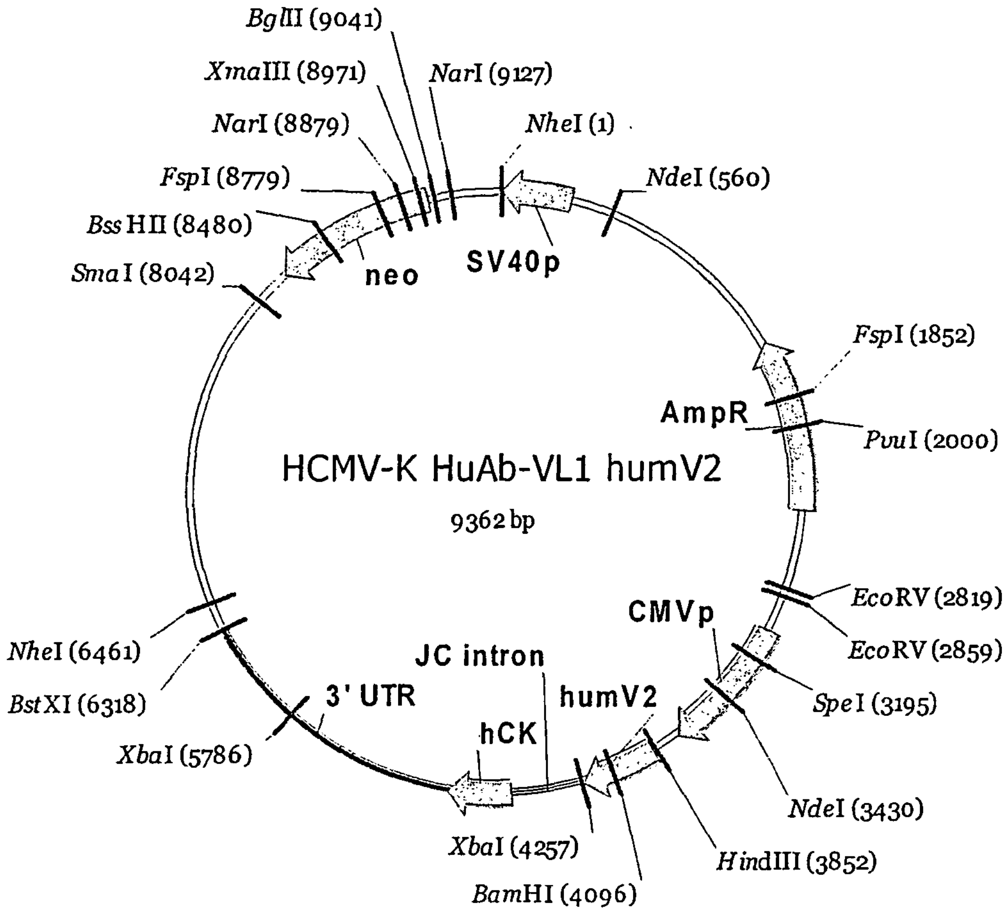

BglII (9041)
XmaIII (8971)
NarI (9127)
NarI (8879)
NheI (1)
FspI (8779)
NdeI (560)
BssHII (8480)
SmaI (8042)
neo
SV40p
FspI (1852)
AmpR
PvuI (2000)
HCMV-K HuAb-VL1 humV2
9362 bp
EcoRV (2819)
CMVp
EcoRV (2859)
NheI (6461)
JC intron
SpeI (3195)
BstXI (6318)
3' UTR
humV2
hCK
XbaI (5786)
NdeI (3430)
XbaI (4257)
HindIII (3852)
BamHI (4096)

Figure 6

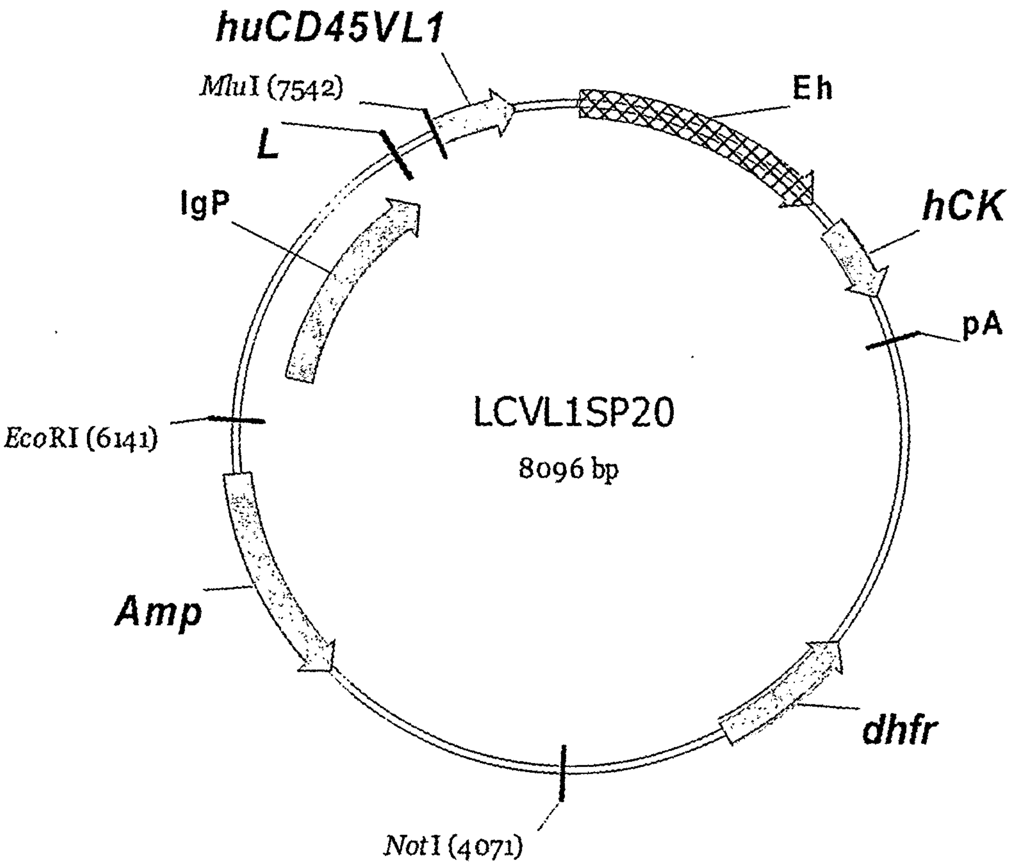
huCD45VL1
MluI (7542)
L
Eh
IgP
hCK
pA
EcoRI (6141)
LCVL1SP20
8096 bp
Amp
dhfr
NotI (4071)

Figure 7

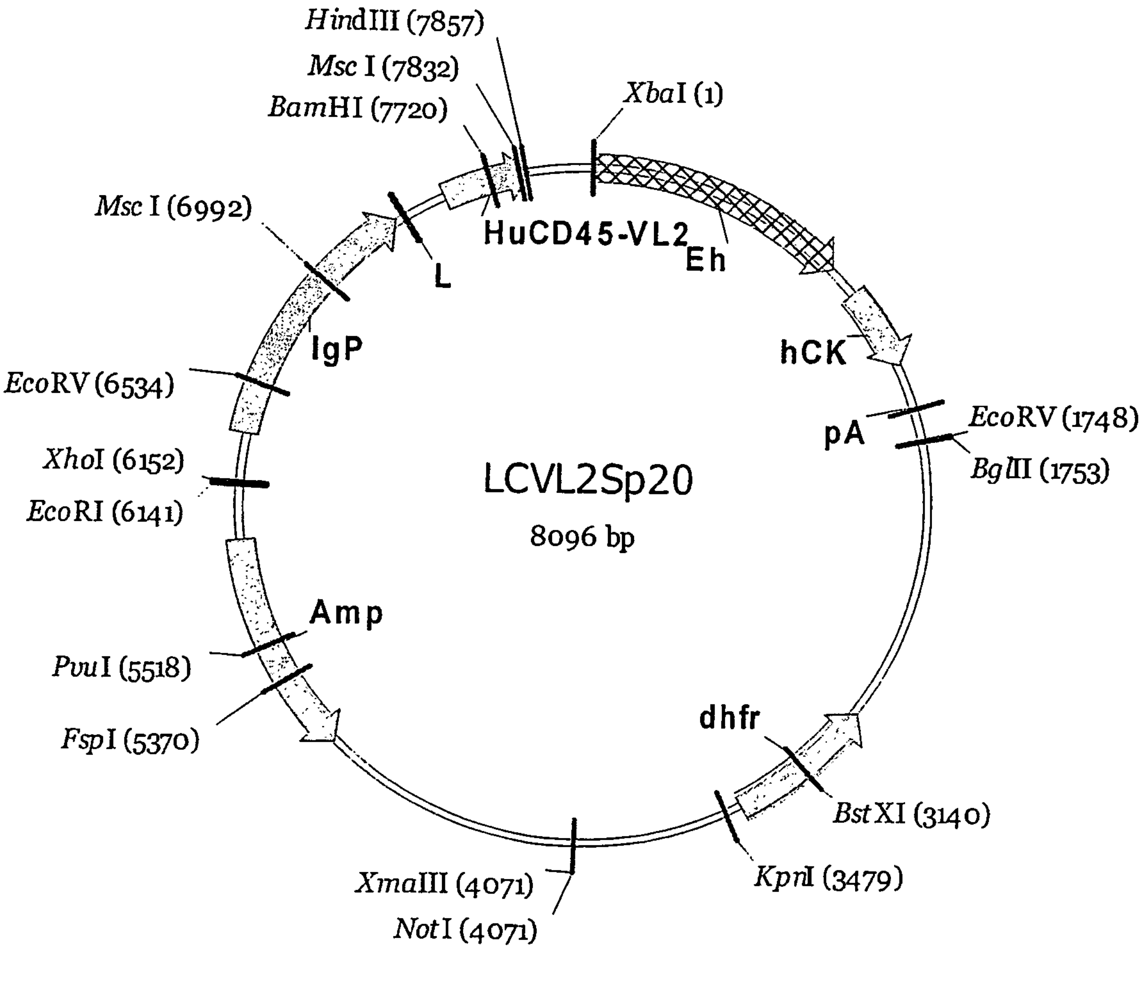
HindIII (7857)
MscI (7832)
BamHI (7720)
XbaI (1)
MscI (6992)
HuCD45-VL2
Eh
L
IgP
hCK
EcoRV (6534)
pA
EcoRV (1748)
BglII (1753)
XhoI (6152)
EcoRI (6141)
LCVL2Sp20
8096 bp
Amp
PvuI (5518)
FspI (5370)
dhfr
BstXI (3140)
KpnI (3479)
XmaIII (4071)
NotI (4071)

Figure 8

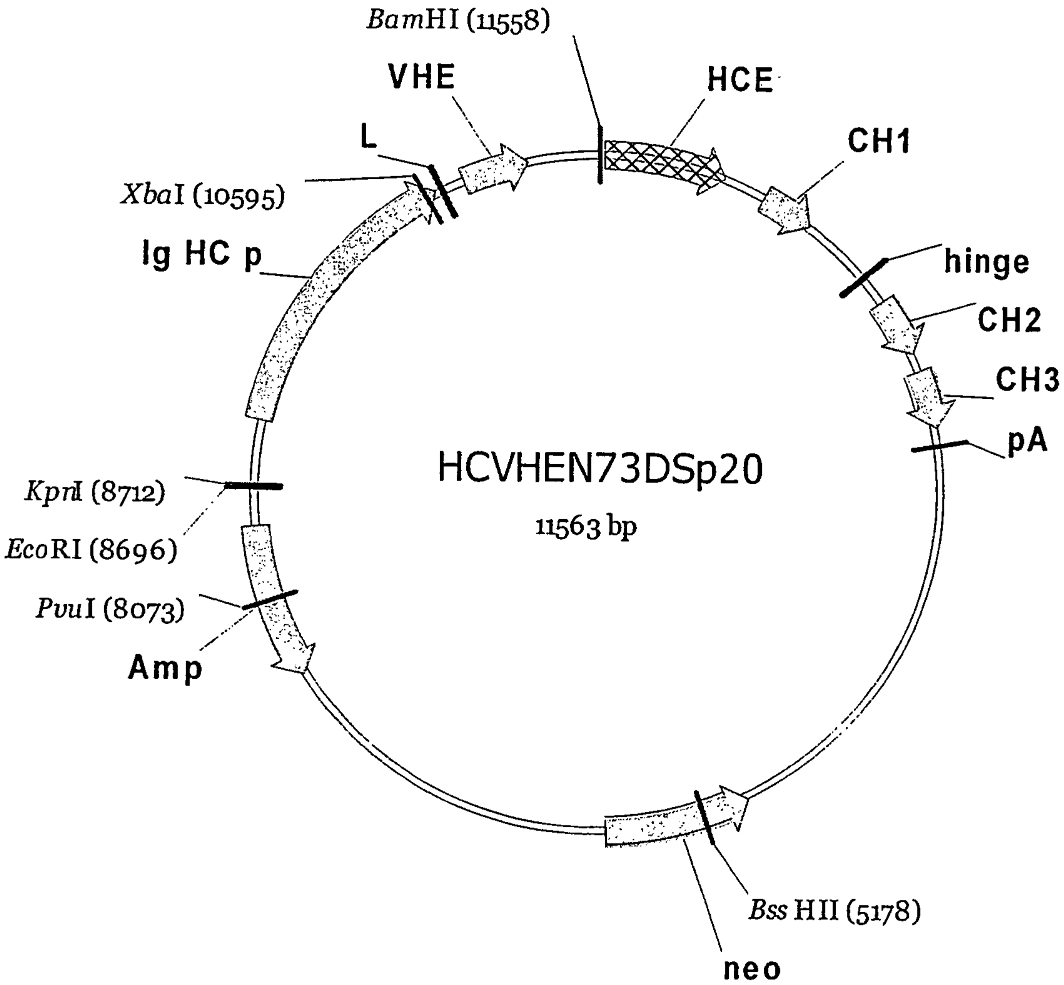

BamHI (11558)
VHE
HCE
L
CH1
XbaI (10595)
Ig HC p
hinge
CH2
CH3
pA
HCVHEN73DSp20
11563 bp
KpnI (8712)
EcoRI (8696)
PvuI (8073)
Amp
BssHII (5178)
neo

Figure 9

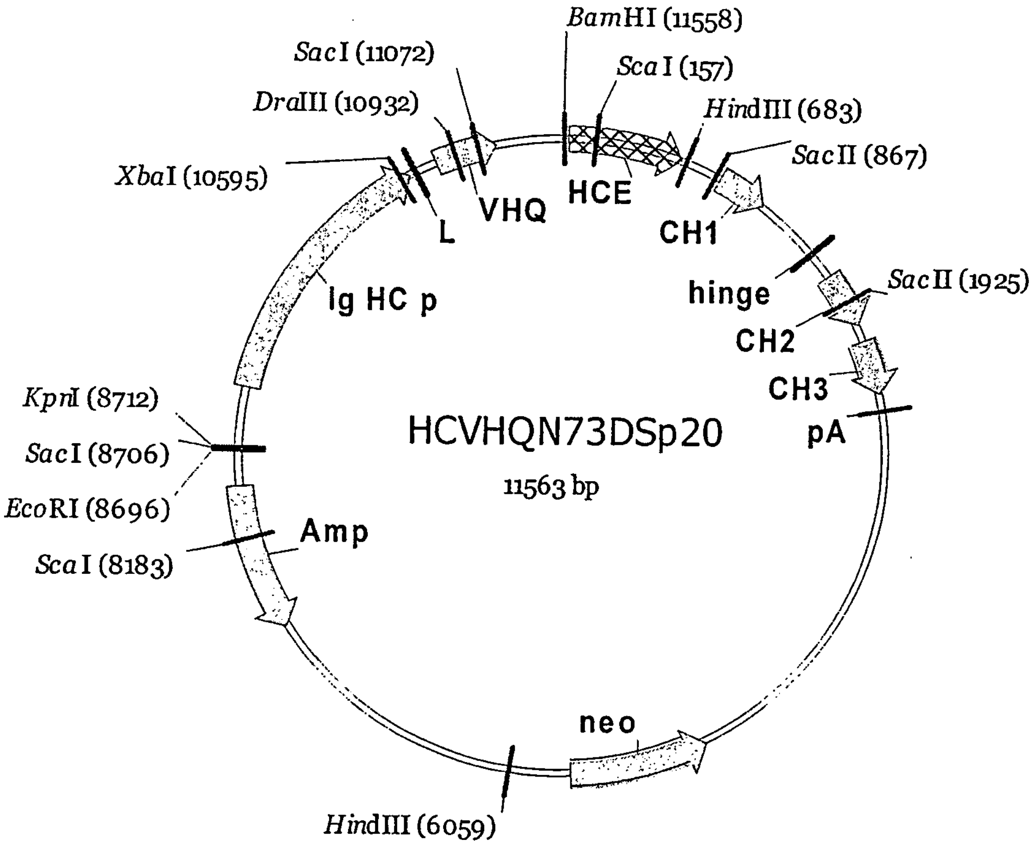
BamHI (11558)
ScaI (157)
SacI (11072)
DraIII (10932)
HindIII (683)
SacII (867)
XbaI (10595)
VHQ
HCE
L
CH1
Ig HC p
hinge
SacII (1925)
CH2
CH3
KpnI (8712)
SacI (8706)
EcoRI (8696)
ScaI (8183)
pA
Amp
HCVHQN73DSp20
11563 bp
neo
HindIII (6059)

Figure 10

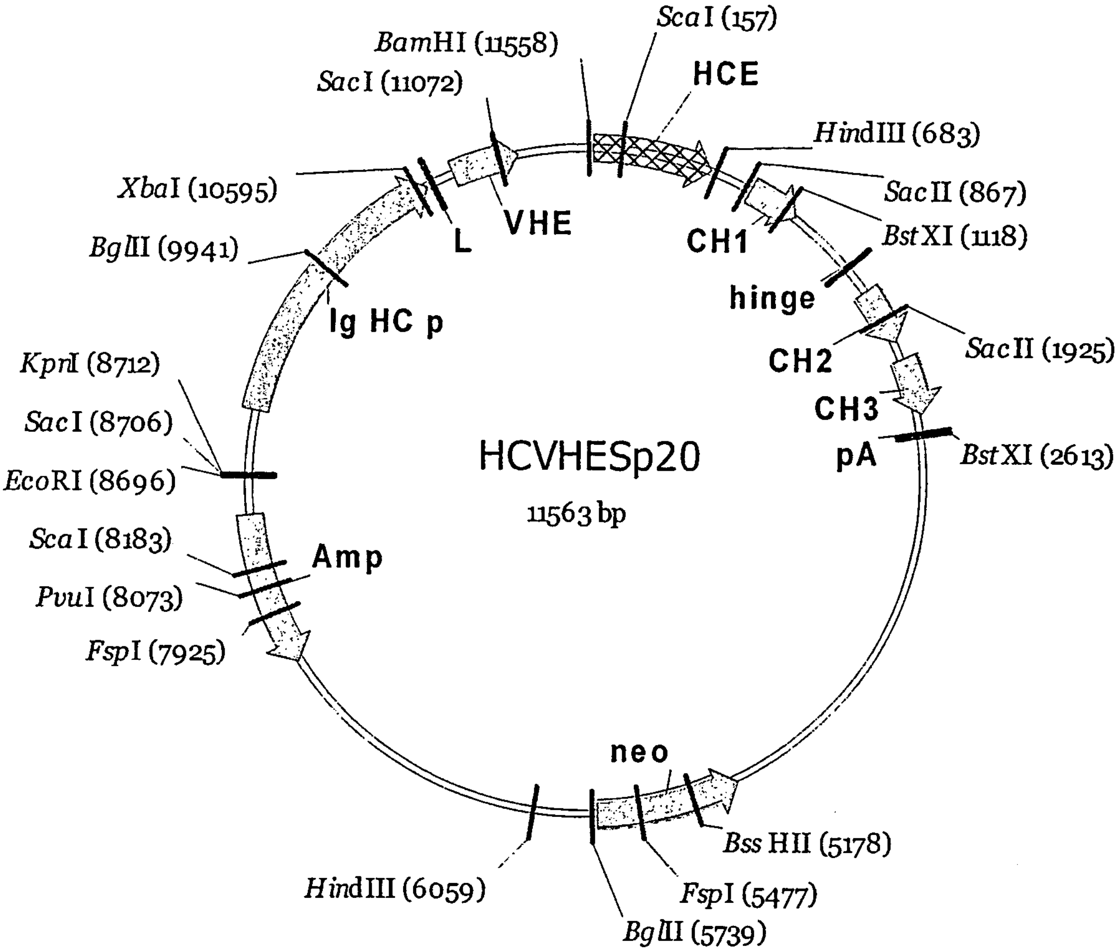
ScaI (157)
BamHI (11558)
HCE
SacI (11072)
HindIII (683)
XbaI (10595)
SacII (867)
VHE
L
CH1
BstXI (1118)
BglII (9941)
hinge
Ig HC p
SacII (1925)
KpnI (8712)
CH2
CH3
SacI (8706)
HCVHESp20
pA
BstXI (2613)
EcoRI (8696)
11563 bp
ScaI (8183)
Amp
PvuI (8073)
FspI (7925)
neo
BssHII (5178)
HindIII (6059)
FspI (5477)
BglII (5739)

Figure 11

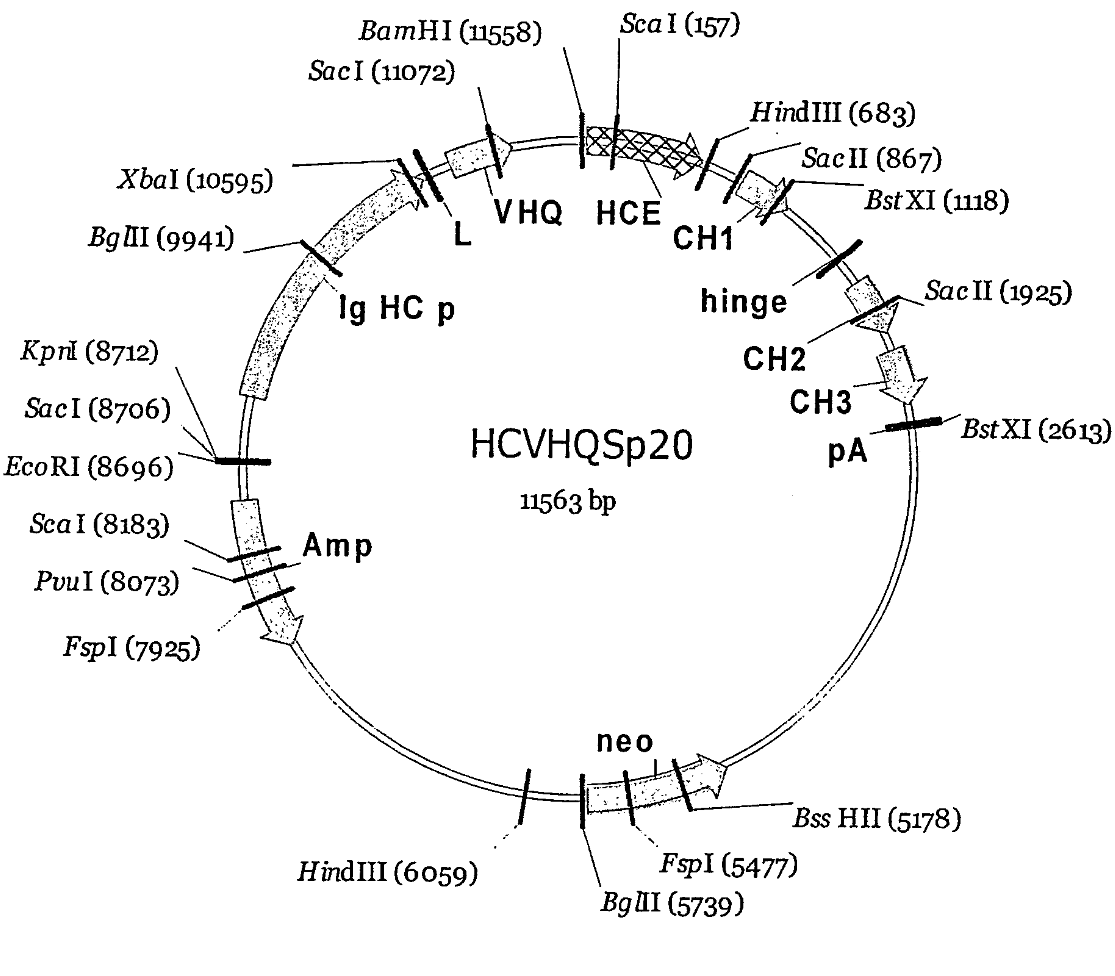
BamHI (11558)
ScaI (157)
SacI (11072)
HindIII (683)
SacII (867)
BstXI (1118)
XbaI (10595)
BglII (9941)
L
VHQ
HCE
CH1
Ig HC p
hinge
SacII (1925)
KpnI (8712)
CH2
CH3
SacI (8706)
EcoRI (8696)
HCVHQSp20
11563 bp
pA
BstXI (2613)
ScaI (8183)
Amp
PvuI (8073)
FspI (7925)
neo
BssHII (5178)
FspI (5477)
HindIII (6059)
BglII (5739)

# Figure 12

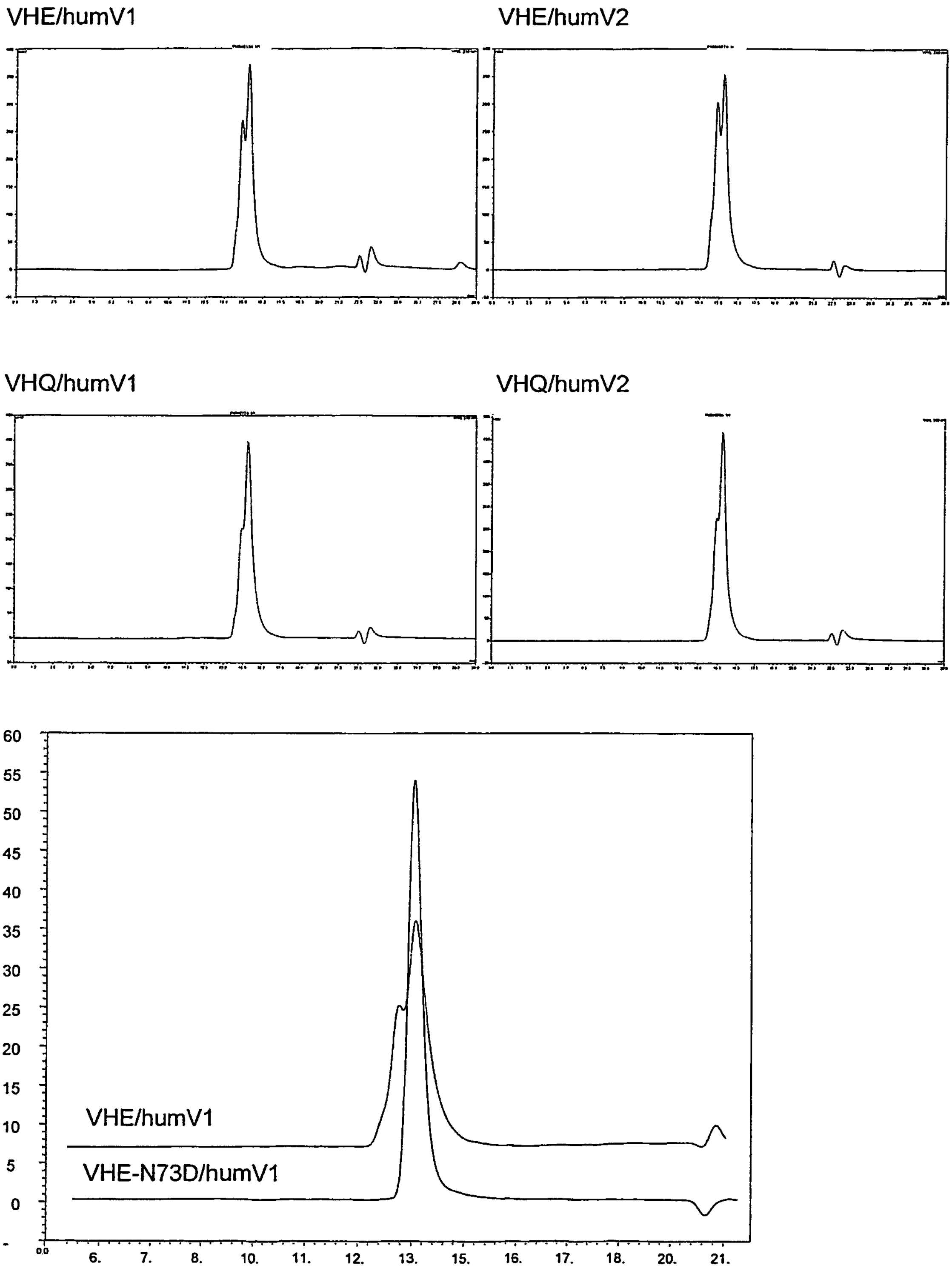
VHE/humV1
VHE/humV2
VHQ/humV1
VHQ/humV2
60
55
50
45
40
35
30
25
20
15
10
5
0
VHE/humV1
VHE-N73D/humV1
0.0
6.
7.
8.
10.
11.
12.
13.
15.
16.
17.
18.
20.
21.

**Figure 13**

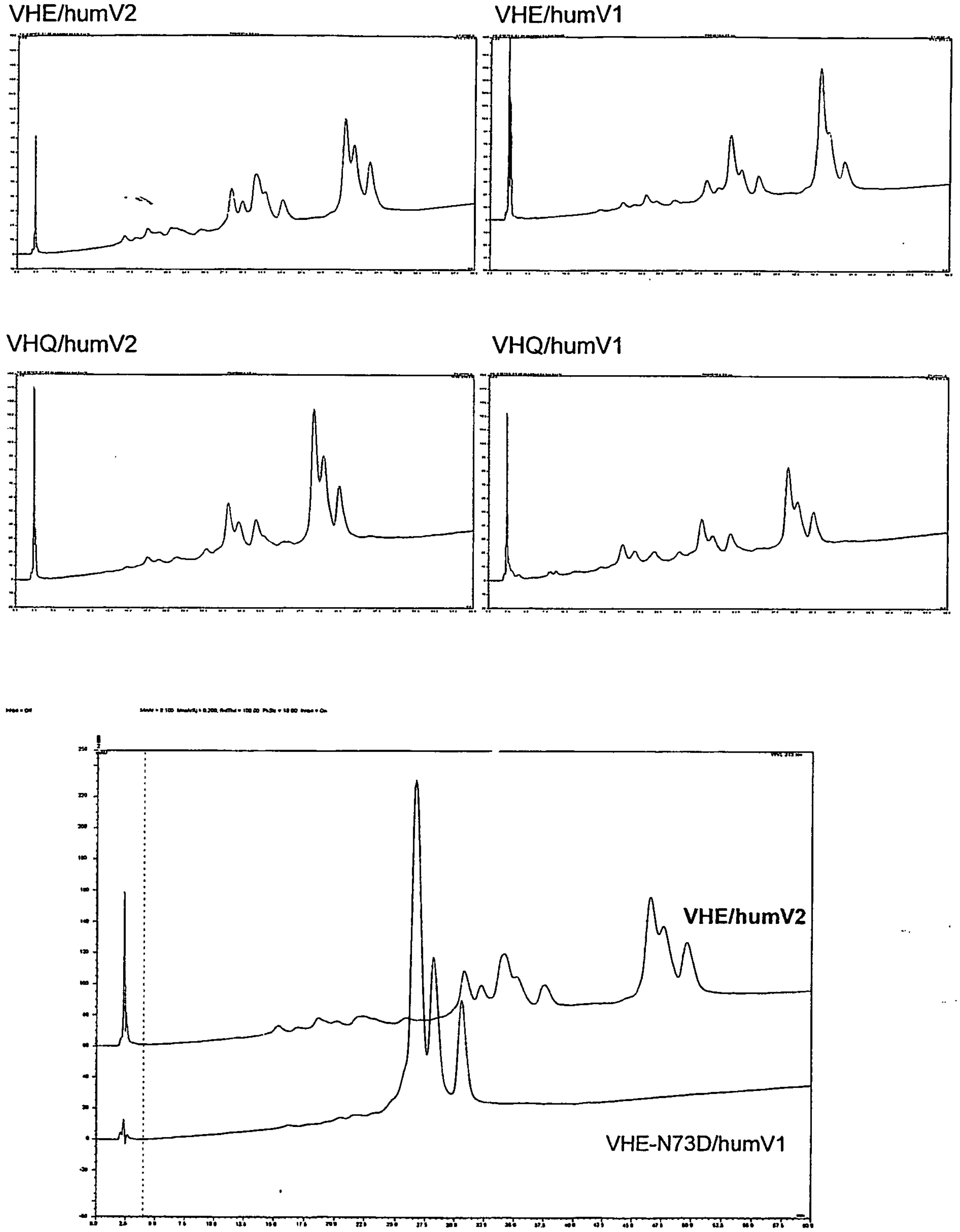
VHE/humV2
VHE/humV1
VHQ/humV2
VHQ/humV1
VHE/humV2
VHE-N73D/humV1

**Figure 14**

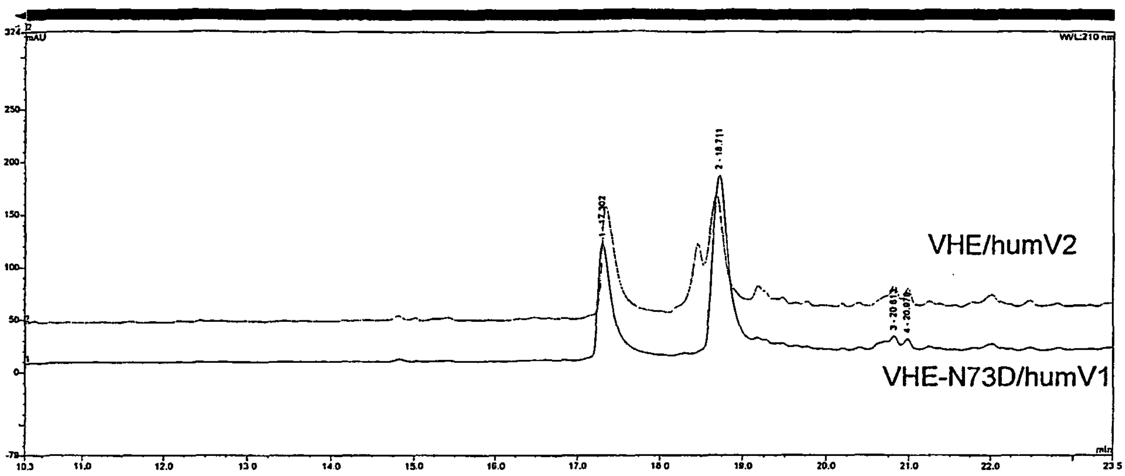

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2002072832 A
- EP 239400 A


### Non-patent literature cited in the description


- **Trowbridge IS et al.** *Annu Rev Immunol.,* 1994, vol. 12, 85-116
- **Streuli MF. et al.** *J. Exp. Med.,* 1987, vol. 166, 1548-1566
- **Thomas ML. et al.** *Immunol. Today,* 1988, vol. 9, 320-325
- **Terry LA et al.** *Immunol.,* 1988, vol. 64, 331-336
- **Dunn-Walters D. et al.** *Molecular Immunology,* 2000, vol. 37, 107-113
- **Friends P. et al.** *Transplantation,* 1999, vol. 68, 1632-1687
- **Aversa et al.** *Cellular Immunology,* 1994, vol. 158, 314-328
- **Kabat, E.A. et al.** Sequences of proteins of immunological interest. US department of health and human services, Public health service
- **T. E. Creighton.** Proteins: Structure and Molecular Properties. W. H. Freeman & Co, 79-86
- **Kolbinger et al.** *Protein Eng.,* November 1993, vol. 6 (8), 971-80
- **Bertuzzi et al.** *Diabetes,* 1999, vol. 48, 1971-8
- **Davalli A.M. et al.** *Diabetes,* 1996, vol. 45, 1161-7